# EUROPEAN PATENT APPLICATION

(11) **EP 4 592 389 A2**
(43) Date of publication of application: **30.07.2025**
(21) Application number: 25177772.8
(22) Date of filing: 04.03.2021
(51) Int. Cl.: C12N 15/11

(54) **ANALYSIS OF TARGET MOLECULES WITHIN A SAMPLE VIA HYBRIDIZATION CHAIN REACTION**

(30) Priority: 06.03.2020 US 202062986436 P
(62) Divisional of application: 21796673.8
(71) Applicant: California Institute of Technology, Pasadena, CA 91125 (US); Molecular Instruments, Inc., Los Angeles, California 90041 (US)
(72) Inventor: PIERCE, Niles A., PASADENA 91125 (US); CHOI, Harry Ming Tak, LOS ANGELES 90041 (US)
(74) Representative: Lavoix

(57) **Abstract**

Methods of analysis of a sample using hybridization chain reaction (HCR) are provided herein. Some embodiments involve one, two, or all three of the following aspects: 1) repeated signal detection, 2) overlapping binding sites, and 3) catalytic reporter deposition (CARD). Compositions and kits relating to these are also provided. Some embodiments encompass a method for repeated signal detection with reporter-labeled HCR hairpins involving providing a sample possibly containing one or more targets as well as possibly other molecules that are not targets, providing one or more probe sets each comprising either: a) one or more HCR initiator-labeled probes, or b) one or more probe units each comprising two or more HCR fractional initiator probes, providing one or more HCR amplifiers (each labeled with one or more reporters), detecting one or more signals from one or more reporters. In some embodiments, a probe unit comprises two or more HCR fractional initiator probes, wherein an HCR fractional initiator probe comprises a target-binding region and a fractional initiator, wherein the target-binding regions within a probe unit are configured to bind to overlapping or non-overlapping binding sites on the target, and wherein the fractional initiators on the probes within each probe unit are configured to bind to overlapping or non-overlapping binding sites on an HCR hairpin. Some embodiments encompass a method for HCR-mediated catalytic reporter deposition (CARD) for signal detection with hapten-labeled HCR hairpins involving providing a sample possibly containing one or more targets as well as possibly other molecules that are not targets, providing one or more probe sets each comprising either: a) one or more HCR initiator-labeled probes, or b) one or more probe units each comprising two or more HCR fractional initiator probes, providing one or more HCR amplifiers (each labeled with one or more haptens), providing one or more anti-haptens labeled with one or more reporter entities, wherein the reporter entity is an enzyme that mediates CARD, providing one or more CARD-substrates leading to deposition of one or more reporters, and detecting one or more signals from one or more reporters.

## Description

### STATEMENT REGARDING FEDERALLY SPONSORED R&D

This invention was made with government support under Grant No. R01EB006192 awarded by the National Institutes of Health and from DARPA under grant HR0011-17-2-0008. The government has certain rights in the invention.

### PRIORITY AND CROSS-REFERENCE TO RELATED APPLICATIONS

This application claims the benefit of U.S. Provisional Application 62/986436 filed on March 6, 2020, which is hereby incorporated by reference in its entirety.

### REFERENCE TO ELECTRONIC SEQUENCE LISTING

The present application is being filed along with an Electronic Sequence Listing. The Electronic Sequence Listing is provided as a file entitled MOINS007WOSEQLIST.txt which is 1,341 bytes in size, created on March 4, 2021. The information in the Electronic Sequence Listing is incorporated herein by reference in its entirety.

### BACKGROUND

### Field

The present invention relates generally to compositions and methods relating to hybridization chain reaction.

### Description of the Related Art

Hybridization Chain Reaction (HCR) is a method for the triggered hybridization of nucleic acid molecules starting from metastable hairpin monomers or other metastable nucleic acid structures. HCR does not require any enzymes and can operate isothermally.

HCR can involve two or more metastable hairpin monomers. The hairpin monomers each have at last one single-stranded toehold, a single-stranded loop, and a double-stranded stem. The energy to drive the self-assembly cascade is stored in the single-stranded loop and toehold segments of the hairpins.

Each monomer is caught in a kinetic trap, preventing the system from rapidly equilibrating. That is, pairs of monomers are unable to hybridize with each other in the absence of an initiator. Introduction of an initiator strand causes the monomers to undergo a chain reaction of hybridization events to form a nicked double-stranded polymer. HCR can be used, for example, to detect the presence of an analyte of interest in a sample by detecting the analyte with an initiator-labeled probe that carries an HCR initiator, which in turn triggers HCR signal amplification. HCR signal amplification makes it possible to increase the signal-to-background ratio for molecular detection and imaging applications by boosting the signal above the background arising from the sample.

### SUMMARY

Some embodiments provided herein provide for even greater signal-to-background ratio using probe units comprising two or more fractional initiator probes each comprising a fractional initiator. If individual fractional initiator probes bind non-specifically in the sample they do not trigger HCR. However, if they bind specifically to their cognate target, the fractional initiators within a probe unit are colocalized to form a full HCR initiator, enabling the triggering of HCR signal amplification. In some embodiments, separating the initiator into two or more fractional initiators, which only effectively co-localize in the presence of a target, provides for automatic background suppression during the detection step. When combined with the automatic background suppression provided by HCR during amplification, this process provides for automatic background suppression throughout the protocol (for example, if a reagent, either an individual probe or an individual hairpin monomer binds in the sample, it will not lead to generation of amplified background).

In some embodiments presented herein, a probe unit comprises two fractional initiator probes, each comprising a target-binding region and a fractional initiator, where the target-binding regions within a probe unit are configured to bind to adjacent binding sites on the target so as to colocalize a full HCR initiator, and wherein the fractional initiators within each probe unit are configured to bind to adjacent binding sites on an HCR hairpin so as to trigger HCR signal amplification. In some embodiments, the junction between the target, the fractional initiator probes, and/or the HCR hairpin is energetically unfavorable. In some embodiments, by configuring the target-binding regions within a probe unit to bind to overlapping binding sites on the target and/or by configuring the fractional initiators within a probe unit to hybridize to overlapping binding sites on an HCR hairpin, the junction can relax into an energetically more favorable conformation, increasing the strength of HCR signal amplification.

In some embodiments provided herein, one or more targets can be analyzed in a sample by generating one or more HCR signals, detecting one or more HCR signals, removing one or more HCR signals, and repeating one or more of these steps.

In some embodiments provided herein, HCR signal amplification is used to mediate catalytic reporter deposition (CARD), leading to even higher signal gain, and allowing for the long-term archival storage of stained samples for regulatory purposes.

In some embodiments, a method for repeated signal detection with a reporter-labeled hairpin is provided. In some embodiments, the method comprises providing a sample possibly containing up to N targets as well as possibly other molecules that are not targets, providing N probe sets (each targeting one of N target types) each comprising either: i) one or more HCR initiator-labeled probes, or ii) one or more probe units each comprising two or more HCR fractional initiator probes, optionally washing the sample, providing M HCR amplifiers (for M ≤ N; each labeled with a distinct reporter) corresponding to M of the N probe sets, optionally washing the sample, detecting M signals corresponding to the M reporters, removing the M signals from the sample; and optionally repeating one or more of the above steps until signal detection has been performed for all N targets. A probe set comprises either one or more HCR initiator-labeled probes, or one or more probe units. An HCR initiator-labeled probe comprises one or more target-binding regions and one or more initiators. A probe unit comprises two or more HCR fractional initiator probes. An HCR fractional initiator probe comprises a target-binding region and a fractional initiator. An HCR amplifier comprises two or more HCR hairpins. An HCR hairpin comprises an input domain comprising a single-stranded toehold and a stem section. An HCR hairpin further comprises an output domain comprising a single-stranded loop and a complement to the stem section. An HCR hairpin further comprises a reporter.

In some embodiments, a method for repeated signal detection with a reporter-labeled hairpin is provided. In some embodiments, the method comprises providing a sample possibly containing one or more targets as well as possibly other molecules that are not targets, providing one or more probe sets each comprising either: i) one or more HCR initiator-labeled probes, or ii) one or more probe units each comprising two or more HCR fractional initiator probes, optionally washing the sample, providing one or more HCR amplifiers (each labeled with one or more reporters), optionally washing the sample, detecting one or more signals from one or more reporters, optionally removing one or more probe sets from the sample, optionally removing one or more HCR amplifiers from the sample, optionally removing one or more reporters from the sample, and optionally removing one or more signals from the sample. A probe set comprises either one or more HCR initiator-labeled probes, or one or more probe units. An HCR initiator-labeled probe comprises one or more target-binding regions and one or more initiators. A probe unit comprises two or more HCR fractional initiator probes. An HCR fractional initiator probe comprises a target-binding region and a fractional initiator. An HCR amplifier comprises two or more HCR hairpins. An HCR hairpin comprises: an input domain comprising a single-stranded toehold and a stem section. An HCR hairpin further comprises an output domain comprising a single-stranded loop and a complement to the stem section. An HCR hairpin further comprises one or more reporters.

In some embodiments, method of repeated signal detection with substrate-labeled hairpins is provided. In some embodiments, the method comprises a) providing a sample possibly containing up to N targets as well as possibly other molecules that are not targets, b) providing N probe sets each comprising either: i) one or more HCR initiator-labeled probes, or ii) one or more probe units each comprising two or more HCR fractional initiator probes, c) optionally washing the sample, d) providing N HCR amplifiers (each labeled with a distinct substrate) corresponding to the N probe sets, e) optionally washing the sample, f) providing M label probes (for M ≤ N; each conjugated to a distinct reporter) corresponding to M of the N distinct substrates, g) optionally washing the sample, h) detecting M signals corresponding to the M distinct reporters, i) removing the M signals from the sample, and j) optionally repeating one or more of steps f-i until signal detection has been performed for all N targets. A probe set comprises either one or more HCR initiator-labeled probes, or one or more probe units. An HCR initiator-labeled probe comprises one or more target-binding regions and one or more initiators. A probe unit comprises two or more HCR fractional initiator probes. An HCR fractional initiator probe comprises a target-binding region and a fractional initiator. An HCR amplifier comprises two or more HCR hairpins. An HCR hairpin comprises an input domain comprising a single-stranded toehold and a stem section. An HCR hairpin further comprises an output domain comprising a single-stranded loop and a complement to the stem section. An HCR hairpin further comprises a substrate. A label probe comprises a substrate-binding region and a reporter.

In some embodiments, a method of repeated signal detection with substrate-labeled hairpins is provided. In some embodiments, the method comprises a) providing a sample possibly containing one or more targets as well as possibly other molecules that are not targets, b) providing one or more probe sets each comprising either: i) one or more HCR initiator-labeled probes, or ii) one or more probe units each comprising two or more HCR fractional initiator probes, c) optionally washing the sample, d) providing one or more HCR amplifiers (each labeled with a substrate) corresponding to one or more probe sets, e) optionally washing the sample, f) providing one or more label probes (each conjugated to a reporter) corresponding to one or more substrates, g) optionally washing the sample, h) detecting one or more signals corresponding to one or more reporters, i) removing one or more signals from the sample, and j) optionally repeating any of steps b-i one or more times in any order. A probe set comprises either one or more HCR initiator-labeled probes, or one or more probe units. An HCR initiator-labeled probe comprises one or more target-binding regions and one or more initiators. A probe unit comprises two or more HCR fractional initiator probes. An HCR fractional initiator probe comprises a target-binding region and a fractional initiator. An HCR amplifier comprises two or more HCR hairpins. An HCR hairpin comprises an input domain comprising a single-stranded toehold and a stem section. An HCR hairpin further comprises an output domain comprising a single-stranded loop and a complement to the stem section. An HCR hairpin further comprises a substrate. A label probe comprises a substrate-binding region and a reporter.

In some embodiments, a method of repeated signal detection with reporter and/or substrate-labeled hairpins is provided. In some embodiments, the method comprises providing a sample possibly containing one or more targets as well as possibly other molecules that are not targets, providing one or more HCR probe sets each comprising either: i) one or more HCR initiator-labeled probes, or ii) one or more probe units each comprising two or more HCR fractional initiator probes, providing one or more HCR amplifiers (each labeled with one or more reporters and/or one or more substrates) corresponding to one or more probe sets, optionally providing one or more label probes (each conjugated to one or more reporters) corresponding to one or more substrates, detecting one or more signals, optionally washing the sample, optionally removing one or more signals from the sample, optionally removing one or more reporters from the sample, optionally removing one or more label probes from the sample, optionally removing one or more HCR amplifiers from the sample, optionally removing one or more probe sets from the sample, and optionally repeating any of the above steps in any order. A probe set comprises either one or more HCR initiator-labeled probes, or one or more probe units. An HCR initiator-labeled probe comprises one or more target-binding regions and one or more initiators. A probe unit comprises two or more HCR fractional initiator probes. An HCR fractional initiator probe comprises a target-binding region and a fractional initiator. An HCR amplifier comprises two or more HCR hairpins. The HCR hairpin comprises an input domain comprising a single-stranded toehold and a stem section. An HCR hairpin further comprises an output domain comprising a single-stranded loop and a complement to the stem section. An HCR hairpin further comprises one or more reporters and/or one or more substrates. A label probe comprises a substrate-binding region and one or more reporters.

In some embodiments, a method of repeated signal detection with reporter and/or substrate-labeled hairpins is provided. In some embodiments, the method comprises providing a sample possibly containing one or more targets as well as possibly other molecules that are not targets, performing any of steps c-g one or more times in any order, providing one or more HCR probe sets each comprising either: i) one or more HCR initiator-labeled probes, or ii) one or more probe units each comprising two or more HCR fractional initiator probes, providing one or more HCR amplifiers that directly or indirectly generate one or more signals, optionally washing the sample, detecting one or more signals, and optionally removing one or more signals, wherein a probe set comprises either one or more HCR initiator-labeled probes, or one or more probe units. An HCR initiator-labeled probe comprises one or more target-binding regions and one or more initiators. A probe unit comprises two or more HCR fractional initiator probes. An HCR fractional initiator probe comprises a target-binding region and a fractional initiator. An HCR amplifier comprises two or more HCR hairpins. An HCR hairpin comprises an input domain comprising a single-stranded toehold and a stem section. An HCR hairpin further comprises an output domain comprising a single-stranded loop and a complement to the stem section. An HCR hairpin further comprises one or more reporters and/or one or more substrates.

In some embodiments, a method of HCR involving overlapping binding sites is provided. In some embodiments, the method comprises providing a sample possibly containing a target as well as possibly other molecules that are not targets, providing a probe set comprising one or more probe units each comprising two or more HCR fractional initiator probes where the target-binding regions on the probes within each probe unit are configured to bind to overlapping binding sites on the target, optionally washing the sample, providing an HCR amplifier labeled with a reporter and/or a substrate, optionally washing the sample, optionally providing a label probe (conjugated to a reporter) corresponding to the substrate, optionally washing the sample, and detecting a signal from the reporter. A probe set comprises one or more probe units. A probe unit comprises two or more HCR fractional initiator probes. An HCR fractional initiator probe comprises a target-binding region and a fractional initiator. The target binding regions on the probes within each probe unit are configured to bind to overlapping binding sites on the target. An HCR amplifier comprises two or more HCR hairpins. An HCR hairpin comprises an input domain comprising a single-stranded toehold and a stem section. An HCR hairpin further comprises an output domain comprising a single-stranded loop and a complement to the stem section. An HCR hairpin further comprises a reporter and/or a substrate. A label probe comprises a substrate-binding region and a reporter.

In some embodiments, a method of HCR involving overlapping binding sites is provided. In some embodiments, the method comprises providing a sample possibly containing a target as well as possibly other molecules that are not targets providing a probe set comprising one or more probe units each comprising two or more HCR fractional initiator probes where the fractional initiators on the probes within each probe unit are configured to bind to overlapping binding sites on an HCR hairpin, optionally washing the sample, providing an HCR amplifier labeled with a reporter and/or a substrate, optionally washing the sample, optionally providing a label probe (conjugated to a reporter) corresponding to the substrate, optionally washing the sample, and detecting a signal from the reporter. A probe set comprises one or more probe units. A probe unit comprises two or more HCR fractional initiator probes. An HCR fractional initiator probe comprises a target-binding region and a fractional initiator. The fractional initiators on the probes within each probe unit are configured to bind to overlapping binding sites on an HCR hairpin. An HCR amplifier comprises two or more HCR hairpins. An HCR hairpin comprises an input domain comprising a single-stranded toehold and a stem section. An HCR hairpin further comprises an output domain comprising a single-stranded loop and a complement to the stem section. An HCR hairpin further comprises a reporter and/or a substrate. A label probe comprises a substrate-binding region and a reporter.

In some embodiments, a method of HCR involving overlapping binding sites with repeated signal detection is provided. In some embodiments, the method comprises providing a sample possibly containing one or more targets as well as possibly other molecules that are not targets providing one or more probe sets each comprising either: i) one or more HCR initiator-labeled probes, or ii) one or more probe units each comprising two or more HCR fractional initiator probes where target-binding regions on the probes within each probe unit are configured to bind to overlapping or non-overlapping binding sites on a target and where fractional initiators on the probes within each probe unit are configured to bind to overlapping or non-overlapping binding sites on an HCR hairpin, optionally washing the sample, providing one or more HCR amplifiers each labeled with one or more reporters and/or substrates, optionally washing the sample, optionally providing one or more label probes (each conjugated to one or more reporters) corresponding to one or more substrates, optionally washing the sample, detecting a signal from one or more reporters, optionally removing one or more signals from the sample, optionally removing one or more reporters from the sample, optionally removing one or more label probes from the sample, optionally removing one or more amplifiers from the sample, optionally removing one or more probe sets from the sample, and optionally repeating any of the above steps in any order. A probe set comprises either one or more HCR initiator-labeled probes, or one or more probe units. An HCR initiator-labeled probe comprises one or more target-binding regions and one or more initiators. A probe unit comprises two or more HCR fractional initiator probes. An HCR fractional initiator probe comprises a target-binding region and a fractional initiator. The target-binding regions within a probe unit are configured to bind to overlapping or non-overlapping binding sites on the target. The fractional initiators on the probes within each probe unit are configured to bind to overlapping or non-overlapping binding sites on an HCR hairpin. An HCR amplifier comprises two or more HCR hairpins. An HCR hairpin comprises an input domain comprising a single-stranded toehold and a stem section. An HCR hairpin further comprises an output domain comprising a single-stranded loop and a complement to the stem section. An HCR hairpin further comprises one or more reporters and/or one or more substrates. A label probe comprises a substrate-binding region and one or more reporters.

In some embodiments, a method comprises providing a first fractional initiator probe comprising a first fractional initiator, a second fractional initiator probe comprising a second fractional initiator, a first hairpin monomer comprising a first input domain comprising a first toehold and a first stem section, a first output domain comprising a first hairpin loop and a complement to the first stem section, and a first hapten molecule, a second hairpin monomer comprising a second input domain comprising a second toehold and a second stem section, a second output domain comprising a second hairpin loop and a complement to the second stem section, and a second hapten molecule, a target molecule, and incubating the first fractional initiator probe and the second fractional initiator probe with the target.

In some embodiments, a method comprises providing at least one initiator-labeled probe comprising at least one initiator, a first hairpin monomer comprising a first input domain comprising a first toehold and a first stem section, a first output domain comprising a first hairpin loop and a complement to the first stem section, and a first hapten molecule, a second hairpin monomer comprising a second input domain comprising a second toehold and a second stem section, a second output domain comprising a second hairpin loop and a complement to the second stem section, and a second hapten molecule, a target molecule, and incubating the at least one initiator-labeled probe comprising at least one initiator with the target.

In some embodiments, a method comprises providing a first fractional initiator probe comprising a first fractional initiator, a second fractional initiator probe comprising a second fractional initiator, a first hairpin monomer comprising a first input domain comprising a first toehold and a first stem section, a first output domain comprising a first hairpin loop and a complement to the first stem section, and a substrate, a second hairpin monomer comprising a second input domain comprising a second toehold and a second stem section, a second output domain comprising a second hairpin loop and a complement to the second stem section, and the substrate a target molecule, and incubating the first fractional initiator probe and the second fractional initiator probe with the target.

In some embodiments, a method comprises providing a first fractional initiator probe comprising a first fractional initiator, a second fractional initiator probe comprising a second fractional initiator, a first hairpin monomer comprising a first input domain comprising a first toehold and a first stem section, a first output domain comprising a first hairpin loop and a complement to the first stem section, and a first fractional substrate, a second hairpin monomer comprising a second input domain comprising a second toehold and a second stem section, a second output domain comprising a second hairpin loop and a complement to the second stem section, and a second fractional substrate, a target molecule, and incubating the first fractional initiator probe and the second fractional initiator probe with the target molecule.

### BRIEF DESCRIPTION OF THE DRAWINGS

FIGs. 1A and 1B depict some embodiments of in situ amplification via hybridization chain reaction (HCR).
FIGs. 2A and 2B depict schematics of in situ HCR using either two-stage or three-stage protocols.
FIGs. 3A and 3B depict some arrangements for two adjacent target sections and two fractional initiator probes that hybridize to these adjacent target sections to colocalize two fractional initiators.
FIGs. 4A-4D depict some embodiments of fractional initiator probes colocalized by a target molecule.
FIGs. 5A-5E depict some embodiments of fractional initiator probes colocalized by a target complex.
FIG. 6 depicts imaging target mRNAs in whole-mount chicken embryos using unoptimized standard probes and fractional initiator probes.
FIGs. 7A-7C depict some embodiments of fractional initiator probes colocalized by a target and displays test tube data demonstrating triggering HCR using fractional initiator probes colocalized by a target.
FIGs. 8A-8B depict some embodiments of in situ HCR using fractional initiator probes.
FIGs. 9A-9D depicts background and signal-to-background using standard probes and fractional initiator probes.
FIGs. 10A-10D depict multiplexed imaging of mRNA expression with high signal-to-background in a fixed whole-mount chicken embryo using fraction initiator probes without probe set optimization.
FIGs. 11A-11B depicts quantitative imaging of mRNA expression with subcellular resolution in fixed whole-mount chicken embryos using fractional initiator probes.
FIG. 12 depicts some embodiments of hybridizing a first fractional initiator probe and a second fractional initiator probe to a target molecule.
FIG. 13 depicts some embodiments of triggered self-assembly of an HCR amplification polymer from hairpin monomers upon initiation by an HCR initiator. 1050 in FIG. 13 depicts a full initiator, from two parts. Only a part of the fractional initiator probes is depicted. I1 (1050) denotes a full initiator formed by two fractional initiator probes colocalized by a target.
FIG. 14 depicts some embodiments of HCR amplification using hairpin monomers triggered by an HCR initiator. Only a part of the fractional initiator probes is depicted. I1 denotes a full initiator formed by two fractional initiator probes colocalized by a target.
FIG. 15 depicts some embodiments of an HCR mechanism using simplified HCR hairpin monomers.
FIGs. 16A-16D depict some embodiments of probe sets comprising one or more probe units and optionally comprising one or more helper probes.
FIGs. 17A-17C depict some embodiments of probe units comprising fractional initiator probes.
FIGs. 18A-18F depict some embodiments of HCR amplifiers.
FIGs. 19A-19B depict some embodiments of HCR amplifiers comprising four HCR hairpins.
FIGs. 20A-20F depict some embodiments of label probes.
FIGs. 21A-21B depict some embodiments of fractional initiator probes designed to be complementary to overlapping regions of an HCR hairpin.
FIG. 22 depicts some embodiments of fractional initiator probes designed to be complementary to overlapping regions of a target.
FIGs. 23A-23O depicts some embodiments for removal of HCR signal from the sample. In some embodiments, any one or more of the steps in the processes can be combined with the other methods of FIGs. 23A-23O and 40A-40N. In some embodiments, any one of more of the steps in the processes of FIG. 23A-23O can be combined with the other methods of FIGs. 26A-26T and 40A-40N.
FIGs. 24A-24B depict some embodiments of increasing the HCR signal strength using fractional initiator probes designed to be complementary to overlapping regions of an HCR hairpin.
FIGs. 25A-25B depict some embodiments of multiplexed in situ hybridization in cultured human cells via repeated reporter detection.
FIGs. 26A-26T depict some embodiments of various methods for detecting one or more targets in a sample using HCR initiator-labeled probes and/or HCR fractional initiator probes in combination with HCR amplifiers. In some embodiments, any one or more of the steps in the processes can be combined with the other methods of FIG. 26A-26T.
FIG. 27 depicts some embodiments of fractional initiator probes designed to be complementary to overlapping regions of an HCR hairpin and designed to be complementary to overlapping regions of a target.
FIGs. 28A-28C depict some embodiments of an example of in vitro optimization of cooperative probe junctions to enhance fractional initiator HCR suppression (OFF state) and conversion (ON state).
FIGs. 29A-29D depict some embodiments of multiplexed HCR immunohistochemistry to image protein targets in formalin-fixed paraffin embedded mouse brain sections using initiator-labeled primary antibody probes.
FIGs. 30A-30D depict some embodiments of multiplexed HCR immunohistochemistry to image protein targets in formalin-fixed paraffin embedded mouse brain sections using unlabeled primary antibody probes and initiator-labeled secondary antibody probes.
FIGs. 31A-31C depict some embodiments of simultaneous HCR RNA in situ hybridization and protein immunohistochemistry in a formalin-fixed paraffin embedded mouse brain section using DNA fractional initiator probes for mRNA targets and initiator-labeled primary antibody probes for protein targets, with HCR signal amplification performed for all targets simultaneously.
FIGs. 32A-32C depict some embodiments of simultaneous HCR RNA in situ hybridization and protein immunohistochemistry in a formalin-fixed paraffin embedded mouse brain section using DNA fractional initiator probes for mRNA targets and unlabeled primary antibody probes and initiator-labeled secondary antibody probes for protein targets, with HCR signal amplification performed for all targets simultaneously.
FIGs. 33A-33E depict some embodiments for using HCR to mediate CARD signal amplification for a variety of target types.
FIGs. 34A-34C depict some embodiments for using HCR to mediate CARD signal amplification for generic target molecules and target complexes.
FIG. 35 depicts some embodiments for the placement of haptens in the context of HCR CARD signal amplification.
FIGs. 36A-36B depict some embodiments of HCR CARD signal amplification using substrate-labeled or fractional-substrate HCR hairpins.
FIGs. 37A-37B depict some embodiments of imaging an RNA target in formalin-fixed paraffin-embedded human kidney and liver sections using HCR-mediated CARD signal amplification.
FIG. 38 depicts some embodiments of fractional initiator probes colocalized by a target molecule or target complex either directly or indirectly.
FIGs. 39A-39N depict some embodiments of initiator-labeled probes bound to a target molecule or a target complex either directly or indirectly.
FIGs. 40A-40N depict some embodiments for removal of HCR signal from the sample. In some embodiments, any one or more of the steps in the processes can be combined with the other methods of FIGs. 23A-23O and 40A-40N. In some embodiments, any one of more of the steps in the processes of FIG. 40A-40N can be combined with the other methods of FIGs. 23A-23O and 26A-26T.
FIGs. 41A-41C depict some embodiments of quantitative imaging of mRNA expression with subcellular resolution in a formalin-fixed paraffin-embedded mouse brain section using fractional initiator probes.
FIGs. 42A-42F depict some embodiments of initiator-labeled probes comprising one or more HCR initiators.
FIGs. 43A-43C depict some embodiments of imaging target microRNAs and mRNAs in whole-mount zebrafish embryos using initiator-labeled probes.
FIGs. 44A-44Z depict some embodiments of shielded initiator-labeled probes comprising one or more shielded HCR initiators.
FIGs. 45A-45B depict some embodiments of an example of using a shielded initiator-labeled probe to reduce background.
FIGs. 46A-46M depict some embodiments of shielded initiators for use in the context of shielded initiator-labeled probes.

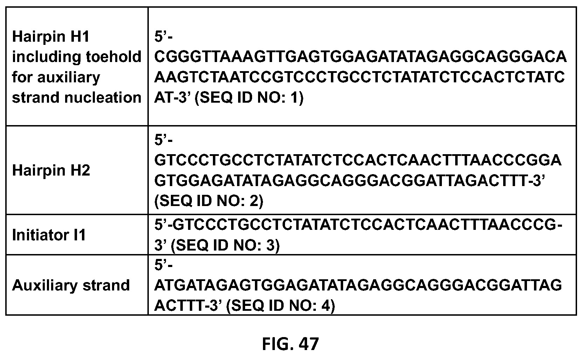

FIG. 47 shows embodiments of sequences used for dehybridizing HCR hairpins from HCR polymers in FIG. 25A (TABLE 2).

### DETAILED DESCRIPTION

Hybridization Chain Reaction (HCR) is a method for the triggered hybridization of nucleic acid molecules starting from metastable hairpin monomers or other metastable nucleic acid structures. See, for example, Dirks, R. and Pierce, N. Proc. Natl. Acad. Sci. USA 101(43): 15275-15278 (2004), and U.S. Patent Application No. 11/087,937, filed March 22, 2005, U.S. Pat. Nos. US 8,105,778, January 31, 2012, 8,507,204, August 13, 2013; and U.S. Pat. Pub. No. 2018/0010166, filed June 30, 2017, each of which is incorporated herein by reference in its entirety. In a simple version of this process, metastable hairpin monomers undergo a chain reaction of hybridization events to form a nicked double-stranded polymer when triggered by a nucleic acid initiator strand. The hairpin monomers store the energy to drive the polymerization process in their single-stranded loops and toeholds.

HCR can involve two or more metastable hairpin monomers. The hairpin monomers each have at last one single-stranded toehold, a single-stranded loop, and a double-stranded stem. The energy to drive the self-assembly cascade is stored in the single-stranded loop and toehold segments of the hairpins.

Each monomer is caught in a kinetic trap, preventing the system from rapidly equilibrating. That is, pairs of monomers are unable to hybridize with each other in the absence of an initiator. Introduction of an initiator strand causes the monomers to undergo a chain reaction of hybridization events to form a nicked double-stranded polymer. HCR can be used, for example, to detect the presence of an analyte of interest in a sample by detecting the analyte with a probe that carries an HCR initiator, which in turn triggers HCR signal amplification. HCR signal amplification makes it possible to increase the signal-to-background ratio for molecular detection and imaging applications by boosting the signal above the background arising from the sample.

Provided herein are embodiments of HCR. Methods of analysis of a sample using hybridization chain reaction (HCR) can involve one, two, or all three of the following aspects: 1) repeated signal detection, 2) overlapping binding sites, and 3) catalytic reporter deposition (CARD).

In some embodiments of a method, a sample, possibly containing up to N targets as well as possibly other molecules that are not targets, is combined with N probe sets (each targeting one of N target types) each comprising one or more probe units each comprising two or more HCR fractional initiator probes. M HCR amplifiers (for M ≤ N; each labeled with a distinct reporter) corresponding to M of the N probe sets are added. Thereafter, M signals corresponding to the M reporters are detected. The steps of the method are repeated until signal detection has been performed for all N targets.

In some embodiments of a method, a sample, possibly containing one or more targets as well as possibly other molecules that are not targets, is combined with one or more probe sets each comprising one or more probe units each comprising two or more HCR fractional initiator probes. One or more HCR amplifiers (each labeled with one or more reporters) is added, and one or more signals from one or more reporters are detected.

In some embodiments of a method, a sample, possibly containing up to N targets as well as possibly other molecules that are not targets, is combined with N probe sets each comprising one or more probe units each comprising two or more HCR fractional initiator probes. N HCR amplifiers (each labeled with a distinct substrate) corresponding to the N probe sets and M label probes (for M≤N; each conjugated to a distinct reporter) corresponding to M of the N distinct substrates are added. Thereafter, M signals corresponding to the M distinct reporters are detected. The steps of the method are repeated until signal detection has been performed for all N targets. In some embodiments, a method of repeated signal detection is provided. The method includes substrate-labeled hairpins, a sample, possibly containing one or more targets as well as possibly other molecules that are not targets is combined with one or more probe sets each comprising one or more probe units each comprising two or more HCR fractional initiator probes, one or more HCR amplifiers (each labeled with a substrate) corresponding to one or more probe sets, and one or more label probes (each conjugated to a reporter) corresponding to one or more substrates. Thereafter, one or more signals corresponding to one or more reporters are detected. The steps of the method are repeated one or more times in any order.

In some embodiments of a method of repeated signal detection with reporter and/or substrate-labeled hairpins, a sample, possibly containing one or more targets as well as possibly other molecules that are not targets is combined with one or more HCR probe sets each comprising one or more probe units each comprising two or more HCR fractional initiator probes, one or more HCR amplifiers (each labeled with one or more reporters and/or one or more substrates) corresponding to one or more probe sets, and one or more label probes (each conjugated to one or more reporters) corresponding to one or more substrates. Thereafter, one or more signals are detected. The steps of the method are repeated in any order.

In some embodiments of a method of repeated signal detection with reporter and/or substrate-labeled hairpins, a sample, possibly containing one or more targets as well as possibly other molecules that are not targets, is combined with one or more HCR probe sets each comprising one or more probe units each comprising two or more HCR fractional initiator probes. One or more HCR amplifiers are added that directly or indirectly generate one or more signals, which are detected. The steps of the method can be performed one or more times in any order.

In some embodiments of a method, a sample, possibly containing a target as well as possibly other molecules that are not targets is combined with a probe set comprising one or more probe units each comprising two or more HCR fractional initiator probes. The target-binding regions on the probes within each probe unit are configured to bind to overlapping binding sites on the target. Thereafter, an HCR amplifier labeled with a reporter and/or a substrate and an optional label probe (conjugated to a reporter) corresponding to the substrate are added, and a signal detected from the reporter. In some embodiments, the amplifier can instead be labeled with reporters, making the label on the probe optional.

In some embodiments of a method, a sample, possibly containing a target as well as possibly other molecules that are not targets is combined with a probe set comprising one or more probe units each comprising two or more HCR fractional initiator probes. The fractional initiators on the probes within each probe unit are configured to bind to overlapping binding sites on an HCR hairpin. An HCR amplifier labeled with a reporter and/or a substrate and an optional label probe (conjugated to a reporter) corresponding to the substrate are added. Thereafter, a signal from the reporter is detected. In some embodiments, the amplifier can instead be labeled with reporters, making the label on the probe optional.

In some embodiments of a method, a sample, possibly containing one or more targets as well as possibly other molecules that are not targets is combined with one or more probe sets each comprising one or more probe units each comprising two or more HCR fractional initiator probes. The target-binding regions on the probes within each probe unit are configured to bind to overlapping or non-overlapping binding sites on a target. The fractional initiators on the probes within each probe unit are configured to bind to overlapping or non-overlapping binding sites on an HCR hairpin. One or more HCR amplifiers each labeled with one or more reporters and/or substrates, and (optionally) one or more optional label probes (each conjugated to one or more reporters) corresponding to one or more substrates are added. A signal from one or more reporters is detected. The steps of the method can be repeated in any order. In some embodiments, the amplifier can instead be labeled with reporters, making the label on the probe optional.

In some embodiments of a method, a first fractional initiator probe comprising a first fractional initiator, a second fractional initiator probe comprising a second fractional initiator, a first hairpin monomer labeled with zero, one, or more haptens, a second hairpin monomer labeled with zero, one, or more haptens, and a target molecule are combined. This results in a binding of the first fractional initiator probe to the target molecule and a binding of the second fractional initiator probe to the target molecule. The first hairpin monomer binds to both the first fractional initiator and the second fractional initiator, and the second hairpin monomer binds to the first hairpin monomer. An anti-hapten molecule labeled with one or more reporter entities is provided. The reporter entity is an enzyme that mediates CARD. One or more CARD-substrates are provided and a signal measured from one or more deposited reporters generated from the CARD-substrates by the enzyme that mediates CARD.

In some embodiments of a method, at least one initiator-labeled probe comprising at least one initiator, a first hairpin monomer labeled with zero, one, or more haptens, a second hairpin monomer labeled with zero, one, or more haptens, and zero, one, or more target molecules are combined. This results in a binding of the at least one initiator-labeled probe comprising at least one initiator to the zero, one, or more target molecules. The first hairpin monomer binds to the at least one initiator, and the second hairpin monomer binds to the first hairpin monomer. An anti-hapten molecule labeled with one or more reporter entities is provided. The reporter entity is an enzyme that mediates CARD. One or more CARD-substrates are provided and a signal measured from one or more deposited reporters generated from the CARD-substrates by the enzyme that mediates CARD.

In some embodiments of a method, a first fractional initiator probe comprising a first fractional initiator, a second fractional initiator probe comprising a second fractional initiator, a first hairpin monomer comprising zero, one, or more substrates, a second hairpin monomer comprising zero, one, or more substrates, and a target molecule are combined. This results in a binding of the first fractional initiator probe to the target molecule and a binding of the second fractional initiator probe to the target molecule. The first hairpin monomer binds to both the first fractional initiator and the second fractional initiator, and the second hairpin monomer binds to the first hairpin monomer. A substrate-binding region labeled with one or more reporter entities is provided. The substrate-binding region binds to the substrate. The reporter entity is an enzyme that mediates CARD. One or more CARD-substrates are provided, and a signal measured from one or more deposited reporters generated from the CARD-substrates by the enzyme that mediates CARD.

In some embodiments of a method, a first fractional initiator probe comprising a first fractional initiator, a second fractional initiator probe comprising a second fractional initiator, a first hairpin monomer comprising a first fractional substrate, a second hairpin monomer comprising a second fractional substrate, and a target molecule are combined. This results in a binding of the first fractional initiator probe to the target molecule and a binding of the second fractional initiator probe to the target molecule. The first hairpin monomer binds to both of the first fractional initiator and the second fractional initiator, and the second hairpin monomer binds to the first hairpin monomer, resulting in a full substrate comprising the first and second fractional substrates. A substrate-binding region labeled with one or more reporter entities is added. The substrate-binding region binds to the full substrate. The reporter entity is an enzyme that mediates CARD. One or more CARD-substrates are provided, and a signal measured from one or more deposited reporters generated from the CARD-substrates by the enzyme that mediates CARD.

In some embodiments, the HCR process can be a fractional HCR process. In some embodiments, the pair or set of fractional initiator probes can have a small amount of overlap between them. In some embodiments, the initiator is shorter or longer than the input domain of an HCR hairpin and/or has incomplete complementarity to the input domain of the hairpin, but is able to hybridize to the input domain of the hairpin to open the hairpin and initiate the HCR polymerization cascade. In some of the embodiments provided herein, the fractional initiators within a probe unit are complementary to overlapping regions of an HCR hairpin (for example, regions that overlap by 1, 2 or more nucleotides), or are substantially complementary to an HCR hairpin (for example, complementary except for 0, 1, 2, a few, or several mismatches).

In some embodiments, the initiators on an initiator-labeled probe can be shielded to enhance penetration into the sample (to increase signal) and/or to reduce non-specific binding of the probe within the sample (to reduce background).

In some embodiments, the HCR process can include a hairpin label that can comprise a substrate that serves to recruit a reporter entity that comprises an enzyme that mediates catalytic reporter deposition (CARD).

In some embodiments, an HCR hairpin label can comprise a fractional substrate such that a label probe conjugated to a reporter molecule (or to a reporter entity) does not strongly bind the fractional substrate on an individual hairpin, but such that following HCR polymerization, neighboring hairpins in the HCR amplification polymer colocalize a full substrate such that the colocalized full substrate strongly binds a label probe conjugated to a reporter molecule (or to a reporter entity comprising an enzyme that mediates CARD signal amplification.

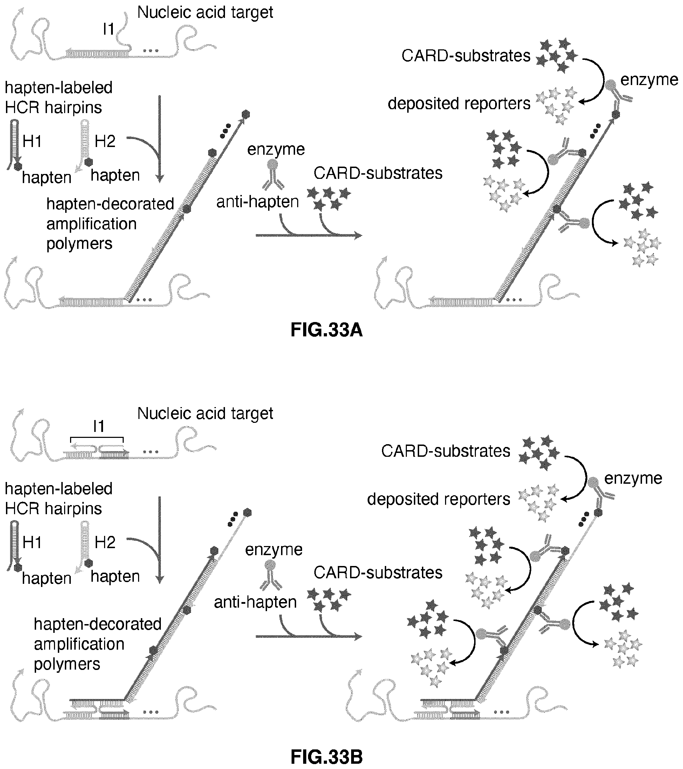

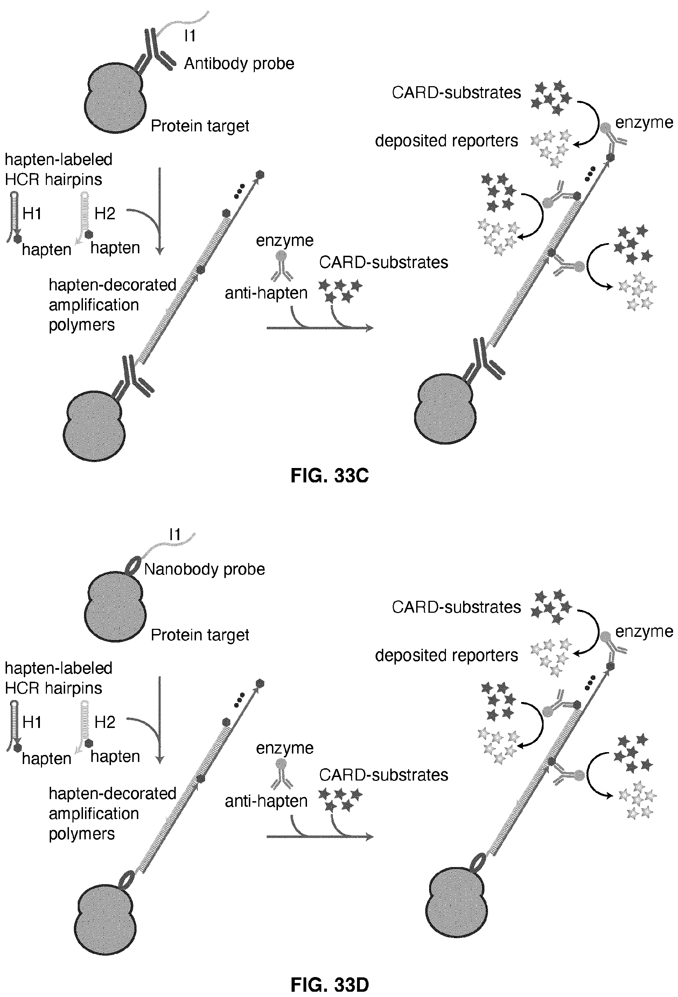

In some embodiments, the HCR process can include one or more haptens (for example see FIGs. 33A-33E, 34A-34C, 35, 36A-36B) that serve to mediate an additional layer of signal amplification via catalytic reporter deposition (CARD).

In some embodiments, a method for repeated signal detection with a reporter-labeled hairpin is provided. In some embodiments, the method comprises: a) providing a sample possibly containing up to N targets as well as possibly other molecules that are not targets; b) providing N probe sets (each targeting one of N target types) each comprising: one or more probe units each comprising two or more HCR fractional initiator probes; c) optionally washing the sample; d) providing M HCR amplifiers (for M ≤ N; each labeled with a distinct reporter) corresponding to M of the N probe sets; e) optionally washing the sample; f) detecting M signals corresponding to the M reporters; g) removing the M signals from the sample; and h) optionally repeating one or more of steps b-g until signal detection has been performed for all N targets.

In some embodiments, a method for repeated signal detection with a reporter-labeled hairpin is provided. In some embodiments, the method comprises: a) providing a sample possibly containing up to N targets as well as possibly other molecules that are not targets; b) providing N probe sets (each targeting one of N target types) each comprising one or more initiator-labeled probes each comprising one or more initiators; c) optionally washing the sample; d) providing M HCR amplifiers (for M ≤ N; each labeled with a distinct reporter) corresponding to M of the N probe sets; e) optionally washing the sample; f) detecting M signals corresponding to the M reporters; g) removing the M signals from the sample; and h) optionally repeating one or more of steps b-g until signal detection has been performed for all N targets.

In some embodiments, a method for repeated signal detection with a reporter-labeled hairpin is provided. In some embodiments, the method comprises: a) providing a sample possibly containing up to N targets as well as possibly other molecules that are not targets; b) providing N probe sets (each targeting one of N target types) each comprising either: 1) one or more HCR initiator-labeled probes each comprising one or more initiators, or 2) one or more probe units each comprising two or more HCR fractional initiator probes; c) optionally washing the sample; d) providing M HCR amplifiers (for M ≤ N; each labeled with a distinct reporter) corresponding to M of the N probe sets; e) optionally washing the sample; f) detecting M signals corresponding to the M reporters; g) removing the M signals from the sample; and h) optionally repeating one or more of steps b-g until signal detection has been performed for all N targets.

In some embodiments, the method comprises: a) providing a sample possibly containing up to N targets as well as possibly other molecules that are not targets; b) providing N probe sets (each targeting one of N target types) each comprising: one or more probe units each comprising two or more HCR fractional initiator probes; c) washing the sample; d) providing M HCR amplifiers (for M ≤ N; each labeled with a distinct reporter) corresponding to M of the N probe sets; e) washing the sample; f) detecting M signals corresponding to the M reporters; g) removing the M signals from the sample; and h) repeating one or more of steps b-g until signal detection has been performed for all N targets.

In some embodiments, the method comprises: a) providing a sample possibly containing one or more targets as well as possibly other molecules that are not targets; b) providing one or more probe sets each comprising one or more probe units each comprising two or more HCR fractional initiator probes; c) optionally washing the sample; d) providing one or more HCR amplifiers (each labeled with one or more reporters); e) optionally washing the sample; f) detecting one or more signals from one or more reporters; g) optionally removing one or more probe sets from the sample; h) optionally removing one or more HCR amplifiers from the sample; i) optionally removing one or more reporters from the sample; and j) optionally removing one or more signals from the sample. The process (all of the steps or a subset) can be repeated as desired.

### Definitions and Embodiments

"Nucleic Acids" as used herein includes oligomers of some form of DNA and/or RNA. Nucleic acids may also include analogs of DNA or RNA having modifications to either the bases or the backbone. For example, nucleic acid, as used herein, includes the use of peptide nucleic acids (PNA). The term "nucleic acids" also includes chimeric molecules. The phrase includes artificial constructs as well as derivatives etc. The phrase includes, for example, any one or more of DNA, RNA, 2'OMe-RNA, LNA, XNA, synthetic nucleic acid analogs, and PNA.

The term "sticky end" refers to a nucleic acid sequence that is available to hybridize with a complementary nucleic acid sequence. The secondary structure of the "sticky end" is such that the sticky end is available to hybridize with a complementary nucleic acid under the appropriate reaction conditions without undergoing a conformational change. Typically the sticky end is a single stranded nucleic acid.

"Monomers" are individual nucleic acid oligomers. Typically, at least two monomers are used in hybridization chain reactions, although three, four, five, six or more monomers may be used. Typically each monomer comprises at least one region that is complementary to at least one other monomer being used for the HCR reaction.

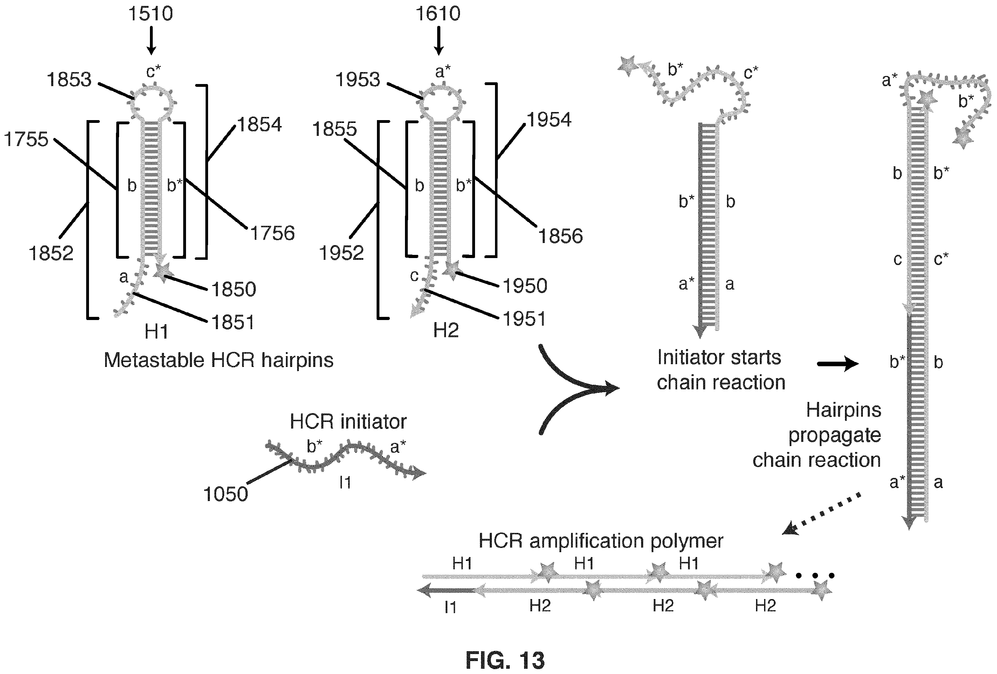

The composition can include (for example, see Figure 13) a first hairpin monomer (1510), comprising: a) a first input domain (1852), comprising a first toehold (1851) and a first stem section, b) a first output domain (1854), comprising a first hairpin loop (1853) and a complement to the first stem section, and c) a first reporter molecule (1850). The composition can further include a second hairpin monomer (1610), comprising: a) a second input domain (1952), comprising a second toehold (1951) and a second stem section, b) a second output domain (1954), comprising a second hairpin loop (1953) and a complement to the second stem section, and c) a second reporter molecule (1950).

In some embodiments, the monomers are "metastable." That is, in the absence of an initiator they are kinetically disfavored from associating with other monomers comprising complementary regions. "HCR" monomers are monomers that are able to assemble upon exposure to an initiator nucleic acid to form a polymer.

As used herein, "polymerization" refers to the association of two or more monomers to form a polymer. The "polymer" can comprise covalent bonds, non-covalent bonds or both. For example, in some embodiments two species of monomers are able to hybridize in an alternating pattern to form a polymer comprising a nicked double-stranded polymer. The polymers are also referred to herein as "HCR products."

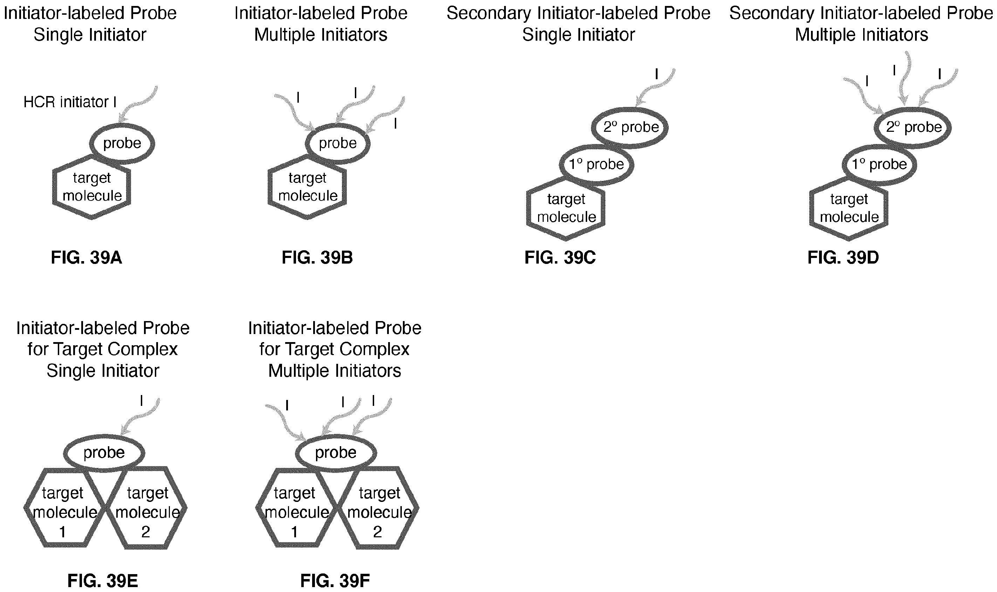

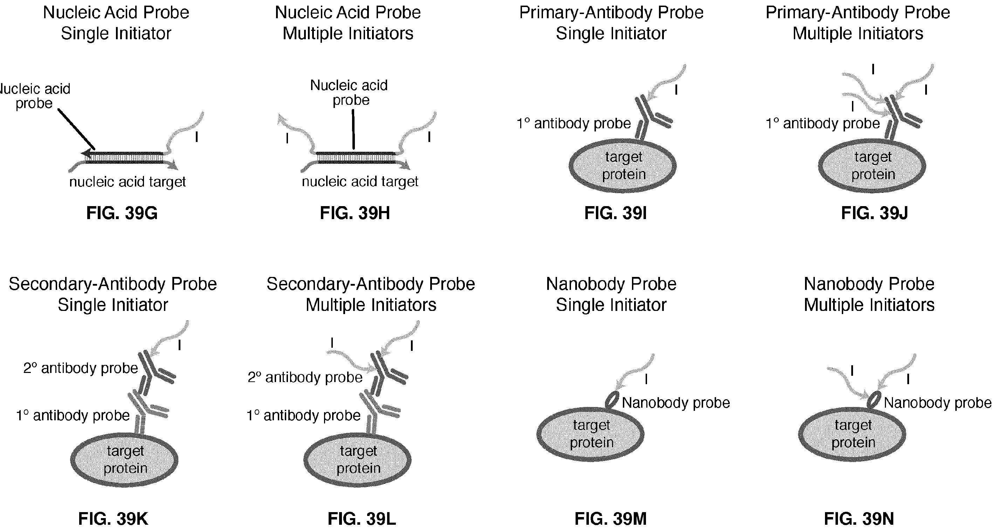

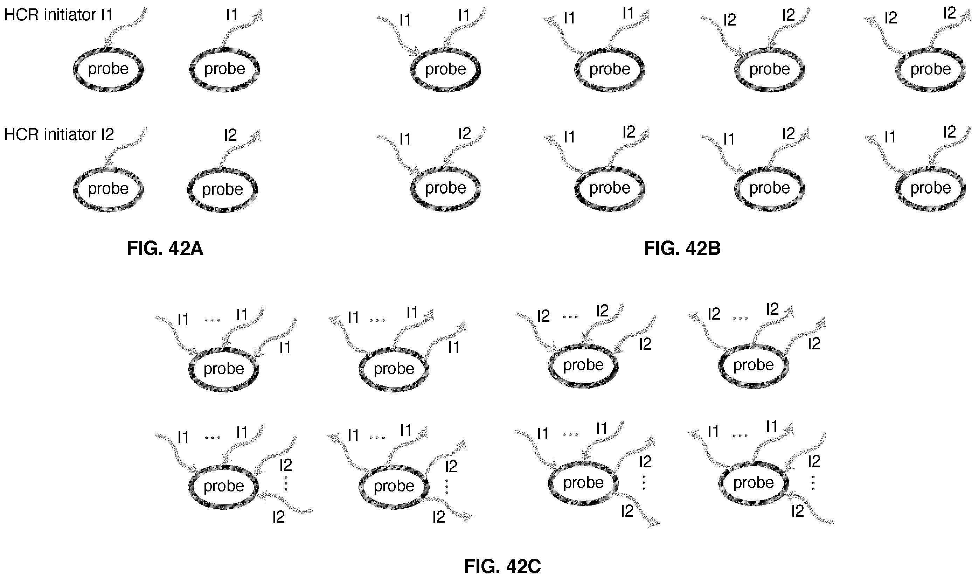

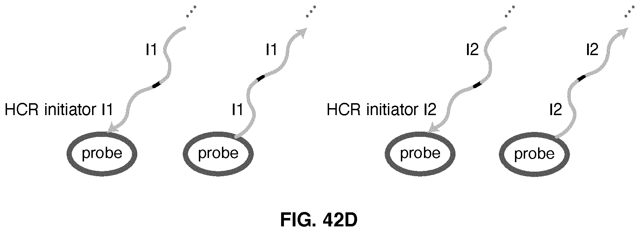

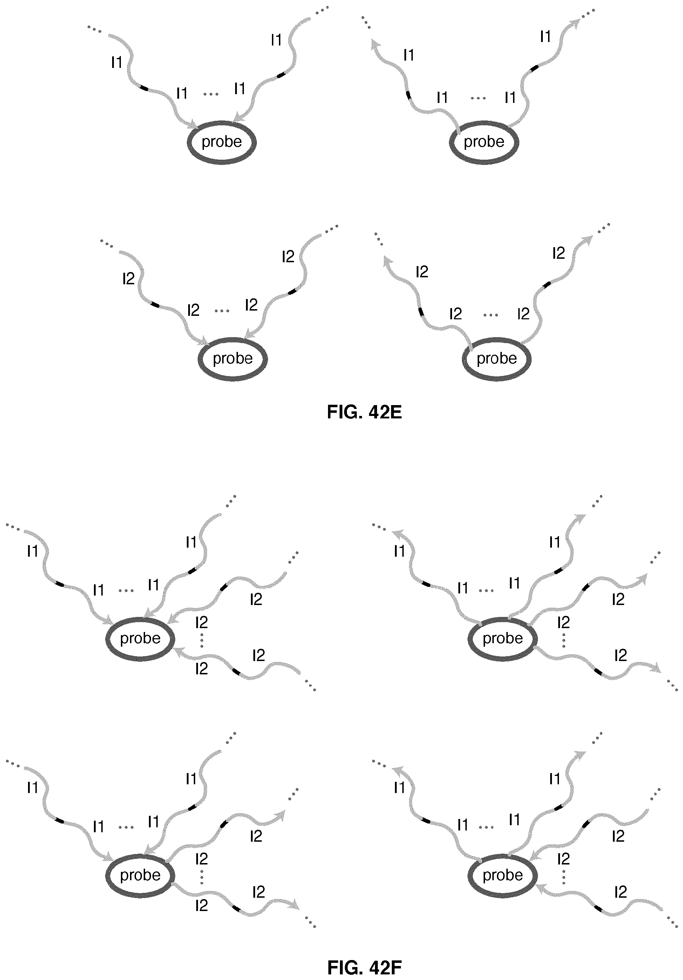

An "initiator-labeled probe" comprises one or more target-binding domains and one or more HCR initiators (for example, FIGs. 39A-39N and 42A-42F).

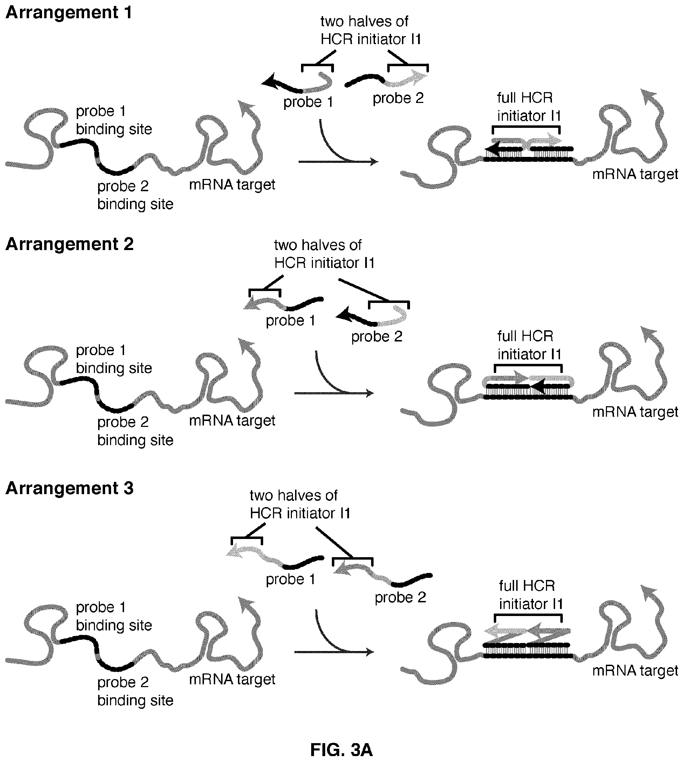

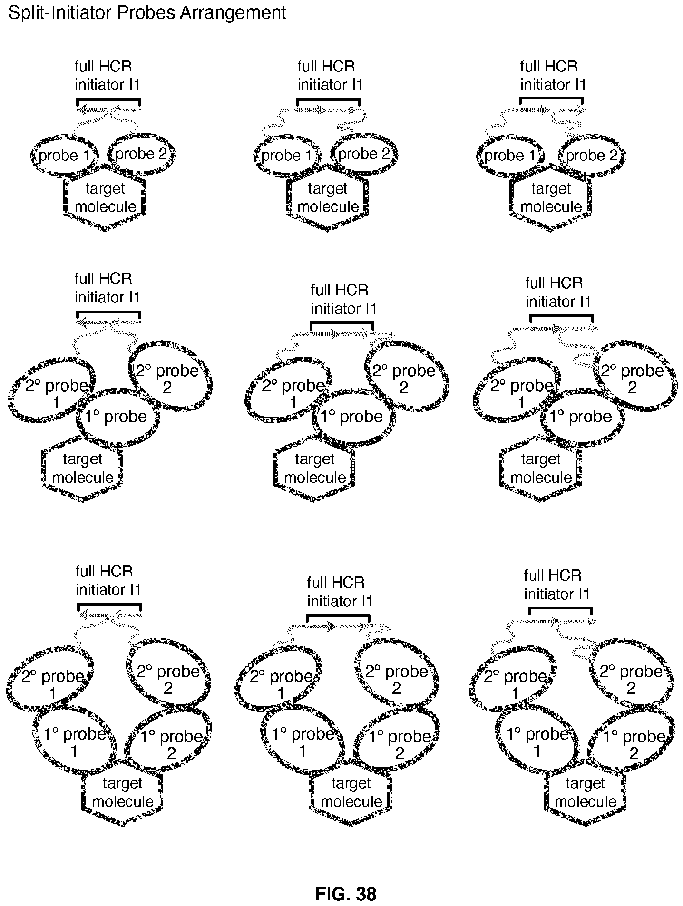

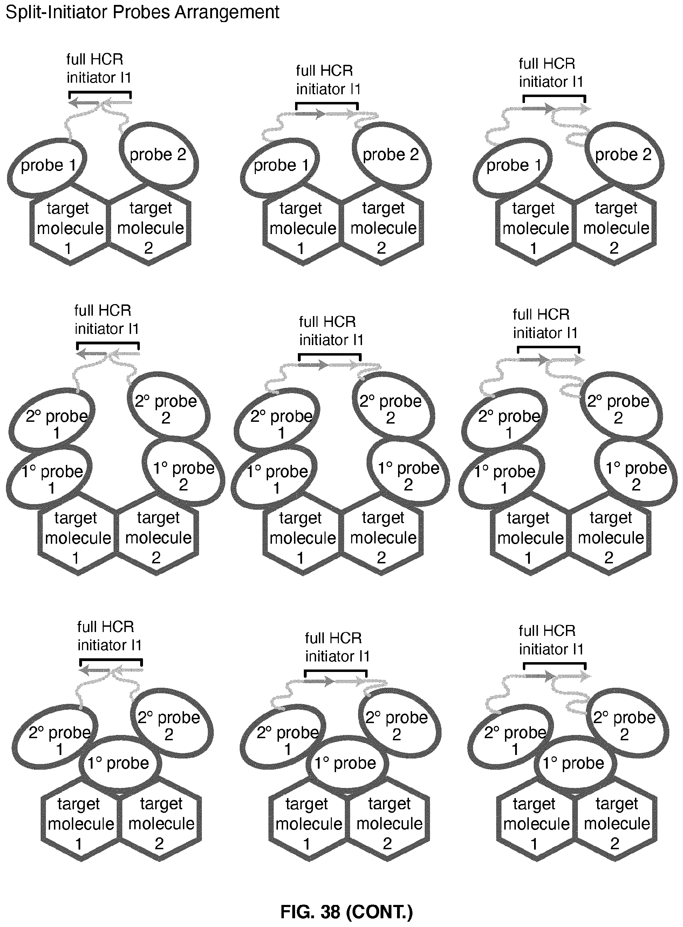

A "fractional initiator probe" comprises one or more target-binding domains and one or more fractional initiator domains (for example, FIGs. 3A-3B, 5A-5E, and 38). The terms "fractional initiator probes" and "fractional-initiator probes" are interchangeable as used herein.

A "full initiator" comes from the combination of two or more fractional initiators that, when colocalized, are able to initiate HCR polymerization. Some initiators comprise a nucleic acid region that is complementary to the initiator complement region of an HCR monomer. A fractional initiator is one that, on its own is insufficient to trigger HCR polymerization, but when colocalized with one (or more) other fractional initiators to form a full initiator, can trigger HCR polymerization.

A "probe unit" comprises two or more fractional initiator probes such that the fractional initiator domains on the fractional initiator probes within the probe unit can be colocalized to create a full initiator.

The following terms, in TABLE 1, are indicated as alternative options (which may vary in breadth depending upon the context) for the terms used herein. The disclosure of the broadest term not only denotes the broadest concept, but also the narrower concept herein. This approach and table are merely being used as a shorthand for denoting both options in a more concise manner.

**TABLE 1**

| | |
|---|---|
| Split-initiator probe | Fractional initiator probe |
| Target-binding sequence | Target-binding section |
| Proximal subsequence | Target section |
| Cognate proximal target site | Target section |
| Target-binding region | Target-binding section |
| HCR Initiator l1 | Full HCR Initiator |
| Hairpin H1 | First Hairpin Monomer |
| Hairpin H2 | Second Hairpin Monomer |
| HCR Hairpin | Hairpin Monomer |
| Split-initiator probe pair | Fractional initiator probe pair |
| Cognate target site | Target section |
| Initiator I1 | Full HCR Initiator |
| Probe 1 | First fractional initiator probe |
| Probe 2 | Second fractional initiator probe |
| Automatic background suppression | Active background suppression |
| Standard probe | Initiator-labeled probe |

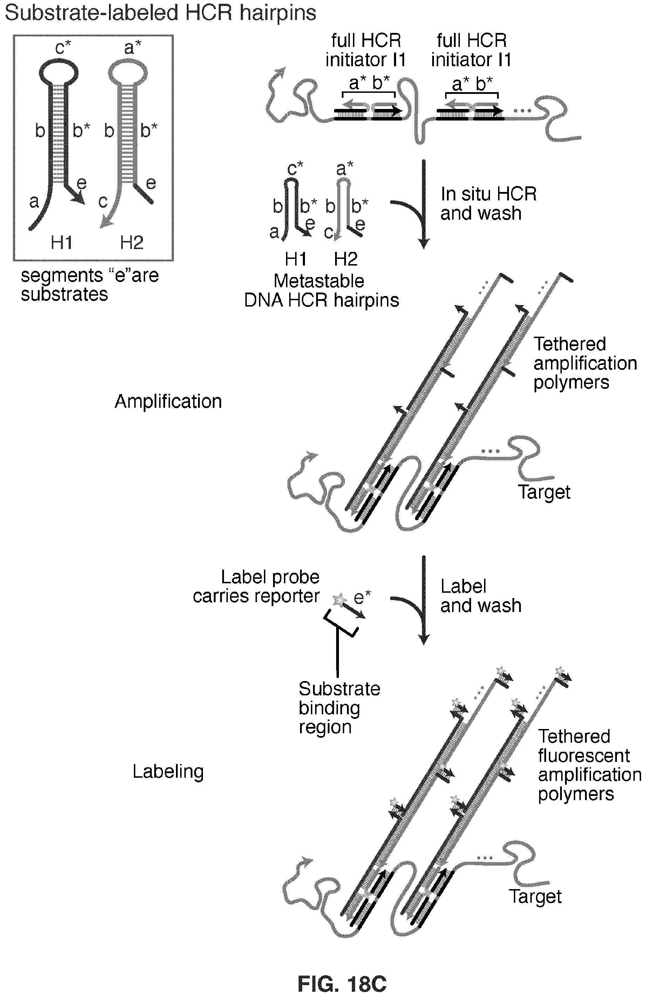

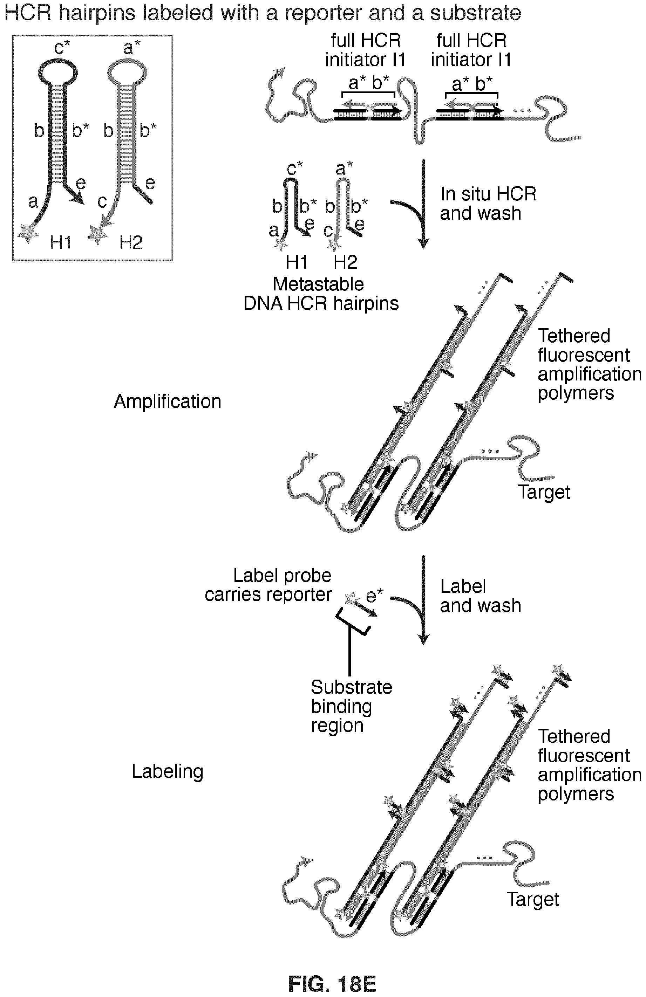

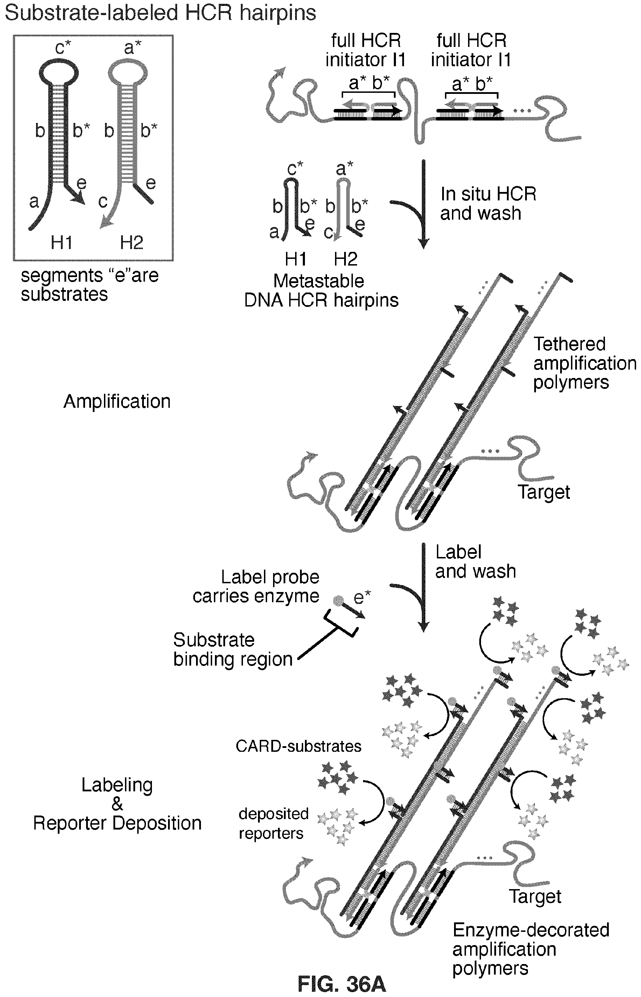

As used herein, "substrate" can denote: 1) a substrate domain on a hairpin (for example, domain "e" in FIGs. 18C, 18E and 36A) that serves as the binding site for the substrate binding region in label probe 2) CARD-substrates that become deposited reporters mediated by the CARD enzyme (for example, FIGs. 33A-33E, 34A-34C, 36A-36B). To avoid confusion, usage (2) substrates are intended to be denoted as "CARD-substrates" so that the word CARD is attached to "substrate" for that context.

The section headings used herein are for organizational purposes only and are not to be construed as limiting the described subject matter in any way. All literature and similar materials cited and/or in this application, including but not limited to, patents, patent applications, articles, books, treatises, and internet web pages are expressly incorporated by reference in their entirety for any purpose. When definitions of terms in incorporated references appear to differ from the definitions provided in the present teachings, the definition provided in the present teachings shall control. It will be appreciated that there is an implied "about" prior to the temperatures, concentrations, times, etc., discussed in the present teachings, such that slight and insubstantial deviations are within the scope of the present teachings herein. In this application, the use of the singular includes the plural unless specifically stated otherwise. Also, the use of "comprise", "comprises", "comprising", "contain", "contains", "containing", "include", "includes", and "including" are not intended to be limiting. It is to be understood that both the foregoing general description and the following detailed description are exemplary and explanatory only and are not restrictive. Unless defined otherwise, technical and scientific terms used herein have the same meaning as commonly understood by one of ordinary skill in the art to which this invention belongs. See, for example Singleton et al., Dictionary of Microbiology and Molecular Biology 2nd ed., J. Wiley & Sons (New York, N.Y. 1994); Sambrook et al., Molecular Cloning, A Laboratory Manual, Cold Springs Harbor Press (Cold Springs Harbor, N.Y. 1989). It is to be understood that both the general description and the detailed description are exemplary and explanatory only and are not restrictive of the invention as claimed. In this application, the use of the singular includes the plural unless specifically stated otherwise. In this application, the use of "or" means "and/or" unless stated otherwise. Furthermore, the use of the term "including", as well as other forms, such as "includes" and "included", is not limiting. Also, terms such as "element" or "component" encompass both elements and components comprising one unit and elements and components that comprise more than one subunit unless specifically stated otherwise. Also, the use of the term "portion" can include part of a moiety or the entire moiety.

In some embodiments, any one or more of the optional elements of any one or more of the figures herein can be combined with any one or more of the other optional steps of any one or more of the figures herein.

In some embodiments, any one or more of the elements that are different for any one or more of the figures herein can be combined with any one or more of the other steps that are different for any one or more of the figures herein.

In some embodiments, any one or more of the optional elements that are different for any one or more of the figures herein can be combined with any one or more of the other optional steps that are different for any one or more of the figures herein.

The term "step" denotes an action that occurs and/or can be performed. It is noted that multiple "steps" can occur at once or in an overlapping period of time and/or sequentially. Unless denoted otherwise (explicitly or implicitly given the context of the disclosure) all options are contemplated herein. Furthermore, the steps can be taken in different order or repeated or have additional intervening or overlapping steps added, unless denoted otherwise.

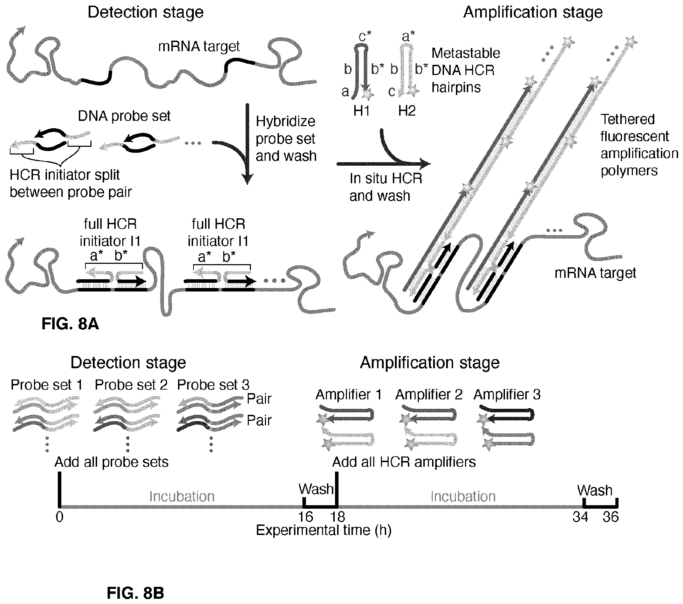

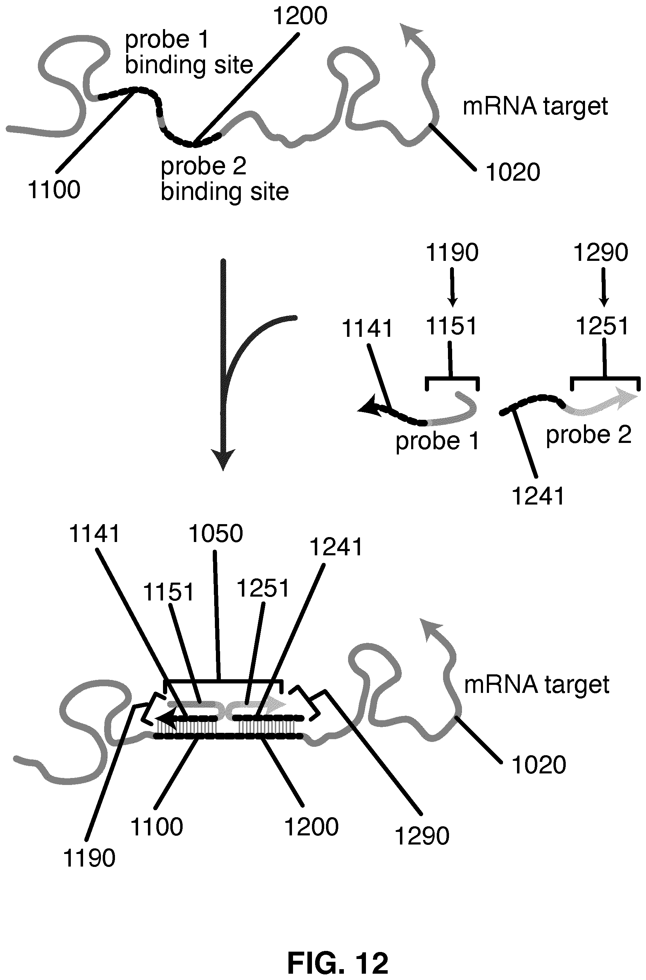

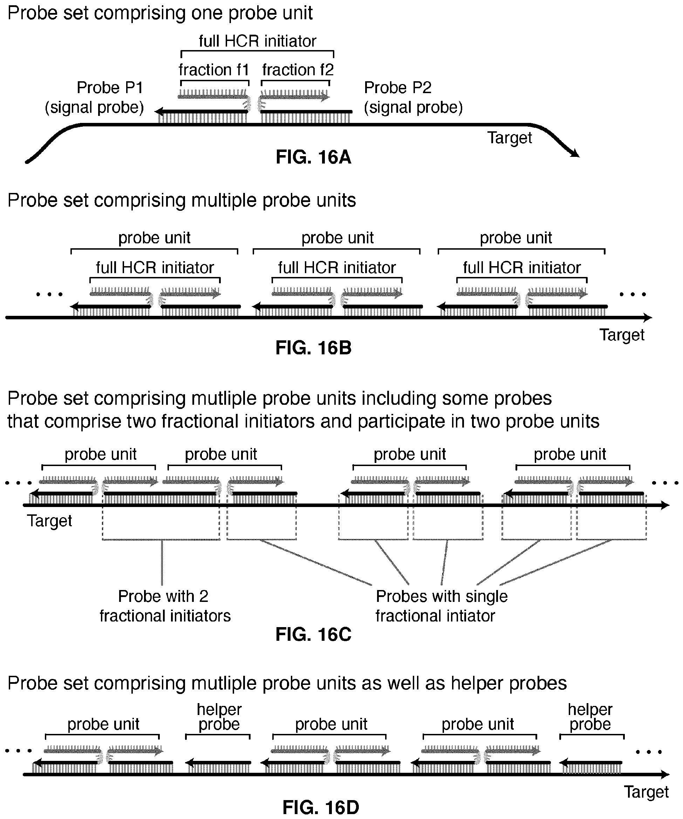

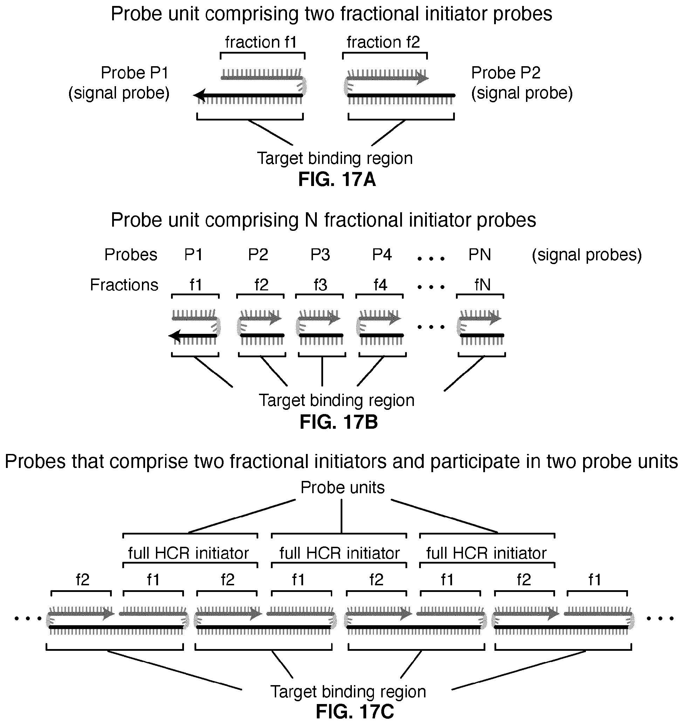

**Analysis of target molecules within a sample via hybridization chain reaction.** In some embodiments, a target is detected using a probe set comprising one or more initiator-labeled probes each comprising one or more HCR initiators (for example, FIGs. 39A-39N, 42A-42F and 43A). In some embodiments, a target is detected within a sample using a probe set comprising one or more probe units (for example see the probe sets of Figures 8A-8B and 16A-16D), where a probe unit comprises two or more HCR fractional initiator probes (for example see the probe units of Figures 3A-5E, and 17A-17C), where each HCR fractional initiator probe comprises a target-binding region and a fractional initiator (for example, see the fractional initiator probes of Figures 12 and 17A-17C). In some embodiments, binding of each probe within a probe unit to adjacent cognate binding sites on the target colocalizes the fractional initiators to form a full HCR initiator (for example, see the full HCR initiators of Figures 3A-5E, 8A-8B, 16A-16D, 21A-22, 27, and 38) capable of hybridizing to an HCR hairpin to trigger HCR signal amplification.

In some embodiments, the HCR signal amplification increases the signal-to-background ratio for molecular detection and imaging applications by boosting the signal above the background arising from the sample by about 5, 10, 15, 20, 25, 30, 40, 50, 75, 100, 500, 1000, or 2000-fold, or a value with a range defined by any two of the aforementioned values.

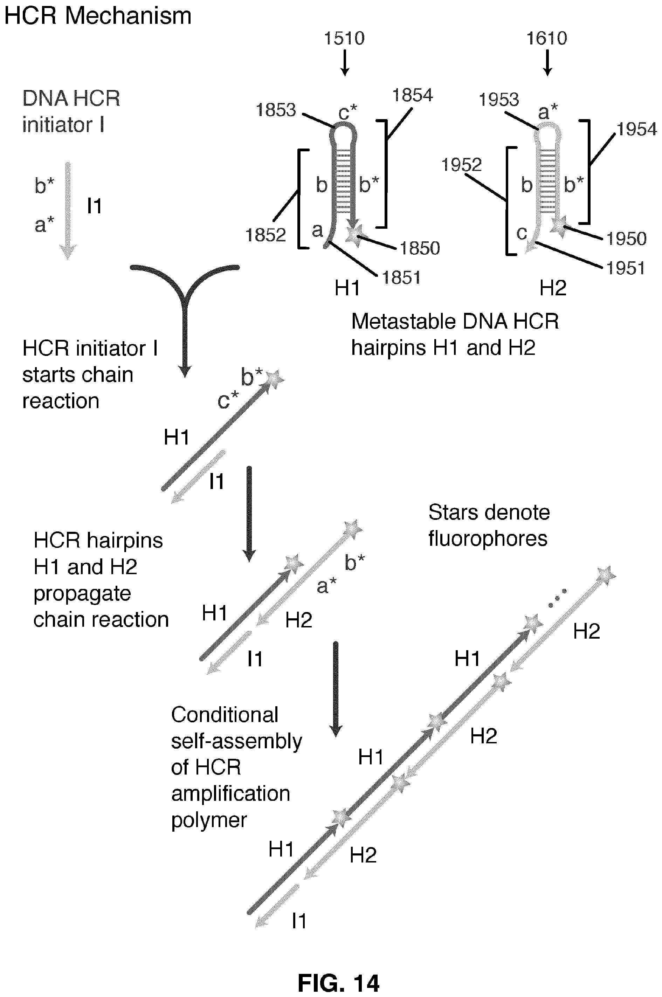

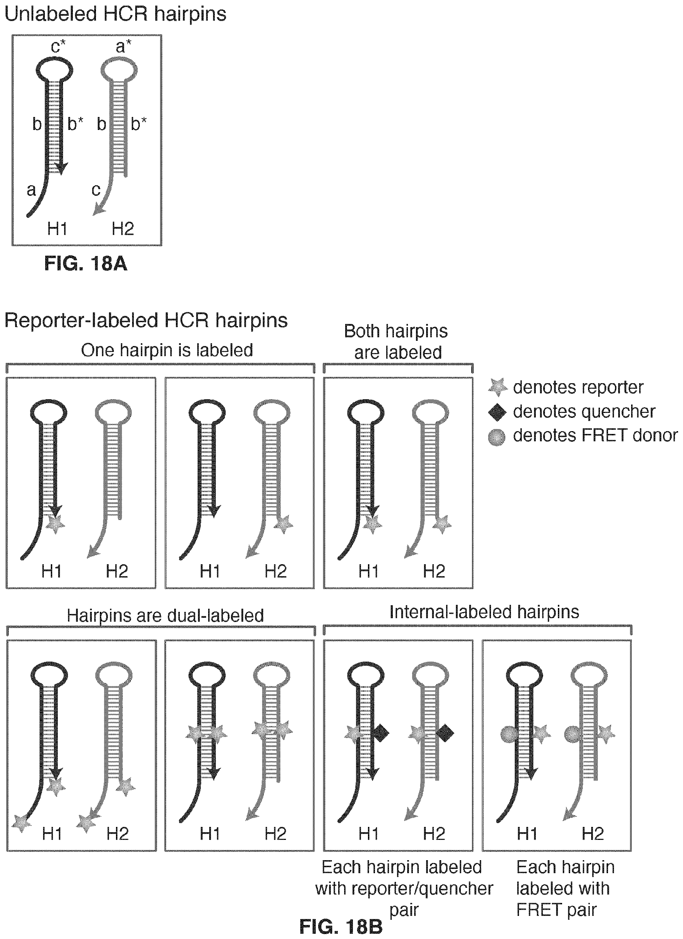

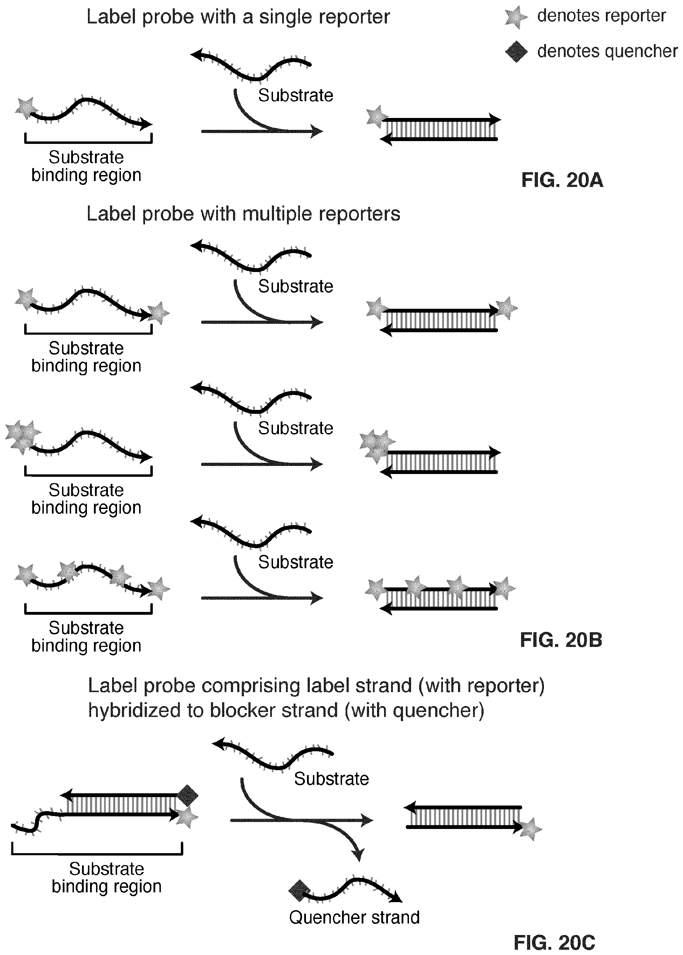

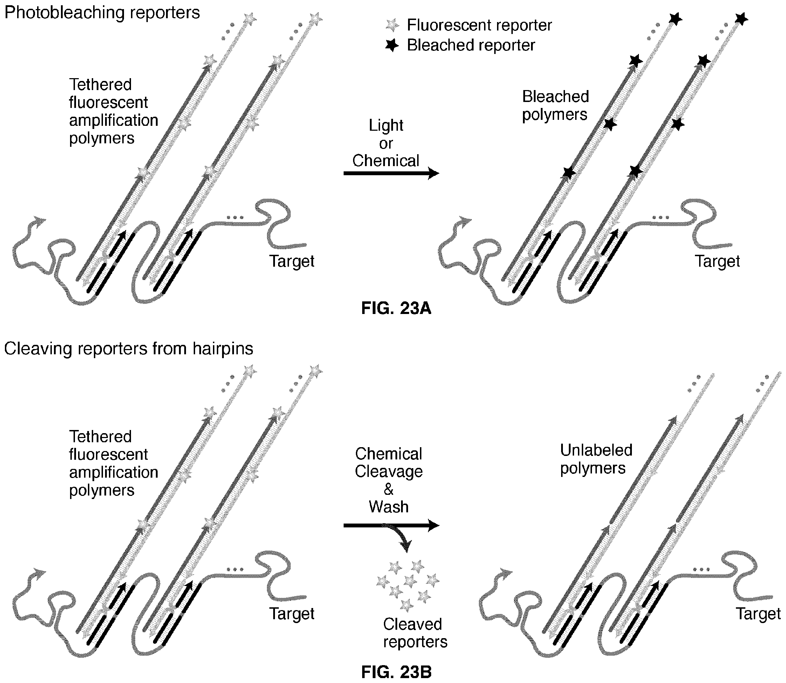

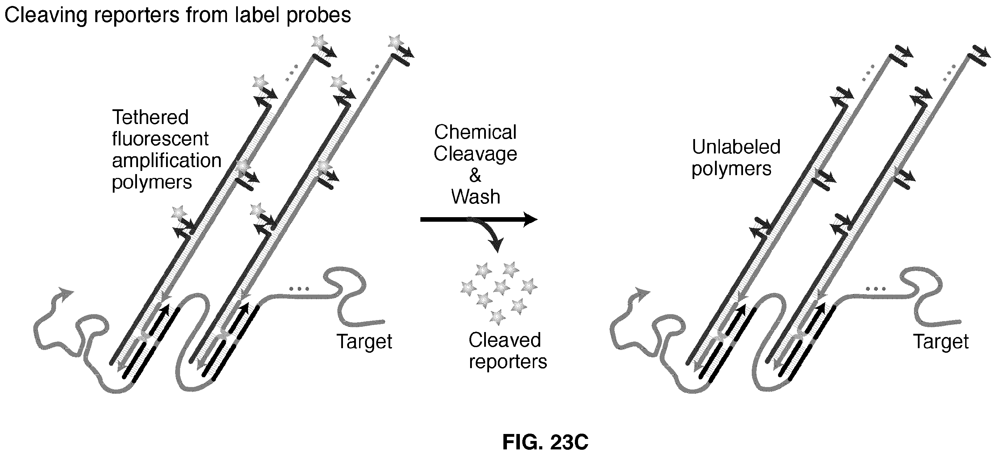

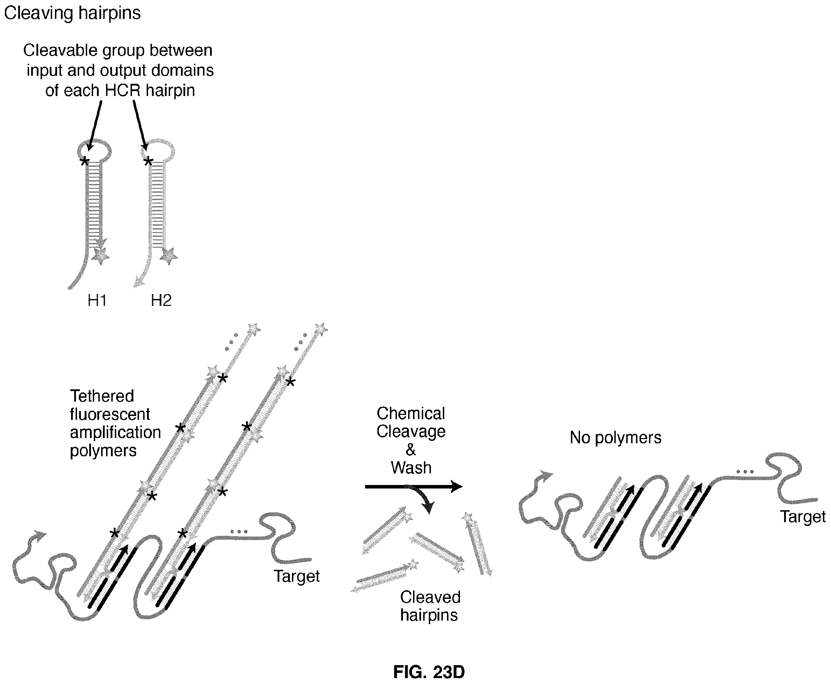

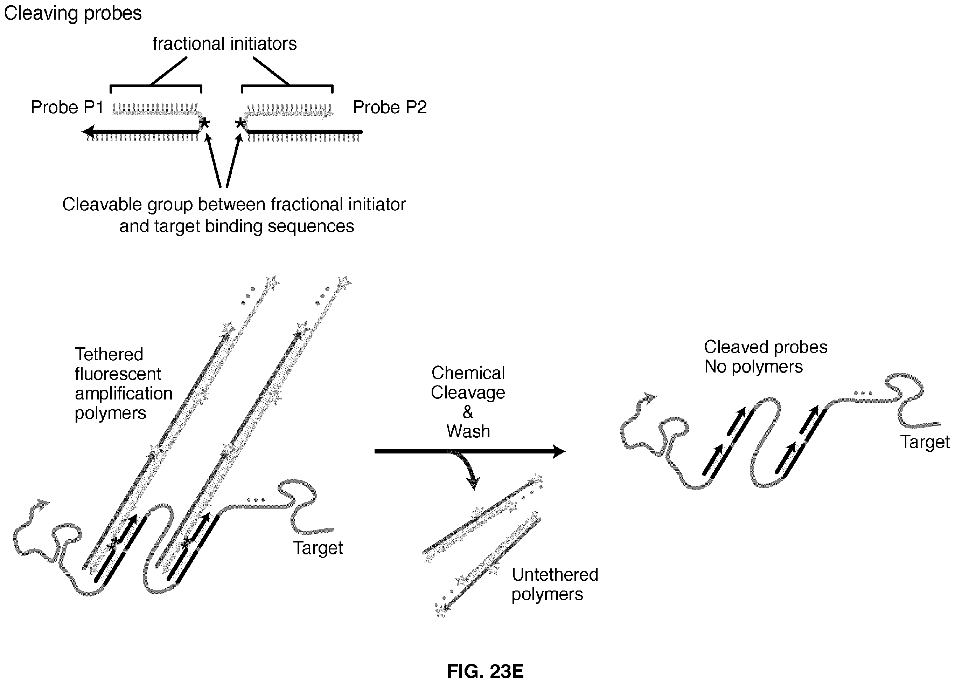

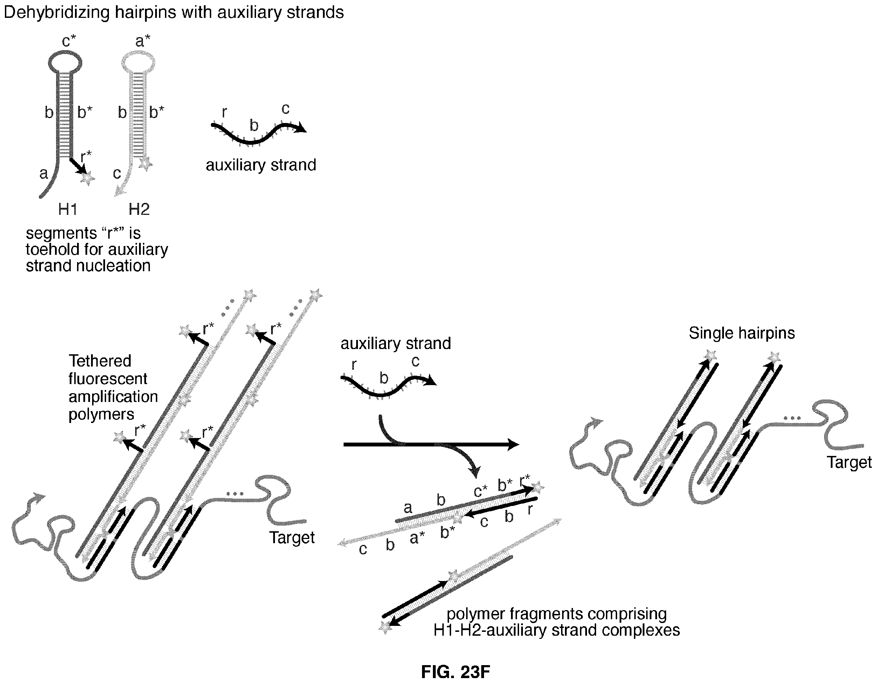

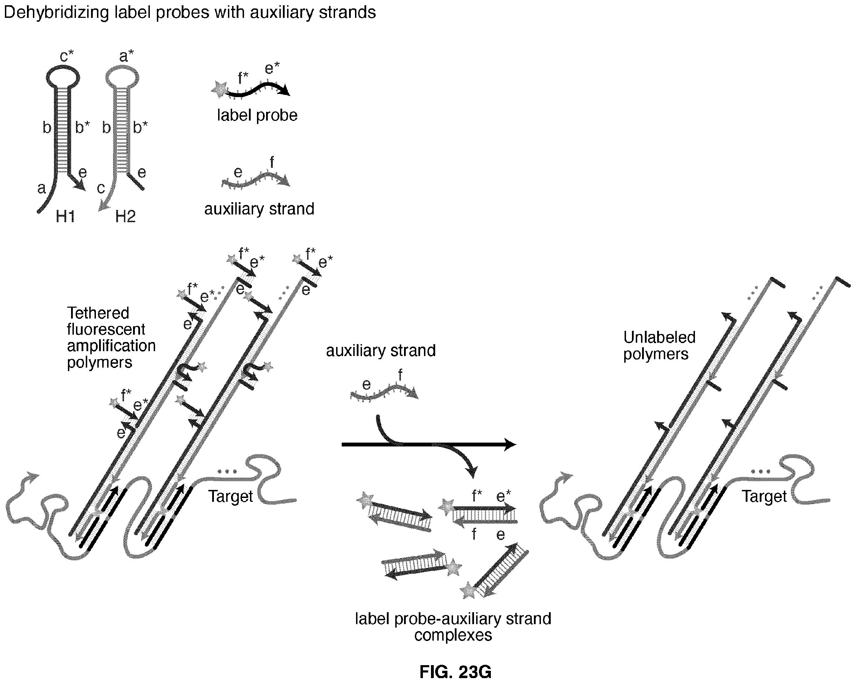

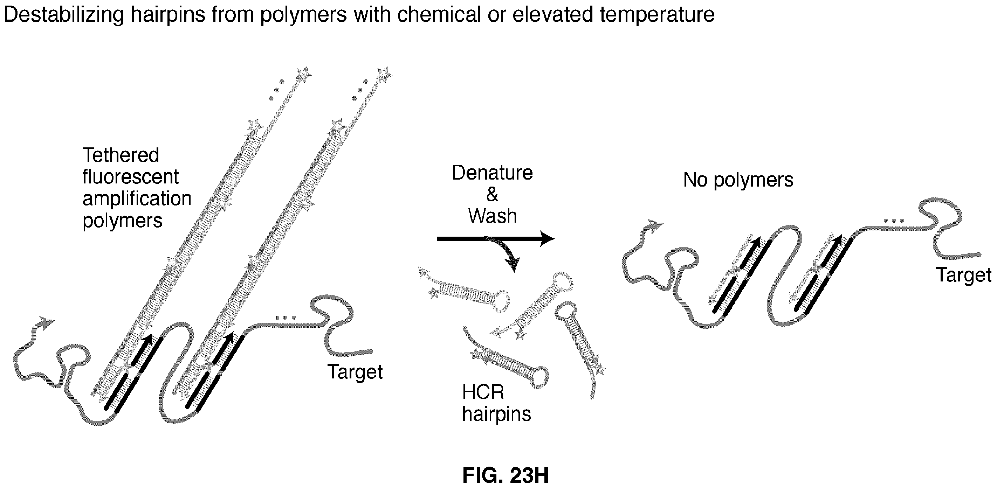

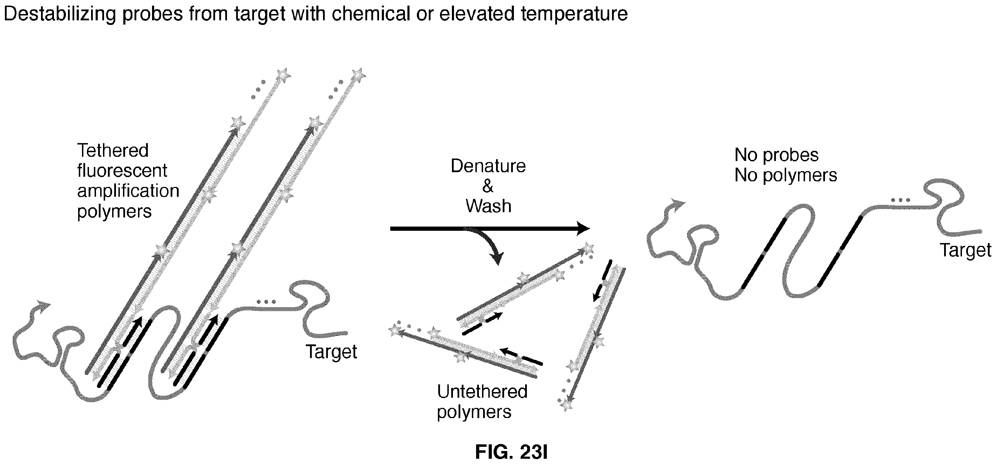

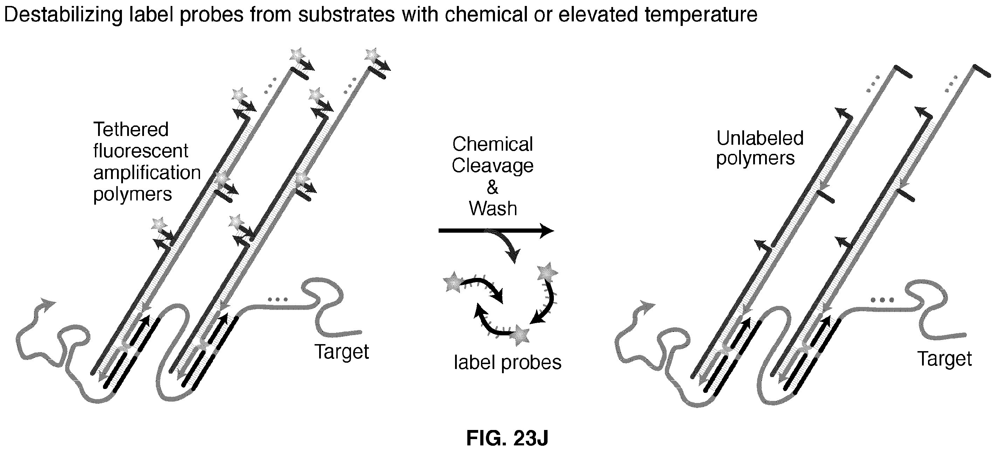

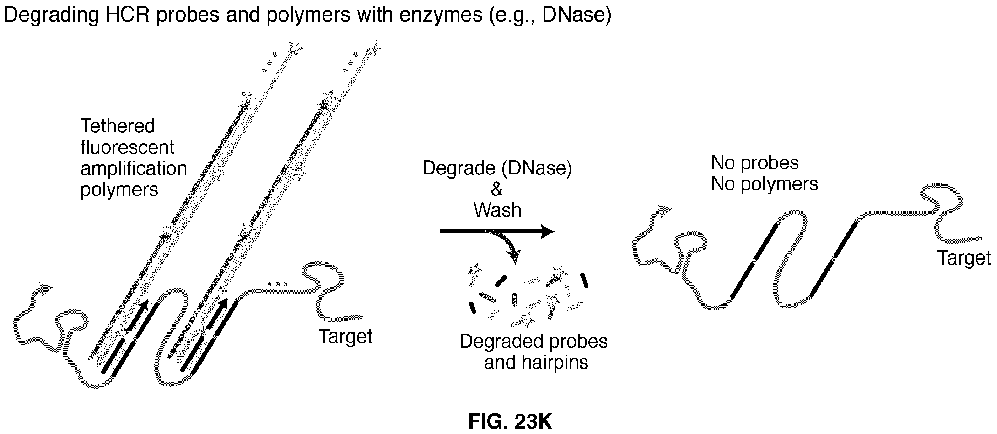

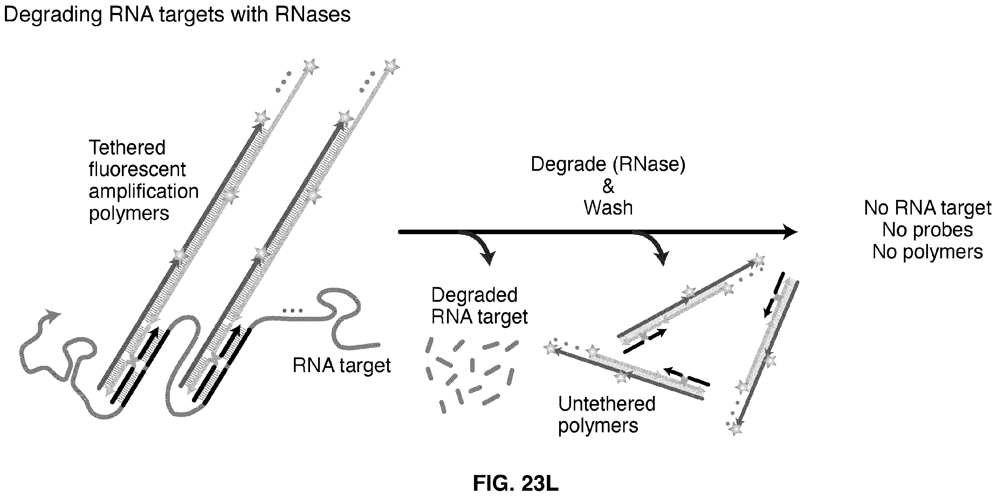

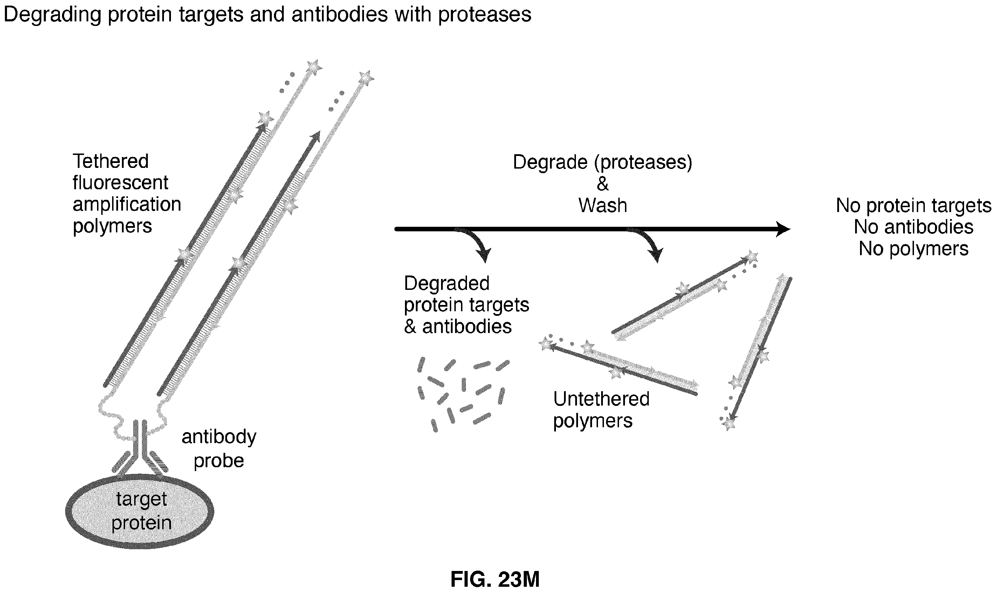

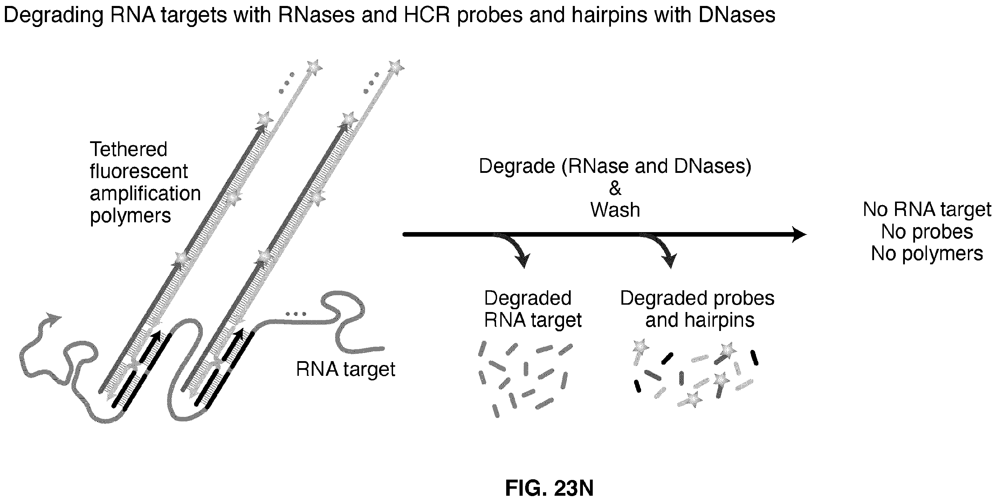

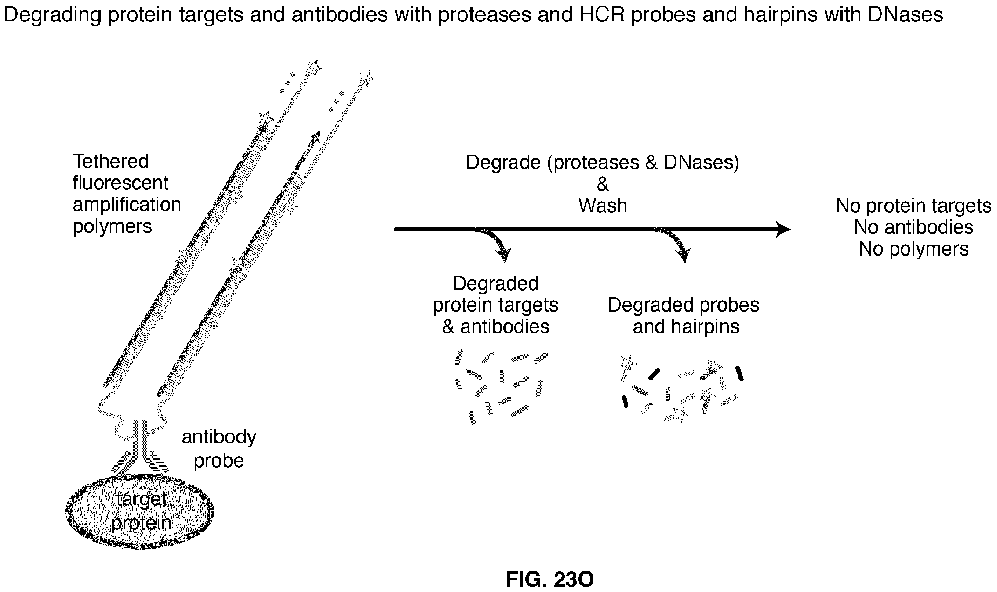

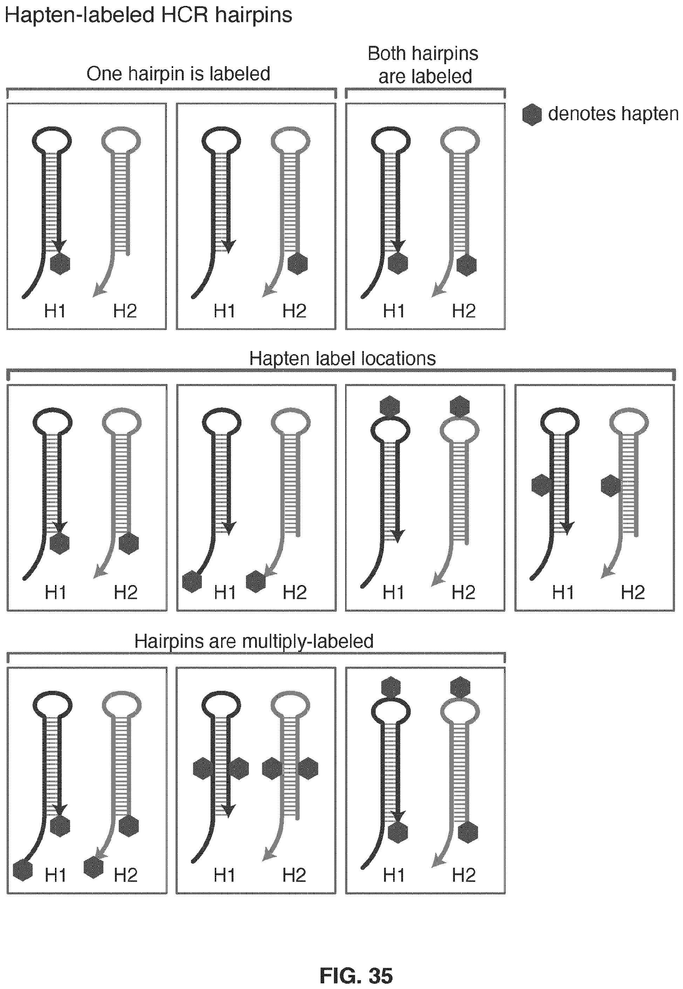

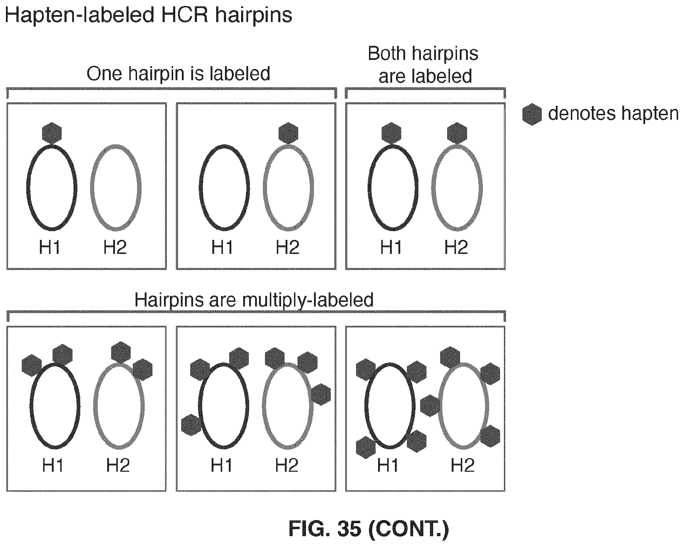

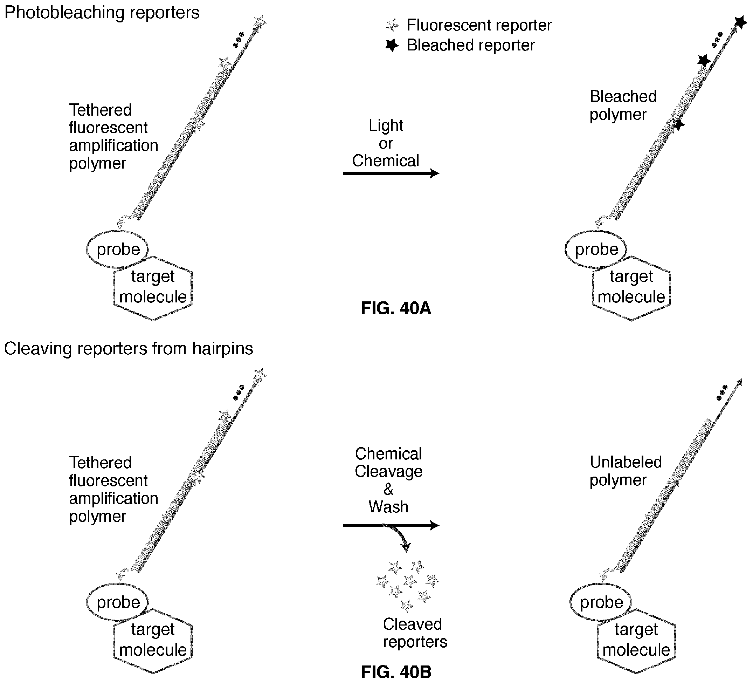

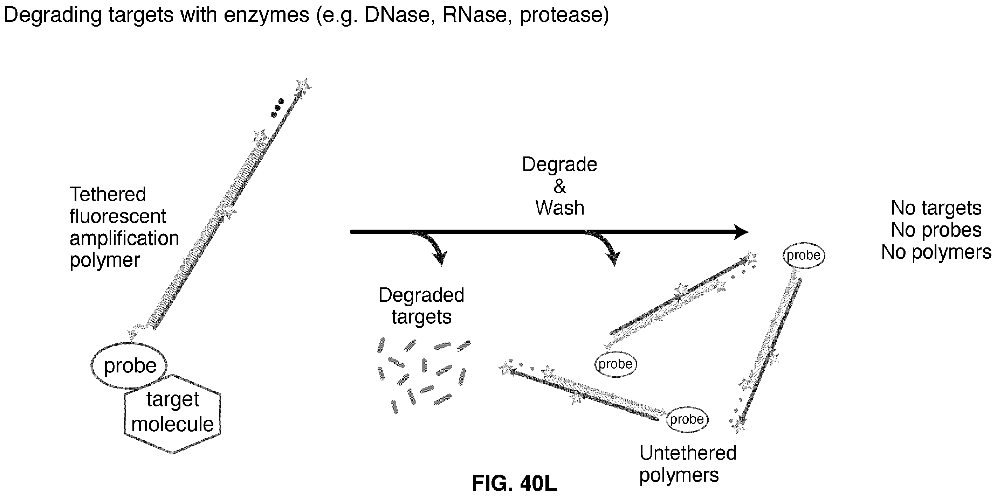

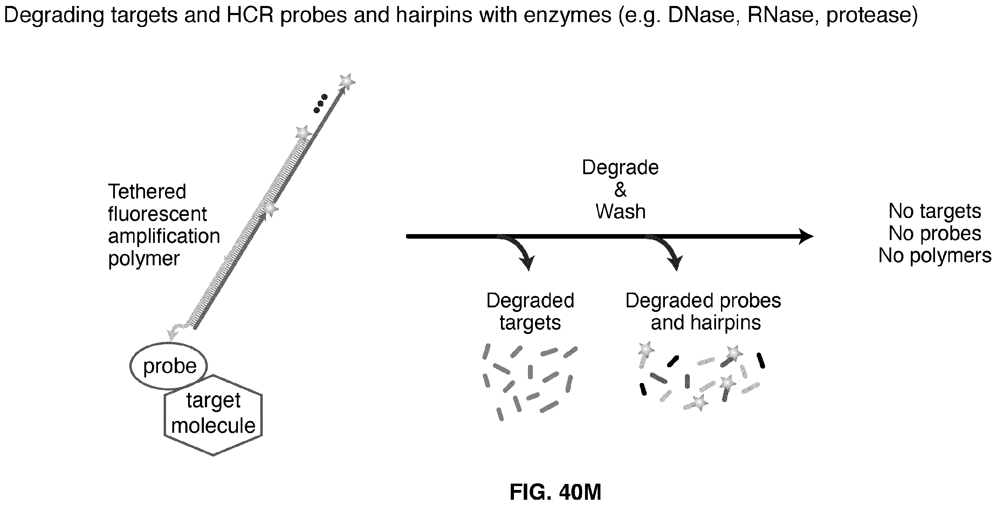

In some embodiments, the target-binding regions within a probe unit are configured to bind to overlapping or non-overlapping regions of the target (for example, see Figures 8A-8B, 21A-21B, 22, and 27). In some embodiments, the fractional initiators within a probe unit are designed to hybridize to overlapping or non-overlapping regions of an HCR hairpin (for example, see Figures 8A-8B, 21A-22, 27, and 28). Individual probes that bind non-specifically in the sample do not colocalize a full HCR initiator, suppressing HCR signal amplification. An HCR amplifier comprises two or more HCR hairpins (for example, see the HCR amplifiers of Figures 8A-8B, 18A-18F, and 19A-19B). In some embodiments, each HCR hairpin comprises an input domain with a single-stranded toehold and a stem section, and an output domain with a single-stranded loop and a complement to the stem section (for example, see the HCR hairpins of Figures 8A-8B, 13, 14, 18A-18F, and 19A-19B). In some embodiments, the one or more HCR initiators on an initiator-labeled probe each initiate a chain reaction of polymerization steps in which the initiator hybridizes to the input domain of a first HCR hairpin, opening the first hairpin to expose its output domain, which in turn hybridizes to the input domain of a second HCR hairpin, opening the second hairpin to expose its output domain, and so on and so forth, leading to a chain reaction in which hairpins polymerize to yield an HCR amplification polymer tethered to the target (for example, see the amplification polymers of Figures 13, 14, 19A-19B, 29A, 30A, 33A, 33C-33E, 34A, and 41A). In the absence of a full HCR initiator, HCR hairpins are kinetically trapped and do not polymerize, suppressing background. However, if the fractional initiator probes within a probe unit bind to their adjacent cognate binding sites on the target to colocalize a full HCR initiator, the full HCR initiator initiates a chain reaction of polymerization steps in which the full initiator hybridizes to the input domain of a first HCR hairpin, opening the first hairpin to expose its output domain, which in turn hybridizes to the input domain of a second HCR hairpin, opening the second hairpin to expose its output domain, and so on and so forth, leading to a chain reaction in which hairpins polymerize to yield an HCR amplification polymer tethered to the target (for example, see the amplification polymers of Figures 8A, 13, 14, 18C-18F, and 19A-19B). In some embodiments, an HCR hairpin further comprises one or more labels, each label comprising a reporter or comprising a substrate (or a fractional substrate) that recruits a label probe comprising one or more reporters (for example, see the reporter-labeled, substrate-labeled, and fractional-substrate-labeled HCR hairpins of Figure 18A-18F). In some embodiments, the zero, one, or more substrates on an HCR hairpin may comprise haptens (for example see FIG. 35) that serve to mediate an additional layer of signal amplification via catalytic reporter deposition (CARD) (see for example, FIGs. 33A-33E, 34A-34D and 37). In some embodiments, a label probe comprises one or more reporters and further comprises a substrate-binding region complementary to a substrate on an HCR hairpin or complementary to a full substrate colocalized within an HCR amplification polymer (for example, see the label probes of FIGs. 20A-20F and 36A-36B). In some embodiments, signal is generated by one or more reporters associated with an HCR amplification polymer tethered to the target within the sample. In some embodiments, signal is removed from the sample (for example, see Figure 23). In some embodiments, HCR signal is generated, detected, and removed from the same sample one or more times (for example, see FIGs. 23A-23O and 40A-40N).

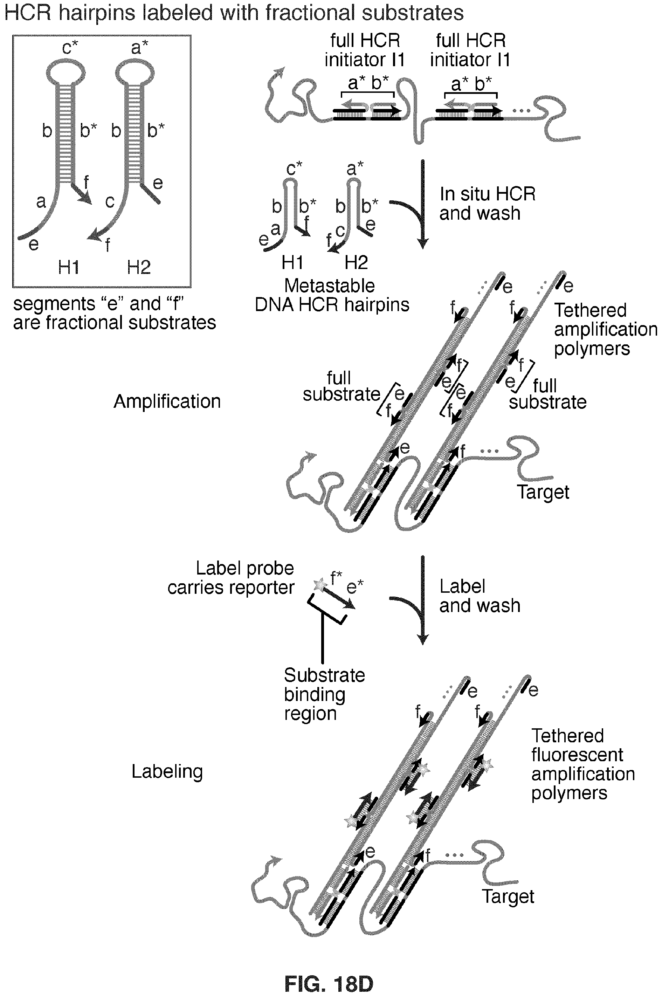

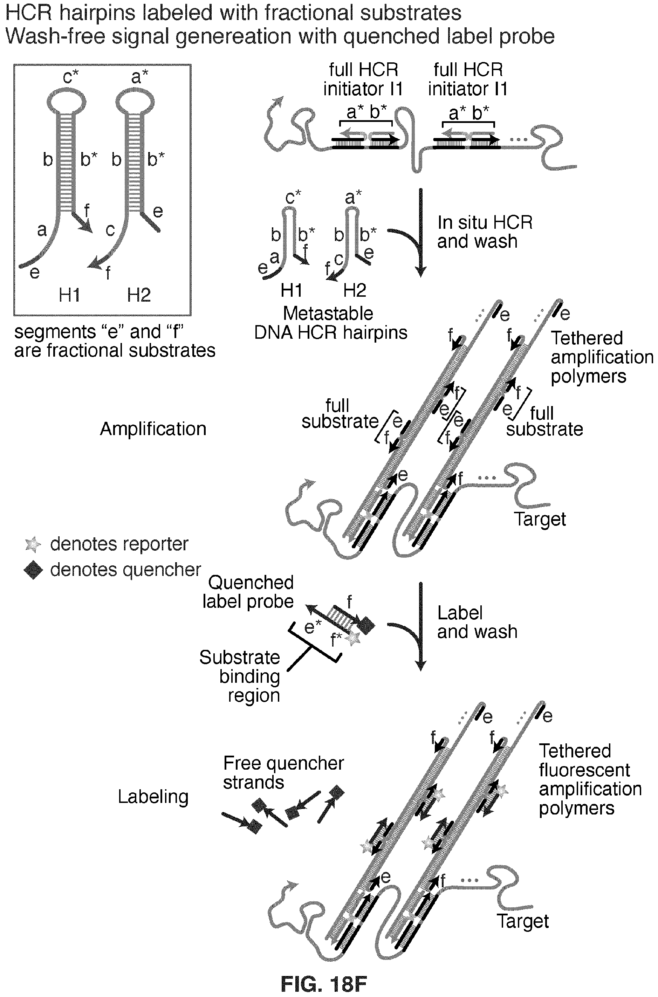

In some embodiments, any one or more of the steps provided in any of the figures provided herein can be combined into one of the other methods provided herein. As used herein, a generic reference to a set of figures (e.g., FIG. 18) denotes all of the different figures contained within that number (e.g., 18A-18F), each combined together, one or more of them, or each in the alternative, unless otherwise denoted.

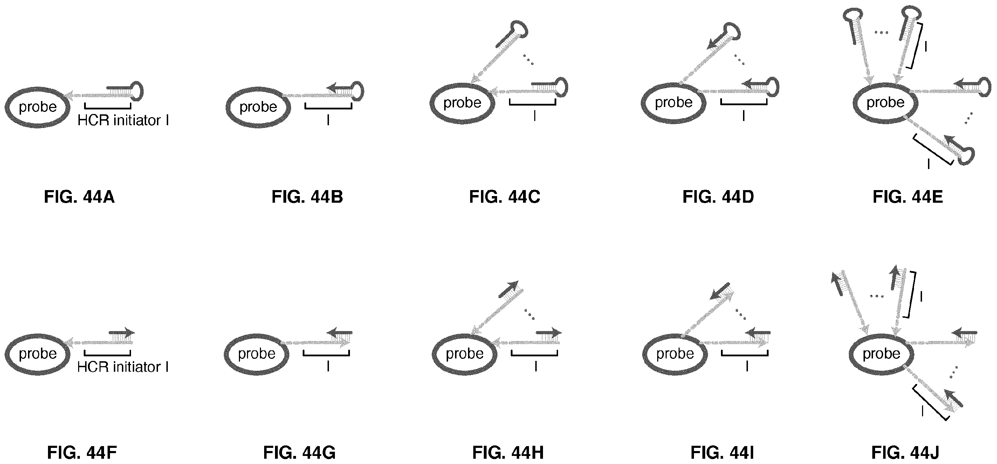

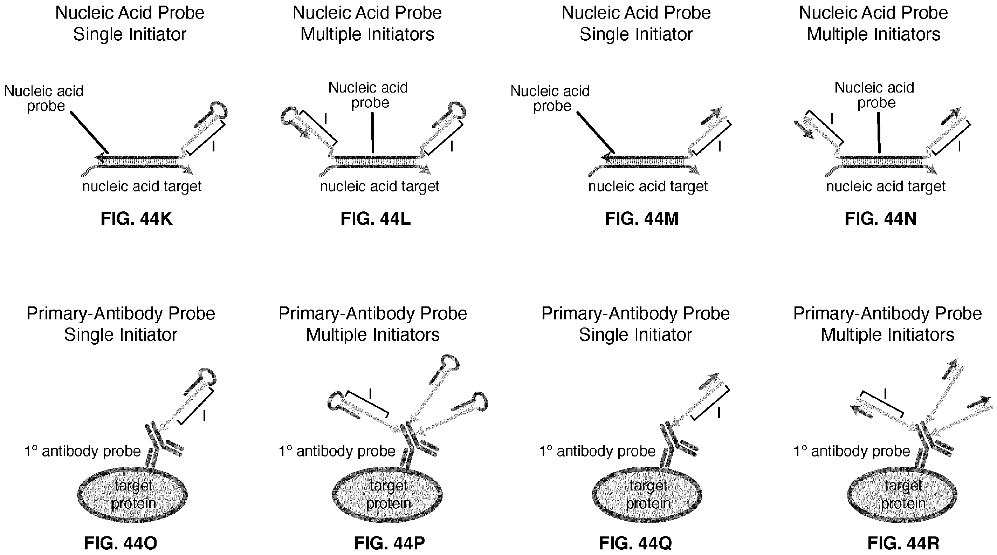

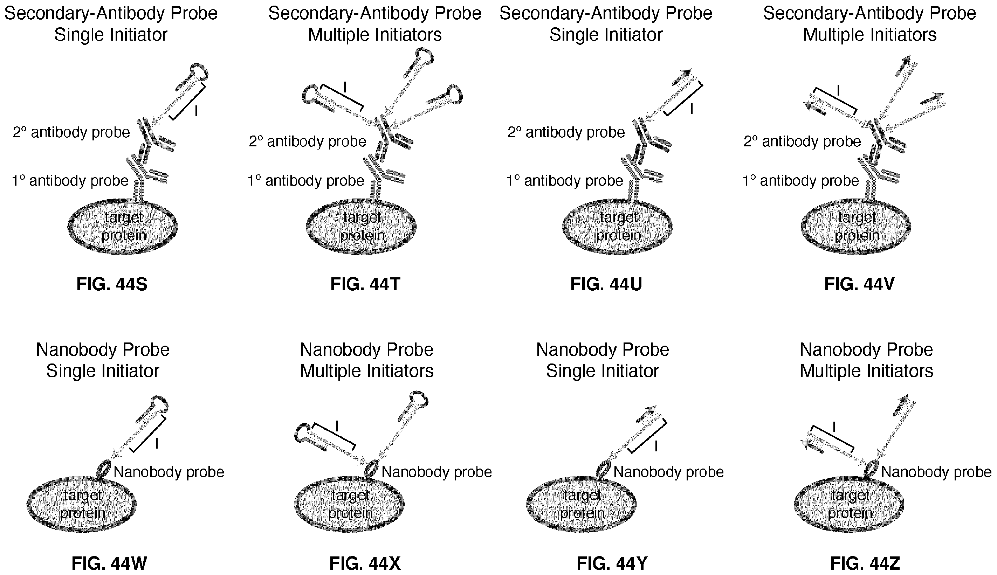

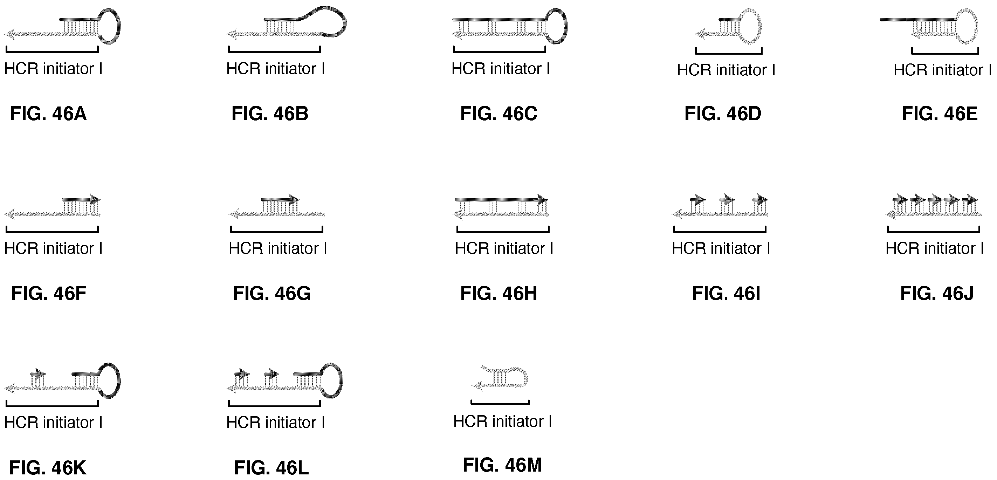

**HCR initiators.** An initiator-labeled probe comprises one or more HCR initiators capable of initiating an HCR polymerization cascade. In some embodiments, an initiator is fully complementary to the input domain of an HCR hairpin such that it hybridizes to the input domain of the hairpin to open the hairpin and initiate the HCR polymerization cascade. In some embodiments, the initiator is partially complementary to the input domain of an HCR hairpin, but sufficiently complementary such that it hybridizes to the input domain of the hairpin to open the hairpin and initiate the HCR polymerization cascade. In some embodiments, the initiator is shorter or longer than the input domain of an HCR hairpin and/or has incomplete complementarity to the input domain of the hairpin, but is able to hybridize to the input domain of the hairpin to open the hairpin and initiate the HCR polymerization cascade. In some embodiments, an HCR initiator might have 60%, 70%, 80%, 90%, or 100% complementarity to the input domain of an HCR hairpin, and hybridize to the input domain of the hairpin to open the hairpin and initiate the HCR polymerization cascade. In some situations, initiator-labeled probes comprising one or more initiators may experience reduced penetration into the sample due to initiators binding non-specifically near the surface of the sample. In some situations, initiator-labeled probes comprising one or more initiators may cause increased background due to non-specific binding of initiators to DNA, RNA, proteins, or other molecules within the sample. In some embodiments, the initiators on an initiator-labeled probe are shielded by base-pairing (see for example FIGs. 44A-44Z, 45A-45B, and 46A-46M) to enhance penetration into the sample (to increase signal) and/or to reduce non-specific binding of the probe within the sample (to reduce background). In some embodiments, the initiator can be shielded by a hairpin structure (for example FIGs. 44A-44E, 44K-44L, 44O-44P, 44S-44T, 44W-44X, 46A-46E, and 46M). In some embodiments, the initiator can be shielded by one or more auxiliary oligos (for example FIGs. 44F-44J, 44M-44N, 44Q-44R, 44U-44V, 44Y-44Z, 46F-46J). In some embodiments, the initiator can be shielded by self-complementarity within the oligo comprising an initiator and/or complementarity to one or more auxiliary strands (for example, FIGs. 44A-44Z and 46A-46M).

**Automatic background suppression with HCR fractional initiator probes.** In some embodiments, fractional initiator probes automatically suppress background because the HCR initiator (I1 or I2) is split between a pair of probes (for example, see Figures 8 and 12). In some embodiments, if probes bind specifically to the target at proximal cognate binding sites, the target colocalizes the two probes within a probe pair to form a full HCR initiator. In some embodiments, individual probes that bind non-specifically do not trigger HCR since each probe carries only a fraction of an HCR initiator, and HCR signal amplification is triggered only if the full HCR initiator is colocalized.

**Automatic background suppression with HCR hairpins.** In some embodiments, HCR hairpins automatically suppress background because HCR hairpins are kinetically trapped so they do not polymerize in the absence of an HCR initiator (I1 or I2). In some embodiments, if both probes within a fractional initiator probe pair bind specifically to their proximal cognate binding sites on the target, the resulting colocalized full HCR initiator (I1 or I2) triggers growth of a tethered HCR amplification polymer (for example, see Figure 8). Individual HCR hairpins that bind non-specifically in the sample do not trigger HCR since they are kinetically trapped.

**Automatic background suppression with HCR fractional initiator probes and HCR hairpins.** The combination of HCR fractional initiator probes for target detection and HCR amplification hairpins for signal amplification provide automatic background suppression throughout the protocol, ensuring that reagents will not generate amplified background even if they bind non-specifically within the sample.

**Full HCR initiator split between 2 or more fractional initiator probes.** We refer to each set of fractional initiator probes that generate a full HCR initiator as a probe unit (for example, see Figure 17). In some embodiments, a full HCR initiator (I1 or I2) is generated by a pair of fractional initiator probes that each carry a fraction of the full HCR initiator such that together they comprise the full HCR initiator (fraction f1 for probe P1 and fraction f2 for probe P2 such that f1 + f2 = 1); in this case, a probe unit is two fractional initiator probes (for example, see Figure 17A). The fractions f1 and f2 are sufficiently small compared to the full HCR initiator (for example, f1 = 0.5 with f2 = 0.5; or f1 = 0.45 with f2 = 0.55; or f1 = 0.4 with f2 = 0.6) such that HCR signal amplification is suppressed if the full HCR initiator is not colocalized by the target.

In some embodiments, an HCR initiator (I1 or I2) is split between three fractional initiator probes (fraction f1 for probe P1, fraction f2 for probe P2, fraction f3 for probe 3 such that f1 + f2 + f3 = 1); in this case, a probe unit consists of three fractional initiator probes. In some embodiments, an HCR initiator (I1 or I2) is split between N fractional initiator probes (fraction f1 for probe P1, fraction f2 for probe P2, ..., fraction fN for probe PN such that f1 + f2 + ... fN = 1; for example, see Figure 17B) with N = 2, 3, 4, or more; in this case, a probe unit consists of N fractional initiator probes. For any of these values of N, HCR signal amplification is suppressed if the full HCR initiator is not colocalized by the target.

In some embodiments, a full HCR initiator is generated by a pair (or set) of probes that each carry a fraction of an HCR initiator such that the sum of the fraction f1 for probe P1 and the fraction f2 for probe P2 (f1 + f2) is sufficiently close to 1 (for example, f1 = 0.47, f2 = 0.47, f1+f2 = 0.94) such that HCR signal amplification is triggered by the colocalized full initiator that results from binding of the pair of probes to their adjacent cognate binding sites on the target. In some embodiments, the fractional initiator probes within a probe unit generate a full HCR initiator corresponding to 100% of an HCR initiator. In some embodiments, the fractional initiator probes within a probe unit generate a sufficient fraction of an HCR initiator to provide efficient HCR signal amplification relative to the rate of signal amplification when no fractional initiator probes are present or when individual fractional initiator probes are present but are not colocalized by the target. In some embodiments, the fraction of a full HCR initiator generated by colocalized probes within a probe unit is 99%, 95%, 90%, 80%, or 60%, including any range above any one of the preceding values or defined between any two of the preceding values of a full HCR initiator. In some embodiments, a probe unit comprises 2, 3, 4, 5 or more fractional initiator probes. In some embodiments, the fractional initiators in the probe unit are sufficient to be functional as an HCR initiator when the probes within the probe unit are colocalized by binding to their adjacent cognate binding sites on the target. In some embodiments, while an HCR initiator may have a sequence of a particular length (e.g., 15 nucleotides), the fractional initiators within a probe unit need not be the exact same length. For example, in some embodiments, their combined length could be 14 or 13 nucleotides, if, when colocalized, they still function as an HCR initiator.

In some embodiments, any two or more fractional initiators can be used, as long as, together, they provide the function of an HCR initiator.

In some embodiments, a full HCR initiator is generated by a pair of probes that each carry a fraction of an HCR initiator further comprising one or a few or several sequence modifications such that the sum of the fraction f1 for probe P1 and the fraction f2 for probe P2 (f1 + f2) is sufficiently close to 1 (for example, f1 = 0.45, f2 = 0.47, f1+f2 = 0.92) such that HCR signal amplification is triggered by the colocalized full initiator that results from binding of the pair of probes to their adjacent cognate binding sites on the target. In some embodiments, the fractional initiator probes within a probe unit generate a full HCR initiator that has 100% sequence identity with an HCR initiator. In some embodiments, the fractional initiator probes within a probe unit generate sufficient sequence identity to an HCR initiator to enable efficient HCR signal amplification relative to the rate of signal amplification when no fractional initiator probes are present or when individual fractional initiator probes are present but are not colocalized by the target. In some embodiments, the full HCR initiator generated by colocalized probes within a probe unit has 99%, 95%, 90%, 80%, or 60% sequence identity with an HCR initiator, including any range above any one of the preceding values or defined between any two of the preceding values.

**Use of large probe sets to enhance signal-to-background ratio.** Signal increases monotonically with the number of probe units so in some embodiments it is advantageous to use large probe sets comprising multiple probe units when target length permits (for example, mRNA, lncRNA, gDNA targets) in order to increase the amount of signal generated per target molecule. Automatic background suppression ensures that only probe units that bind specifically to the target trigger HCR signal amplification, so it is typically unnecessary to test individual probes within a probe set in order to remove those that bind non-specifically. As a result, automatic background suppression increases ease-of-use by eliminating the need for probe set optimization when using a new probe set for a new target. Because signal increases monotonically with probe set size and background is automatically suppressed for all probe pairs, it is typically advantageous to increase the probe set size in order to increase the signal-to-background ratio. A probe set comprises 1 or more probe units, each comprising two or more fractional initiator probes (for example, see Figure 8 and Figure 16). For example, the number of probe units in a probe set could be in the range 1 to 1000, or 10 to 100, or 20 to 50, or 30 to 40. Increasing the number of probe units in a probe set could increase the signal-to-background ratio by 2-fold, or 5-fold, or 10-fold, or 100-fold, or more. Increasing the number of probe units in a probe set could increase the signal-to-background ratio to be 2, or 5, or 10, or 50, or 100, or 200, or 500, or 1000 or more depending on the number of probe units in the probe set, the level of background inherent to the sample, and the abundance of the target within the sample.

**Probe set size constrained by transcriptomes and/or genomes within the sample.** In some situations, it is desirable to discriminate one target within a sample from one or more other targets in the sample that are closely related in sequence. For example: 1) a target mRNA within an organism might have a similar sequence to another mRNA within the organism, including the possibility that the target mRNA might be one splice variant that needs to be distinguished from other splice variants, 2) a target mRNA might contain multiple regions that each have sequence similarity to one or more other RNAs within the transcriptome of the organism, 3) a target mRNA contained in one organism might be present in a multi-species sample such that the target mRNA has regions that are similar in sequence to one or more RNAs within the transcriptome of another species, 4) a target rRNA or gDNA contained in one organism might be similar in sequence to rRNAs or gDNAs in other organisms contained in the sample. One way to discriminate between the target nucleic acid and all other nucleic acids present in the sample, is to design a probe set for the target nucleic acid that contains only probe pairs that are selective for the target nucleic acid relative to the transcriptomes and/or genomes present in the sample. In some situations, this requirement for selectivity can substantially constrain the size of the probe set. For example, the sequence of the target nucleic acid might be so similar to one or more other nucleic acids within the sample that the probe set might contain only a single fractional initiator probe pair.

**Cooperative target binding using probe sets comprising multiple fractional initiator probe pairs.** Probes that bind a target nucleic acid (for example, RNA or DNA) compete energetically with native secondary structure (i.e., base-pairing) within the target nucleic acid. For example, a single probe may be unable to bind a domain within a target nucleic acid that is predominantly base-paired to another domain within the target. The binding yield for a given probe is the fraction of target molecules with the probe bound at the cognate probe binding site, varying between 0 and 1 depending on the accessibility of that portion of target, the degree of affinity between the probe and its cognate probe binding site, and other factors. Using a probe set comprising multiple fractional initiator probe pairs, the binding of one probe to the target can improve the binding yield of one or more of the other probes to the target, leading to cooperative effects in which probes within the probe set collectively improve the binding yield of other probes within the probe set. Hence, compared to a probe set with N probes, a probe set with N+M probes can generate more signal not only because the M new probes will generate signal, but also because the N original probes will have higher binding yields to the target and thus generate more signal per target molecule on average.

**Increasing signal using helper probes.** In order to maintain selectivity for the target nucleic acid in the context of the one or more transcriptomes and/or genomes present in the sample, the probe set size is sometimes constrained to be no more than 1 probe unit, or no more than 2 probe units, or no more than N probe units, where N is less than the number of probe units, N+M, that would be preferentially used based on sensitivity considerations and/or on the length of the target nucleic acid. In this scenario where the probe set size is constrained by selectivity considerations, the amount of signal generated can be less relative to detection of the same target using N+M probe units both because the probe set has the potential for generating M fewer full initiators for triggering HCR signal amplification and because the absence of the M additional probe units can reduce the binding yield of the N remaining probe units due to cooperative effects.

In some embodiments, probes carry fractional initiators as signal probes (for example, see Figure 16A). In some embodiments, probes do not carry fractional initiators as helper probes. In order to increase signal without decreasing selectivity, a probe set comprising N probe units can be augmented with M helper probes (for example, see Figure 16D).

For example: 1) to detect a target RNA with a unique splice junction, a probe set could comprise one probe unit that binds to the target spanning the splice junction, and further comprise 30 (or any number of) helper probes that bind elsewhere to the target RNA to cooperatively improve the binding yield of the probe unit and thus increase signal without decreasing selectivity; 2) to detect a target RNA or DNA for which selectivity considerations lead to a probe set comprising 5 probe units, the probe set can further comprise 45 helper probes; 3) to detect a target RNA or DNA for which selectivity considerations lead to a probe set comprising N probe units (with N less than 40), the probe set can further comprise 40-N helper probes; 4) to detect a target nucleic acid for which selectivity considerations lead to a probe set comprising N probe units, and the length of the target nucleic acid restricts the total number of probes to 2N+M, the probe set can further comprise M helper probes.

In some embodiments, helper probes will be designed for a target nucleic acid with less stringent selectivity requirements than those used to design signal probes for the same target nucleic acid. In some embodiments, helper probes will be used for a target nucleic acid even though they are equally selective for other off-target nucleic acids within the transcriptomes and/or genomes present in the sample. In some embodiments, the target-binding region on signal probes will be the same length as the target-binding region on helper probes. In some embodiments, the target-binding region on signal probes will be shorter or longer than the target binding region on helper probes. In some embodiments, the target binding region may vary across a range of lengths (number of nucleotides) for different signal and/or helper probes. In some embodiments, the affinity between signal probes and the target nucleic acid will be comparable to the affinity between helper probes and the target nucleic acid. In some embodiments, the affinity between signal probes and the target nucleic acid will be lower or higher than the affinity between helper probes and the target nucleic acid.

**Increasing signal using signal probes that carry one or two fractional initiators.** In some embodiments, a probe unit comprises two fractional initiator probes that together generate a full HCR initiator (fraction f1 for probe P1 and fraction f2 for probe P2 such that f1 + f2 = 1). In this situation, each probe participates in one probe unit. If both probes within the probe unit bind to a given target RNA molecule, a single full HCR initiator is generated, triggering growth of one HCR amplification polymer tethered to the probe unit. Consider a situation where the length of the target RNA constrains the probe set size to be N probe units corresponding to 2N signal probes. In the case where each signal probe participates in only one probe unit, the maximum number of full HCR initiators would be N, corresponding to the case where the target mRNA was bound by all of the signal probes in the probe set. Hence, the maximum number of HCR polymers tethered to a target RNA would be N. In order to increase the signal per target molecule, now consider a case where a probe can carry two fractional initiators that contribute to two different probe units (for example, see Figure 16C). In some embodiments, the following three possibilities are contemplated:
1. In an embodiment, where all 2N signal probes hybridize to adjacent subsequences along the target RNA, the signal probes at the 5' and 3' ends of the target each have one neighboring signal probe and all other intervening signal probes have two neighboring signal probes. In this scenario, if each probe except for the 5'-most signal probe and the 3'-most signal probe carries two fractional initiators that contribute to two different probe units, the total number of probe units increases from N to (2N - 1). As a result, the maximum number of full HCR initiators increases from N to (2N - 1) and the maximum number of HCR polymers tethered to the target RNA increases from N to (2N - 1). Hence, the signal generated per target molecule is increased relative to the case where each signal probe carries a single fractional initiator.
2. In another embodiment, selectivity considerations resulting from the transcriptomes and/or genomes present in the sample may lead to a probe set comprising 2N signal probes and N probe units where no probe unit is proximal to another along the target nucleic acid. In this scenario, each signal probe carries only a single fractional initiator and contributes to only one probe unit so the maximum number of full HCR initiators is N and the maximum number of tethered HCR amplification polymers is N.
3. In another embodiment, representing an intermediate case, some signal probes will be proximal to more than one neighbor so the 2N signal probes will participate in a number of probe units that is intermediate between N and 2N - 1. For example, if there are 2N signal probes and M of them tile one portion of the target (with the 5' and 3' of these signal probes carrying one fractional initiator and contributing to one probe unit and the other M-2 signal probes carrying two fractional initiators and contributing to two probe units) and the other L probes (with 2N = M+L with L even) each bind to the target in pairs with each signal probe proximal to only one other signal probe (each of L signal probes carrying one fractional initiator and contributing to one probe unit), then the total number of probe units will be M-1 + L/2 which will be intermediate between N and 2N - 1. For example, if there are 40 signal probes (i.e., N = 20) and 20 of them tile the target proximally (i.e., M = 20) and the other 20 bind to the target in proximal pairs (i.e., L = 20), the total number of probe units will be M-1 + L/2 = 20 - 1 + 20/2 = 29.

In the related case where a probe unit comprises more than two signal probes, for example three signal probes, or four or more signal probes, the number of probe units can again be increased without increasing the number of signal probes, by using some probes that comprise two fractional initiators and participate in two probe units (one fractional initiator per probe unit).

**HCR amplifiers with 2 hairpins.** In some embodiments, an HCR amplifier comprises two hairpins (H1 and H2; for example, see Figures 8 and 18). In some embodiments, each hairpin comprises an input domain with a single-stranded toehold and a stem section, and an output domain with a single-stranded loop and a complement to the stem section. In the absence of an HCR initiator (I1 or I2), hairpins H1 and H2 coexist metastably, that is, they are kinetically trapped and do not polymerize.

Initiation with full initiator I1. In some embodiments, an initiator I1 comprises a domain complementary to the toehold of hairpin H1 and a domain complementary to the stem section of H1. If an H1 hairpin encounters a full initiator I1, the full initiator I1 hybridizes to the input domain of hairpin H1 via toehold-mediated strand displacement, opening hairpin H1 to expose the output domain of hairpin H1 and form complex I1-H1. The output domain of hairpin H1 comprises a domain complementary to the toehold of hairpin H2 and a domain complementary to the stem section of H2. If an H2 hairpin encounters an I1-H1 complex, the exposed output domain of H1 hybridizes to the input domain of hairpin H2 via toehold-mediated strand displacement, opening hairpin H2 to expose the output domain of hairpin H2 and form complex I1-H1-H2. The output domain of hairpin H2 comprises a domain complementary to the toehold of hairpin H1 and a domain complementary to the stem section of H1. If an H1 hairpin encounters an I1-H1-H2 complex, the exposed output domain of H2 hybridizes to the input domain of hairpin H1 via toehold-mediated strand displacement, opening hairpin H1 to expose the output domain of hairpin H1 and form complex I1-H1-H2-H1. This polymerization process can repeat with alternating H1 and H2 polymerization steps to generate polymers of the form I1-H1-H2-H1-H2-H1-H2-..., which we may denote I1-(H1-H2)_{N} for a polymer that incorporates N alternating copies of hairpins H1 and H2. For example, a polymer might incorporate several H1 and H2 molecules, or dozens of H1 and H2 molecules, or hundreds of H1 and H2 molecules, or thousands of H1 and H2 molecules, or tens of thousands of H1 and H2 molecules, or more. It is also possible for a polymer to end with either H1 or H2, so I1-(H1-H2)_{N}-H1 and I1-(H1-H2)_{N}-H1-H2 are both possible, the latter being equivalent to I1-(H1-H2)_{N+1}.

**Initiation with full initiator I2.** In some embodiments, an initiator I2 comprises a domain complementary to the toehold of hairpin H2 and a domain complementary to the stem section of H2. If an H2 hairpin encounters a full initiator I2, the full initiator I2 hybridizes to the input domain of hairpin H2 via toehold-mediated strand displacement, opening hairpin H2 to expose the output domain of hairpin H2 and form complex I2-H2. If an H1 hairpin encounters an I2-H2 complex, the exposed output domain of H2 hybridizes to the input domain of hairpin H1 via toehold-mediated strand displacement, opening hairpin H1 to expose the output domain of hairpin H1 and form complex I2-H2-H1. If an H2 hairpin encounters an I2-H2-H1 complex, the exposed output domain of H1 hybridizes to the input domain of hairpin H2 via toehold-mediated strand displacement, opening hairpin H2 to expose the output domain of hairpin H2 and form complex I2-H2-H1-H2. This polymerization process can repeat with alternating H2 and H1 polymerization steps to generate polymers of the form I2-H2-H1-H2-H1-H2-H1..., which can be denoted I2-(H2-H1)_{N} for a polymer that incorporates N alternating copies of H2 and H1. For example, a polymer might incorporate several H1 and H2 molecules, or dozens of H1 and H2 molecules, or hundreds of H1 and H2 molecules, or thousands of H1 and H2 molecules, or tens of thousands of H1 and H2 molecules, or more. It is also possible for a polymer to end with either H1 or H2, so I2-(H2-H1)_{N}-H2 and I2-(H2-H1)_{N}-H2-H1 are both possible, the latter being equivalent to I2-(H2-H1)_{N+1}.

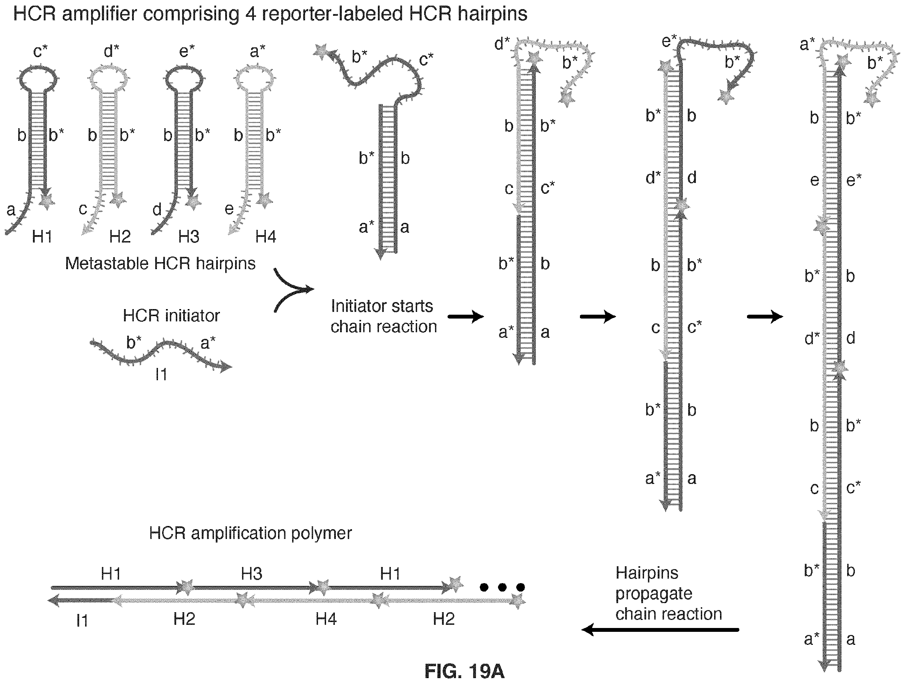

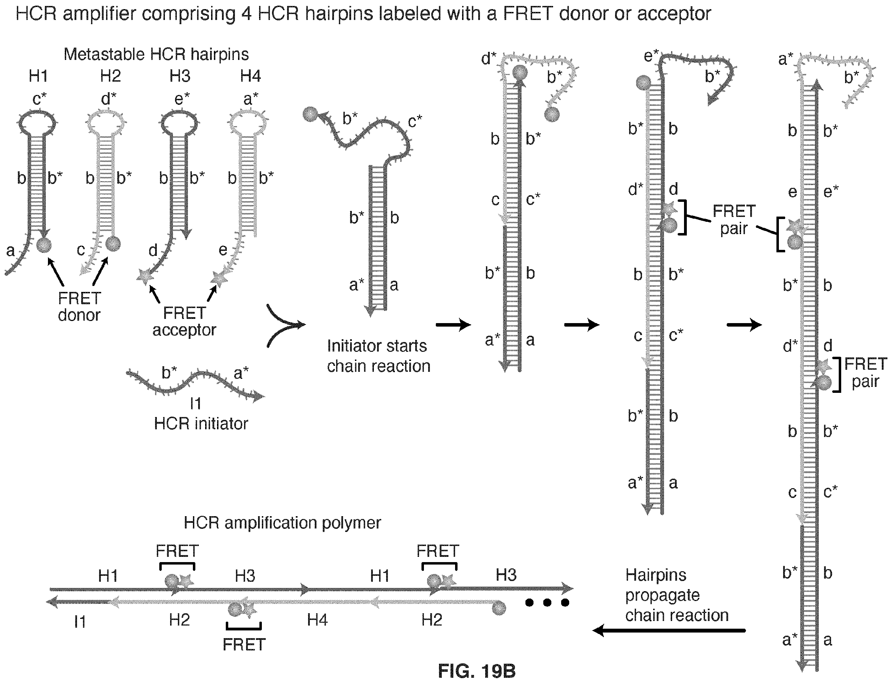

**HCR amplifiers with 4 hairpins.** In some embodiments, an HCR amplifier can comprise more than 2 hairpins. For example, an HCR amplifier might comprise 4 hairpins H1, H2, H3, H4 (for example, see Figure 19). Just as for 2-hairpin HCR, each hairpin comprises an input domain comprising a single-stranded toehold and a stem section, and an output domain comprising a single-stranded loop and a complement to the stem section. In the absence of an HCR initiator (I1, I2, I3, or I4), hairpins H1, H2, H3, H4 coexist metastably, that is, they are kinetically trapped and do not polymerize. The output domain of hairpin H1 comprises a domain complementary to the toehold of hairpin H2 and a domain complementary to the stem section of H2. The output domain of hairpin H2 comprises a domain complementary to the toehold of hairpin H3 and a domain complementary to the stem section of H3. The output domain of hairpin H3 comprises a domain complementary to the toehold of hairpin H4 and a domain complementary to the stem section of H4. The output domain of hairpin H4 comprises a domain complementary to the toehold of hairpin H1 and a domain complementary to the stem section of H1. Initiator I1 comprises a domain complementary to the toehold of hairpin H1 and a domain complementary to the stem section of H1. Initiator I2 comprises a domain complementary to the toehold of hairpin H2 and a domain complementary to the stem section of H2. Initiator I3 comprises a domain complementary to the toehold of hairpin H3 and a domain complementary to the stem section of H3. Initiator I4 comprises a domain complementary to the toehold of hairpin H4 and a domain complementary to the stem section of H4. Analogous to the case of 2-hairpin HCR, if a hairpin H1 encounters a full HCR initiator I1, the full initiator I1 opens hairpin H1 to form complex I1-H1 with an exposed H1 output domain, which in turn opens hairpin H2 to form complex I1-H1-H2 with an exposed H2 output domain, which in turn opens hairpin H3 with an exposed output domain to form complex I1-H1-H2-H3 with an exposed H3 output domain, which in turn opens hairpin H4 to form complex I1-H1-H2-H3-H4 with an exposed H4 output domain, which in turn opens hairpin H1 to form complex 11-H1-H2-H3-H4-H1 with an exposed H1 output domain, and so forth, leading to polymerization via alternating H1, H2, H3, and H4 polymerization steps to generate polymers of the form I1-H1-H2-H3-H4-H1-H2-H3-H4-H1-H2-H3-H4..., which can be denoted I1-(H1-H2-H3-H4)_{N} for a polymer that incorporates N alternating copies of H1, H2, H3, and H4. It is possible for a polymer to end with H1, H2, H3, or H4, so I1-(H1-H2-H3-H4)_{N}-H1_{,} I1-(H1-H2-H3-H4)_{N}-H1-H2_{,} I1-(H1-H2-H3-H4)_{N}-H1-H2-H3, and I1-(H1-H2-H3-H4)_{N}-H1-H2-H3-H4 are all possible, the latter being equivalent to I1-(H1-H2-H3-H4)_{N+1}. It is possible for HCR polymerization to be triggered by any of the cognate full initiators (I1, I2, I3, or I4). For example, initiator by full initiator I3 could generate polymers of the form I3-(H3-H4-H1-H2)_{N}. HCR amplifiers with 4 hairpins are convenient for generating a signal that is absent in the monomer state and present in the polymer state (for example, Figure 19B illustrates FRET pairs that are colocalized to generate a FRET signal only when hairpins are colocalized within an amplification polymer, providing a basis for wash-free methods since unused hairpins that are not washed from the sample will not participate in FRET, and hence will avoid generating background).

**HCR amplifiers with 2 or more hairpins.** More generally, in some embodiments, an HCR amplifier may comprise M HCR hairpins (H1, H2, ..., HM) with M an integer of 2 or more. In the absence of an HCR initiator (I1, I2, ..., IM), hairpins H1, H2, ..., HM coexist metastably, that is, they are kinetically trapped and do not polymerize. In the presence of a cognate full HCR initiator, polymerization occurs via alternating polymerization steps analogous to 2-hairpin or 4-hairpin HCR. For example, initiator I1 would lead to growth of polymers of the form I1-(H1-H2-...-HM)_{N} for a polymer that incorporates N alternating copies of H1, H2,. ..,HM. It is possible for a polymer to end with any of H1,H2,...,HM, so I1-(H1-H2-...-HM)_{N}-H1_{,} I1-(H1-H2-...-HM)_{N}-H1-H2_{,} ..., and I1-(H1-H2-...-HM)_{N}-H1-H2-...-HM are all possible, the latter being equivalent to I1-(H1-H2-...-HM)_{N+1}. It is possible for HCR polymerization to be triggered by any of the cognate full initiators (I1, I2, ..., IM). For example, initiator by full initiator I3 could generate polymers of the form I3-(H3-...-HM-H1-H2)_{N}.

**HCR hairpin labels.** For a given HCR amplifier, each HCR hairpin comprises zero, one, or more labels. Labels on different hairpins within an amplifier may be the same or different. For example, an amplifier comprising hairpins H1 and H2 might have: 1) the same label on H1 and H2, 2) different labels on H1 and H2, 3) a label on H1 but no label on H2, 4) a label on H2 but no label on H1, 5) no label on H1 or H2, 6) zero, one, or more labels on H1 of which zero, one, or more of them are the same or different as zero, one, or more labels on H2. Similarly, for an HCR amplifier comprising hairpins H1, H2, H3, H4, each hairpin may comprise zero, one, or more labels (for example 3, 5, or 10 labels) of which zero, one, or more of them may be the same as zero, one, or more labels on each of the other hairpins. In some embodiments, one or more of the labels for a given hairpin can be unique within a mixture of hairpins and/or hairpin labels. In some embodiments, there are 1, 10, 100, 1000, 10,000, 100,000 or more unique labels within a mixture (including any range defined between any two of the previous numbers).

**HCR hairpin labels as reporters.** In some embodiments, an HCR hairpin label may comprise a reporter molecule that facilitates measurement of a signal, for example by generating a signal, by altering a signal, or by eliminating a signal. For example, a reporter could be a fluorophore, a chromophore, a luminophore, a phosphor, a FRET pair, a member of a FRET pair, a quencher, a fluorophore/quencher pair, a rare-earth element or compound, a radioactive molecule, a magnetic molecule, or any other molecule that facilitates measurement of a signal.

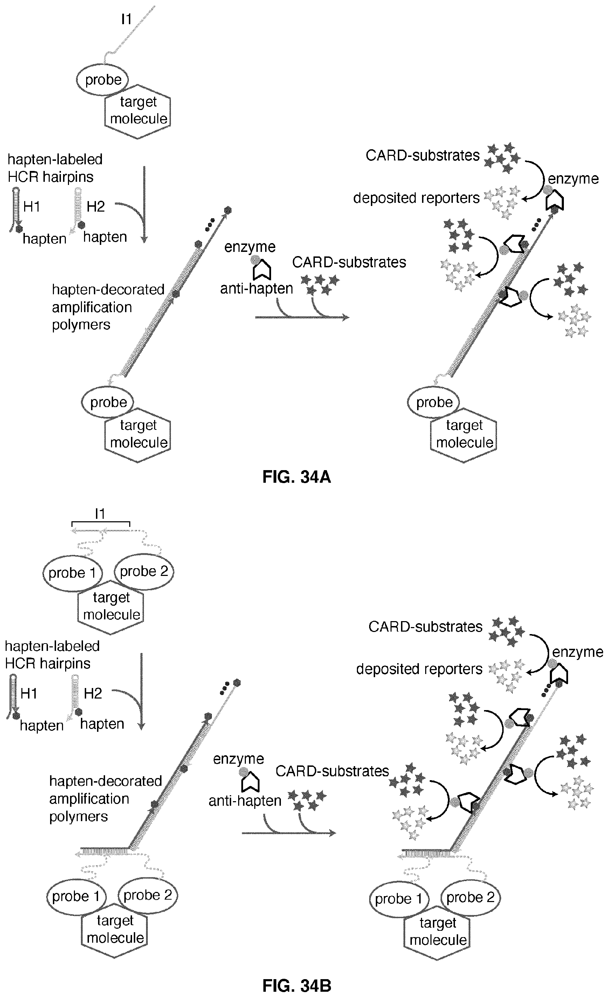

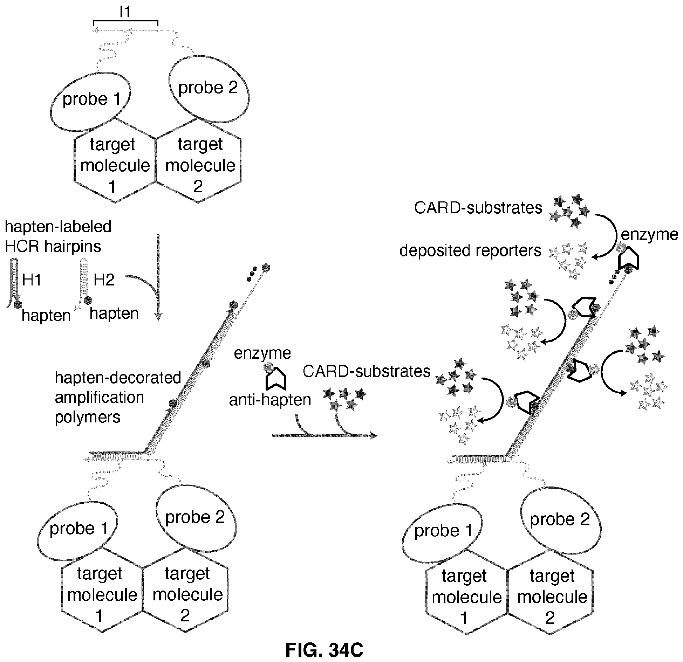

**HCR hairpin labels as substrates.** In some embodiments, an HCR hairpin label may comprise a substrate that serves to recruit a reporter entity that directly or indirectly mediates localization of reporters in the vicinity of the hairpin label. For example:
1. the hairpin label can comprise digoxigenin (DIG) that recruits anti-DIG antibody as the reporter entity, where the anti-DIG is directly labeled with one or more reporters, or with one or more substrates or reporter entities that serve to directly or indirectly mediate localization of reporters in the vicinity of the hairpin label.
2. the hairpin label can comprise a nucleic acid domain that serves as a substrate with full or partial sequence complementarity to a domain within a label probe that carries one or more reporters (for example FIG. 18C),
3. the hairpin label can comprise a nucleic acid domain that serves as a substrate with full or partial sequence complementarity to a domain within a label probe that carries one or more substrates that serve to mediate localization of reporters in the vicinity of the hairpin label.
4. the hairpin label can comprise a nucleic acid domain that serves as a substrate for a reporter entity that directly or indirectly mediates localization of reporters in the vicinity of the hairpin label.
5. the hairpin label can comprise a substrate that serves to recruit a reporter entity that indirectly mediates localization of reporters in the vicinity of the hairpin label.
6. the hairpin label can comprise a substrate that serves to recruit a reporter entity that comprises an enzyme that mediates catalytic reporter deposition (CARD) in the vicinity of the hairpin label (for example FIG. 36A).
7. the hairpin label can comprise biotin that recruits streptavidin as the reporter entity, where the streptavidin is directly labeled with one or more reporters, or with one or more substrates or reporter entities that serve to directly or indirectly mediate localization of reporters in the vicinity of the hairpin label.
8. the hairpin label can comprise a hapten that recruits an anti-hapten antibody or anti-hapten nanobody that directly or indirectly mediates localization of reporters in the vicinity of the hairpin label via CARD signal amplification. For example, the anti-hapten antibody or nanobody may comprise a reporter entity that is an enzyme that mediates CARD (for example, FIG. 33A-33E).
9. the hairpin label can comprise a hapten that recruits an anti-hapten that directly or indirectly mediates localization of reporters in the vicinity of the hairpin label. For example, the anti-hapten may comprise a reporter entity that is an enzyme that mediates CARD (for example, FIG. 34A-34C).
10. the hairpin label can comprise an enzyme that mediates CARD signal amplification to deposit reporter molecules in the vicinity of the hairpin.
11. the hairpin label can comprise zero, one, or more haptens (for example FIG. 35) that mediate, directly or indirectly, localization of reporters in the vicinity of haptens.

In some embodiments, a hairpin label can comprise a hapten that recruits an anti-hapten (for example, an antibody, a nanobody, streptavidin, or another molecule) that is labeled with reporters.

In some embodiments provided herein, HCR signal amplification is used to mediate catalytic reporter deposition (CARD), leading to even higher signal gain. In some embodiments, the even higher single gain is about 5, 10, 15, 20, 25, 30, 40, 50, 75, 100, 500, 1000, 2000, 5000, or 10,000-fold, or a value with a range defined by any two of the aforementioned values.

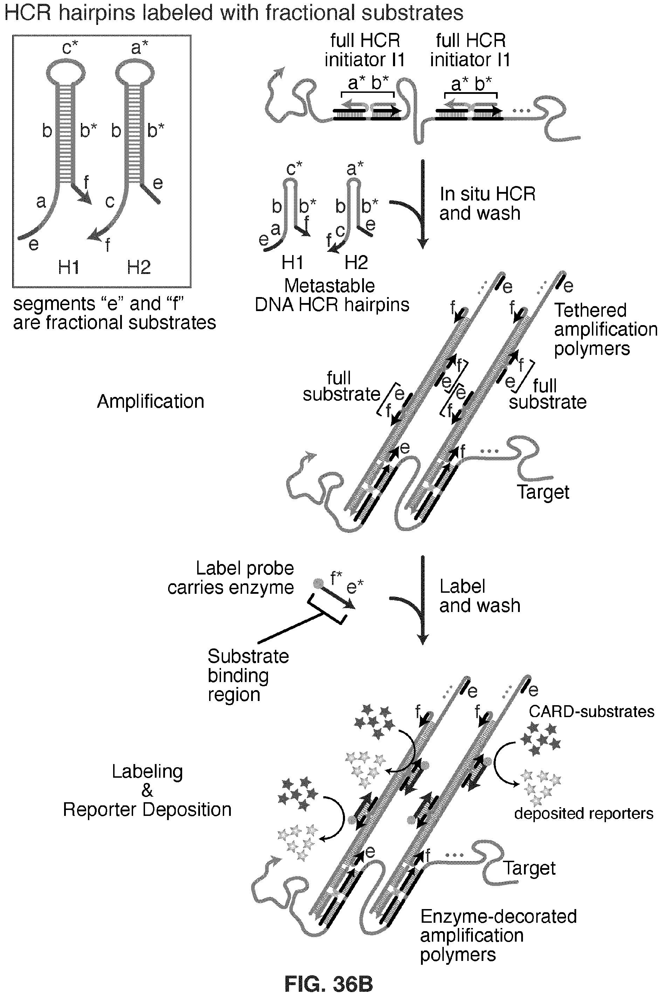

In some embodiments, an HCR hairpin label may comprise a fractional substrate such that a label probe conjugated to a reporter molecule does not strongly bind the fractional substrate on an individual hairpin, but such that following HCR polymerization, neighboring hairpins in the HCR amplification polymer colocalize a full substrate such that the colocalized full substrate strongly binds a label probe conjugated to a reporter molecule (for example FIG. 18D), or to a reporter entity comprising an enzyme that mediates CARD signal amplification (for example FIG. 36B).

**Haptens and anti-haptens.** In some embodiments, hairpin labels that are substrates comprising a hapten could for example be digoxygenin (DIG), dinitrophenyl (DNP), a fluorophore, biotin, or any small molecule, biological molecule, or non-biological molecule that can recruit an anti-hapten. Examples of anti-haptens include antibodies, nanobodies, streptavidin, aptamers, or any other molecule or complex of molecules that selectively binds a hapten.

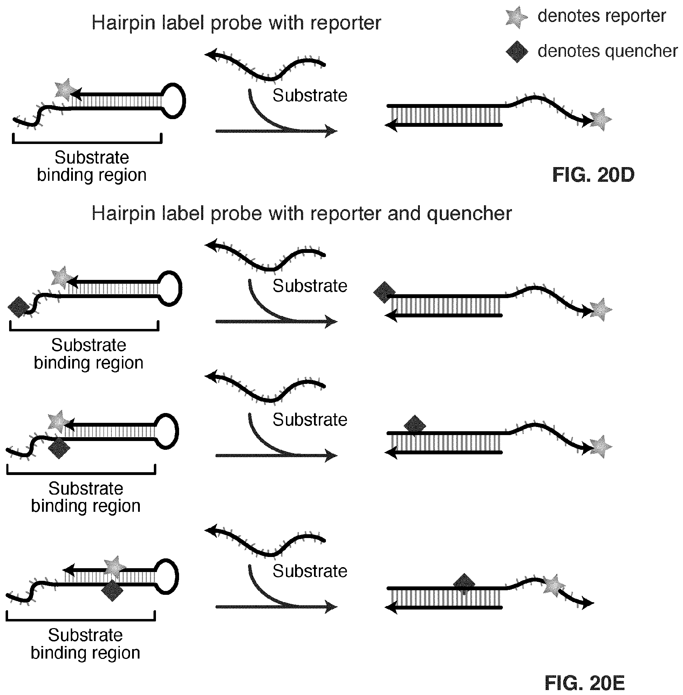

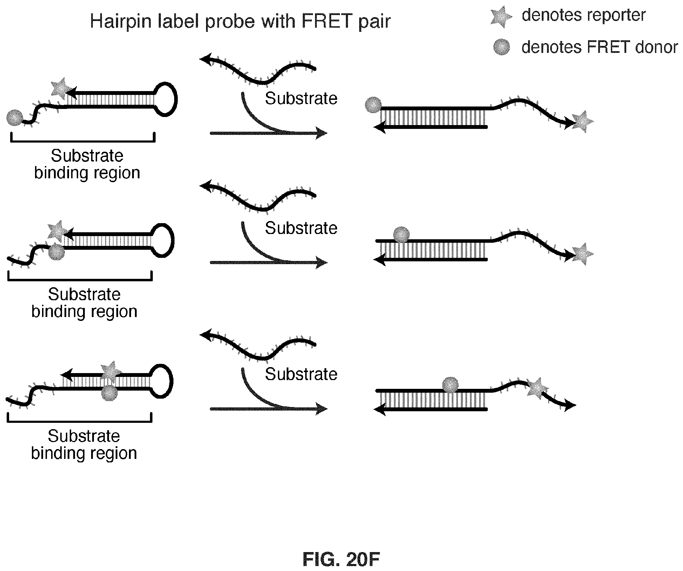

**Label probes.** In some embodiments, a label probe comprises a substrate-binding region and one or more reporters (for example, see FIGs. 18C-E and FIG. 20) or reporter entities (for example, see FIGs. 33A-33E and 34A-34D). In some embodiments, a label probe comprises an unstructured strand labeled with one reporter molecule (for example, see FIG. 20A). In some embodiments, a label probe comprises multiple reporter molecules (for example, see FIG. 20B). In some embodiments, a label probe comprises a label strand (conjugated to a reporter) hybridized to a blocker strand (conjugated to a quencher) (for example, see FIG. 20C). In some embodiments, a label probe has a hairpin structure (for example, see the label probe of FIG. 20D) or other intramolecular base-pairing. In some embodiments, a hairpin label probe is conjugated to one or more reporters, quenchers, and/or FRET pairs (for example, see FIGs. 20E and 20F). In some embodiments, a label probe comprises an anti-hapten antibody or nanobody that recognizes a hapten label on the HCR hairpin and further comprises a reporter entity comprising an enzyme that mediates CARD (for example, FIGs. 33A-33E). In some embodiments, a label probe comprises an anti-hapten entity that recognizes a hapten label on the HCR hairpin and futher comprises a reporter entity comprising an enzyme that mediates CARD (for example, FIGs. 34A-34D). In some embodiments, a label probe comprises a substrate complement that recognizes a substrate label on the HCR hairpin, or that recognizes a full substrate colocalized within an HCR amplification polymer, and further comprises a reporter entity comprising an enzyme that mediates CARD

### (for example, FIGs. 36A-36B)

**Enzymes for HCR-mediated Catalytic Reporter Deposition (CARD).** In some embodiments, HCR hairpins mediate signal amplification via catalytic reporter deposition (CARD) by a reporter entity comprising an enzyme that catalyzes a CARD-substrate leading to deposition of reporters in the vicinity of the hairpin (see for example FIGs. 33A-33E, 34A-34C, 36A-36B). For example:
1. the enzyme could be horseradish peroxidase (HRP) (or polymer HRP comprising multiple HRP enzymes) that acts on a CARD-substrate to catalyze deposition a chromogenic reporter such as AEC, DAB, TMB, or StayYellow, or that catalyzes a CARD-substrate to catalyze deposition of a fluorescent reporter such as fluophore-labeled tyramide, or that catalyzes deposition of a hapten-labeled CARD-substrate such as biotin-labeled tyramide, where the hapten serves to mediate localization of reporters in the vicinity of the hairpin label.
2. the enzyme could be alkaline phosphatase (AP) (or polymer AP comprising multiple AP enzymes) that acts on a CARD-substrate to catalyze deposition of reporters, for example a chromogenic reporter such as but not limited to BCIP/NBT, BCIP/TNBT, Napthol AS-MX phosphate + FastBlue BB, Napthol AS-MX phosphate + FastRed TR, StayGreen.
3. the enzyme could be glucose oxidase that acts on a CARD-substrate to catalyze deposition of reporters, for example NBT,
4. the enzyme could be any molecule or complex that directly or indirectly mediates localization of reporters in the vicinity of a hairpin label.

In some embodiments, the enzyme that mediates CARD is deactivated (aka inactivated) after reporter deposition (for example, using chemical or heat denaturation). For example, the enzyme that mediates CARD can be deactivated using any combination of:
1. Heat (for example, 65 °C or above)
2. Fixative (for example, 4% PFA)
3. Acid (for example, 0.1 M glycine-HCl with 1% Tween 20 at pH 2.2, 0.2N HCl, 10% acetic acid, 10 mM HCl)
4. Other chemicals (for example, hydrogen peroxide (H₂O₂), hydrogen peroxide + phenol, sodium azide, DEPC, MAB with 10 mM EDTA)

In some embodiments, HRP is inactivated using H₂O₂. In some embodiments, AP is inactivated using a combination of heat and acid. In some embodiments, AP is inactivated with fixative. In some embodiments, deactivation of the enzyme that mediates CARD enables repeated CARD using the same enzyme in combination with different substrates for different targets to enable multiplexed target analysis using HCR-mediated CARD. In some embodiments, deactivation of the enzyme that mediates CARD enables repeated CARD using different enzymes in combination with different substrates for different targets to enable multiplexed target analysis using HCR-mediated CARD.

In some embodiments, CARD enables storage of stained samples for 10 or more years to enable reimaging in compliance with regulatory requirements for biopharma. In some embodiments, the CARD based stained sample is adequately stable for 1, 2, 3, 4, 5, 6, 7, 8, 9, 10, 11, 12, 13, 14, 15 or more years, and still in compliance with regulatory requirements for biopharma. In some embodiments, CARD provides for storage of formalin fixed paraffin embedded (FFPE) samples for decades. In some embodiments, CARD provides for storage of pathology samples for decades to provide for retrospective scientific and medical studies. In some embodiments, CARD staining provides for long-term storage of archival samples.

**Target types.** In some embodiments, an initiator-labeled HCR probe comprises one or more target-binding regions and further comprises one or more HCR initiators. In some embodiments, an initiator-labeled probe can detect a target comprising:
1. any molecule including but not limited to an RNA molecule (for example, mRNA, rRNA, lncRNA, siRNA, shRNA, microRNA, non-coding RNA, synthetic RNA, or modified RNA), a DNA molecule, a non-natural nucleic acid molecule, a protein molecule, a small molecule, a biological molecule, a chemically modified biological molecule, a non-biological molecule
2. any complex of molecules comprising any combination of RNA, DNA, protein, small molecules, biological molecules, and/or non-biological molecules (for example, an RNA/RNA complex, an RNA/protein complex, a DNA/protein complex, and RNA/DNA/protein complex, a protein/protein complex).

A fractional initiator HCR probe comprises one or more target-binding regions and further comprises one or more fractional initiator regions. A probe unit comprises two or more fractional initiator probes such that the fractional initiator probes in the probe unit combine to create a full HCR initiator. A probe unit can detect a target comprising:
1. any molecule including but not limited to an RNA molecule (for example, mRNA, rRNA, lncRNA, siRNA, shRNA, microRNA, non-coding RNA, synthetic RNA, or modified RNA), a DNA molecule, a non-natural nucleic acid molecule, a protein molecule, a small molecule, a biological molecule, a chemically modified biological molecule, a non-biological molecule,
2. any complex of molecules comprising any combination of RNA, DNA, protein, small molecules, biological molecules, and/or non-biological molecules (for example, an RNA/RNA complex, an RNA/protein complex, a DNA/protein complex, and RNA/DNA/protein complex, a protein/protein complex)
3. any collection of proximal molecules or complexes such that the fractional initiators in the probe unit can colocalize to form a full HCR initiator when the fractional initiator probes comprising the probe unit are bound to their respective targets within the collection of proximal molecules or complexes.

In any of the embodiments provided herein, the fractional initiators within a probe unit are designed to be (or are) complementary to non-overlapping regions of an HCR hairpin (for example, regions separated by 0, 1, 2, or more nucleotides), or are designed to be (or are) complementary to overlapping regions of an HCR hairpin (for example, regions that overlap by 1, 2 or more nucleotides), or are designed to be (or are) substantially complementary to an HCR hairpin (for example, complementary except for 0, 1, 2, a few, or several mismatches).

In any of the embodiments provided herein, the target-binding regions within a probe unit are configured to bind to non-overlapping regions of the target (for example, regions separated by 0, 1, 2, or more nucleotides or regions separated by 0, 1, 2, or more nm), or are configured to bind to overlapping regions of the target (for example, regions that overlap by 1, 2 or more nucleotides, or regions that overlap by 1, 2, or more nm).

**Signal probes configured to bind to overlapping or non-overlapping regions of the target and/or designed to have fractional initiators that hybridize to overlapping or non-overlapping regions of an HCR hairpin.** In some embodiments, a probe unit comprises two or more fractional initiator probes each comprising a target-binding region and a fractional initiator. In some embodiments, the fractional initiators within a probe unit:
1. are designed to be complementary to adjacent regions of an HCR hairpin,
2. or are designed to be complementary to non-overlapping regions of an HCR hairpin (for example, regions separated by 0, 1, 2, or more nucleotides),
3. or are designed to be complementary to overlapping regions of an HCR hairpin (for example, regions that overlap by 1, 2 or more nucleotides),
4. or are designed to be substantially complementary to an HCR hairpin (for example, complementary except for 0, 1, 2, a few, or several mismatches)
5. or are designed to hybridize to adjacent regions of an HCR hairpin,
6. or are designed to hybridize to non-overlapping regions of an HCR hairpin,
7. or are designed to hybridize to overlapping regions of an HCR hairpin,
8. or are designed to have sequences that are complementary to adjacent regions of an HCR hairpin,
9. or are designed to have sequences that are complementary to non-overlapping regions of an HCR hairpin,
10. or are designed to have sequences that are complementary to overlapping regions of an HCR hairpin,
11. or are designed to have sequences that are substantially complementary to adjacent regions of an HCR hairpin,
12. or are designed to have sequences that are substantially complementary to non-overlapping regions of an HCR hairpin,
13. or are designed to have sequences that are substantially complementary to overlapping regions of an HCR hairpin,

In some embodiments, the target-binding regions within a probe unit:
1. are configured to bind to adjacent regions of the target,
2. or are configured to bind to non-overlapping regions of the target (for example, regions separated by 0, 1, 2, or more nucleotides, or regions separated by 0, 1, 2, or more nm),
3. or are configured to bind to overlapping regions of the target (for example, regions overlapping by 1, 2, or more nucleotides, or regions overlapping by 1, 2, or more nm),
4. are designed to bind to adjacent regions of the target,
5. or are designed to bind to non-overlapping regions of the target,
6. or are designed to bind to overlapping regions of the target,
7. or are designed to have sequences that hybridize to adjacent regions of the target,
8. or are designed to have sequences that hybridize to non-overlapping regions of the target,
9. or are designed to have sequences that hybridize to overlapping regions of the target,

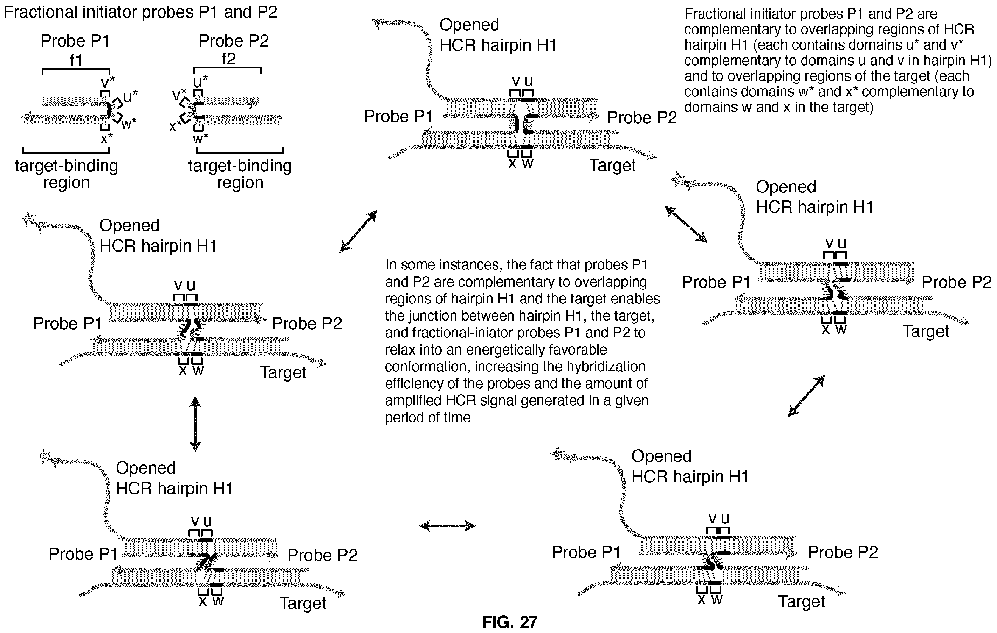

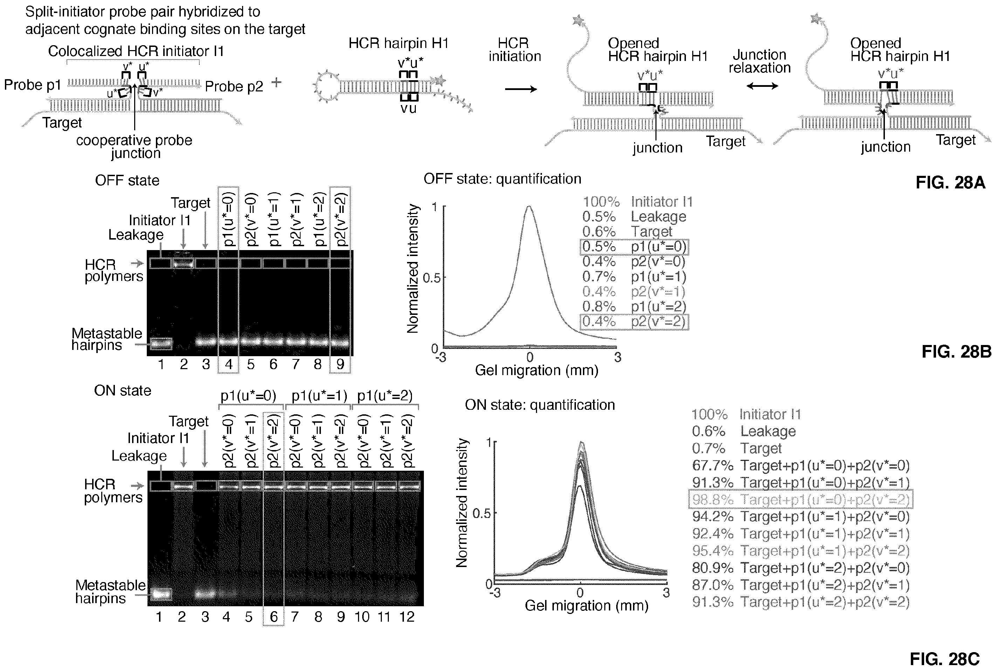

In some embodiments, a probe unit comprises two fractional initiator probes. The two fractional initiator probes bind to the cognate target to colocalize a full HCR initiator. The colocalized full HCR initiator then binds to the cognate HCR hairpin to initiate HCR polymerization, with one fractional initiator hybridizing to the hairpin to form a first duplex and the other fractional initiator hybridizing to the hairpin to form a second duplex. In some embodiments, there is an energetically unfavorable junction between the two duplexes. In some embodiments, by configuring the fractional initiators to bind to overlapping regions of the hairpin, the location of the junction along the hairpin and the tertiary structure of the two probes and the hairpin in the vicinity of the junction can relax into an energetically more favorable conformation, increasing the affinity between the colocalized full initiator and the HCR hairpin, increasing the amount of amplified HCR signal generated in a given period of time (for example, FIGs. 27 and 28). In some embodiments the affinity between the two probes and the cognate target can be increased by configuring the target-binding regions of the two probes to bind to overlapping regions of the target so as to permit the junction between the molecules to relax to an energetically favorable conformation.

In some embodiments, probe sets are designed for multiplexed experiments in which 2, 3, 4, 5, 10, 20, or 100 or more probe sets are used to bind to different targets in the same sample, where 1, 2, 3, 4, 5, 10, 20, or 100 or more of the probe sets comprise one or more initiator-labeled probes. In some embodiments, probe sets are designed for multiplexed experiments in which 2, 3, 4, 5, 10, 20, or 100 or more probe sets are used in the same sample, where more than 1%, more than 2%, more than 5%, more than 10%, more than 30%, more than 50%, or 100% of the probe sets comprise one or more initiator-labeled probes.

In some embodiments, probe sets are designed for multiplexed experiments in which 2, 3, 4, 5, 10, 20, or 100 or more probe sets are used to bind to different targets in the same sample, where 1, 2, 3, 4, 5, 10, 20, or 100 or more of the probe sets comprise one or more probe units comprising fractional initiators that are designed to hybridize to overlapping regions of an HCR hairpin. In some embodiments, probe sets are designed for multiplexed experiments in which 2, 3, 4, 5, 10, 20, or 100 or more probe sets are used in the same sample, where more than 1%, more than 2%, more than 5%, more than 10%, more than 30%, more than 50%, or 100% of the probe sets comprise one or more probe units comprising fractional initiators with sequences that are designed to be complementary to overlapping regions of an HCR hairpin.

In some embodiments, probe sets are designed for multiplexed experiments in which 2, 3, 4, 5, 10, 20, or 100 or more probe sets are used to bind to different targets in the same sample, where 1, 2, 3, 4, 5, 10, 20, or 100 or more of the probe sets comprise one or more probe units comprising target-binding regions that are designed to bind to overlapping regions of a target. In some embodiments, probe sets are designed for multiplexed experiments in which, 3, 4, 5, 10, 20, or 100 or more probe sets are used in the same sample, where more than 1%, more than 2%, more than 5%, more than 10%, more than 30%, more than 50%, or 100% of the probe sets comprise one or more probe units comprising target-binding regions that are designed to bind to overlapping regions of a target.

In some embodiments, probe sets are designed for multiplexed experiments in which 2, 3, 4, 5, 10, 20, or 100 or more probe sets are used to bind to different targets in the same sample, where 1 or more of the probe sets comprise one or more initiator-labeled probes and 1 or more of the probe sets comprise one or more probe units each comprising two or more fractional initiator probes. In some embodiments, probe sets are designed for multiplexed experiments in which 2, 3, 4, 5, 10, 20, or 100 or more probe sets are used in the same sample, where more than 0.1%, more than 1%, more than 2%, more than 5%, more than 10%, more than 30%, or more than 50% of the probe sets comprise one or more initiator-labeled probes, and where more than 0.1%, more than 1%, more than 2%, more than 5%, more than 10%, more than 30%, or more than 50% of the probe sets comprise one or more probe units each comprising two or more fractional initiator probes.

In some embodiments, a probe unit comprises fractional initiators that are designed to bind to overlapping regions of an HCR hairpin, where the overlapping regions overlap by 1 base, or 2 bases, or 3 bases, or 4 bases, or 5 bases, or more bases.

In some embodiments, a probe unit comprises target-binding regions that are designed to bind to overlapping regions of the target, where the overlapping regions overlap by at least 0.1 nm, or at least 0.2 nm, or at least .3 nm, or at least 0.5 nm, or at least 1 nm, or at least 2 nm, or at least 3 nm, or at least 5 nm. In some embodiments, a probe unit comprises target-binding regions comprising sequences that are designed to bind to overlapping regions of the target, where the overlapping regions overlap by at least 1 base, or 2 bases, or 3 bases, or 4 bases, or 5 bases, or more bases.

**Materials and compositions of initiator-labeled probes.** In some embodiments, an initiator-labeled probe comprises one or more target-binding domains and one or more HCR initiators (for example, FIGs. 39A-39N and 42A-42F). Each domain may comprise one or more materials including DNA, RNA, 2'OMe-RNA, PNA, XNA, chemically modified nucleic acids, synthetic nucleic acid analogs, amino acids, synthetic amino acid analogs, and/or any other molecule suited for the purpose of the domain. For example:
1. an initiator-labeled probe may comprise one or more initiators made of DNA and a target-binding domain made of DNA.
2. an initiator-labeled probe may comprise one or more initiators made of DNA and a target-binding domain made of amino acids (for example, an antibody or a nanobody or an antibody fragment).
3. an initiator-labeled probe may comprise an initiator made of a synthetic nucleic acid analog and a target-binding domain made of a combination of DNA and 2'OMe-RNA.
4. an initiator-labeled probe may comprise an initiator made of 2'OMe-RNA and a target-binding domain made of a combination of RNA and protein.
5. an initiator-labeled probe may comprise an initiator made of DNA and a target-binding domain made of PNA.
6. an initiator-labeled probe may comprise one or more initiators made of any nucleic acid or nucleic acid analog and one or more target-binding domains made of any combination of materials suitable for binding the target molecule.

In some embodiments, an initiator-labeled probe may comprise a single covalently linked molecule or may comprise two or more molecules (each covalently linked) that interact non-covalently to form a complex. For example:
1. an initiator-labeled probe may comprise an initiator made of DNA that is covalently linked to a target-binding domain made of DNA.
2. an initiator-labeled probe may comprise one or more initiators made of DNA that are covalently linked to dCas9 (or another Cas) which is non-covalently bound to a guide RNA (gRNA) such that the target-binding domain comprises the gRNA:dCas9 complex (or gRNA:Cas complex using another Cas).
3. an initiator-labeled probe may comprise one or more initiators made of DNA that are covalently linked to a gRNA that is non-covalently bound to dCas9 (or another Cas) such that the target-binding domain comprises the gRNA:dCas9 complex (or gRNA:Cas complex using another Cas).
4. an initiator-labeled probe may comprise an initiator made of a nucleic acid or nucleic acid analog that is covalently linked or non-covalently bound to a target-binding domain comprising one or more molecules.

**Materials and composition of fractional initiator probes.** In some embodiments, a fractional initiator probe comprises one or more target-binding domains and one or more fractional initiator domains (for example, FIGs. 3A-3B, 5A-5E, and 38). Each domain may comprise one or more materials including DNA, RNA, 2'OMe-RNA, PNA, XNA, chemically modified nucleic acids, synthetic nucleic acid analogs, amino acids, synthetic amino acid analogs, and/or any other molecule suited for the purpose of the domain. For example:
1. a fractional initiator probe may comprise one or more fractional initiator domains made of DNA and a target-binding domain made of DNA.
2. a fractional initiator probe may comprise one or more fractional initiator domains made of DNA and a target-binding domain made of amino acids (for example, an antibody or a nanobody or an antibody fragment).
3. a fractional initiator probe may comprise a fractional initiator domain made of a synthetic nucleic acid analog and a target-binding domain made of a combination of DNA and 2'OMe-RNA.
4. a fractional initiator probe may comprise a fractional initiator domain made of 2'OMe-RNA and a target-binding domain made of a combination of RNA and protein.
5. a fractional initiator probe may comprise a fractional initiator domain made of DNA and a target-binding domain made of PNA.
6. a fractional initiator probe may comprise one or more fractional initiator domains made of any nucleic acid or nucleic acid analog and one or more target-binding domains made of any combination of materials suitable for binding the target molecule.

In some embodiments, a fractional initiator probe may comprise a single covalently linked molecule or may comprise two or more molecules (each covalently linked) that interact non-covalently to form a complex. For example:
1. a fractional initiator probe may comprise a fractional initiator domain made of DNA that is covalently linked to a target-binding domain made of DNA.
2. a fractional initiator probe may comprise one or more fractional initiator domains made of DNA that are covalently linked to dCas9 (or another Cas) which is non-covalently bound to a guide RNA (gRNA) such that the target-binding domain comprises the gRNA:dCas9 complex (or gRNA:Cas complex using another Cas).
3. a fractional initiator probe may comprise one or more fractional initiator domains made of DNA that are covalently linked to a gRNA that is non-covalently bound to dCas9 (or another Cas) such that the target-binding domain comprises the gRNA:dCas9 complex (or gRNA:Cas complex using another Cas).
4. a fractional initiator probe may comprise a fractional initiator domain made of a nucleic acid or nucleic acid analog that is covalently linked or non-covalently bound to a target-binding domain comprising one or more molecules. Each fractional initiator probe within a probe unit may have the same or different material compositions from the other fractional initiator probes in the probe unit. Each fractional initiator probe within a probe unit may have target-binding regions that bind to different detection sites on the same target molecule, or to different detection sites within a target molecular complex, or to different detection sites within a target collection of proximal molecules or complexes.

**Wash-free signal generation using fractional initiator probes and HCR signal amplification.** In some embodiments, fractional initiator probes and HCR amplifiers are used to generate signal using a wash-free protocol where unused probes and amplifiers are not removed from the sample. In some embodiments, the two or more fractional initiator probes within a probe unit each comprise a fractional initiator and a target-binding region such that individual fractional initiator probes are not sufficient to efficiently trigger HCR signal amplification, but such that upon binding of the target-binding domain of each probe to the cognate detection site on the target, the fractional initiators are colocalized to generate a full HCR initiator and trigger growth of a tethered HCR amplification polymer. In some embodiments, each HCR hairpin is labeled with one or more reporters, and/or one or more quenchers, and/or one or more components of a FRET pair. In some embodiments, the HCR amplifier comprises two HCR hairpins H1 and H2 that undergo a polymerization cascade of alternating H1 and H2 polymerization steps to grow a tethered HCR amplification polymer. In some embodiments, the HCR amplifier comprises four HCR hairpins H1, H2, H3, H4 that undergo a polymerization cascade of alternating H1, H2, H3, and H4 polymerization steps to grow a tethered HCR amplification polymer. In some embodiments, one or more reporters on an HCR hairpin are in a quenched state prior to polymerization and in an unquenched state after polymerization. In some embodiments, two reporters that comprise a FRET pair are not sufficiently close to perform efficient FRET prior to HCR polymerization but are sufficiently close to perform efficient FRET after polymerization. In some embodiments, HCR signal generation is conformation-dependent such that the total HCR signal in the sample is lower before HCR polymerization than after HCR polymerization due to: a) the conformational change that HCR monomers undergo when they open to join an HCR polymer, and/or b) the colocalization of two or more HCR hairpins within an HCR polymer. In some embodiments, HCR signal is concentrated at the site of targets due to the growth of tethered HCR amplification polymers. In some embodiments, fractional initiator probes and HCR amplifiers are used to generate signal using a wash-free protocol where unused probes and amplifiers are not removed from the sample. In some embodiments, fractional initiator probes and HCR amplifiers are used to generate signal using a wash-free protocol for a sample comprising one or more targets within any of: living cells, living organisms, tissue sections, brain slices, a bulk solution, fixed cells, fixed tissue, fixed embryos. In some embodiments, the targets are not crosslinked, and/or are not fixed within the sample, and/or are not captured to a solid support. In some embodiments, HCR hairpins comprise labels that are fractional substrates such that HCR amplification colocalize full substrates (for example see Figure 18F). In some embodiments, label probes comprise a label strand (conjugated to a reporter) hybridized to a blocker strand (conjugated to a quencher) such that hybridization of a label strand to a full colocalized within an HCR amplification polymer displaces the blocker strand from the label strand, separating the reporter from the quencher and generating a signal.

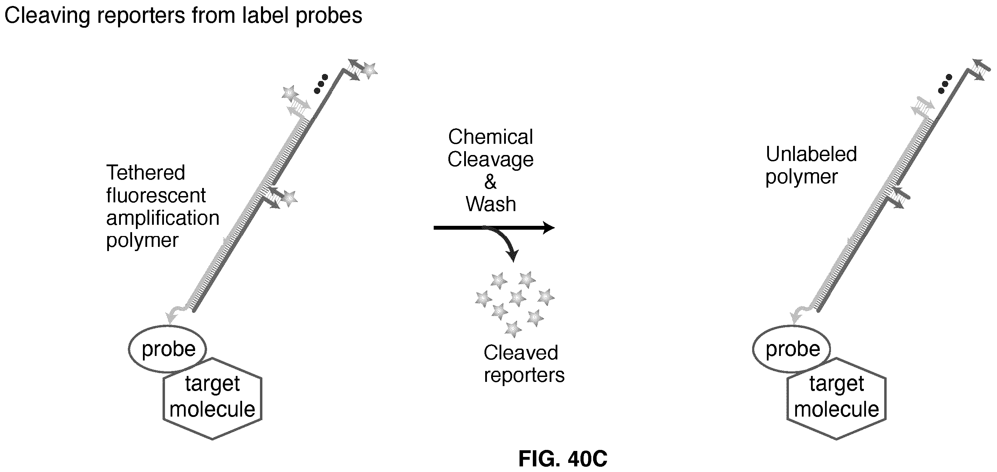

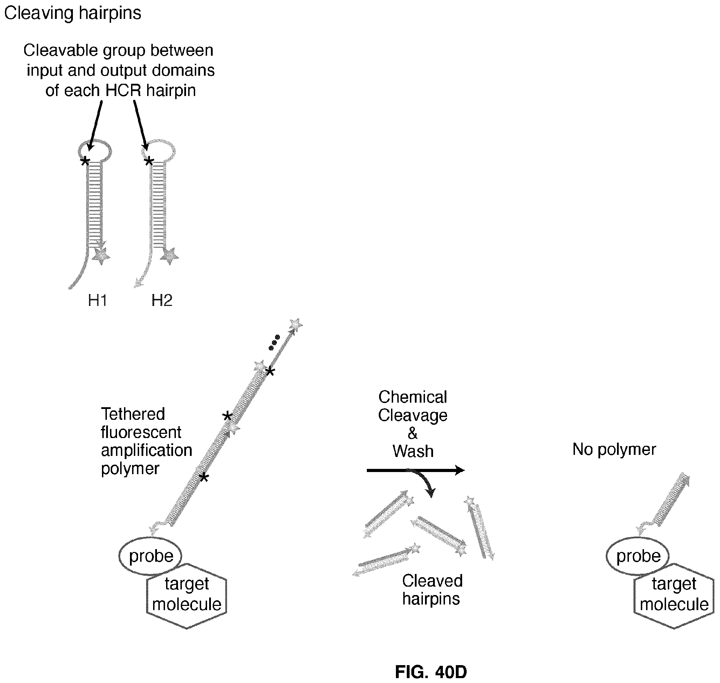

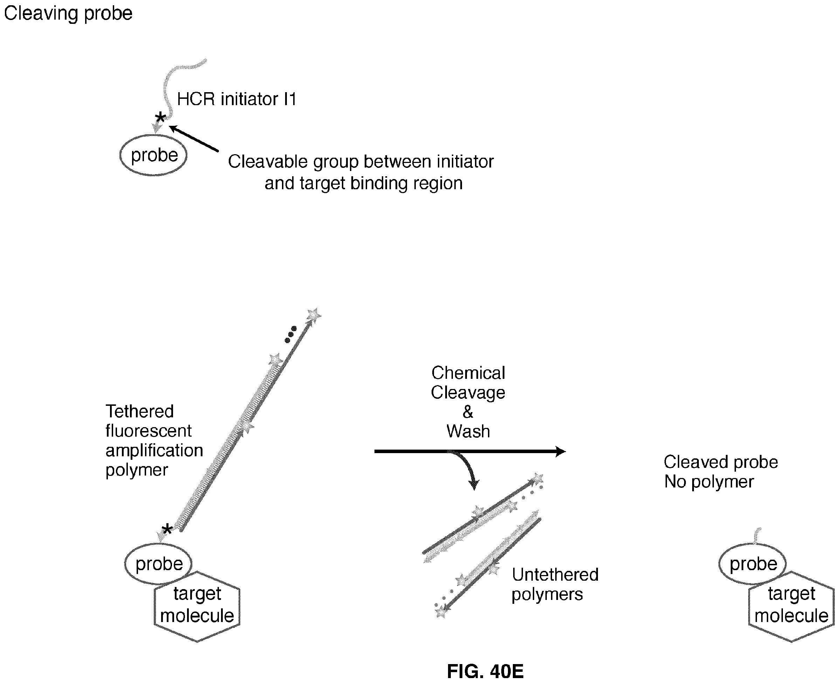

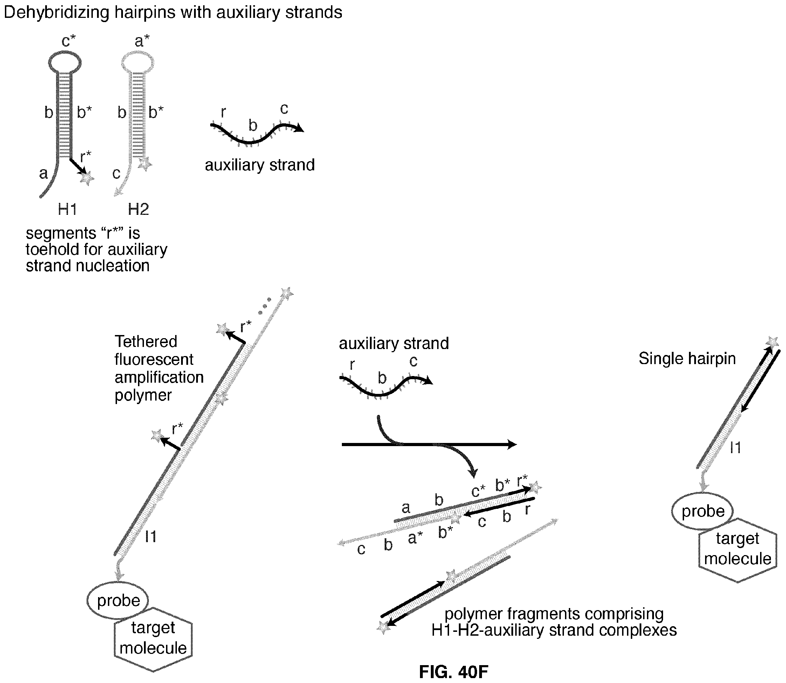

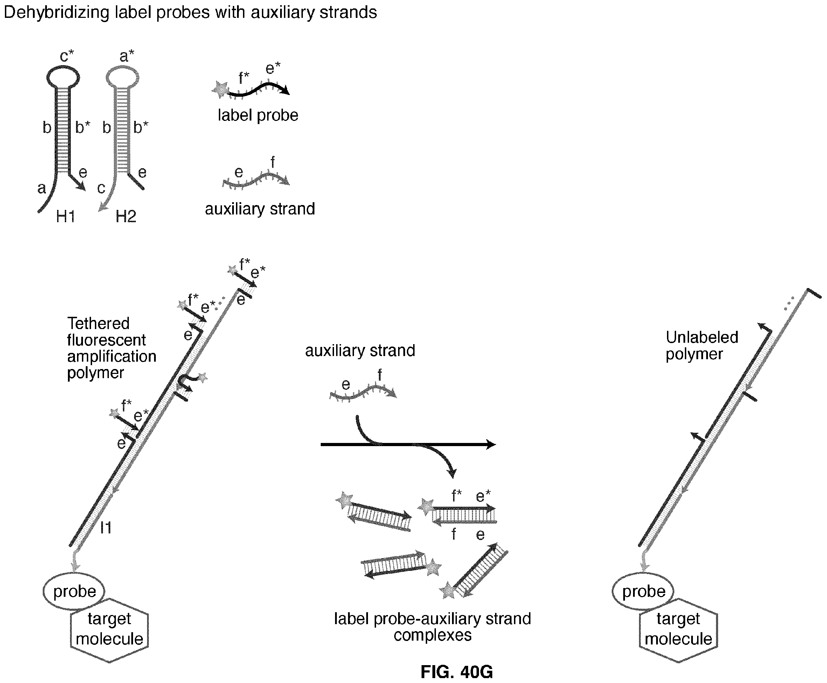

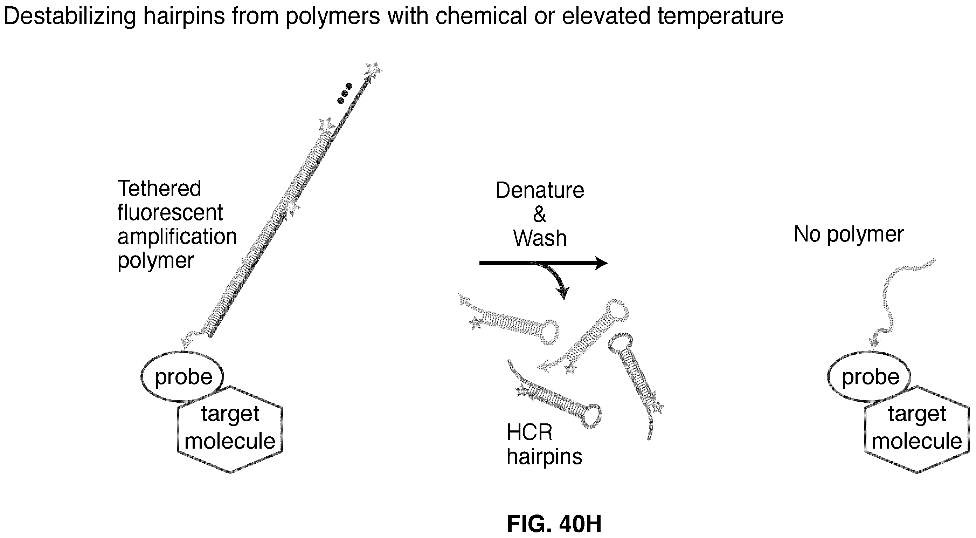

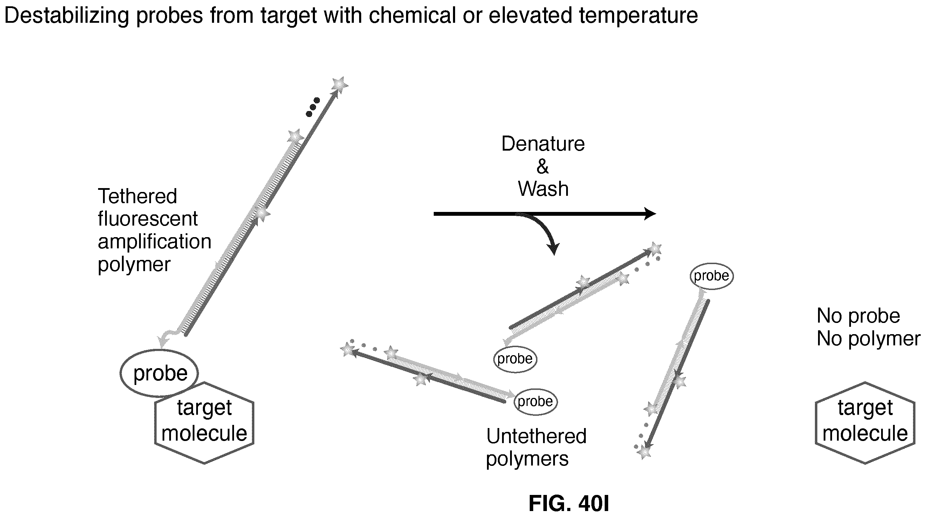

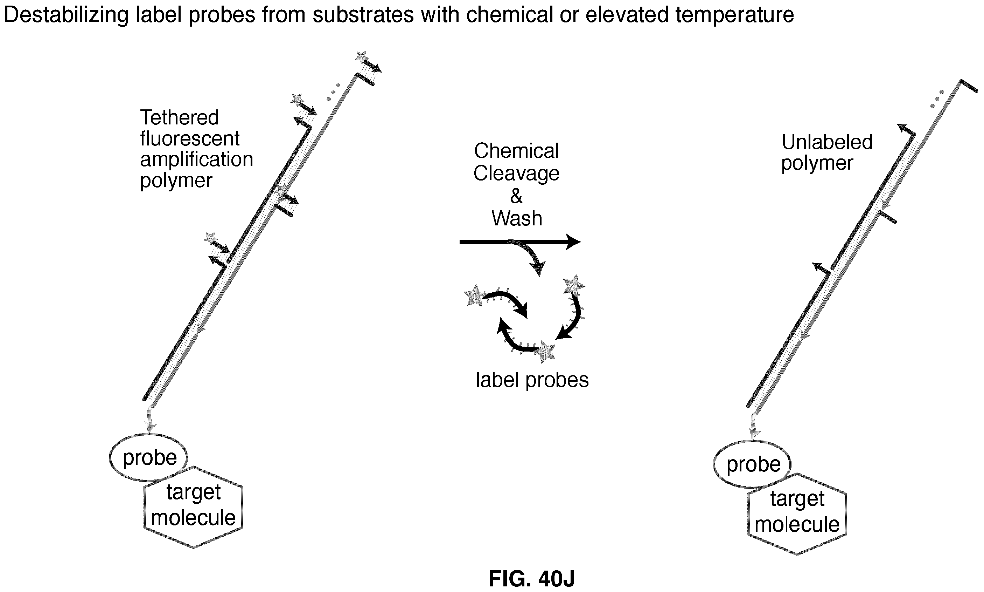

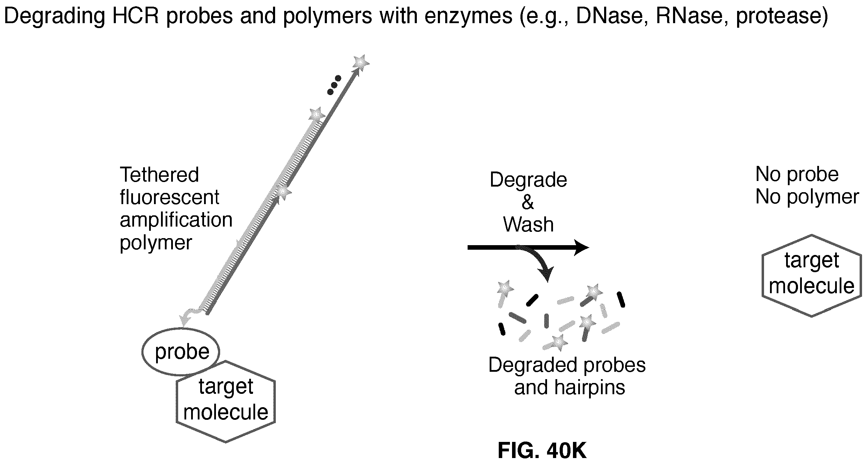

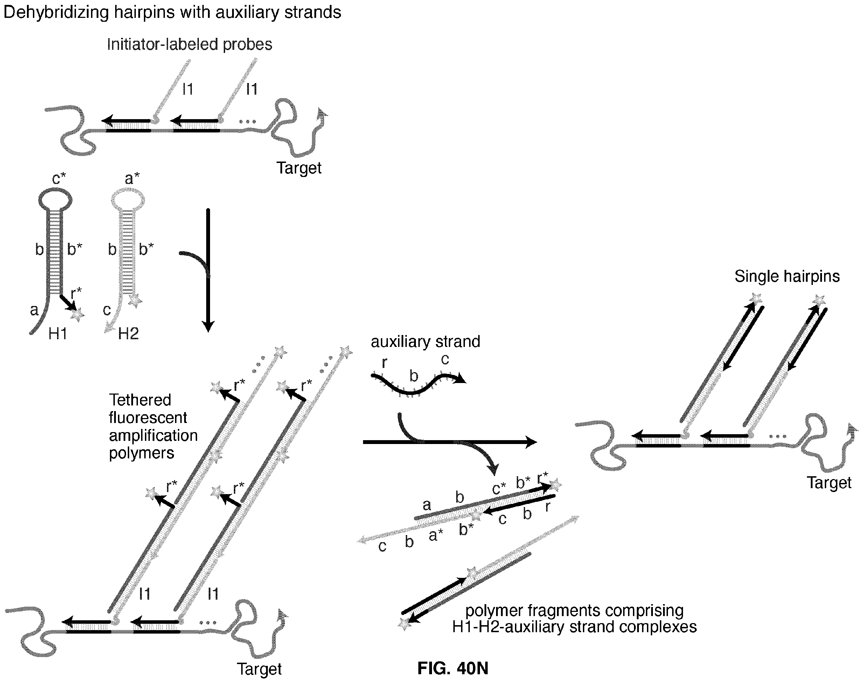

**Removal of signal from the sample.** In some embodiments, HCR signal is removed from the sample after detecting the signal (for example, see FIG. 23A-23N and FIG. 40A-40N). Signal can be removed from the sample by any method that reduces the number of signal-generating reporters in the sample. For example:
- photobleaching fluorescent reporter molecules using light and/or chemical reagents (for example, see FIGs. 23A and 40A),
- chemically cleaving reporters from HCR hairpins and washing them from the sample (e.g., TCEP) (for example, see FIGs. 23B and 40B),
- chemically cleaving reporters from label probes and washing them from the sample (for example, see FIGs. 23C and 40C),
- chemically cleaving hairpins to fragment HCR amplification polymers and washing the fragments from the sample (for example, see FIGs. 23D and 40D),
- chemically cleaving probes to untether HCR amplification polymers from the target and washing the untethered amplification polymers from the sample (for example, see FIGs. 23E and 40E),
- using an auxiliary strand to dehybridize hairpins from HCR amplification polymers and washing the hairpins from the sample (for example, see FIGs. 23F, 40F and 40N),
- using an auxiliary strand to dehybridize label probes from HCR amplification polymers and washing the label probes from the sample (for example, see FIGs. 23G and 40G),
- using chemical denaturants and/or elevated temperature to destabilize HCR amplification polymers and then washing the hairpins from the sample (for example, see FIGs. 23H and 40H),
- using chemical denaturants and/or elevated temperature to destabilize the interaction between probes and their targets and then washing the untethered amplification polymers from the sample (for example, see FIGs. 23I and 40I),
- using chemical denaturants and/or elevated temperature to destabilize the interaction between label probes and their substrates and then washing the label probes from the sample (for example, see FIGs. 23J and 40J),
- using enzymes to degrade amplification polymers and/or probes and washing the degraded molecules from the sample (for example, see FIGs. 23K and 40K-40M),
- using DNases to degrade DNA amplification polymers and/or DNA probes and/or DNA targets and washing the resulting molecules from the sample (for example, see FIGs. 23K, 40K-40M),
- using RNases to degrade RNA targets and then washing the untethered amplification polymers from the sample (for example, see FIGs. 23L and 40K-40M),
- using proteases to degrade protein targets and then washing the untethered amplification polymers from the sample (for example, see FIGs. 23M and 40K-40M)
- using a combination of RNases to degrade RNA targets and DNases to degrade DNA amplification polymers and/or DNA probes and washing the resulting molecules from the sample (for example, see FIGs. 23N and 40K-40M)
- using a combination of proteases to degrade protein targets and DNases to degrade DNA amplification polymers and/or DNA probes and/or DNA targets and washing the resulting molecules from the sample (for example, see FIGs. 23O and 40K-40M),
- using two or more of the above methods at the same time or at different times.

**Assay formats.** In some embodiments, an HCR signal can be measured in different assay formats including but not limited to: blots, northern blots, western blots, Southern blots, spot blots, paper assays, flow cytometry assays, fluorescent flow cytometry assays, cell sorting assays, fluorescence-activated cell sorting assays, magnetic-activated cell sorting assays, microscopy assays, light microscopy assays, epifluorescence microscopy assays, confocal microscopy assays, light sheet microscopy assays, microarray assays, bead-based assays, mass spectrometry assays, fluorescent microscopy assays, mass spectrometry microscopy assays, mass spectrometry flow cytometry assays, fluorescence assays, chemiluminescence assays, bioluminescence assays, colorimetric assays, electrochemical impedance assays, electrochemical chemiluminescence assays, energy dissipation assays, assays using the human eye, assays using a cell phone camera, gel electrophoresis assays, in situ hybridization (ISH) assays, RNA-ISH assays, DNA-ISH assays, immunohistochemistry (IHC) assays, autoradiography assays, or any assay capable of detecting a signal generated by an HCR amplification polymer.

**Sample types.** In some embodiments, an HCR initiator-labeled probes and/or HCR fractional initiator probes can be used with HCR amplification hairpins to detect a target in a sample, the target comprising a molecule, a complex, or a collection of proximal molecules or complexes. The target molecule may be contained within a sample, including for example: a bacterium, a zebrafish embryo, a chicken embryo, a mouse embryo, a human biopsy specimen, a human tissue section, an FFPE tissue section, a urine sample, a blood sample, a stool sample, a mouse tissue section, a brain slice, a sea urchin embryo, a nematode larva, a fruit fly embryo, a model organism, a non-model organism, a multi-species mixture of organisms, an environmental sample containing unknown organisms, a consortium of organisms (for example, a mixture of protists and bacteria within the gut of another organism), a termite, a microbiome, a clinical specimen, a diagnostic sample, a sputum sample, a tumor biopsy sample, a research sample, a sample comprising material from a human, a sample comprising material from a pet (for example, a dog, cat, rabbit, lizard, snake, or fish), material from a wild animal (for example, a cheetah, elephant, rhinoceros, or chimpanzee), material from an extinct animal (for example, a woolly mammoth, a dodo, a giant auk, a triceratops, or a passenger pigeon), living cells (for example, bacteria or cultured mammalian cells), or a living organism (for example a living mouse or a living human).

In some embodiments, the target may be free in solution within the sample. For example, the target may be free in solution within: a test tube, a cell, an embryo, an organism, a tissue section, a biological specimen, or other sample.

In some embodiments, the target may be covalently crosslinked or non-covalently bound to one or more capture probes covalently or non-covalently attached to a solid support. For example, bound directly or indirectly to a capture probe covalently linked to a microarray or bead.

In some embodiments, the target may be fixed, covalently crosslinked, or non-covalently bound directly or indirectly to a solid support. For example, the target may be bound, fixed, or covalently cross-linked to a slide, a blot, a membrane, a paper substrate, or any other substrate. The target may be fixed or covalently crosslinked to a cell, embryo, organism, tissue section, biological specimen, or any other sample. The target may be covalently linked within a sample that is fixed and permeabilized, fixed but not permeabilized, or not fixed but permeabilized.

In some embodiments, the target may be free within a living cell, living embryo, living organism, living ecosystem, or consortium of organisms (for example, the microbiome within the gut of a mammal). The target may be associated with but exterior to a cell or organism, or it may be contained within a cell or organism. The target may be covalently crosslinked within a living cell, living embryo, living organism, living ecosystem, or living consortium of organisms. The target may be present within or absent from one or more cell types within the sample. The target may be present within or absent from one or more species of organism within the sample. The target may be present in a sample that contains one or more off-targets that have different degrees of similarity to the target molecule. The target may be present within an expanded sample. The target may be present within a compressed sample. The sample may be expanded prior to detecting the target so as to increase the spatial separation between molecules. The sample may be compressed prior to detecting the target so as to decrease the spatial separation between molecules. The target and/or other molecules may be crosslinked to an expanded sample so as to maintain the relative position between molecules in the sample as the sample expands. The target and/or other molecules may be crosslinked to a gel, matrix, or other reagents introduced to the sample so as to expand the sample while maintaining the relative position and/or orientation of molecules in the sample as the sample expands. The sample may be differentially expanded and/or compressed with different expansion and/or compression factors in different tissues and/or organs within the sample.

**Fixing the sample.** In some embodiments, an target molecules can be crosslinked to the sample so that they are retained during subsequent steps in an experiment. For example, target molecules can be crosslinked to the sample using chemical reagents (for example, formaldehyde, paraformaldehyde, EDC (1-ethyl-3-(3-dimethylaminopropyl) carbodiimide)).

**Permeabilizing the sample.** In some embodiments, the sample can be treated to enhance the accessibility of target molecules to HCR probes and amplifiers. For example, the sample (for example, cells, tissue sections, or whole-mount embryos) can be permeabilized using chemical reagents (for example, methanol, ethanol, detergent) or enzymes (for example, proteinase K). Target accessibility can also be enhanced via sample homogenization, microdissection, electroporation, sectioning, heat treatment (for example, Smith JJ, Gunasekera TS, Barardi CR, Veal D, Vesey G (2004) J Appl Microbiol 96(2):409-417), and/or microwave treatment (for example, Lan HY, Mu W, NG YY, Nikolic-Paterson DH, & Atkins RC (1996) J Histochem Cytochem 44(3):281-287). Another option is to deliver HCR probes and amplifiers across the cell membrane using chemical transfection reagents.

**Sample washes to remove unbound reagents from the sample.** In some embodiments, background can be reduced by washing unused imaging reagents from the sample. For example, washes can be used to remove probes, HCR initiator-labeled probes, HCR fractional initiator probes, HCR amplification hairpins, amplification reagents, label probes, antibodies, and/or other imaging reagents from the sample. Washes can be performed at a temperature using chemical reagents such that imaging reagents that are bound specifically are predominantly not removed (retaining signal) and imaging reagents that are bound non-specifically are predominantly removed (reducing background). For example, wash buffers could include denaturing agents (e.g., formamide, urea), salt buffer (e.g., sodium chloride sodium citrate (SSC), phosphate buffered saline (PBS)), acids (e.g., citric acid), surfactants (e.g., Tween 20, Triton-X, SDS), or blocking agents (e.g., tRNA, salmon sperm DNA, BSA, ficoll, polyvinilypyrolidone, heparin). Wash buffer can be combined with wash temperature (e.g., 25-80 °C) to optimize wash stringency.

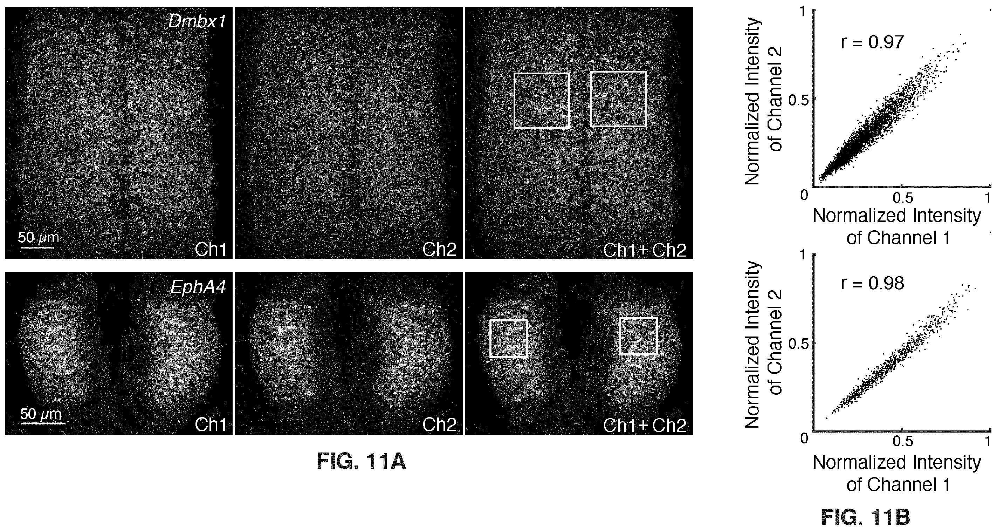

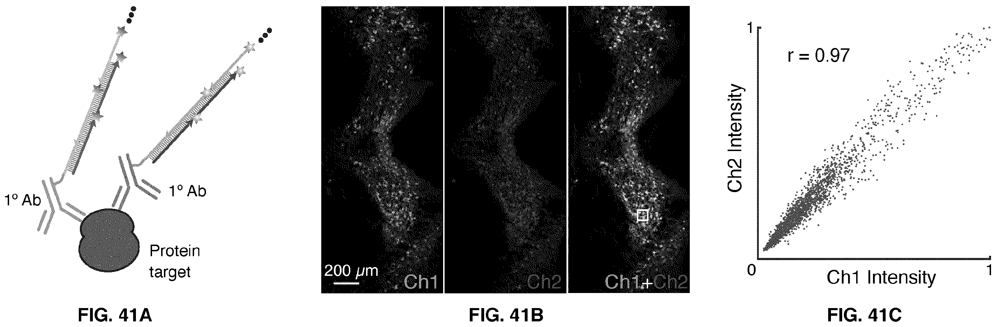

**Accurate and precise target quantitation.** In some embodiments, HCR probes and HCR amplifiers provide quantitative analysis of target molecules in an anatomical context, generating a signal that scales approximately linearly with the number of target molecules per imaging voxel. This quantitative property follows from summation of signal that occurs at three levels during imaging: 1) summation over one or more initiator-labeled probes per target molecule or over one or more probe units (each comprising two or more fractional initiator probes) per target molecule. 2) Summation over multiple HCR amplification hairpins per amplification polymer tethered to an initiator-labeled probe or to a probe unit of fractional initiator probes that colocalize a full initiator. 3) Summation over zero, one, or more target molecules in an imaging pixel. Quantitative precision can be further increased while still maintaining subcellular resolution by defining imaging voxels that average the intensities of neighboring pixels. For example, accurate and precise quantitative imaging with subcellular resolution is demonstrated for mRNA targets in FIGs. 11A-11B and for protein targets in FIGs. 41A-41C. The quantitative nature of HCR signal follows from the binding properties of HCR probes, the polymerization properties of HCR amplification hairpins, and the central limit theorem, which leverage summation and averaging during and after image acquisition to generate signals that scale approximately linearly with target abundance. The same quantitative properties apply to other assay formats, with summation and/or averaging occurring during and/or after data acquisition (for example, by a flow cytometer or a blot scanner).

**Multiplexing using initiator-labeled probes.** In some embodiments, HCR probe sets comprising initiator-labeled probes and HCR amplifiers comprising HCR hairpins can be used for multiplexed target analysis (for example, target analysis via imaging, blotting, flow cytometry, mass cytometry, gel analysis, or any other analysis mode) in which multiple targets are analyzed in the same sample at the same time. Consider a sample containing some or all of N target types of interest as well as zero, one, or more additional off-target species that are not of interest. Each target can be detected using a probe set comprising one or more initiator-labeled probes (each comprising one or more HCR initiators) that selectively bind the cognate target. In some embodiments, the probe set for each of N target types is labeled with HCR initiators for a different HCR amplifier. For example, Target 1 can be detected with Probe Set 1 labeled with HCR initiators for HCR Amplifier 1, Target 2 can be detected with Probe Set 2 labeled with HCR initiators for HCR Amplifier 2, and so on, with Probe Set N labeled with HCR initiators for HCR Amplifier N.

In some embodiments, the N probe sets operate orthogonally such that each probe set selectively binds its cognate target independent of whether the other probe sets and/or targets are present in the sample. In some embodiments, the N amplifiers operate orthogonally such that; 1) the hairpins for each amplifier coexist metastably in the absence of a cognate HCR initiator, 2) each amplifier is selectively triggered to polymerize if its cognate initiator is present independent of whether the other amplifiers are present in the sample.

In some embodiments, the labels carried by each HCR amplifier are orthogonal such that the analysis method is able to measure the signal generated by each HCR amplifier whether or not the other labels are present in the sample (for example, fluorescent labels that can be distinguished using fluorescence microscopy, or rare earth labels that can be distinguished using mass cytometry).

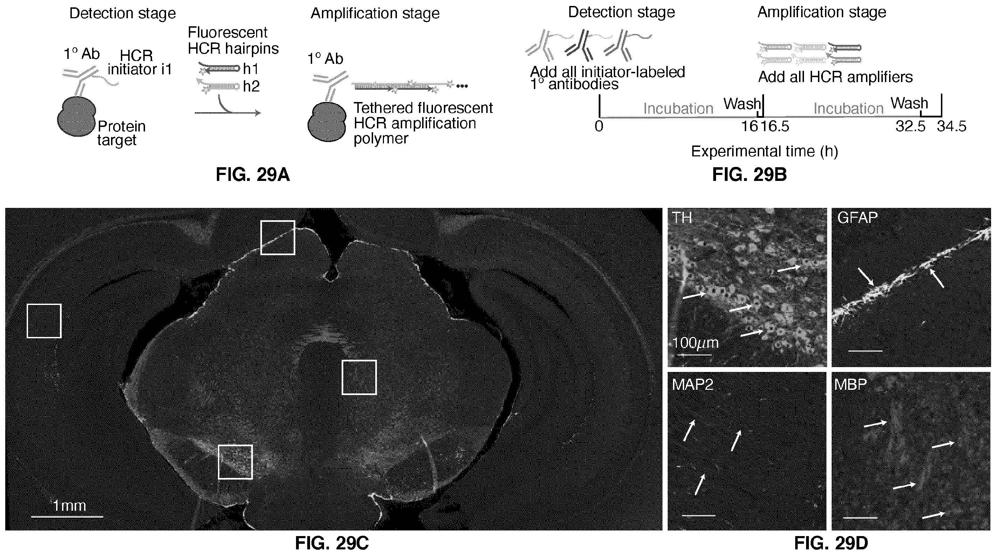

For example, multiplexed imaging using initiator-labeled probes and simultaneous HCR signal amplification for all targets are shown in FIGs. 29, 30, 41, and 43.

In some embodiments, multiplexed target analysis for N target types (types j = 1,...,N) can be achieved as follows:
1. Using N orthogonal HCR initiator-labeled probe sets to detect all targets simultaneously (with initiator-labeled probes from probe set j binding to target j for target types j = 1,...,N),
2. Using N orthogonal HCR amplifiers to amplify the signal for all target types simultaneously (with hairpins from amplifier j polymerizing in response to initiator j to form an amplification polymer j tethered to target type j for j=1,...,N),
3. Analyzing the sample using a measurement device to detect reporter j either directly carried by one or more of the hairpins in amplifier j or indirectly bound to one or more of the hairpins in amplifier j, for target types j=1,...,N.

**Multiplexing using fractional initiator probes.** In some embodiments, HCR probe sets comprising fractional initiator probes and HCR amplifiers comprising HCR hairpins can be used for multiplexed target analysis (for example, target analysis via imaging, blotting, flow cytometry, mass cytometry, gel analysis, or any other analysis mode) in which multiple targets are analyzed in the same sample at the same time. Consider a sample containing some or all of N target types of interest as well as zero, one, or more additional off-target species that are not of interest. Each target can be detected using a probe set comprising one or more probe units (each comprising two or more fractional initiator probes) that selectively bind the cognate target so that each bound probe unit colocalizes a full HCR initiator. In some embodiments, the probe set for each of N target types colocalizes full HCR initiators for a different HCR amplifier. For example, Target 1 can be detected with Probe Set 1 that colocalizes one or more full HCR initiators for HCR Amplifier 1, Target 2 can be detected with Probe Set 2 that colocalizes one or more full HCR initiators for HCR Amplifier 2, and so on, with Probe Set N colocalizing one or more full HCR initiators for HCR Amplifier N.

In some embodiments, the N probe sets operate orthogonally such that each probe set selectively binds its cognate target independent of whether the other probe sets and/or targets are present in the sample. In some embodiments, the N amplifiers operate orthogonally such that; 1) the hairpins for each amplifier coexist metastably in the absence of a cognate full initiator colocalized by the cognate target, 2) each amplifier is selectively triggered to polymerize if its cognate full initiator is colocalized by its cognate target independent of whether the other amplifiers are present in the sample.

In some embodiments, the labels carried by each HCR amplifier are orthogonal such that the analysis method is able to measure the signal generated by each HCR amplifier whether or not the other labels are present in the sample (for example, fluorescent labels that can be distinguished using fluorescence microscopy, or rare earth labels that can be distinguished using mass cytometry).

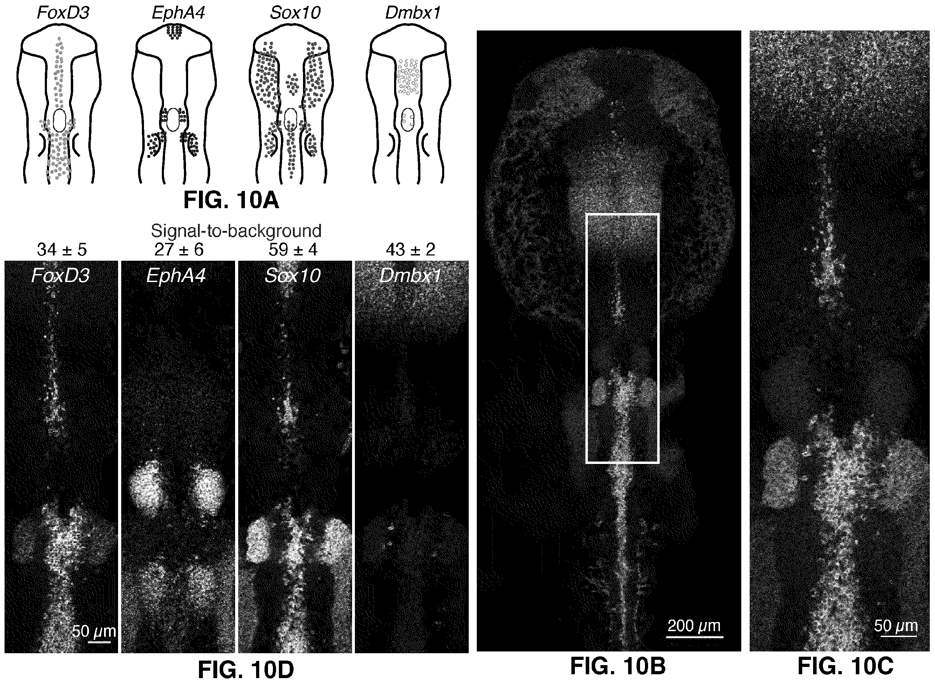

For example, multiplexed imaging using fractional initiator probes and simultaneous HCR signal amplification for all targets are shown in FIGs. 10 and 11.

In some embodiments, multiplexed target analysis for N target types (types j = 1,...,N) can be achieved as follows:
1. Using N orthogonal HCR fractional initiator probe sets to detect all targets simultaneously (with fractional initiator probes from probe set j binding to target j so as to colocalize a full HCR initiator j for each probe unit in probe set j for target types j = 1,...,N),
2. Using N orthogonal HCR amplifiers to amplify the signal for all target types simultaneously (with hairpins from amplifier j polymerizing in response to full HCR initiator j to form an amplification polymer j tethered to target type j for j=1,...,N),
3. Analyzing the sample using a measurement device to detect reporter j either directly carried by one or more of the hairpins in amplifier j or indirectly bound to one or more of the hairpins in amplifier j, for target types j=1,...,N.

**Multiplexing using a combination of initiator-labeled probes and fractional initiator probes.** In some embodiments, multiplexed analysis is performed in a sample using initiator-labeled probes to detect one or more targets (of possibly different types) and fractional initiator probes to detect one or more other targets (of possibly different types). For example, in the same sample, one or more protein targets and one or more small RNA targets could be detected with orthogonal initiator-labeled probes, one or more mRNA targets and/or DNA targets could be detected with orthogonal fractional initiator probes, and one or more complex targets (comprising a complex of two or more non-covalently linked molecules) could be detected with fractional initiator probes. In some embodiments, the probe set for each target (comprising one or more initiator-labeled probes or one or more probe units each comprising two or more fractional initiator probes) would trigger an orthogonal HCR amplifier that generates (directly or indirectly) an orthogonal signal. In some embodiments, HCR signal amplification is performed for all target types simultaneously.

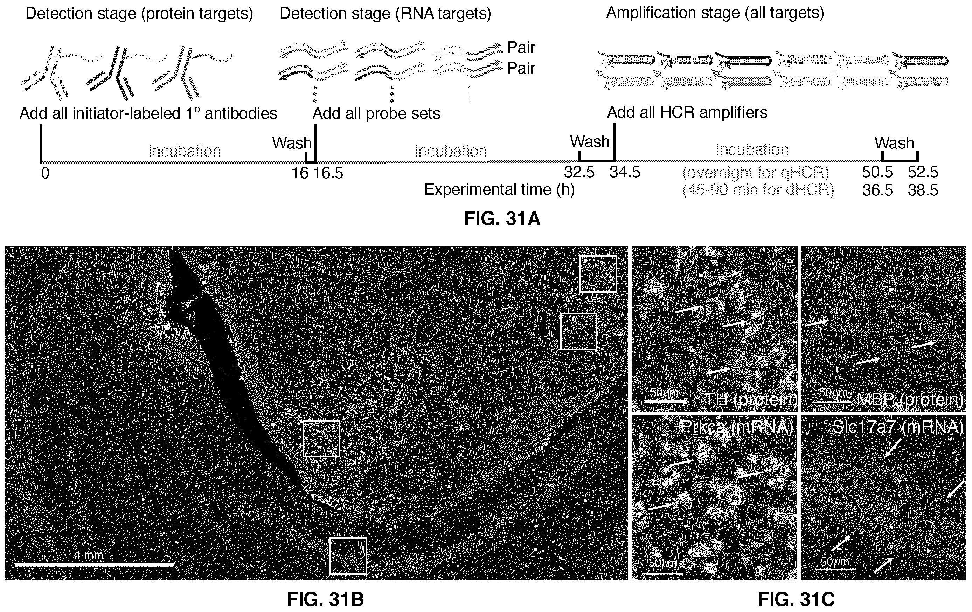

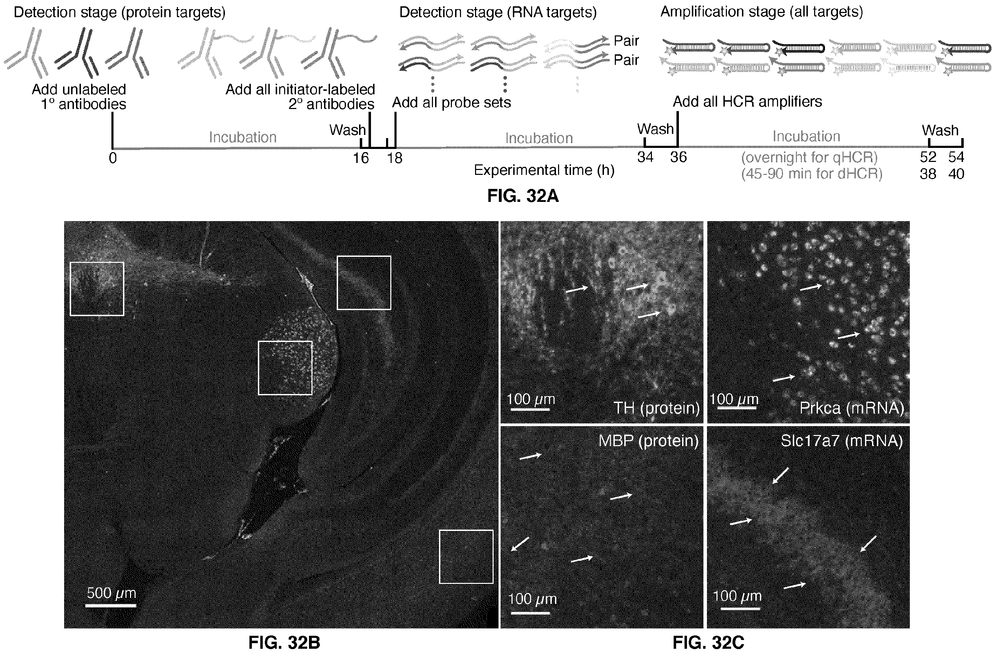

For example, multiplexed imaging using initiator-labeled probes for one or more targets and fractional initiator probes for one or more targets with simultaneous HCR signal amplification for all targets are shown in FIGs. 31 and 32.

**Multiplexing using spectral imaging.** In some embodiments, the number of labels that can be distinguished from each other can be increased using spectral analysis. For example, if two fluorophores have overlapping emissions spectra such that measurement of emissions intensity using a bandpass filter would not be able to distinguish between the two labels, they can be distinguished using spectral imaging in which multiple emissions measurements at different wavelengths are used to distinguish between the signal coming from the two labels even though the emissions spectra of the labels are substantially overlapping. Using spectral imaging, in some embodiments, 10 fluorescent dyes can be spectrally distinguished, or 20 fluorescent dyes can be spectrally distinguished, or 30 or more fluorescent dyes can be spectrally distinguished.

**Multiplexing using hybrid spectra using multi-reporter polymers.** HCR polymerization proceeds via alternating H1 and H2 polymerization steps so the resulting HCR amplification polymer contains either: 1) the same number of H1 and H2 hairpins, 2) one more H1 hairpin, 3) or one more H2 hairpin. As the length of the polymer increases, the fraction of H1 hairpins in the polymer approaches 0.5 and the fraction of H2 hairpins in the polymer also approaches 0.5. In some embodiments, hairpin H1 is labeled with reporter R1 and hairpin H2 is labeled with reporter R2. The signal produced by the HCR amplification polymer is a 1:1 blend of the signal produced by reporter R1 and reporter R2 with a new hybrid spectrum. Consider a set of N reporters with distinct spectra. The N reporters can be used to create N*(N-1)/2 hybrid spectra corresponding to the number of distinct pairs of reporters that can be selected from the set of N reporters. For example: 1) with 6 reporters it is possible to create 6*5/2 = 15 hybrid reporter spectra, 2) with 8 reporters it is possible to create 8*7/2 = 28 hybrid reporter spectra, 3) with 15 reporters it is possible to create 15*14/2 = 105 hybrid reporter spectra, 4) with 50 reporters it is possible to create 50*49/2 = 1225 hybrid reporter spectra, 5) with 100 reporters it is possible to create 100*99/2 = 4950 hybrid reporter spectra.

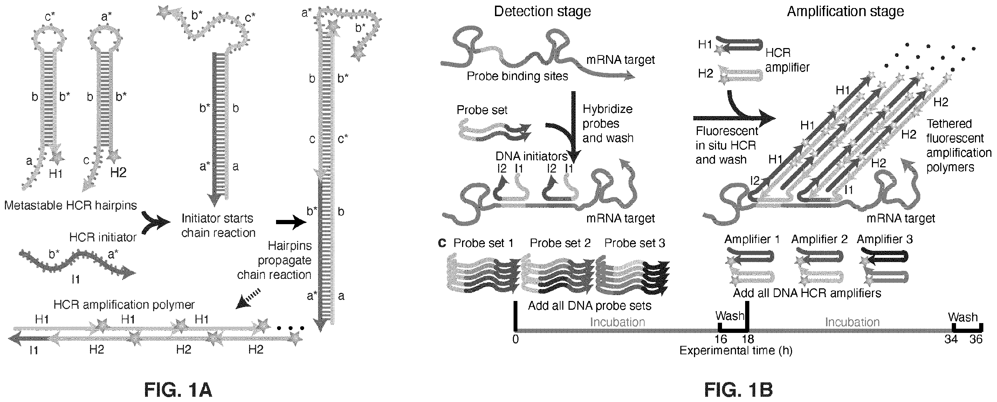

**Computational sequence design of orthogonal HCR amplifiers using NUPACK.** In some embodiments, a set of orthogonal HCR amplifiers (with or without substrates and/or auxiliary strands) is designed using the reaction pathway designer within the NUPACK the software suite.^{56,57} In some embodiments, sequence design is formulated as a multistate optimization problem using a set of target test tubes to represent elementary steps in the reaction pathway as well as to model global crosstalk.⁵⁶ In some embodiments, each elementary step tube contains a set of desired on-target complexes (each with a target secondary structure and target concentration), corresponding to the on-pathway hybridization products for a given step, and a set of undesired off-target complexes (each with vanishing target concentration), corresponding to on-pathway reactants and off-pathway hybridization crosstalk for a given step.⁵⁶ In this scenario, these elementary step tubes promote full conversion of cognate reactants into cognate products and against local hybridization crosstalk between these same reactants. In some embodiments, to simultaneously design N orthogonal systems, elementary step tubes are specified for each orthogonal system. In some embodiments, to design against off-pathway interactions between systems, a single global crosstalk tube is also specified.⁵⁶ In some embodiments, in the global crosstalk tube, the on-target complexes correspond to all reactive species generated during all elementary steps for all systems (for example, the single-stranded output domains of HCR hairpins that have been opened via polymerization). In some embodiments, in the global crosstalk tube, the off-target complexes correspond to noncognate interactions between these reactive species. In some embodiments, the global crosstalk tube ensemble omits the cognate products that the reactive species are intended to form (they appear as neither on-targets nor off-targets). In this scenario, all reactive species in the global crosstalk tube can be forced to either perform no reaction (remaining as desired on-targets) or to undergo a crosstalk reaction (forming undesired off-targets), providing the basis for minimization of global crosstalk during sequence optimization. In some embodiments, sequence design is performed subject to complementarity constraints inherent to the reaction pathway (for example in FIG. 1A, domain "a" complementary to domain "a*", domain "b" complementary to domain "b*").⁵⁶ In some embodiments, sequences are optimized by reducing the ensemble defect quantifying the average fraction of incorrectly paired nucleotides over the multi-tube ensemble.⁵⁶ In some embodiments, defect weights are applied within the ensemble defect to prioritize design effort.⁵⁶ Optimization of the ensemble defect implements both a positive design paradigm, explicitly design for on-pathway elementary steps, and a negative design paradigm, explicitly design against off-pathway crosstalk.⁵⁶

**Multiplexing using repeated reporter detection.** In some embodiments, the number of targets that can be analyzed in a sample can be increased using the same N labels to detect multiple targets in successive rounds of analysis. For example, N targets can be imaged using N labels and then removing the signal from the sample and detecting another set of N targets using the same N labels. This approach is applicable for imaging multiple target types in the same sample: 1) regardless of whether the expression levels of the different target types are high, low, variable within each target type, and/or variable across different target types, 2) regardless of whether the expression patterns of the different target types are spatially overlapping or non-overlapping within the sample. Example methods for multiplexed analysis using repeated reporter detection include but are not limited to those described in Methods AT below. In some embodiments, in one, more, and/or all of the steps of Methods A-T below, the choice of probe type can be different for different targets and can, optionally, be mixed within any process. In some embodiments of methods A-T: 1) all targets may be detected with initiator-labeled probes, or 2) all targets may be detected with fractional initiator probes, or 3) one or more targets may be detected with initiator-labeled probes and other targets may be detected with fractional initiator probes. Thus, the disclosure of one option herein provides the options for the other and for their combination. For example the statement "Providing N probe sets each comprising either: a) one or more HCR initiator-labeled probes, or b) one or more probe units each comprising two or more HCR fractional initiator probes" implies that any one of the N probe sets can be of either type (a or b), including the possibility that all probe sets are of the same type (all of type a or all of type b) and the possibility that some probe sets are of one type and some probe sets are of the other type (some of type a and some of type b). It is also appreciated that in the disclosures in the specification, the types can be mixed for any of the embodiments where the "either...or" is denoted in this context (unless explicitly noted otherwise).

In some embodiments, any one of the Methods A-T below can be combined with CARD, enzyme deactivation, and/or repeated CARD. When the phrase "optionally be modified further by CARD, enzyme deactivation, and/or repeated CARD)" is used, it denotes that any of the embodiments being discussed can also be combined with any one or more of the discussed embodiments involving CARD, enzyme deactivation, and/or repeated CARD. This explicitly allows for the further combination of the various methods provided herein. Similarly, when the phrase "optionally be modified further by multiplexing, CARD, enzyme deactivation, repeated CARD, repeated reporter detection, and/or repeated signal removal" is used, it denotes that any of the embodiments being discussed can also be combined with any one or more of the discussed embodiments involving multiplexing, CARD, enzyme deactivation, repeated CARD, repeated reporter detection, and/or repeated signal removal. These phrases are used as shorthand notations to simplify the disclosure by relying on the other disclosure parts by reference, rather than repeating them.

In some embodiments, any one or more of the optional steps of any one or more of the methods herein can be combined with any one or more of the other optional steps of any one or more of the methods herein.

In some embodiments, any one or more of the steps that are different for any one or more of the methods herein can be combined with any one or more of the other steps that are different for any one or more of the methods herein.

In some embodiments, any one or more of the optional steps that are different for any one or more of the methods herein can be combined with any one or more of the other optional steps that are different for any one or more of the methods herein.

In some embodiments, a method for multiplexed analysis using repeated reporter detection is **Method A** (Example 15) (which can optionally be modified further by CARD, enzyme deactivation, and/or repeated CARD) comprising:
1. Providing a sample possibly containing a target as well as possibly other molecules that are not targets
2. Optionally fixing the sample
3. Optionally permeabilizing the sample
4. Providing a probe set comprising either: a) one or more HCR initiator-labeled probes, or b) one or more probe units each comprising two or more HCR fractional initiator probes
5. Optionally washing the sample
6. Providing an HCR amplifier labeled with a reporter
7. Optionally washing the sample
8. Detecting a signal from the reporter

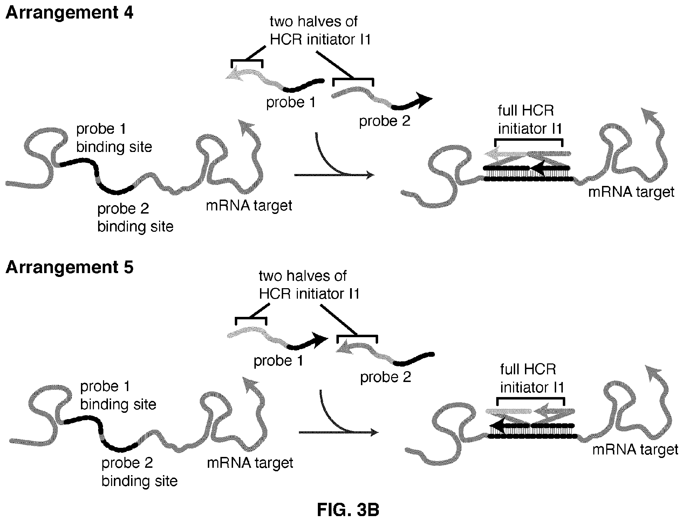

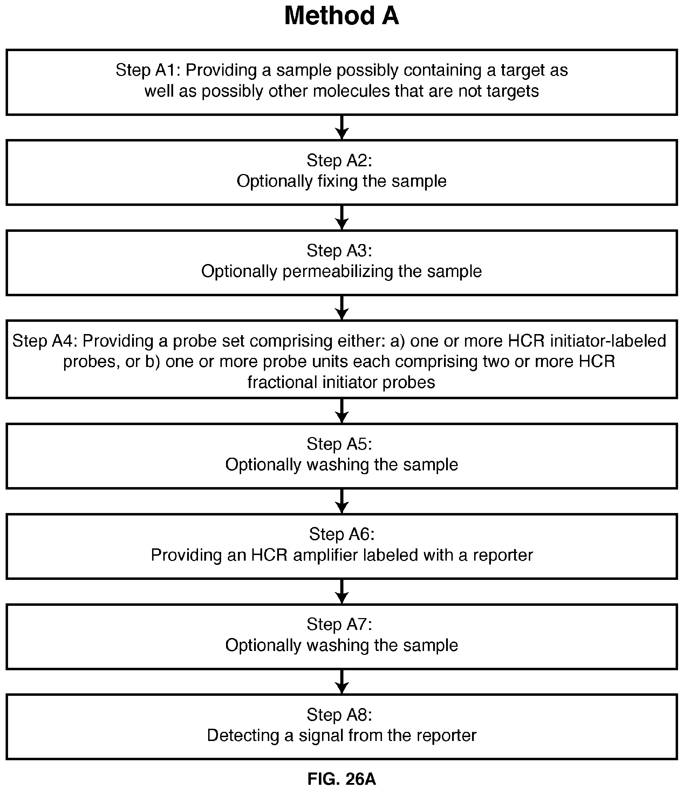

In some embodiments, Method A (Example 15) comprises (see Figure 26A): Step A1: Providing a sample possibly containing a target as well as possibly other molecules that are not targets; Step A2: Optionally fixing the sample; Step A3: Optionally permeabilizing the sample; Step A4: providing a probe set comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17); Step A5: Optionally washing the sample; Step A6: Providing an HCR amplifier labeled with a reporter (for example, see the reporter-labeled amplifiers of Figures 8 and 18); Step A7: Optionally washing the sample; Step A8: Detecting a signal from the reporter.

In some embodiments, Method A (e.g., an example of which is shown in Example 15) comprises (see Figure 26A): Step A1: Providing a sample possibly containing a target as well as possibly other molecules that are not targets; Step A2: fixing the sample; Step A3: permeabilizing the sample; Step A4: providing a probe set comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17); Step A5: washing the sample; Step A6: Providing an HCR amplifier labeled with a reporter (for example, see the reporter-labeled amplifiers of Figures 8 and 18); Step A7: washing the sample; Step A8: Detecting a signal from the reporter.

In some embodiments, a method for multiplexed analysis using repeated reporter detection includes **Method B** (e.g., Example 16) (which can optionally be modified further by CARD, enzyme deactivation, and/or repeated CARD) comprising:
1. Providing a sample possibly containing up to N targets as well as possibly other molecules that are not targets
2. Optionally fixing the sample
3. Optionally permeabilizing the sample
4. Providing N probe sets each comprising either: a) one or more HCR initiator-labeled probes, or b) one or more probe units each comprising two or more HCR fractional initiator probes
5. Optionally washing the sample
6. Providing N HCR amplifiers (each labeled with a distinct reporter) corresponding to the N probe sets
7. Optionally washing the sample
8. Detecting N signals from the N distinct reporters

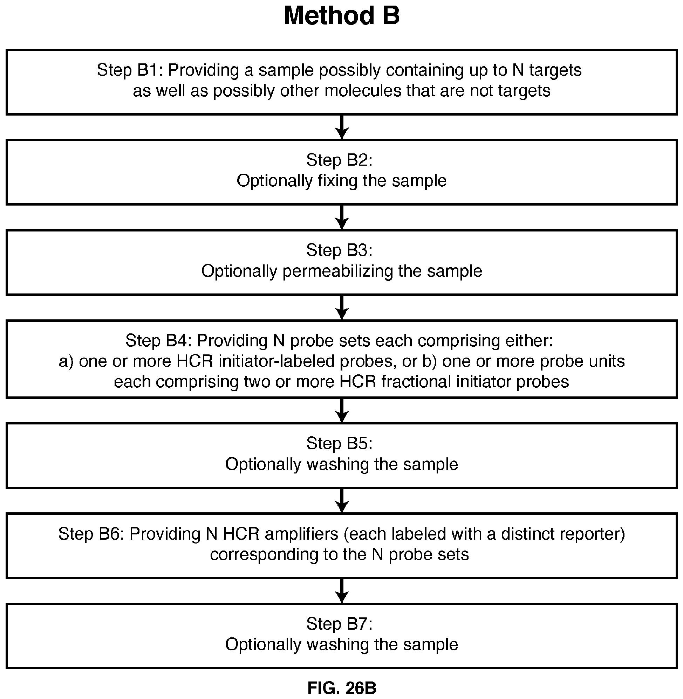

In some embodiments, Method B (e.g., Example 16) comprises (see Figure 26B): Step B1: Providing a sample possibly containing up to N targets as well as possibly other molecules that are not targets; Step B2: Optionally fixing the sample; Step B3: Optionally permeabilizing the sample; Step B4: Providing N probe sets each comprising either: a) one or more HCR initiator-labeled probes (for example, see the probes of Figures 39A-39N, 41A, 42A-42F, and 43A), or b) one or more probe units (for example see the probe sets of Figures 8 and 16) each comprising two or more HCR fractional initiator probes (for example, see the probe units of Figures 3, 4, 5, and 17); Step B5: Optionally washing the sample; Step B6: Providing N HCR amplifiers (each labeled with a distinct reporter) corresponding to the N probe sets (for example, see the reporter-labeled HCR amplifiers of Figures 8 and 18); Step B7: Optionally washing the sample; Step B8: Detecting N signals from the N distinct reporters.

In some embodiments, Method B (e.g., Example 16) comprises (see Figure 26B): Step B1: Providing a sample possibly containing up to N targets as well as possibly other molecules that are not targets; Step B2: fixing the sample; Step B3: permeabilizing the sample; Step B4: Providing N probe sets each comprising either: a) one or more HCR initiator-labeled probes (for example, see the probes of Figures 39A-39N, 41A, 42A-42F, and 43A), or b) one or more probe units (for example see the probe sets of Figures 8 and 16) each comprising two or more HCR fractional initiator probes (for example, see the probe units of Figures 3, 4, 5, and 17); Step B5: washing the sample; Step B6: Providing N HCR amplifiers (each labeled with a distinct reporter) corresponding to the N probe sets (for example, see the reporter-labeled HCR amplifiers of Figures 8 and 18); Step B7: washing the sample; Step B8: Detecting N signals from the N distinct reporters.

In some embodiments, a method for multiplexed analysis using repeated reporter detection is **Method C** (e.g., Example 17) (which can optionally be modified further by CARD, enzyme deactivation, and/or repeated CARD) comprising:
1. Providing a sample possibly containing up to N targets as well as possibly other molecules that are not targets
2. Optionally fixing the sample
3. Optionally permeabilizing the sample
4. Providing a probe set (targeting one of the N target types) comprising either: a) one or more HCR initiator-labeled probes, or b) one or more probe units each comprising two or more HCR fractional initiator probes
5. Optionally washing the sample
6. Providing an HCR amplifier (labeled with a reporter) corresponding to the provided probe set
7. Optionally washing the sample
8. Detecting a signal from the reporter
9. Removing the signal from the sample
10. Optionally repeating one or more of Steps C4-C9 until signal detection has been performed for all N targets

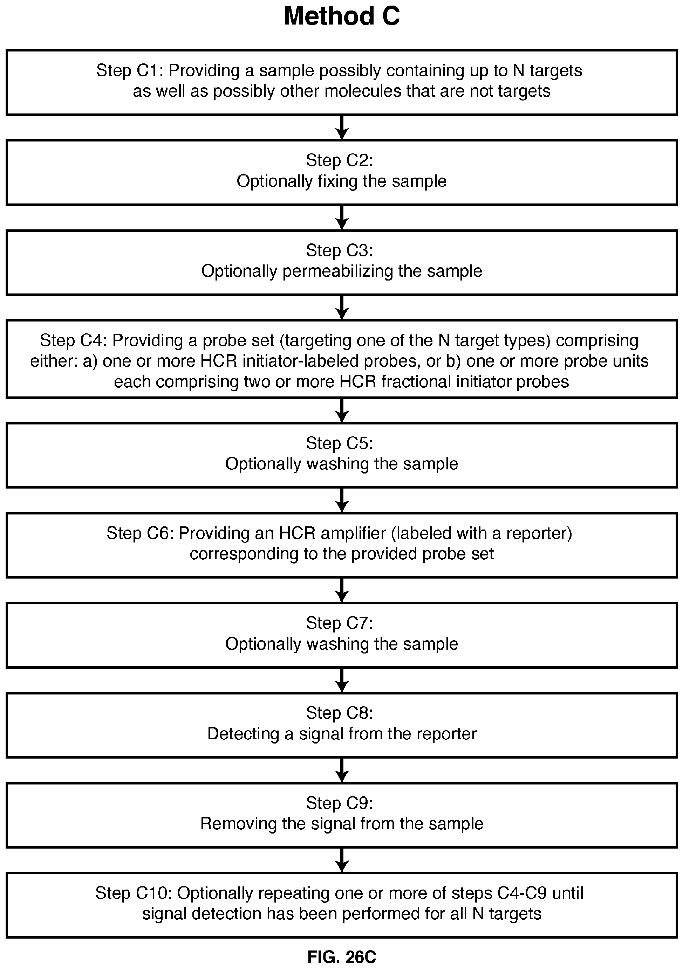

In some embodiments, Method C (e.g., Example 17) comprises (see Figure 26C): Step C1: providing a sample possibly containing up to N targets as well as possibly other molecules that are not targets; Step C2: optionally fixing the sample; Step C3: optionally permeabilizing the sample, Step C4: providing a probe set (targeting one of the N target types) comprising either: a) one or more HCR initiator-labeled probes (for example, see the probes of Figures 39A-39N, 41A, 42A-42F, and 43A), or b) one or more probe units (for example see the probe sets of Figures 8 and 16) each comprising two or more HCR fractional initiator probes (for example, see the probe units of Figures 3, 4, 5, and 17); Step C5: optionally washing the sample, Step C6: providing an HCR amplifier (labeled with a reporter) corresponding to the provided probe set (for example, see the reporter-labeled HCR amplifiers of Figures 8 and 18); Step C7: optionally washing the sample, Step C8: detecting a signal from the reporter; Step C9: removing the signal from the sample (for example, see Figure 23); Step C10: optionally repeating one or more of Steps C4-C9 until signal detection has been performed for all N targets.

In some embodiments, Method C (e.g., Example 17) comprises (see Figure 26C): Step C1: providing a sample possibly containing up to N targets as well as possibly other molecules that are not targets; Step C2: fixing the sample; Step C3: permeabilizing the sample, Step C4: providing a probe set (targeting one of the N target types) comprising either: a) one or more HCR initiator-labeled probes (for example, see the probes of Figures 39A-39N, 41A, 42A-42F, and 43A), or b) one or more probe units (for example see the probe sets of Figures 8 and 16) each comprising two or more HCR fractional initiator probes (for example, see the probe units of Figures 3, 4, 5, and 17); Step C5: washing the sample, Step C6: providing an HCR amplifier (labeled with a reporter) corresponding to the provided probe set (for example, see the reporter-labeled HCR amplifiers of Figures 8 and 18); Step C7: washing the sample, Step C8: detecting a signal from the reporter; Step C9: removing the signal from the sample (for example, see Figure 23); Step C10: repeating one or more of Steps C4-C9 until signal detection has been performed for all N targets.

In some embodiments, a method for multiplexed analysis using repeated reporter detection is **Method D** (e.g., Example 18) (which can optionally be modified further by CARD, enzyme deactivation, and/or repeated CARD) comprising:
1. Providing a sample possibly containing up to N targets as well as possibly other molecules that are not targets
2. Optionally fixing the sample
3. Optionally permeabilizing the sample
4. Providing M probe sets (for M ≤ N; each targeting one of M target types) each comprising either: a) one or more HCR initiator-labeled probes, or b) one or more probe units each comprising two or more HCR fractional initiator probes
5. Optionally washing the sample
6. Providing M HCR amplifiers (each labeled with a distinct reporter) corresponding to the M probe sets
7. Optionally washing the sample
8. Detecting M signals corresponding to the M distinct reporters
9. Removing the M signals from the sample
10. Optionally repeating one or more of steps 4-9 until signal detection has been performed for all N targets

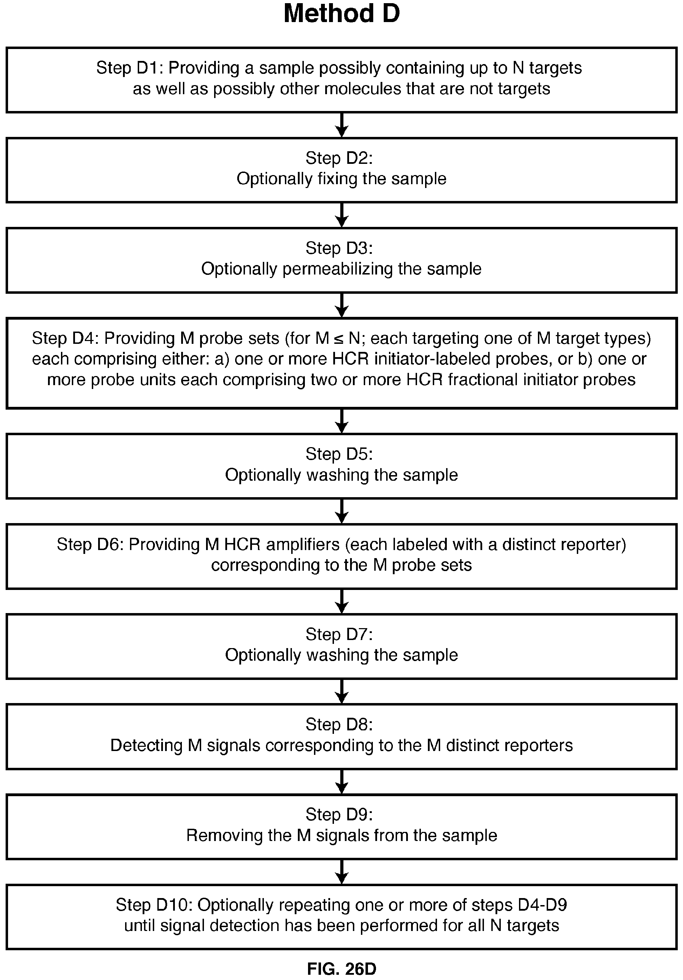

In some embodiments, Method D (e.g., Example 18) comprises (see Figure 26D): Step D1: Providing a sample possibly containing up to N targets as well as possibly other molecules that are not targets; Step D2: Optionally fixing the sample; Step D3: Optionally permeabilizing the sample; Step D4: Providing M probe sets (for M ≤ N; each targeting one of M target types) each comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17); Step D5: Optionally washing the sample; Step D6: Providing M HCR amplifiers (each labeled with a distinct reporter) corresponding to the M probe sets (for example, see the reporter-labeled HCR amplifiers of Figures 8 and 18); Step D7: Optionally washing the sample; Step D8: Detecting M signals corresponding to the M distinct reporters; Step D9: Removing the M signals from the sample (for example, see Figure 23); Step D10: Optionally repeating one or more of steps 4-9 until signal detection has been performed for all N targets.

In some embodiments, Method D (e.g., Example 18) comprises (see Figure 26D): Step D1: Providing a sample possibly containing up to N targets as well as possibly other molecules that are not targets; Step D2: fixing the sample; Step D3: permeabilizing the sample; Step D4: Providing M probe sets (for M ≤ N; each targeting one of M target types) each comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17); Step D5: washing the sample; Step D6: Providing M HCR amplifiers (each labeled with a distinct reporter) corresponding to the M probe sets (for example, see the reporter-labeled HCR amplifiers of Figures 8 and 18); Step D7: washing the sample; Step D8: Detecting M signals corresponding to the M distinct reporters; Step D9: Removing the M signals from the sample (for example, see Figure 23); Step D10: repeating one or more of steps 4-9 until signal detection has been performed for all N targets.

In some embodiments, a method for multiplexed analysis using repeated reporter detection is **Method E** (e.g., Example 19) (which can optionally be modified further by CARD, enzyme deactivation, and/or repeated CARD) comprising:
1. Providing a sample possibly containing up to N targets as well as possibly other molecules that are not targets
2. Optionally fixing the sample
3. Optionally permeabilizing the sample
4. Providing N probe sets (each targeting one of N target types) each comprising either: a) one or more HCR initiator-labeled probes, or b) one or more probe units each comprising two or more HCR fractional initiator probes
5. Optionally washing the sample
6. Providing an HCR amplifier (labeled with a reporter) corresponding to one of the probe sets
7. Optionally washing the sample
8. Detecting a signal from the reporter
9. Removing the signal from the sample
10. Optionally repeating one or more of steps 6-9 until signal detection has been performed for all N targets

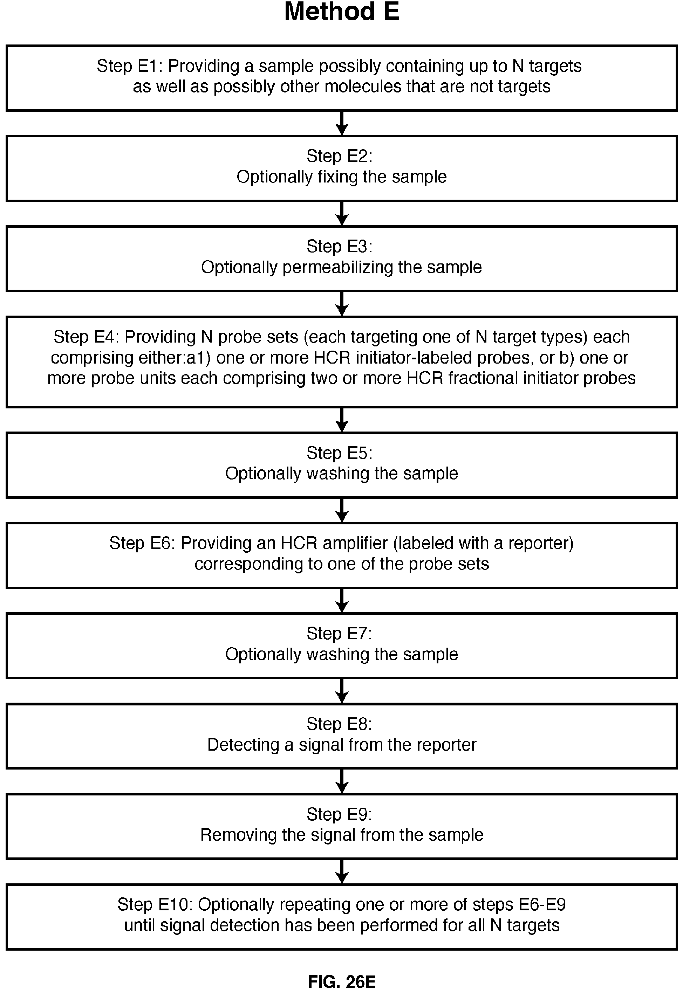

In some embodiments, Method E (e.g., Example 19) comprises (see Figure 26E): Step E1: Providing a sample possibly containing up to N targets as well as possibly other molecules that are not targets; Step E2: Optionally fixing the sample; Step E3: Optionally permeabilizing the sample; Step E4: Providing N probe sets (each targeting one of N target types) each comprising either: a) one or more HCR initiator-labeled probes (for example, see the probes of Figures 39A-39N, 41A, 42A-42F, and 43A), or b) one or more probe units (for example, see the probe sets of Figure 8 and 16) each comprising two or more HCR fractional initiator probes (for example, see the probe units of Figures 3, 4, 5, and 17); Step E5: Optionally washing the sample; Step E6: Providing an HCR amplifier (labeled with a reporter) corresponding to one of the probe sets (for example, see the reporter-labeled amplifiers of Figure 8 and 18); Step E7: Optionally washing the sample: Step E8: Detecting a signal from the reporter; Step E9: Removing the signal from the sample (for example, see Figure 23); Step E10: Optionally repeating one or more of steps 6-9 until signal detection has been performed for all N targets.

In some embodiments, Method E (e.g., Example 19) comprises (see Figure 26E): Step E1: Providing a sample possibly containing up to N targets as well as possibly other molecules that are not targets; Step E2: fixing the sample; Step E3: permeabilizing the sample; Step E4: Providing N probe sets (each targeting one of N target types) each comprising either: a) one or more HCR initiator-labeled probes (for example, see the probes of Figures 39A-39N, 41A, 42A-42F, and 43A), or b) one or more probe units (for example, see the probe sets of Figure 8 and 16) each comprising two or more HCR fractional initiator probes (for example, see the probe units of Figures 3, 4, 5, and 17); Step E5: washing the sample; Step E6: Providing an HCR amplifier (labeled with a reporter) corresponding to one of the probe sets (for example, see the reporter-labeled amplifiers of Figure 8 and 18); Step E7: washing the sample: Step E8: Detecting a signal from the reporter; Step E9: Removing the signal from the sample (for example, see Figure 23); Step E10: repeating one or more of steps 6-9 until signal detection has been performed for all N targets.

In some embodiments, a method for multiplexed analysis using repeated reporter detection is **Method F** (e.g., Example 20) (which can optionally be modified further by CARD, enzyme deactivation, and/or repeated CARD) comprising:
1. Providing a sample possibly containing up to N targets as well as possibly other molecules that are not targets
2. Optionally fixing the sample
3. Optionally permeabilizing the sample
4. Providing N probe sets (each targeting one of N target types) each comprising either: a) one or more HCR initiator-labeled probes, or b) one or more probe units each comprising two or more HCR fractional initiator probes
5. Optionally washing the sample
6. Providing M HCR amplifiers (for M ≤ N; each labeled with a distinct reporter) corresponding to M of the N probe sets
7. Optionally washing the sample
8. Detecting M signals corresponding to the M reporters
9. Removing the M signals from the sample
10. Optionally repeating one or more of steps 6-9 until signal detection has been performed for all N targets

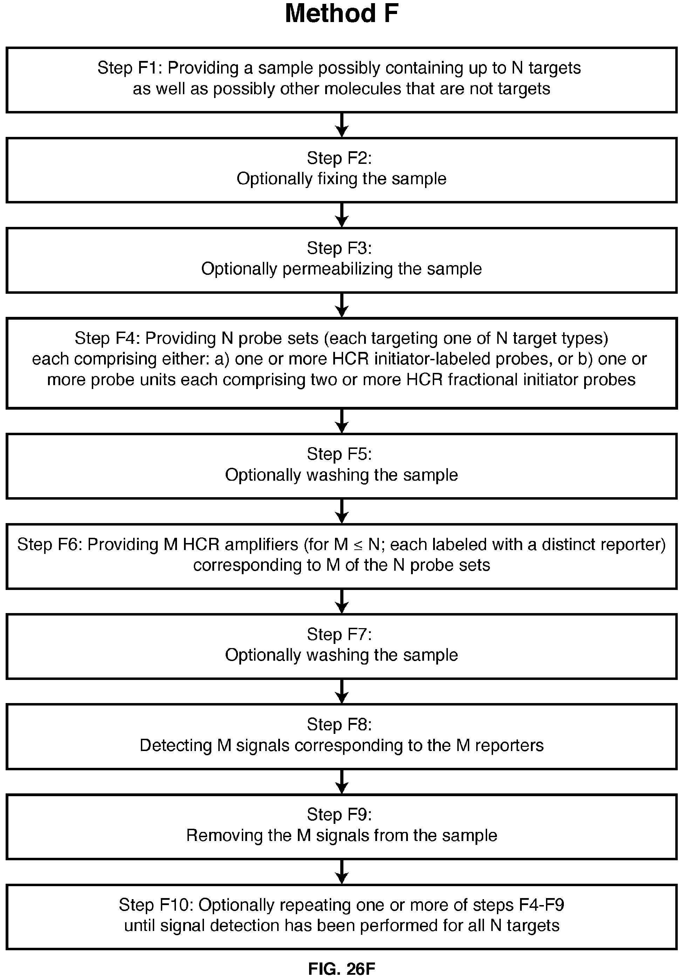

In some embodiments, Method F (e.g., Example 20) comprises (see Figure 26F): Step F1: Providing a sample possibly containing up to N targets as well as possibly other molecules that are not targets; Step F2: Optionally fixing the sample; Step F3: Optionally permeabilizing the sample; Step F4: Providing N probe sets (each targeting one of N target types) each comprising either: a) one or more HCR initiator-labeled probes (for example, see the probes of Figures 39A-39N, 41A, 42A-42F, and 43A), or b) one or more probe units (for example, see the probe sets of Figure 8 and 16) each comprising two or more HCR fractional initiator probes (for example, see the probe units of Figures 3, 4, 5, and 17); Step F5: Optionally washing the sample; Step F6: Providing M HCR amplifiers (for M ≤ N; each labeled with a distinct reporter) corresponding to M of the N probe sets (for example, see the reporter-labeled amplifiers of Figure 8 and 18); Step F7: Optionally washing the sample; Step F8: Detecting M signals corresponding to the M reporters; Step F9: Removing the M signals from the sample (for example, see Figure 23); Step F10: Optionally repeating one or more of steps 6-9 until signal detection has been performed for all N targets.

In some embodiments, Method F (e.g., Example 20) comprises (see Figure 26F): Step F1: Providing a sample possibly containing up to N targets as well as possibly other molecules that are not targets; Step F2: fixing the sample; Step F3: permeabilizing the sample; Step F4: Providing N probe sets (each targeting one of N target types) each comprising either: a) one or more HCR initiator-labeled probes (for example, see the probes of Figures 39A-39N, 41A, 42A-42F, and 43A), or b) one or more probe units (for example, see the probe sets of Figure 8 and 16) each comprising two or more HCR fractional initiator probes (for example, see the probe units of Figures 3, 4, 5, and 17); Step F5: washing the sample; Step F6: Providing M HCR amplifiers (for M ≤ N; each labeled with a distinct reporter) corresponding to M of the N probe sets (for example, see the reporter-labeled amplifiers of Figure 8 and 18); Step F7: washing the sample; Step F8: Detecting M signals corresponding to the M reporters; Step F9: Removing the M signals from the sample (for example, see Figure 23); Step F10: repeating one or more of steps 6-9 until signal detection has been performed for all N targets.

In some embodiments, a method for multiplexed analysis using repeated reporter detection is **Method G** (e.g., Example 21) (which can optionally be modified further by CARD, enzyme deactivation, and/or repeated CARD) comprising:
1. Providing a sample possibly containing one or more targets as well as possibly other molecules that are not targets
2. Optionally fixing the sample
3. Optionally permeabilizing the sample
4. Providing one or more probe sets each comprising either: a) one or more HCR initiator-labeled probes, or b) one or more probe units each comprising two or more HCR fractional initiator probes
5. Optionally washing the sample
6. Providing one or more HCR amplifiers (each labeled with one or more reporters)
7. Optionally washing the sample
8. Detecting one or more signals from one or more reporters
9. Optionally removing one or more probe sets from the sample
10. Optionally removing one or more HCR amplifiers from the sample
11. Optionally removing one or more reporters from the sample
12. Optionally removing one or more signals from the sample
13. Optionally repeating any of steps 2-12 one or more times in any order

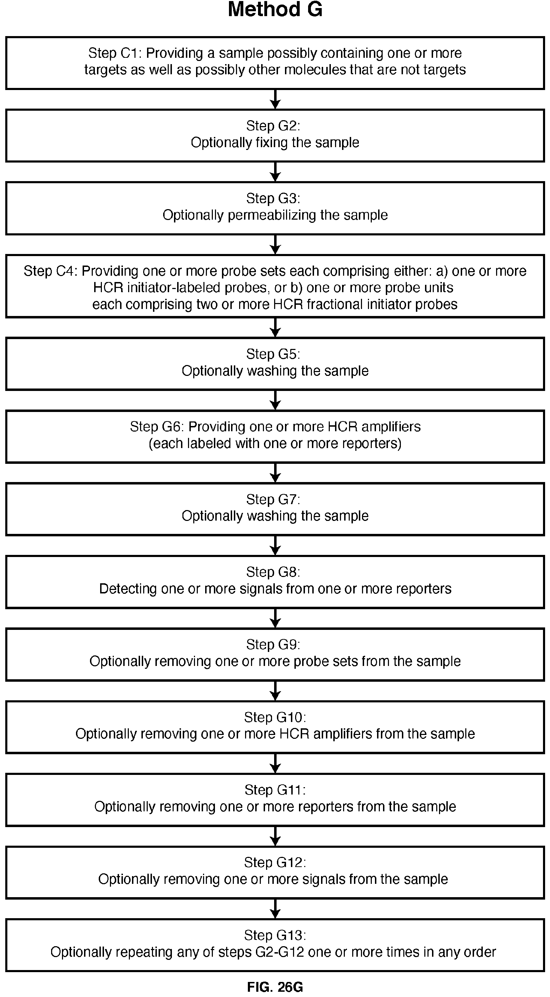

In some embodiments, Method G (e.g., Example 21) comprises (see Figure 26G): Step G1: Providing a sample possibly containing one or more targets as well as possibly other molecules that are not targets; Step G2: Optionally fixing the sample; Step G3: Optionally permeabilizing the sample; Step G4: Providing one or more probe sets each comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17); Step G5: Optionally washing the sample; Step G6: Providing one or more HCR amplifiers (each labeled with one or more reporters) (for example, see the reporter-labeled HCR amplifiers of Figure 8 and 18); Step G7: Optionally washing the sample; Step G8: Detecting one or more signals from one or more reporters; Step G9: Optionally removing one or more probe sets from the sample; Step G10; Optionally removing one or more HCR amplifiers from the sample; Step G11: Optionally removing one or more reporters from the sample; Step G12: Optionally removing one or more signals from the sample (for example, see Figure 23); Step G13: Optionally repeating any of steps 2-12 one or more times in any order.

In some embodiments, Method G (e.g., Example 21) comprises (see Figure 26G): Step G1: Providing a sample possibly containing one or more targets as well as possibly other molecules that are not targets; Step G2: fixing the sample; Step G3: permeabilizing the sample; Step G4: Providing one or more probe sets each comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17); Step G5: washing the sample; Step G6: Providing one or more HCR amplifiers (each labeled with one or more reporters) (for example, see the reporter-labeled HCR amplifiers of Figure 8 and 18); Step G7: washing the sample; Step G8: Detecting one or more signals from one or more reporters; Step G9: removing one or more probe sets from the sample; Step G10; removing one or more HCR amplifiers from the sample; Step G11: removing one or more reporters from the sample; Step G12: removing one or more signals from the sample (for example, see Figure 23); Step G13: repeating any of steps 2-12 one or more times in any order.

In some embodiments, a method for multiplexed analysis using repeated reporter detection is **Method H** (e.g., Example 22) (which can optionally be modified further by CARD, enzyme deactivation, and/or repeated CARD) comprising:
1. Providing a sample possibly containing a target as well as possibly other molecules that are not targets
2. Optionally fixing the sample
3. Optionally permeabilizing the sample
4. Providing a probe set comprising either: a) one or more HCR initiator-labeled probes, or b) one or more probe units each comprising two or more HCR fractional initiator probes
5. Optionally washing the sample
6. Providing an HCR amplifier labeled with a substrate
7. Optionally washing the sample
8. Providing a label probe (conjugated to a reporter) corresponding to the substrate
9. Optionally washing the sample
10. Detecting a signal from the reporter

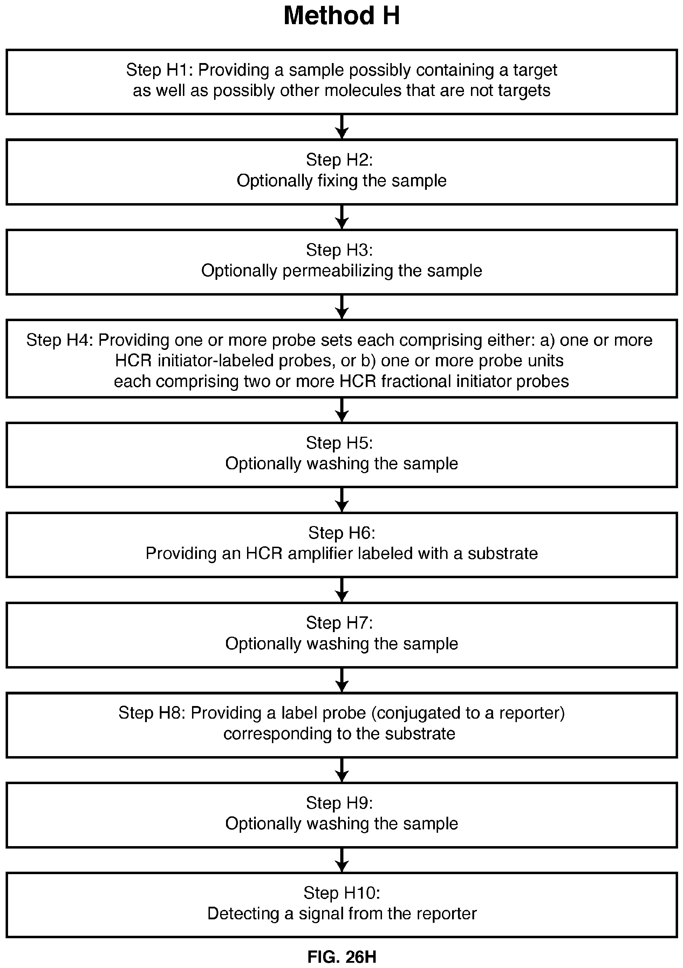

In some embodiments, Method H (e.g., Example 22) comprises (see Figure 26H): Step H1: Providing a sample possibly containing a target as well as possibly other molecules that are not targets; Step H2: Optionally fixing the sample; Step H3: Optionally permeabilizing the sample; Step H4: Providing a probe set comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17); Step H5: Optionally washing the sample; Step H6: Providing an HCR amplifier labeled with a substrate (for example, see the substrate-labeled HCR amplifiers of Figure 18); Step H7: Optionally washing the sample; Step H8: Providing a label probe (conjugated to a reporter) corresponding to the substrate (for example, see the label probes of Figure 20); Step H9: Optionally washing the sample; Step H10: Detecting a signal from the reporter.

In some embodiments, Method H (e.g., Example 22) comprises (see Figure 26H): Step H1: Providing a sample possibly containing a target as well as possibly other molecules that are not targets; Step H2: fixing the sample; Step H3: permeabilizing the sample; Step H4: Providing a probe set comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17); Step H5: washing the sample; Step H6: Providing an HCR amplifier labeled with a substrate (for example, see the substrate-labeled HCR amplifiers of Figure 18); Step H7: washing the sample; Step H8: Providing a label probe (conjugated to a reporter) corresponding to the substrate (for example, see the label probes of Figure 20); Step H9: washing the sample; Step H10: Detecting a signal from the reporter.

In some embodiments, a method for multiplexed analysis using repeated reporter detection is **Method I** (e.g., Example 23) (which can optionally be modified further by CARD, enzyme deactivation, and/or repeated CARD) comprising:
1. Providing a sample possibly containing up to N targets as well as possibly other molecules that are not targets
2. Optionally fixing the sample
3. Optionally permeabilizing the sample
4. Providing N probe sets each comprising either: a) one or more HCR initiator-labeled probes, or b) one or more probe units each comprising two or more HCR fractional initiator probes
5. Optionally washing the sample
6. Providing N HCR amplifiers (each labeled with a distinct substrate) corresponding to the N probe sets
7. Optionally washing the sample
8. Providing N label probes (each conjugated to a distinct reporter) corresponding to the N distinct substrates
9. Detecting the N signals from the N distinct reporters

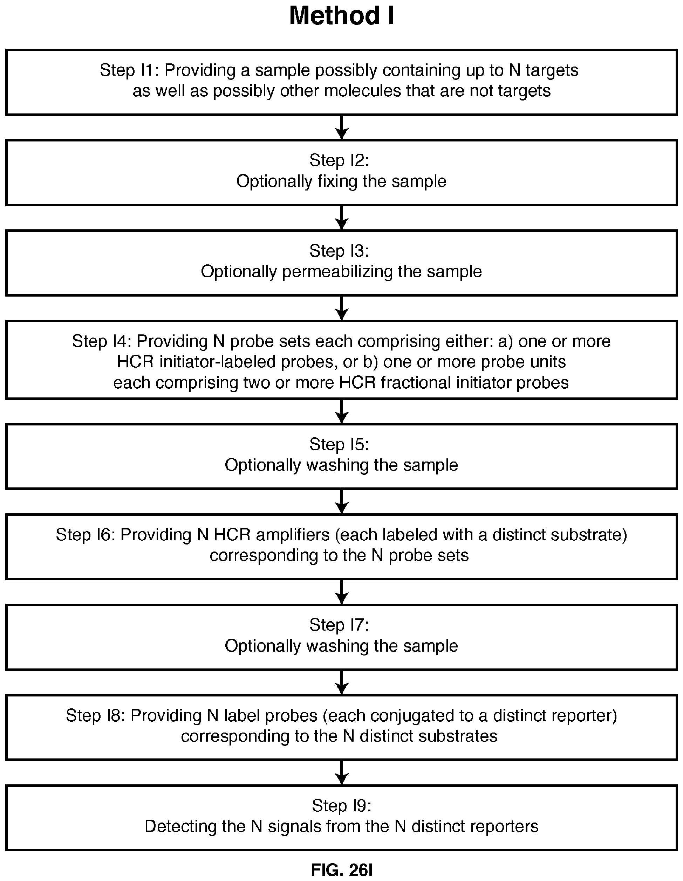

In some embodiments, Method I (e.g., Example 23) comprises (see Figure 26I): Step I1: Providing a sample possibly containing up to N targets as well as possibly other molecules that are not targets; Step I2: Optionally fixing the sample; Step I3: Optionally permeabilizing the sample; Step I4: Providing N probe sets each comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17); Step I5: Optionally washing the sample; Step I6: Providing N HCR amplifiers (each labeled with a distinct substrate) corresponding to the N probe sets (for example, see the substrate-labeled HCR amplifiers of Figure 18); Step I7: Optionally washing the sample; Step I8: Providing N label probes (each conjugated to a distinct reporter) corresponding to the N distinct substrates (for example, see the label probes of Figure 20); Step I9: Detecting the N signals from the N distinct reporters.

In some embodiments, Method I (e.g., Example 23) comprises (see Figure 26I): Step I1: Providing a sample possibly containing up to N targets as well as possibly other molecules that are not targets; Step I2: fixing the sample; Step I3: permeabilizing the sample; Step I4: Providing N probe sets each comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17); Step I5: washing the sample; Step I6: Providing N HCR amplifiers (each labeled with a distinct substrate) corresponding to the N probe sets (for example, see the substrate-labeled HCR amplifiers of Figure 18); Step I7: washing the sample; Step I8: Providing N label probes (each conjugated to a distinct reporter) corresponding to the N distinct substrates (for example, see the label probes of Figure 20); Step I9: Detecting the N signals from the N distinct reporters.

In some embodiments, a method for multiplexed analysis using repeated reporter detection is **Method J** (e.g., Example 24) (which can optionally be modified further by CARD, enzyme deactivation, and/or repeated CARD) comprising:
1. Providing a sample possibly containing up to N targets as well as possibly other molecules that are not targets
2. Optionally fixing the sample
3. Optionally permeabilizing the sample
4. Providing N probe sets each comprising either: a) one or more HCR initiator-labeled probes, or b) one or more probe units each comprising two or more HCR fractional initiator probes
5. Optionally washing the sample
6. Providing N HCR amplifiers (each labeled with a distinct substrate) corresponding to the N probe sets
7. Optionally washing the sample
8. Providing M label probes (for M ≤ N; each conjugated to a distinct reporter) corresponding to M of the N distinct substrates
9. Optionally washing the sample
10. Detecting M signals corresponding to the M distinct reporters
11. Removing the M signals from the sample
12. Optionally repeating one or more of steps 8-11 until signal detection has been performed for all N targets

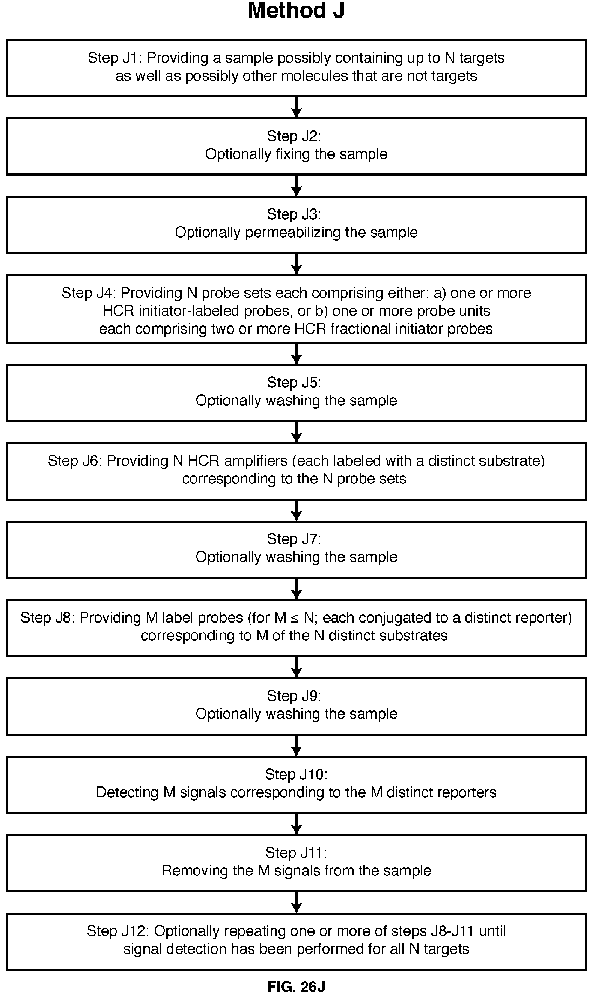

In some embodiments, Method J (e.g., Example 24) comprises (see Figure 26J): Step J1: Providing a sample possibly containing up to N targets as well as possibly other molecules that are not targets; Step J2: Optionally fixing the sample; Step J3: Optionally permeabilizing the sample; Step J4: Providing N probe sets each comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17); Step J5: Optionally washing the sample; Step J6: Providing N HCR amplifiers (each labeled with a distinct substrate) corresponding to the N probe sets (for example, see the substrate-labeled HCR amplifiers of Figure 18); Step J7: Optionally washing the sample; Step J8: Providing M label probes (for M ≤ N; each conjugated to a distinct reporter) corresponding to M of the N distinct substrates (for example, see the label probes of Figure 20); Step J9: Optionally washing the sample; Step J10: Detecting M signals corresponding to the M distinct reporters; Step J11: Removing the M signals from the sample (for example, see Figure 23); Step J12: Optionally repeating one or more of steps J8-J11 until signal detection has been performed for all N targets.

In some embodiments, Method J (e.g., Example 24) comprises (see Figure 26J): Step J1: Providing a sample possibly containing up to N targets as well as possibly other molecules that are not targets; Step J2: fixing the sample; Step J3: permeabilizing the sample; Step J4: Providing N probe sets each comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17); Step J5: washing the sample; Step J6: Providing N HCR amplifiers (each labeled with a distinct substrate) corresponding to the N probe sets (for example, see the substrate-labeled HCR amplifiers of Figure 18); Step J7: washing the sample; Step J8: Providing M label probes (for M ≤ N; each conjugated to a distinct reporter) corresponding to M of the N distinct substrates (for example, see the label probes of Figure 20); Step J9: washing the sample; Step J10: Detecting M signals corresponding to the M distinct reporters; Step J11: Removing the M signals from the sample (for example, see Figure 23); Step J12: repeating one or more of steps J8-J11 until signal detection has been performed for all N targets.

In some embodiments, a method for multiplexed analysis using repeated reporter detection is **Method K** (e.g., Example 25) (which can optionally be modified further by CARD, enzyme deactivation, and/or repeated CARD) comprising:
1. Providing a sample possibly containing one or more targets as well as possibly other molecules that are not targets
2. Optionally fixing the sample
3. Optionally permeabilizing the sample
4. Providing one or more probe sets each comprising either: a) one or more HCR initiator-labeled probes, or b) one or more probe units each comprising two or more HCR fractional initiator probes
5. Optionally washing the sample
6. Providing one or more HCR amplifiers (each labeled with a substrate) corresponding to one or more probe sets
7. Optionally washing the sample
8. Providing one or more label probes (each conjugated to a reporter) corresponding to one or more substrates
9. Optionally washing the sample
10. Detecting one or more signals corresponding to one or more reporters
11. Removing one or more signals from the sample
12. Optionally repeating any of steps 4-11 one or more times in any order

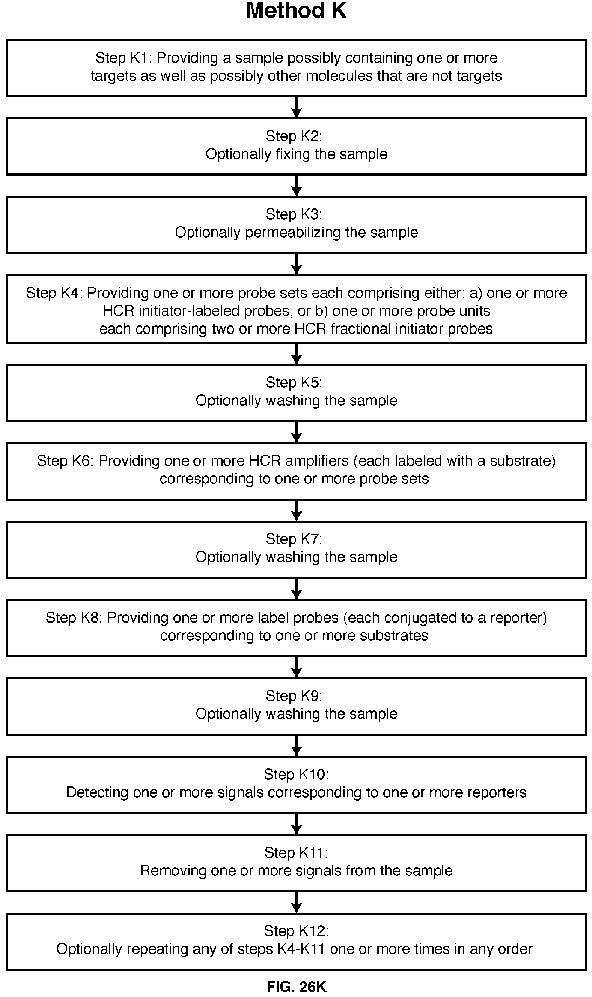

In some embodiments, Method K (e.g., Example 25) comprises (see Figure 26K): Step K1: Providing a sample possibly containing one or more targets as well as possibly other molecules that are not targets; Step K2: Optionally fixing the sample; Step K3: Optionally permeabilizing the sample; Step K4: Providing one or more probe sets each comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17); Step K5: Optionally washing the sample; Step K6: Providing one or more HCR amplifiers (each labeled with a substrate) corresponding to one or more probe sets (for example, see the substrate-labeled HCR amplifiers of Figure 18); Step K7: Optionally washing the sample; Step K8: Providing one or more label probes (each conjugated to a reporter) corresponding to one or more substrates (for example, see the label probes of Figure 20); Step K9: Optionally washing the sample; Step K10: Detecting one or more signals corresponding to one or more reporters; Step K11: Removing one or more signals from the sample (for example, see Figure 23); Step K12: Optionally repeating any of steps K4-K11 one or more times in any order.

In some embodiments, Method K (e.g., Example 25) comprises (see Figure 26K): Step K1: Providing a sample possibly containing one or more targets as well as possibly other molecules that are not targets; Step K2: fixing the sample; Step K3: permeabilizing the sample; Step K4: Providing one or more probe sets each comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17); Step K5: washing the sample; Step K6: Providing one or more HCR amplifiers (each labeled with a substrate) corresponding to one or more probe sets (for example, see the substrate-labeled HCR amplifiers of Figure 18); Step K7: washing the sample; Step K8: Providing one or more label probes (each conjugated to a reporter) corresponding to one or more substrates (for example, see the label probes of Figure 20); Step K9: washing the sample; Step K10: Detecting one or more signals corresponding to one or more reporters; Step K11: Removing one or more signals from the sample (for example, see Figure 23); Step K12: repeating any of steps K4-K11 one or more times in any order.

In some embodiments, a method for multiplexed analysis using repeated reporter detection is **Method L** (e.g., Example 26) (which can optionally be modified further by CARD, enzyme deactivation, and/or repeated CARD) comprising:
1. Providing a sample possibly containing one or more targets as well as possibly other molecules that are not targets
2. Optionally fixing the sample
3. Optionally permeabilizing the sample
4. Providing one or more HCR probe sets each comprising either: a) one or more HCR initiator-labeled probes, or b) one or more probe units each comprising two or more HCR fractional initiator probes
5. Providing one or more HCR amplifiers (each labeled with one or more reporters and/or one or more substrates) corresponding to one or more probe sets
6. Optionally providing one or more label probes (each conjugated to one or more reporters) corresponding to one or more substrates
7. Detecting one or more signals
8. Optionally washing the sample
9. Optionally removing one or more signals from the sample
10. Optionally removing one or more reporters from the sample
11. Optionally removing one or more label probes from the sample
12. Optionally removing one or more HCR amplifiers from the sample
13. Optionally removing one or more probe sets from the sample
14. Optionally repeating any of the above steps in any order

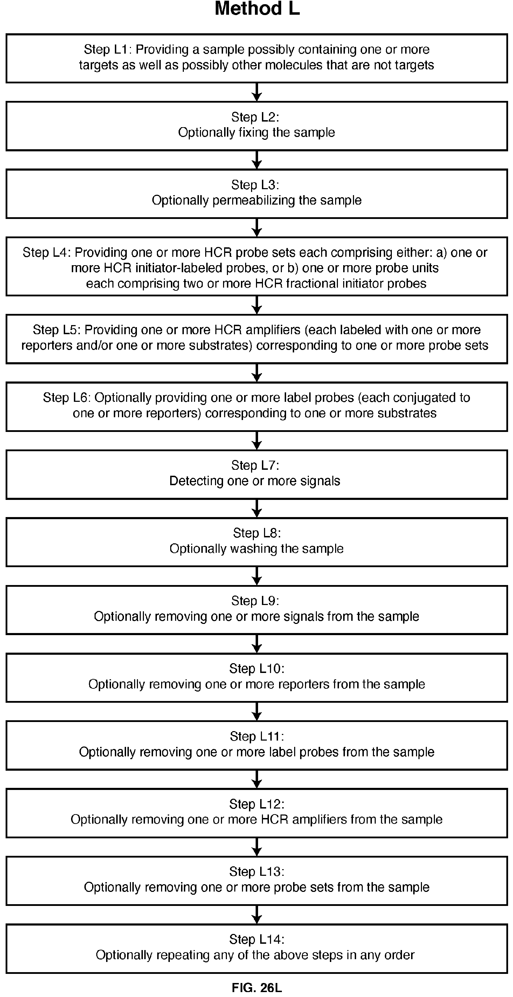

In some embodiments, Method L (e.g., Example 26) comprises (see Figure 26L): Step L1: Providing a sample possibly containing one or more targets as well as possibly other molecules that are not targets; Step L2: Optionally fixing the sample; Step L3: Optionally permeabilizing the sample; Step L4: Providing one or more HCR probe sets each comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17); Step L5: Providing one or more HCR amplifiers (each labeled with one or more reporters and/or one or more substrates) corresponding to one or more probe sets (for example, see the HCR amplifiers of Figures 8 and 18); Step L6: Optionally providing one or more label probes (each conjugated to one or more reporters) corresponding to one or more substrates (for example, see the label probes of Figure 20); Step L7: Detecting one or more signals; Step L8: Optionally washing the sample; Step L9: Optionally removing one or more signals from the sample (for example, see Figure 23); Step L10: Optionally removing one or more reporters from the sample; Step L11: Optionally removing one or more label probes from the sample; Step L12: Optionally removing one or more HCR amplifiers from the sample; Step L13: Optionally removing one or more probe sets from the sample; Step L14: Optionally repeating any of the above steps in any order.

In some embodiments, Method L (e.g., Example 26) comprises (see Figure 26L): Step L1: Providing a sample possibly containing one or more targets as well as possibly other molecules that are not targets; Step L2: fixing the sample; Step L3: permeabilizing the sample; Step L4: Providing one or more HCR probe sets each comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17); Step L5: Providing one or more HCR amplifiers (each labeled with one or more reporters and/or one or more substrates) corresponding to one or more probe sets (for example, see the HCR amplifiers of Figures 8 and 18); Step L6: providing one or more label probes (each conjugated to one or more reporters) corresponding to one or more substrates (for example, see the label probes of Figure 20); Step L7: Detecting one or more signals; Step L8: washing the sample; Step L9: removing one or more signals from the sample (for example, see Figure 23); Step L10: removing one or more reporters from the sample; Step L11: removing one or more label probes from the sample; Step L12: removing one or more HCR amplifiers from the sample; Step L13: removing one or more probe sets from the sample; Step L14: repeating any of the above steps in any order.

In some embodiments, a method for multiplexed analysis using repeated reporter detection is **Method M** (e.g., Example 27) (which can optionally be modified further by CARD, enzyme deactivation, and/or repeated CARD) comprising:
1. Providing a sample possibly containing one or more targets as well as possibly other molecules that are not targets
2. Optionally fixing the sample
3. Optionally permeabilizing the sample
4. Performing any of steps 5-9 one or more times in any order:
5. Providing one or more HCR probe sets each comprising either: a) one or more HCR initiator-labeled probes, or b) one or more probe units each comprising two or more HCR fractional initiator probes
6. Providing one or more HCR amplifiers that directly or indirectly generate one or more signals
7. Optionally washing the sample
8. Detecting one or more signals
9. Optionally removing one or more signals

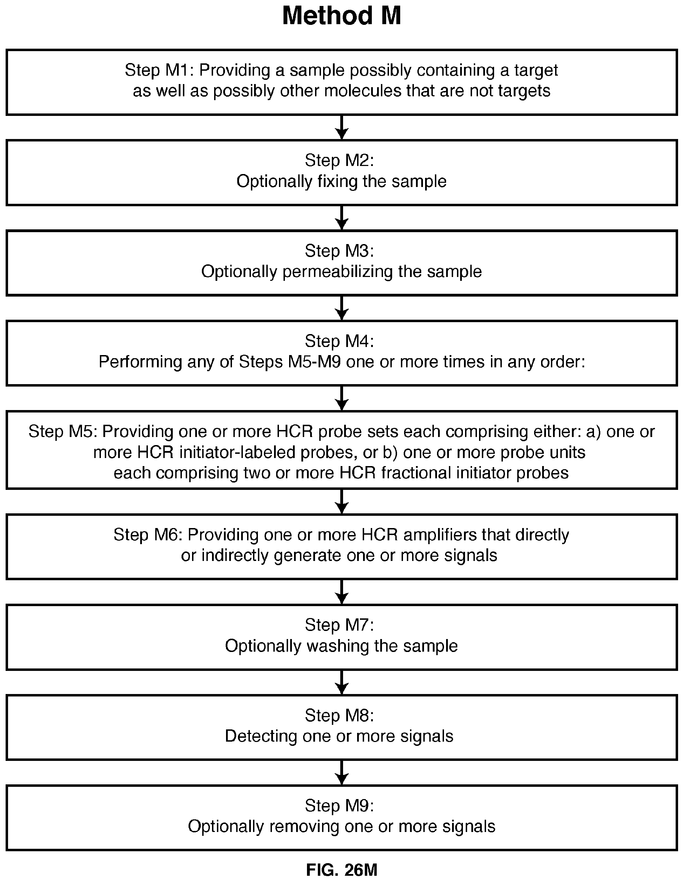

In some embodiments, Method M (e.g., Example 27) comprises (see Figure 26M): Step M1: Providing a sample possibly containing one or more targets as well as possibly other molecules that are not targets; Step M2: Optionally fixing the sample; Step M3: Optionally permeabilizing the sample; Step M4: Performing any of Steps M5-M9 one or more times in any order; Step M5: Providing one or more HCR probe sets each comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17); Step M6: Providing one or more HCR amplifiers that directly or indirectly generate one or more signals (for example, see Figures 8, 18, and 20); Step M7: Optionally washing the sample; Step M8: Detecting one or more signals; Step M9: Optionally removing one or more signals (for example, see Figure 23).

In some embodiments, Method M (e.g., Example 27) comprises (see Figure 26M): Step M1: Providing a sample possibly containing one or more targets as well as possibly other molecules that are not targets; Step M2: fixing the sample; Step M3: permeabilizing the sample; Step M4: Performing any of Steps M5-M9 one or more times in any order; Step M5: Providing one or more HCR probe sets each comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17); Step M6: Providing one or more HCR amplifiers that directly or indirectly generate one or more signals (for example, see Figures 8, 18, and 20); Step M7: washing the sample; Step M8: Detecting one or more signals; Step M9: removing one or more signals (for example, see Figure 23).

In some embodiments, a method for target analysis is **Method N** (e.g., Example 28) (which can optionally be modified further by multiplexing, CARD, enzyme deactivation, repeated CARD, repeated reporter detection, and/or repeated signal removal) comprising:
1. Providing a sample possibly containing a target as well as possibly other molecules that are not targets
2. Optionally fixing the sample
3. Optionally permeabilizing the sample
4. Providing a probe set comprising either: a) one or more HCR initiator-labeled probes, or b) one or more probe units each comprising two or more HCR fractional initiator probes where the target binding regions on the probes within each probe unit are configured to bind to overlapping binding sites on the target
5. Optionally washing the sample
6. Providing an HCR amplifier labeled with a reporter and/or a substrate
7. Optionally washing the sample
8. Optionally providing a label probe (conjugated to a reporter) corresponding to the substrate
9. Optionally washing the sample
10. Detecting a signal from the reporter

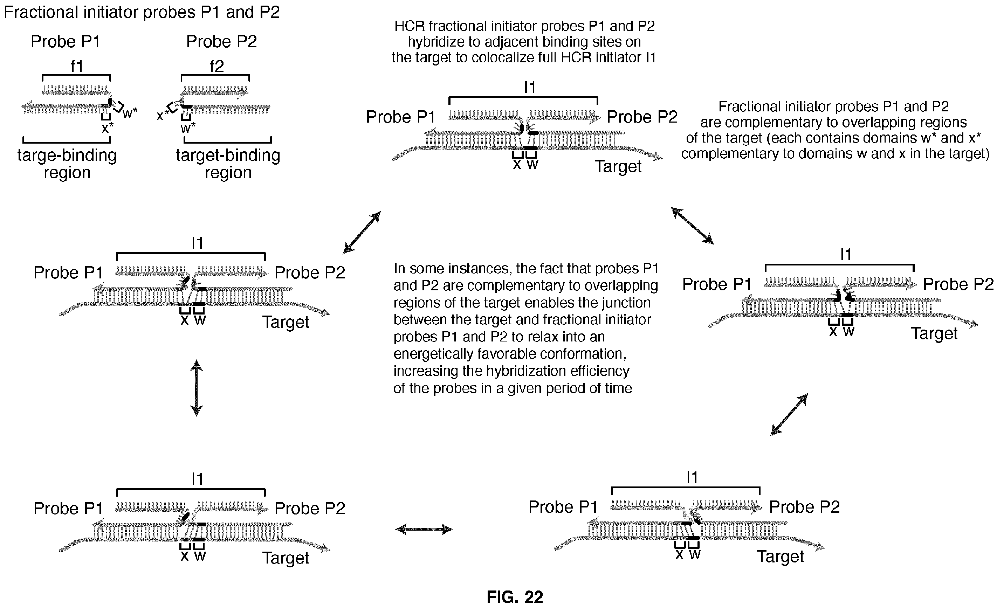

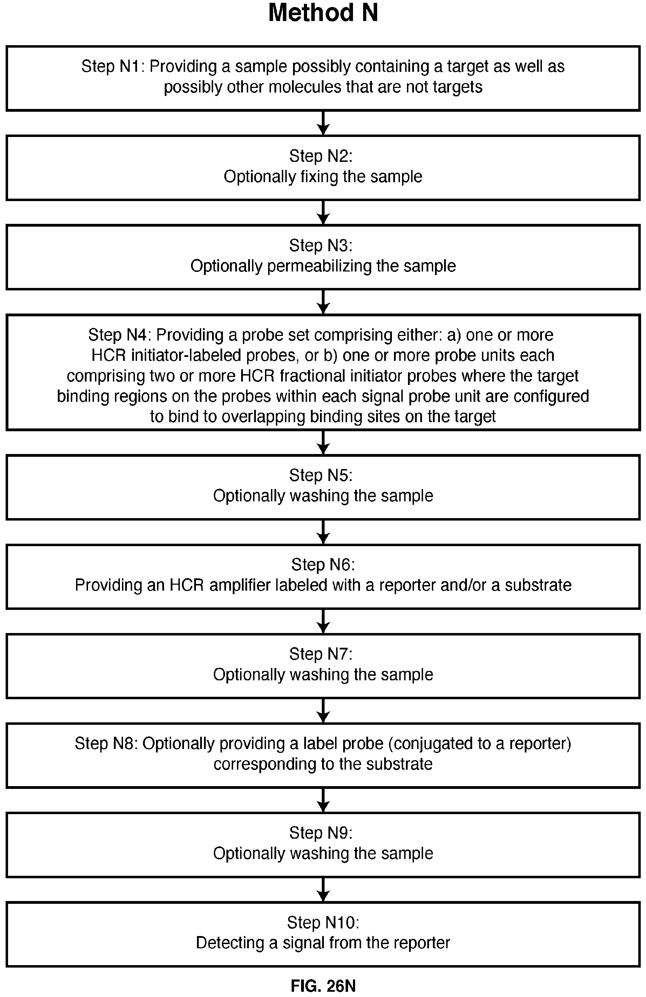

In some embodiments, Method N (e.g., Example 28) comprises (see Figure 26N): Step N1: Providing a sample possibly containing a target as well as possibly other molecules that are not targets; Step N2: Optionally fixing the sample; Step N3: Optionally permeabilizing the sample; Step N4: Providing a probe set comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes where the target-binding regions on the probes within each probe unit are configured to bind to overlapping binding sites on the target (for example, see the probe units of Figure 22); Step N5: Optionally washing the sample; Step N6: Providing an HCR amplifier labeled with a reporter and/or a substrate (for example, see the HCR amplifiers of Figures 8 and 18); Step N7: Optionally washing the sample; Step N8: Optionally providing a label probe (conjugated to a reporter) corresponding to the substrate (for example, see the label probes of Figure 20); Step N9: Optionally washing the sample; Step N10: Detecting a signal from the reporter.

In some embodiments, Method N (e.g., Example 28) comprises (see Figure 26N): Step N1: Providing a sample possibly containing a target as well as possibly other molecules that are not targets; Step N2: fixing the sample; Step N3: permeabilizing the sample; Step N4: Providing a probe set comprising either: a) one or more HCR initiator-labeled probes (for example, see the probes of Figures 39A-39N,41A, 42A-42F, and 43A), or b) one or more probe units (for example, see the probe sets of Figures 8 and 16) each comprising two or more HCR fractional initiator probes where the target-binding regions on the probes within each probe unit are configured to bind to overlapping binding sites on the target (for example, see the probe units of Figure 22); Step N5: washing the sample; Step N6: Providing an HCR amplifier labeled with a reporter and/or a substrate (for example, see the HCR amplifiers of Figures 8 and 18); Step N7: washing the sample; Step N8: providing a label probe (conjugated to a reporter) corresponding to the substrate (for example, see the label probes of Figure 20); Step N9: washing the sample; Step N10: Detecting a signal from the reporter.

In some embodiments, a method for target analysis is **Method O** (e.g., Example 29) (which can optionally be modified further by multiplexing, CARD, enzyme deactivation, repeated CARD, repeated reporter detection, and/or repeated signal removal) comprising:
1. Providing a sample possibly containing a target as well as possibly other molecules that are not targets
2. Optionally fixing the sample
3. Optionally permeabilizing the sample
4. Providing a probe set comprising either: a) one or more HCR initiator-labeled probes, or b) one or more probe units each comprising two or more HCR fractional initiator probes where the fractional initiators on the probes within each probe unit are configured to bind to overlapping binding sites on an HCR hairpin
5. Optionally washing the sample
6. Providing an HCR amplifier labeled with a reporter and/or a substrate
7. Optionally washing the sample
8. Optionally providing a label probe (conjugated to a reporter) corresponding to the substrate
9. Optionally washing the sample
10. Detecting a signal from the reporter

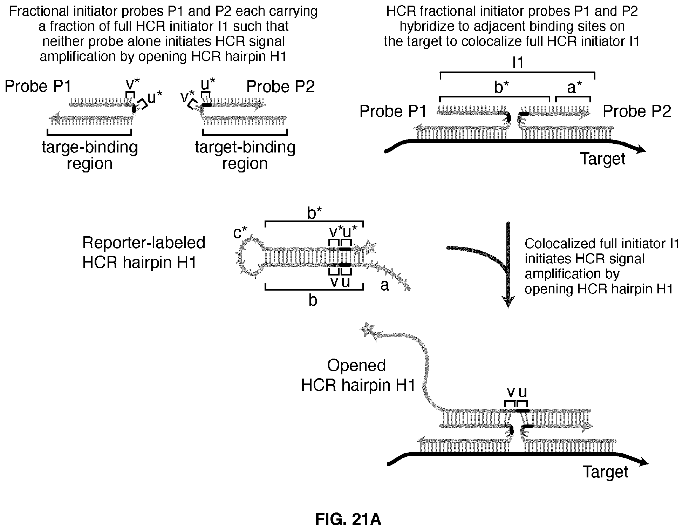

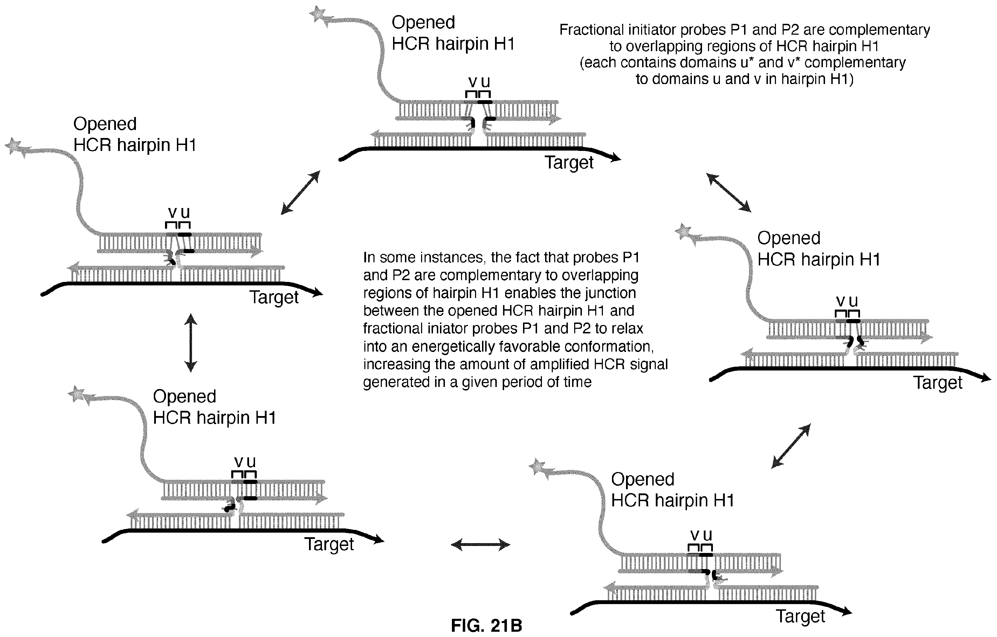

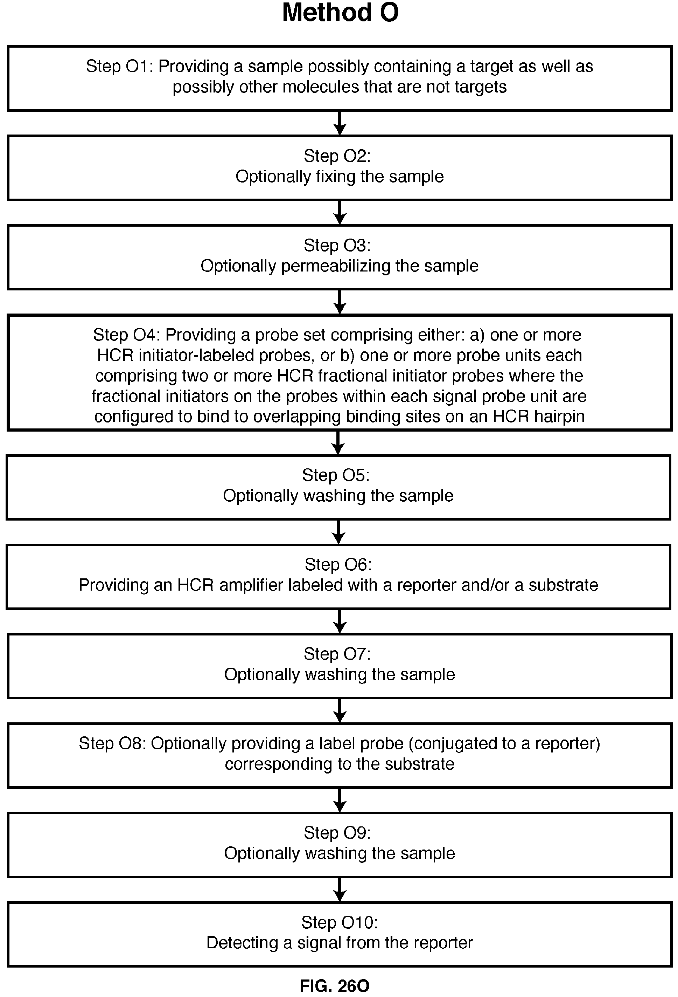

In some embodiments, Method O (e.g., Example 29) comprises (see Figure 26O): Step O1: Providing a sample possibly containing a target as well as possibly other molecules that are not targets; Step O2: Optionally fixing the sample; Step O3: Optionally permeabilizing the sample; Step O4: Providing a probe set comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes where the fractional initiators on the probes within each probe unit are configured to bind to overlapping binding sites on an HCR hairpin (for example, see the probe units of Figure 21); Step O5: Optionally washing the sample; Step O6: Providing an HCR amplifier labeled with a reporter and/or a substrate (for example, see the HCR amplifiers of Figures 8 and 18); Step O7: Optionally washing the sample; Step O8: Optionally providing a label probes (conjugated to a reporter) corresponding to the substrate (for example, see the label probes of Figure 20); Step O9: Optionally washing the sample; Step O10: Detecting a signal from the reporter.

In some embodiments, Method O (e.g., Example 29) comprises (see Figure 26O): Step O1: Providing a sample possibly containing a target as well as possibly other molecules that are not targets; Step O2: fixing the sample; Step O3: permeabilizing the sample; Step O4: Providing a probe set comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes where the fractional initiators on the probes within each probe unit are configured to bind to overlapping binding sites on an HCR hairpin (for example, see the probe units of Figure 21); Step O5: washing the sample; Step O6: Providing an HCR amplifier labeled with a reporter and/or a substrate (for example, see the HCR amplifiers of Figures 8 and 18); Step O7: washing the sample; Step O8: providing a label probes (conjugated to a reporter) corresponding to the substrate (for example, see the label probes of Figure 20); Step O9: washing the sample; Step O10: Detecting a signal from the reporter.

In some embodiments, a method for target analysis is **Method P** (e.g., Example 30) (which can optionally be modified further by multiplexing, CARD, enzyme deactivation, repeated CARD, repeated reporter detection, and/or repeated signal removal) comprising:
1. Providing a sample possibly containing a target as well as possibly other molecules that are not targets
2. Optionally fixing the sample
3. Optionally permeabilizing the sample
4. Providing a probe set comprising either: a) one or more HCR initiator-labeled probes, or b) one or more probe units each comprising two or more HCR fractional initiator probes where the target binding regions on the probes within each probe unit are configured to bind to overlapping binding sites on the target and where the fractional initiators on the probes within each probe unit are configured to bind to overlapping binding sites on an HCR hairpin
5. Optionally washing the sample
6. Providing an HCR amplifier labeled with a reporter and/or a substrate
7. Optionally washing the sample
8. Optionally providing a label probe (conjugated to a reporter) corresponding to the substrate
9. Optionally washing the sample
10. Detecting a signal from the reporter

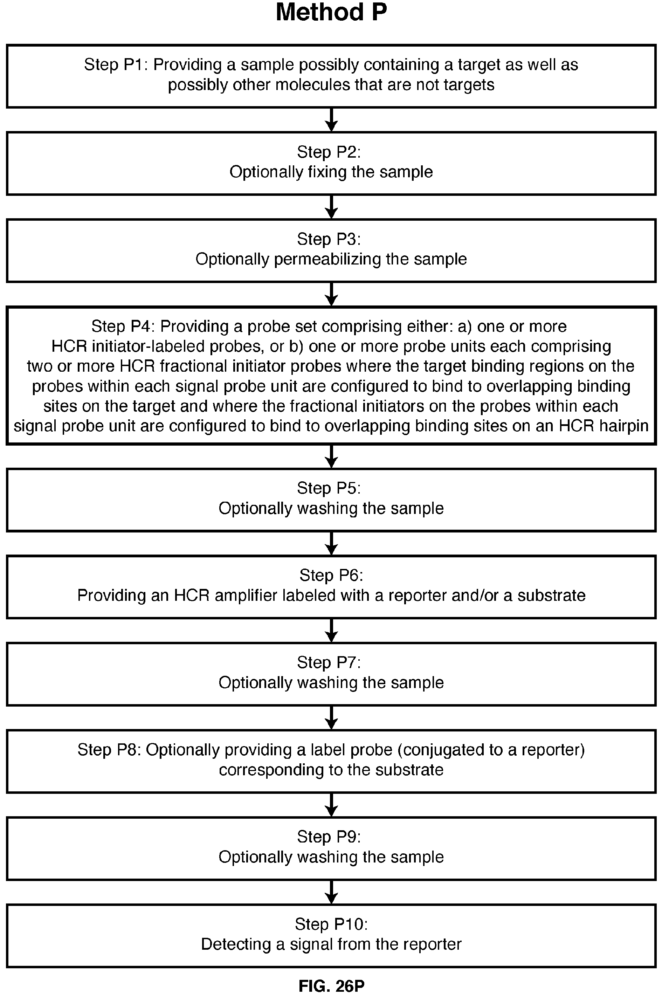

In some embodiments, Method P (e.g., Example 30) comprises (see Figure 26P): Step P1: Providing a sample possibly containing a target as well as possibly other molecules that are not targets; Step P2: Optionally fixing the sample; Step P3: Optionally permeabilizing the sample; Step P4: Providing a probe set comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes where the target binding regions on the probes within each probe unit are configured to bind to overlapping binding sites on the target and where the fractional initiators on the probes within each probe unit are configured to bind to overlapping binding sites on an HCR hairpin (for example, see the probe units of Figure 27); Step P5: Optionally washing the sample; Step P6: Providing an HCR amplifier labeled with a reporter and/or a substrate (for example, see the HCR amplifiers of Figures 8 and 18); Step P7: Optionally washing the sample; Step P8: Optionally providing a label probe (conjugated to a reporter) corresponding to the substrate (for example, see the label probes of Figure 20); Step P9: Optionally washing the sample; Step P10: Detecting a signal from the reporter.

In some embodiments, Method P (e.g., Example 30) comprises (see Figure 26P): Step P1: Providing a sample possibly containing a target as well as possibly other molecules that are not targets; Step P2: fixing the sample; Step P3: permeabilizing the sample; Step P4: Providing a probe set comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes where the target binding regions on the probes within each probe unit are configured to bind to overlapping binding sites on the target and where the fractional initiators on the probes within each probe unit are configured to bind to overlapping binding sites on an HCR hairpin (for example, see the probe units of Figure 27); Step P5: washing the sample; Step P6: Providing an HCR amplifier labeled with a reporter and/or a substrate (for example, see the HCR amplifiers of Figures 8 and 18); Step P7: washing the sample; Step P8: providing a label probe (conjugated to a reporter) corresponding to the substrate (for example, see the label probes of Figure 20); Step P9: washing the sample; Step P10: Detecting a signal from the reporter.

In some embodiments, a method for target analysis is **Method Q** (e.g., Example 31) (which can optionally be modified further by multiplexing, CARD, enzyme deactivation, repeated CARD, repeated reporter detection, and/or repeated signal removal) comprising:
1. Providing a sample possibly containing a target as well as possibly other molecules that are not targets
2. Optionally fixing the sample
3. Optionally permeabilizing the sample
4. Providing a probe set comprising either: a) one or more HCR initiator-labeled probes, or b) one or more probe units each comprising two or more HCR fractional initiator probes where the target binding regions on the probes within each probe unit are configured to bind to non-overlapping binding sites on the target and where the fractional initiators on the probes within each probe unit are configured to bind to overlapping binding sites on an HCR hairpin
5. Optionally washing the sample
6. Providing an HCR amplifier labeled with a reporter and/or a substrate
7. Optionally washing the sample
8. Optionally providing a label probe (conjugated to a reporter) corresponding to the substrate
9. Optionally washing the sample
10. Detecting a signal from the reporter

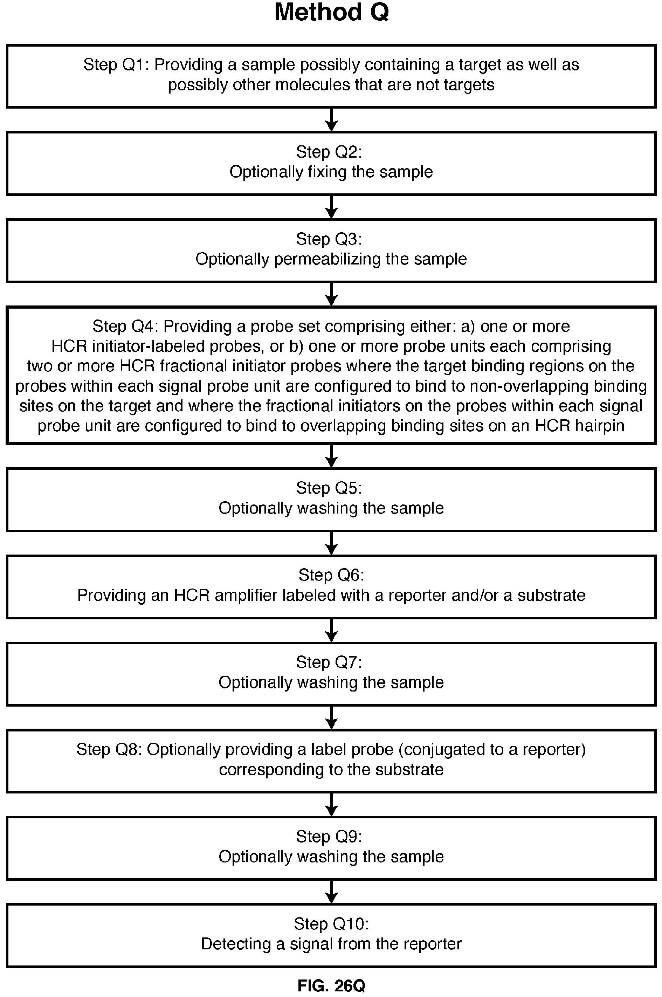

In some embodiments, Method Q (e.g., Example 31) comprises (see Figure 26Q): Step Q1: Providing a sample possibly containing a target as well as possibly other molecules that are not targets; Step Q2: Optionally fixing the sample; Step Q3: Optionally permeabilizing the sample; Step Q4: Providing a probe set comprising either: a) one or more HCR initiator-labeled probes (for example, see the probes of Figures 39A-39N, 41A, 42A-42F, and 43A), or b) one or more probe units each comprising two or more HCR fractional initiator probes where the target binding regions on the probes within each probe unit are configured to bind to non-overlapping binding sites on the target and where the fractional initiators on the probes within each probe unit are configured to bind to overlapping binding sites on an HCR hairpin (for example, see the probe units of Figure 21); Step Q5: Optionally washing the sample; Step Q6: Providing an HCR amplifier labeled with a reporter and/or a substrate (for example, see the HCR amplifiers of Figures 8 and 18); Step Q7: Optionally washing the sample; Step Q8: Optionally providing a label probe (conjugated to a reporter) corresponding to the substrate (for example, see the label probes of Figure 20); Step Q9: Optionally washing the sample; Step Q10: Detecting a signal from the reporter.

In some embodiments, Method Q (e.g., Example 31) comprises (see Figure 26Q): Step Q1: Providing a sample possibly containing a target as well as possibly other molecules that are not targets; Step Q2: fixing the sample; Step Q3: permeabilizing the sample; Step Q4: Providing a probe set comprising either: a) one or more HCR initiator-labeled probes (for example, see the probes of Figures 39A-39N, 41A, 42A-42F, and 43A), or b) one or more probe units each comprising two or more HCR fractional initiator probes where the target binding regions on the probes within each probe unit are configured to bind to non-overlapping binding sites on the target and where the fractional initiators on the probes within each probe unit are configured to bind to overlapping binding sites on an HCR hairpin (for example, see the probe units of Figure 21); Step Q5: washing the sample; Step Q6: Providing an HCR amplifier labeled with a reporter and/or a substrate (for example, see the HCR amplifiers of Figures 8 and 18); Step Q7: washing the sample; Step Q8: providing a label probe (conjugated to a reporter) corresponding to the substrate (for example, see the label probes of Figure 20); Step Q9: washing the sample; Step Q10: Detecting a signal from the reporter.

In some embodiments, a method for target analysis is **Method R** (e.g., Example 32) (which can optionally be modified further by multiplexing, CARD, enzyme deactivation, repeated CARD, repeated reporter detection, and/or repeated signal removal) comprising:
1. Providing a sample possibly containing a target as well as possibly other molecules that are not targets
2. Optionally fixing the sample
3. Optionally permeabilizing the sample
4. Providing a probe set comprising either: a) one or more HCR initiator-labeled probes, or b) one or more probe units each comprising two or more HCR fractional initiator probes where the target binding regions on the probes within each probe unit are configured to bind to overlapping binding sites on the target and where the fractional initiators on the probes within each probe unit are configured to bind to non-overlapping binding sites on an HCR hairpin
5. Optionally washing the sample
6. Providing an HCR amplifier labeled with a reporter and/or a substrate
7. Optionally washing the sample
8. Optionally providing a label probe (conjugated to a reporter) corresponding to the substrate
9. Optionally washing the sample
10. Detecting a signal from the reporter

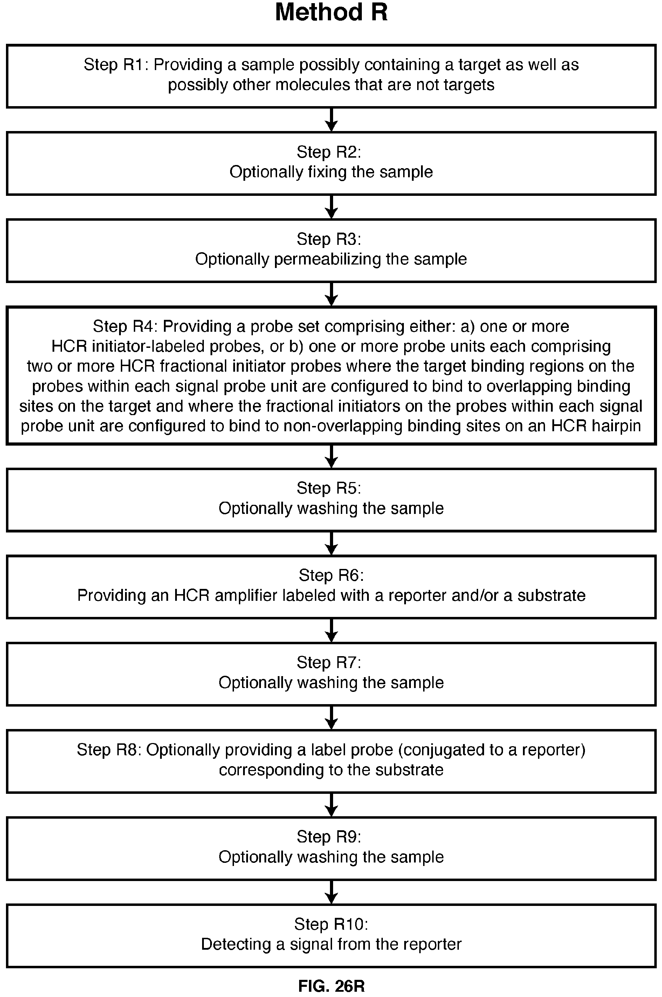

In some embodiments, Method R (e.g., Example 32) comprises (see Figure 26R): Step R1: Providing a sample possibly containing a target as well as possibly other molecules that are not targets; Step R2: Optionally fixing the sample; Step R3: Optionally permeabilizing the sample; Step R4: Providing a probe set comprising either: a) one or more HCR initiator-labeled probes (for example, see the probes of Figures 39A-39N, 41A, 42A-42F, and 43A), or b) one or more probe units each comprising two or more HCR fractional initiator probes where the target binding regions on the probes within each probe unit are configured to bind to overlapping binding sites on the target and where the fractional initiators on the probes within each probe unit are configured to bind to non-overlapping binding sites on an HCR hairpin (for example, see the probe units of Figure 22); Step R5: Optionally washing the sample; Step R6: Providing an HCR amplifier labeled with a reporter and/or a substrate (for example, see the HCR amplifiers of Figures 8 and 18); Step R7: Optionally washing the sample; Step R8: Optionally providing a label probe (conjugated to a reporter) corresponding to the substrate (for example, see the label probes of Figure 20); Step R9: Optionally washing the sample; Step R10: Detecting a signal from the reporter.

In some embodiments, Method R (e.g., Example 32) comprises (see Figure 26R): Step R1: Providing a sample possibly containing a target as well as possibly other molecules that are not targets; Step R2: fixing the sample; Step R3: permeabilizing the sample; Step R4: Providing a probe set comprising either: a) one or more HCR initiator-labeled probes (for example, see the probes of Figures 39A-39N, 41A, 42A-42F, and 43A), or b) one or more probe units each comprising two or more HCR fractional initiator probes where the target binding regions on the probes within each probe unit are configured to bind to overlapping binding sites on the target and where the fractional initiators on the probes within each probe unit are configured to bind to non-overlapping binding sites on an HCR hairpin (for example, see the probe units of Figure 22); Step R5: washing the sample; Step R6: Providing an HCR amplifier labeled with a reporter and/or a substrate (for example, see the HCR amplifiers of Figures 8 and 18); Step R7: washing the sample; Step R8: providing a label probe (conjugated to a reporter) corresponding to the substrate (for example, see the label probes of Figure 20); Step R9: washing the sample; Step R10: Detecting a signal from the reporter.

In some embodiments, a method for target analysis is **Method S** (e.g., Example 33) (which can optionally be modified further by multiplexing, CARD, enzyme deactivation, repeated CARD, repeated reporter detection, and/or repeated signal removal) comprising:
1. Providing a sample possibly containing a target as well as possibly other molecules that are not targets
2. Optionally fixing the sample
3. Optionally permeabilizing the sample
4. Providing a probe set comprising either: a) one or more HCR initiator-labeled probes, or b) one or more probe units each comprising two or more HCR fractional initiator probes where the target binding regions on the probes within each probe unit are configured to bind to non-overlapping binding sites on the target and where the fractional initiators on the probes within each probe unit are configured to bind to non-overlapping binding sites on an HCR hairpin
5. Optionally washing the sample
6. Providing an HCR amplifier labeled with a reporter and/or a substrate
7. Optionally washing the sample
8. Optionally providing a label probe (conjugated to a reporter) corresponding to the substrate
9. Optionally washing the sample
10. Detecting a signal from the reporter

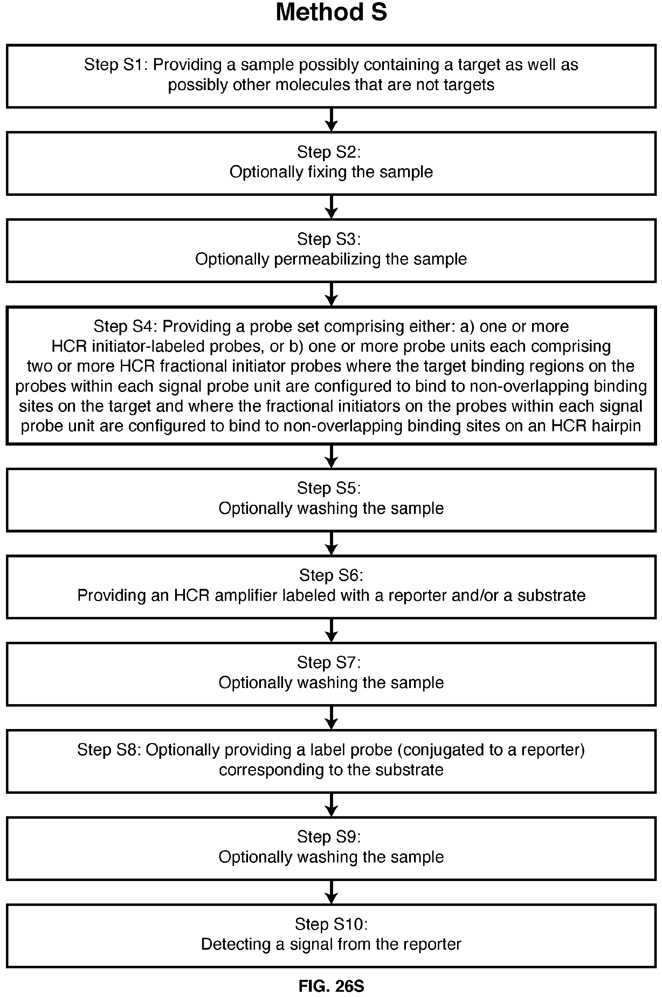

In some embodiments, Method S (e.g., Example 33) comprises (see Figure 26S): Step S1: Providing a sample possibly containing a target as well as possibly other molecules that are not targets; Step2: Optionally fixing the sample; Step S3: Optionally permeabilizing the sample; Step S4: Providing a probe set comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes where the target binding regions on the probes within each probe unit are configured to bind to non-overlapping binding sites on the target and where the fractional initiators on the probes within each probe unit are configured to bind to non-overlapping binding sites on an HCR hairpin (for example, see the probe units of Figures 3, 4, 5, 17); Step S5: Optionally washing the sample; Step S6: Providing an HCR amplifier labeled with a reporter and/or a substrate (for example, see the HCR amplifiers of Figures 8 and 18); Step S7: Optionally washing the sample; Step S8: Optionally providing a label probe (conjugated to a reporter) corresponding to the substrate (for example, see the label probes of Figure 20); Step S9: Optionally washing the sample; Step S10: Detecting a signal from the reporter.

In some embodiments, Method S (e.g., Example 33) comprises (see Figure 26S): Step S1: Providing a sample possibly containing a target as well as possibly other molecules that are not targets; Step2: fixing the sample; Step S3: permeabilizing the sample; Step S4: Providing a probe set comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes where the target binding regions on the probes within each probe unit are configured to bind to non-overlapping binding sites on the target and where the fractional initiators on the probes within each probe unit are configured to bind to non-overlapping binding sites on an HCR hairpin (for example, see the probe units of Figures 3, 4, 5, 17); Step S5: washing the sample; Step S6: Providing an HCR amplifier labeled with a reporter and/or a substrate (for example, see the HCR amplifiers of Figures 8 and 18); Step S7: washing the sample; Step S8: providing a label probe (conjugated to a reporter) corresponding to the substrate (for example, see the label probes of Figure 20); Step S9: washing the sample; Step S10: Detecting a signal from the reporter.

In some embodiments, a method for multiplexed analysis using repeated reporter detection is **Method T** (e.g., Example 34) (which can optionally be modified further by CARD, enzyme deactivation, and/or repeated CARD) comprising:
1. Providing a sample possibly containing one or more targets as well as possibly other molecules that are not targets
2. Optionally fixing the sample
3. Optionally permeabilizing the sample
4. Providing one or more probe sets each comprising either: a) one or more HCR initiator-labeled probes, or b) one or more probe units each comprising two or more HCR fractional initiator probes where the target binding regions on the probes within each probe unit are configured to bind to overlapping or non-overlapping binding sites on a target and where the fractional initiators on the probes within each probe unit are configured to bind to overlapping or non-overlapping binding sites on an HCR hairpin
5. Optionally washing the sample
6. Providing one or more HCR amplifiers each labeled with one or more reporters and/or substrates
7. Optionally washing the sample
8. Optionally providing one or more label probes (each conjugated to one or more reporters) corresponding to one or more substrates
9. Optionally washing the sample
10. Detecting a signal from one or more reporters
11. Optionally removing one or more signals from the sample
12. Optionally removing one or more reporters from the sample
13. Optionally removing one or more label probes from the sample
14. Optionally removing one or more amplifiers from the sample
15. Optionally removing one or more probe sets from the sample
16. Optionally repeating any of the above steps in any order

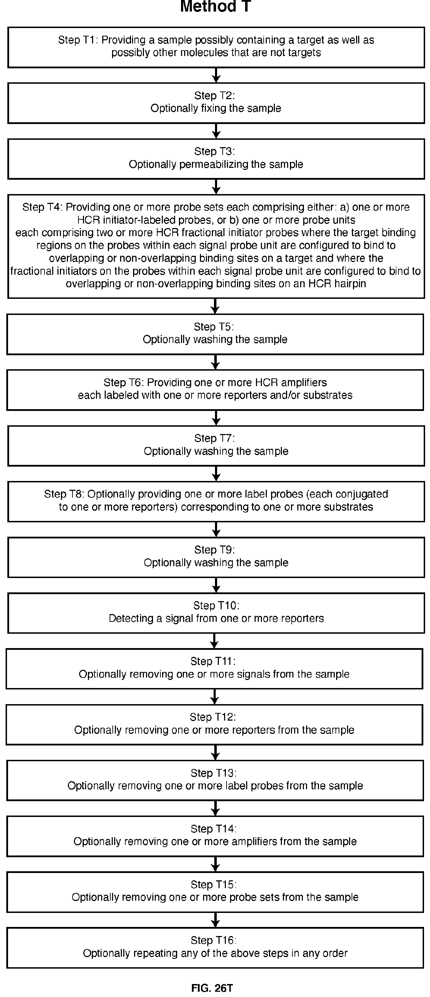

In some embodiments, Method T (e.g., Example 34) comprises (see Figure 26T): Step T1: Providing a sample possibly containing one or more targets as well as possibly other molecules that are not targets; Step T2: Optionally fixing the sample; Step T3: Optionally permeabilizing the sample; Step T4: Providing one or more probe sets each comprising either: a) one or more HCR initiator-labeled probes (for example, see the probes of Figures 39A-39N, 41A, 42A-42F, and 43A), or b) one or more probe units each comprising two or more HCR fractional initiator probes where the target binding regions on the probes within each probe unit are configured to bind to overlapping or non-overlapping binding sites on a target and where the fractional initiators on the probes within each probe unit are configured to bind to overlapping or non-overlapping binding sites on an HCR hairpin (for example, see the probe units of Figure 3, 4, 5, 17, 21, 22, 27); Step T5: Optionally washing the sample; Step T6: Providing one or more HCR amplifiers each labeled with one or more reporters and/or substrates (for example, see the HCR amplifiers of Figures 8 and 18); Step T7: Optionally washing the sample; Step T8: Optionally providing one or more label probes (each conjugated to one or more reporters) corresponding to one or more substrates (for example, see the label probes of Figure 20); Step T9: Optionally washing the sample; Step T10: Detecting a signal from one or more reporters; Step T11: Optionally removing one or more signals from the sample (for example, see Figure 23); Step T12: Optionally removing one or more reporters from the sample; Step T13: Optionally removing one or more label probes from the sample; Step T14: Optionally removing one or more amplifiers from the sample; Step T15: Optionally removing one or more probe sets from the sample; Step T16: Optionally repeating any of the above steps in any order.

In some embodiments, Method T (e.g., Example 34) comprises (see Figure 26T): Step T1: Providing a sample possibly containing one or more targets as well as possibly other molecules that are not targets; Step T2: fixing the sample; Step T3: permeabilizing the sample; Step T4: Providing one or more probe sets each comprising either: a) one or more HCR initiator-labeled probes (for example, see the probes of Figures 39A-39N, 41A, 42A-42F, and 43A), or b) one or more probe units each comprising two or more HCR fractional initiator probes where the target binding regions on the probes within each probe unit are configured to bind to overlapping or non-overlapping binding sites on a target and where the fractional initiators on the probes within each probe unit are configured to bind to overlapping or non-overlapping binding sites on an HCR hairpin (for example, see the probe units of Figure 3, 4, 5, 17, 21, 22, 27); Step T5: washing the sample; Step T6: Providing one or more HCR amplifiers each labeled with one or more reporters and/or substrates (for example, see the HCR amplifiers of Figures 8 and 18); Step T7: washing the sample; Step T8: providing one or more label probes (each conjugated to one or more reporters) corresponding to one or more substrates (for example, see the label probes of Figure 20); Step T9: washing the sample; Step T10: Detecting a signal from one or more reporters; Step T11: removing one or more signals from the sample (for example, see Figure 23); Step T12: removing one or more reporters from the sample; Step T13: removing one or more label probes from the sample; Step T14: removing one or more amplifiers from the sample; Step T15: removing one or more probe sets from the sample; Step T16: Optionally repeating any of the above steps in any order.

**Multiplexing using single-molecule barcoding.** In some embodiments, the number of targets that can be analyzed in a sample can be increased by analyzing each target molecule in multiple analysis rounds such that the labels used for different target types in different analysis rounds are varied so as to create a distinct barcode for each species of target molecule. The barcode for a given target molecule is then read out as a barcode of signal measurements. For example, using single-molecule imaging to read out the signal of each target molecule as a diffraction-limited dot, consider 3 rounds of imaging. For each given target type, consider assigning a probe set comprising one or more probe units such that each probe unit in a probe set colocalizes a full HCR initiator corresponding to an HCR amplifier comprising HCR hairpins labeled with either red or green reporters depending on the target type and the round of imaging. Then, for example, a target molecule of type 1 can be read out with barcode (red, red, green; denoting a red dot for round 1 using an HCR amplifier labeled with red reporters, a red dot for round 2 using an HCR amplifier labeled with red reporters, and a green dot for round 3 using an HCR amplifier labeled with green rerporters), a target molecule of type 2 can have barcode (red, green, red), a target molecule of type 3 can have barcode (red, red, red), a target molecule of type 4 can have barcode (green, red, green), and so on.

In some embodiments, the number of targets that can be analyzed in a sample can be increased by detecting each target molecule in only a fraction of the barcoding rounds. For example, in an experiment with 4 rounds, a target molecule of type 1 can be read out with barcode (red, ---, ---, red; denoting a red dot for round 1, no dot for round 2, no dot for round 3, a red dot for round 4), a target molecule of type 2 can be read out with barcode (green, red, ---, ---), and so on.

### Additional Embodiments

Any of the embodiments and/or methods provided herein can be employed with, or in the alternative form of, any of the following. Thus, for example, the above noted methods can employ any of the compositions or methods noted below. Similarly, the above noted methods should be understood to also provide methods employing the methods below or as being part of the methods noted below.

Similarly, the embodiments and/or methods provided herein should also be understood to provide embodiments involved in the method, e.g., compositions, components of the method, kits, etc. In some embodiments, any of the ingredients in one or more of the methods and/or steps provided herein can be provided as a kit including one or more of the noted ingredients (and optionally the target or target sequence or sample).

### Compositions

Some embodiments of compositions are outlined in FIGs. 12 and 13, as well as other figures provided herein. In some embodiments, a composition is provided that comprises a first fractional initiator probe (1190) that comprises a first fractional initiator (1151), and a second fractional initiator probe (1290) that comprises a second fractional initiator (1251). In some embodiments, the first and second fractional initiators (1151, 1251) together form a full initiator (1050), from which HCR can progress, via first and second hairpin monomers (1510, 1610). In some embodiments, the first fractional initiator probe (1190) further comprises a first target binding section (1141) and the second fractional initiator probe (1290) further comprises a second target binding section (1241), wherein the first target binding section (1141) is configured to bind to a first target section (1100) and the second target binding section (1241) is configured to bind to a second target section (1200). These target binding sections are located effectively adjacent on the target molecule, such that when both fractional initiator probes are bound to both targets, the first and second fractional initiators (within the fractional initiator probes) are close enough to form a full initiator (1050), from which HCR can occur. In some embodiments, these fractional initiator probes can be provided as a kit, along with hairpin monomers for HCR polymerization.

In some embodiments, a composition is provided comprising a first hairpin monomer (1510), a second hairpin monomer (1610), a first fractional initiator probe (1190) comprising a first fractional initiator (1151), and a second fractional initiator probe (1290) comprising a second fractional initiator (1251). In some embodiments, the first and second fractional initiators (1151, 1251) together form a full initiator (1050), from which HCR can progress, via the first and second hairpin monomers (1510, 1610).

In some embodiments, the fractional initiator probes and the hairpin monomers can be introduced to the same sample at the same time. In some embodiments, the fractional initiator probes can be introduced to the sample, followed by a wash, and then the hairpin monomers can be introduced to the sample, followed by a wash. In some embodiments, the fractional initiator probes and hairpin monomers can be introduced to the sample at different times.

In some embodiments, one or more of the hairpin monomers can include a reporter molecule, such that polymerization of the hairpin monomers (first and second, and optionally more), will result in a signaling event that is detectable. In some embodiments, the reporter molecule can be covalently associated with the hairpin monomer(s). In some embodiments, the reporter molecule can be subsequently bound to the HCR polymer after polymerization (e.g., in a subsequent hybridization event). In some embodiments, the first hairpin monomer (1510) comprises a label-binding site (not depicted) that is configured to hybridize to a complement to the label-binding site (not depicted). In some embodiments, the complement to the label-binding site further comprises a reporter molecule.

In some embodiments, a composition is provided that includes a first hairpin monomer (1510), comprising: a) a first input domain (1852), comprising a first toehold (1851) and a first stem section (1755), b) a first output domain (1854), comprising a first hairpin loop (1853) and a complement to the first stem section (1756), and c) a first reporter molecule (1850). The composition can further include a second hairpin monomer (1610), comprising: a) a second input domain (1952), comprising a second toehold (1951) and a second stem section (1855), b) a second output domain (1954), comprising a second hairpin loop (1953) and a complement to the second stem section (1856), and c) a second reporter molecule (1950). The composition can further include a) a first fractional initiator probe (1190) comprising a first fractional initiator (1151), and b) a second fractional initiator probe (1290) comprising a second fractional initiator (1251). As noted above, the first and second hairpin monomers can be introduced to the sample together with or separately from the first and second fractional initiator probes. In some embodiments, the monomers can be provided together, but separate from the first and second fractional initiator probes. In some embodiments, the hairpin monomers and the fractional initiator probes can be introduced to the same sample at the same time. In some embodiments, the hairpin monomers and the fractional initiator probes can be introduced to the same sample, but at different, non-overlapping times (with a wash to remove unbound molecules).

In some embodiments, the first stem section has the same sequence as the second stem section. In some embodiments, the complement to the first stem section has the same sequence as the complement to the second stem section. In some embodiments, the complement to the first stem section has the same sequence as the complement to the second stem section, and the first stem section has the same sequence as the second stem section. In some embodiments, the toehold sequence of the two hairpin monomers is the same and the loop sequence of the two hairpin monomers is the same (although the polarity of the two hairpin monomers is reversed, so the two hairpin monomers are not identical).

In some embodiments, the first toehold (1851) is complementary to the second hairpin loop. In some embodiments, the second toehold is complementary to the first hairpin loop. In some embodiments, this circularity allows for the hybridization chain reaction to occur. In some embodiments, the first toehold is not 100% complementary to the second hairpin loop, but is sufficient to allow for hybridization.

In some embodiments, more than two different input domains can be employed, for example, 3, 4, 5, 6, 7, 8, 9, 10, 100, 1000, 2000, 4000 or more input domains can be employed. In some embodiments, a corresponding number of subparts can be used for each input domain.

In some embodiments, any of the compositions described herein comprise a target molecule (1020) that comprises a first target section (1100) and a second target section (1200) (as shown, for example, in FIG. 12). In some embodiments, the target molecule comprises additional target sections, for example, three target sections, four target sections, five target sections, six target sections, seven target sections or more (e.g., 10, 50, 100, etc.). In some embodiments, the number of target sections will mean there are a corresponding number of target-binding sections (e.g., 1141, 1241). In some embodiments, this allows for greater specificity/selectivity for the initial formation of the full initiator (as it requires more target binding sections to bind to the target sections). In some embodiments, this allows for the parallel assaying of more than one target sequence at a time (thus allowing one to assay for multiple targets at once, each one involving two or more target binding sections/fractional initiator probes).

In some embodiments, any of the first fractional initiator probes (1190) described herein further comprises a first target binding section (1141) and any of the second fractional initiator probes (1290) described herein further comprises a second target binding section (1241). In some embodiments, the first target binding section (1141) is configured to bind to the first target section (1100). In some embodiments, the second target binding section (1241) is configured to bind to the second target section (1200). In some embodiments, the first and second fractional initiator probes comprise additional target binding sections, for example, two target binding sections, three target binding sections, four target binding sections, five target binding sections, 10, 20, 30, 40, 50, 100, 1000, 10,000, or more.

In some embodiments, the first target binding section is configured to bind to the first target section through selective protein-protein interactions (such as via an antibody as the first target-binding section). In some embodiments, the first target binding section is configured to bind to the first target section through selective nucleic acid-protein interactions (such as an aptamer as the first target-binding section). In some embodiments, the second target binding section is configured to bind to the second target section through selective protein-protein interactions (such as via an antibody as the second target-binding section). In some embodiments, the second target binding section is configured to bind to the second target section through selective nucleic acid-protein interactions (such as an aptamer as the second target-binding section). In some embodiments, the first target binding section is configured to bind to the first target section through hybridization. In some embodiments, the second target binding section is configured to bind to the second target section through hybridization. In some embodiments, the first target binding section is configured to bind to the first target section through covalent bonding or ionic bonding. In some embodiments, the second target binding section is configured to bind to the second target section through covalent or ionic bonding.

In some embodiments, any reporter molecule whose presence or absence can be monitored can be employed. In some embodiments, the reporter molecule comprises a fluorescent molecule such as a fluorophore, or a colorimetric compound, that allows the resulting polymers to be visualized. In some embodiments, the reporter molecule is directly observable. In some embodiments, the reporter molecule is indirectly observable. In some embodiments, the reporter molecule comprises an enzyme or is enzymatic, and/or can mediate enzymatic signaling after HCR polymerization. In some embodiments, reporting is achieved by catalyzed reporter deposition ("CARD"). In some embodiments, a label binding site on each hairpin monomer can provide binding of a complement to the label binding site, wherein the complement to the label binding site carries a reporter molecule. In some embodiments, one type of reporter molecule carried by the hairpin monomers or the complement to the label binding site can mediate enzymatic signal amplification (CARD) after HCR polymerization such that a second type of reporter molecules deposited in the vicinity of HCR polymers/target molecules will then be detected. In some embodiments, the reporter molecule is at least one of a luminescent molecule, FRET molecules, fluorophore/quencher molecular pairs, or other detectable markers. In some embodiments, the reporter molecule can allow for a secondary molecule (such as a secondary antibody) to be employed for detection of the polymerization event. In some embodiments, the hairpin monomers can be labeled with reporter molecules (e.g., a fluorophore and a quencher) such that hairpin monomers are quenched but that the conformation change that occurs during HCR polymerization leads to fluorescent HCR amplification polymers.

In some embodiments, any of the ingredients in one or more of the methods and/or steps provided herein can be provided as a composition including one or more of the noted ingredients (and optionally the target or target sequence or sample). In some embodiments, any one or more of the molecules or combination of molecules in any one or more of FIGs. 1A, 1B, 2A, 2B, 3A, 3B, 4A, 4B, 4C, 4D, 5A, 5B, 5C, 5D, 5E, 8A, 8B, 12, 13, 14, 15, 16A, 16B, 16C, 16D, 17A, 17B, 17C, 18A, 18B, 18C, 18D, 18E, 18F, 19A, 19B, 20A, 20B, 20C, 20D, 20E, 20F, 21A, 21B, 22, 23A, 23B, 23C, 23D, 23E, 23F, 23G, 23H, 23I, 23J, 23K, 23L, 23M, 23N, 23O, 27, 28A, 29A, 29B, 30A, 30B, 31A, 32A, 33A, 33B, 33C, 33D, 33E, 34A, 34B, 34C, 35, 36A, 36B, 37A, 38, 39A, 39B, 39C, 39D, 39E, 39F, 39G, 39H, 39I, 39J, 39K, 39L, 39M, 39N, 40A, 40B, 40C, 40D, 40E, 40F, 40G, 40H, 40I, 40J, 40K, 40L, 40M, 40N, 41A, 42A, 42B, 42C, 42D, 42E, 42F, 43A, 44A, 44B, 44C, 44D, 44E, 44F, 44G, 44H, 44I, 44J, 44K, 44L, 44M, 44N, 44O, 44P, 44Q, 44R, 44S, 44T, 44U, 44V, 44W, 44X, 44Y, 44Z, 46A, 46B, 46C, 46D, 46E, 46F, 46G, 46H, 46I, 46J, 46K, 46L, and/or 46M envisioned as their denoted structural components as a composition. In some embodiments, the composition or components are those added to the sample. In some embodiments, the compositions or components are those added at each step of the process as denoted in the figures. In some embodiments, the compositions or components are those added initially in the reaction and/or those added at any reaction step denoted in the figures. In some embodiments, the composition is one that includes hybridization of a molecule to a target, as denoted in any one of FIGs. 1A, 1B, 2A, 2B, 3A, 3B, 4A, 4B, 4C, 4D, 5A, 5B, 5C, 5D, 5E, 8A, 8B, 12, 13, 14, 15, 16A, 16B, 16C, 16D, 17A, 17B, 17C, 18A, 18B, 18C, 18D, 18E, 18F, 19A, 19B, 20A, 20B, 20C, 20D, 20E, 20F, 21A, 21B, 22, 23A, 23B, 23C, 23D, 23E, 23F, 23G, 23H, 23I, 23J, 23K, 23L, 23M, 23N, 23O, 27, 28A, 29A, 29B, 30A, 30B, 31A, 32A, 33A, 33B, 33C, 33D, 33E, 34A, 34B, 34C, 35, 36A, 36B, 37A, 38, 39A, 39B, 39C, 39D, 39E, 39F, 39G, 39H, 39I, 39J, 39K, 39L, 39M, 39N, 40A, 40B, 40C, 40D, 40E, 40F, 40G, 40H, 40I, 40J, 40K, 40L, 40M, 40N, 41A, 42A, 42B, 42C, 42D, 42E, 42F, 43A, 44A, 44B, 44C, 44D, 44E, 44F, 44G, 44H, 44I, 44J, 44K, 44L, 44M, 44N, 44O, 44P, 44Q, 44R, 44S, 44T, 44U, 44V, 44W, 44X, 44Y, 44Z, 46A, 46B, 46C, 46D, 46E, 46F, 46G, 46H, 46I, 46J, 46K, 46L, and/or 46M. In some embodiments, the compositions or components are those in the last step of the process or protocol, as denoted in any one of FIGs. 1A, 1B, 2A, 2B, 3A, 3B, 4A, 4B, 4C, 4D, 5A, 5B, 5C, 5D, 5E, 8A, 8B, 12, 13, 14, 15, 16A, 16B, 16C, 16D, 17A, 17B, 17C, 18A, 18B, 18C, 18D, 18E, 18F, 19A, 19B, 20A, 20B, 20C, 20D, 20E, 20F, 21A, 21B, 22, 23A, 23B, 23C, 23D, 23E, 23F, 23G, 23H, 23I, 23J, 23K, 23L, 23M, 23N, 23O, 27, 28A, 29A, 29B, 30A, 30B, 31A, 32A, 33A, 33B, 33C, 33D, 33E, 34A, 34B, 34C, 35, 36A, 36B, 37A, 38, 39A, 39B, 39C, 39D, 39E, 39F, 39G, 39H, 39I, 39J, 39K, 39L, 39M, 39N, 40A, 40B, 40C, 40D, 40E, 40F, 40G, 40H, 40I, 40J, 40K, 40L, 40M, 40N, 41A, 42A, 42B, 42C, 42D, 42E, 42F, 43A, 44A, 44B, 44C, 44D, 44E, 44F, 44G, 44H, 44I, 44J, 44K, 44L, 44M, 44N, 44O, 44P, 44Q, 44R, 44S, 44T, 44U, 44V, 44W, 44X, 44Y, 44Z, 46A, 46B, 46C, 46D, 46E, 46F, 46G, 46H, 46I, 46J, 46K, 46L, and/or 46M. In some embodiments, the compositions or components are those with label attached or associated with a target as denoted in any one of the appropriate (those having label attached or associated with a target) FIGs. 1A, 1B, 2A, 2B, 3A, 3B, 4A, 4B, 4C, 4D, 5A, 5B, 5C, 5D, 5E, 8A, 8B, 12, 13, 14, 15, 16A, 16B, 16C, 16D, 17A, 17B, 17C, 18A, 18B, 18C, 18D, 18E, 18F, 19A, 19B, 20A, 20B, 20C, 20D, 20E, 20F, 21A, 21B, 22, 23A, 23B, 23C, 23D, 23E, 23F, 23G, 23H, 23I, 23J, 23K, 23L, 23M, 23N, 23O, 27, 28A, 29A, 29B, 30A, 30B, 31A, 32A, 33A, 33B, 33C, 33D, 33E, 34A, 34B, 34C, 35, 36A, 36B, 37A, 38, 39A, 39B, 39C, 39D, 39E, 39F, 39G, 39H, 39I, 39J, 39K, 39L, 39M, 39N, 40A, 40B, 40C, 40D, 40E, 40F, 40G, 40H, 40I, 40J, 40K, 40L, 40M, 40N, 41A, 42A, 42B, 42C, 42D, 42E, 42F, 43A, 44A, 44B, 44C, 44D, 44E, 44F, 44G, 44H, 44I, 44J, 44K, 44L, 44M, 44N, 44O, 44P, 44Q, 44R, 44S, 44T, 44U, 44V, 44W, 44X, 44Y, 44Z, 46A, 46B, 46C, 46D, 46E, 46F, 46G, 46H, 46I, 46J, 46K, 46L, and/or 46M.

### Methods

In some embodiments a method is provided. The method comprises (i) providing a first fractional initiator probe, (1190) a second fractional initiator probe (1290), a first hairpin monomer (1510), a second hairpin monomer (1610), and a target molecule (1020) (ii) incubating to allow for binding, and (iii) detecting a signal.

In some embodiments, a method of performing HCR is provided. The method comprises (i) adding a first fractional initiator (1151) and a second fractional initiator (1251) to a sample, wherein together the first fractional initiator (1151) and the second fractional initiator (1251) provide a full HCR initiator and (ii) adding a set of HCR hairpin monomers to the sample so as to allow HCR to occur in the presence of the full HCR initiator. The set of HCR hairpin monomers are configured so as to polymerize via HCR.

In some embodiments, a method is provided. The method comprises (a) providing: I. a first fractional initiator probe (1190) comprising a first fractional initiator (1151), II. a second fractional initiator probe (1290) comprising a second fractional initiator (1251). The method can further comprise providing III. a first hairpin monomer (1510), comprising: a. a first input domain (1852), comprising a first toehold (1851) and a first stem section (1755), b. a first output domain (1854), comprising a first hairpin loop (1853) and a complement to the first stem section (1756), and c. a first reporter molecule (1850). Further provided is IV. a second hairpin monomer (1610), comprising: a. a second input domain (1952), comprising a second toehold (1951) and a second stem section (1855), b. a second output domain (1954), comprising a second hairpin loop (1953) and a complement to the second stem section (1856), and c. a second reporter molecule (1950). Further provided is V. a target molecule (1020). The method further comprises (b) incubating the provided first fractional initiator probe and the second fractional initiator probe with a target. As noted herein, the fractional initiator probes can further include target binding sections, so as to colocalize two fractional initiators to thereby form the full initiator.

In some embodiments, the fractional initiator probes can be added initially to the sample that may include a target and then the bulk solution washed away, keeping the bound fractional initiator probes, and then the hairpin monomers can be added so that fractional initiator probes that are specifically bound to the target will colocalize fractional initiators and trigger HCR, but individual fractional initiator probes that are bound non-specifically will not colocalize a full initiator and will not trigger HCR.

In any of the methods described herein, any one or more of the following can be detected and/or assayed for: molecules, DNA molecules, RNA molecules, protein molecules, small molecules, synthetic molecules, or complexes of molecules. In some embodiments, the target is more than one target, such as a complex of proteins, or a complex of a protein and a nucleic acid, etc. Thus, the association of proteins can be assayed by the present fractional initiator approach. In some embodiments, inorganic or non-organic materials can also be assayed for. In some embodiments, any target can be detected, as long as there is a corresponding target binding section that can bind to the target that can be made part of the fractional initiator probe. In some embodiments, the target is any nucleic acid molecule. In some embodiments, the target is a protein. In some embodiments, the target consists of at least one of: mRNA, miRNA, lncRNA, rRNA, non-coding RNA, or genomic DNA. In some embodiments, the target is comprised of an amino acid sequence. In some embodiments, the target is comprised of a complex of molecules. In some embodiments, the target is at least one of: DNA, RNA, protein, or small molecule target molecules or complexes in vitro, in situ, or in vivo. In some embodiments, the target is a complex of molecules that is made up of at least one of: DNA, RNA, protein, or small molecule target molecules. In some embodiments, the target comprises a molecule or complex in vitro, in situ, or in vivo.

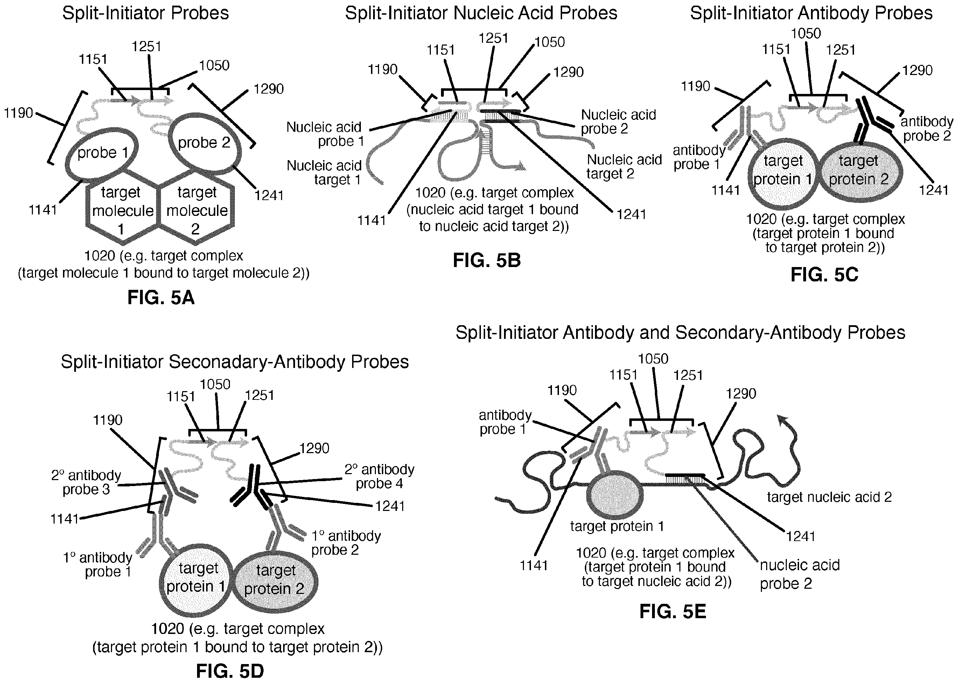

In some embodiments, the target molecule can be a complex of molecules such that when the target binding sites within a fractional initiator (aka a split-initiator) probe pair bind specifically to their target sites within the complex, the two halves of the HCR initiator are brought into proximity, such that the full initiator becomes capable of initiating HCR signal amplification. Figure 5 illustrates detection of a target complex using fractional initiator (aka a split-initiator) probes (panel a), detection of a target complex of nucleic acids using fractional initiator (aka a split-initiator) nucleic acid probes (panel b), detection of a target complex of proteins using fractional initiator (aka a split-initiator) antibody probes (panel c), detection of a target complex of proteins using primary-antibody probes and fractional initiator (aka a split-initiator) secondary-antibody probes (panel d), detection of a target protein/nucleic acid complex using split-initiator antibody and nucleic acid probes (panel e).

In some embodiments, any of the methods described herein can be used as part of an in situ process to image DNA, RNA, protein, or small molecule targets, including DNA in situ hybridization (ISH), RNA in situ hybridization (ISH), or protein immunohistochemistry (IHC).

In some embodiments, any of the methods described herein further comprise applying one or more of the components to a target. In some embodiments, the target is hydrated. In some embodiments, the target is in a solution, but can be immobilized to a solid support. In some embodiments, the target is in a solution. In some embodiments, the target is immobilized on a bead or other support. In some embodiments, the support is a mesh or a gel or a rigid surface. In some embodiments, the target is not immobilized. In some embodiments, the detection occurs in vivo, in vitro, or in situ. In some embodiments, an HCR method is provided that comprises an in vitro method in which the target is immobilized on a bead or microarray. In some embodiments, the target is immobilized on a bead. In some embodiments, the target is immobilized on a microarray. In some embodiments, an HCR method is provided that comprises an in vivo or in vitro method in which the target is not immobilized. In some embodiments, an HCR method is provided that comprises an in vivo or in vitro method in which the target is immobilized.

In some embodiments, incubating results in binding the first fractional initiator probe (1190) to the target molecule and in binding the second fractional initiator probe (1290) to a target molecule. The target molecule can be a single molecule or multiple associated molecules. In some embodiments, incubating occurs at room temperature. In some embodiments, incubating occurs at 4 °C. In some embodiments, incubating occurs at 37°C. In some embodiments, incubating occurs at 45 °C. In some embodiments, incubating occurs at 50 °C. In some embodiments, incubating occurs at 55 °C. In some embodiments, incubating occurs at 60 °C. In some embodiments, incubating comprises an incubation period of at least 1 minute, for example, 5 minutes, 15 minutes, 30 minutes or 1 hour. In some embodiments, the incubation period exceeds 1 hour, for example, 2 hours, 4 hours, 12 hours, 16 hours, or 24 hours. In some embodiments, incubating occurs in hybridization buffer containing 0% formamide. In some embodiments, incubating occurs in hybridization buffer comprises formamide. In some embodiments, the percent concentration of formamide is between 1% and 80%, for example, between 10% and 70%, or between 30% and 60%. In some embodiments, the hybridization buffer comprises citric acid. In some embodiments, the molar concentration of citric acid is between 1nM and 30nM, for example, between 5nM and 15nM or between 8nM and 12nM. In some embodiments, the hybridization buffer comprises Tween. In some embodiments, the percent concentration of Tween is between 0% and 1.0%, for example, between 0.05% and 0.5%. In some embodiments, the hybridization buffer comprises heparin. In some embodiments, the concentration of heparin is between 20µg/mL and 80µg/mL, for example, between 30µg/mL and 70 µg/mL, for example, between 40µg/mL and 60µg/mL. In some embodiments, the hybridization buffer comprises Denhardt's solution. In some embodiments, the hybridization buffer comprises dextran sulfate. In some embodiments, the percent concentration of dextran sulfate is between 1% and 60%, for example, between 40% and 60%.

In some embodiments the first fractional initiator (1151) of any of the methods described herein is part of a first fractional initiator probe (1190) and the second fractional initiator (1251) is part of a second fractional initiator probe (1290). In some embodiments, the first fractional initiator probe (1190) further comprises a first target-binding section (1141) and the second fractional initiator probe (1290) further comprises a second target-binding section (1241). In some embodiments, the first target-binding section (1141) is configured to bind adjacent to the second target-binding section (1241) on a target, when the first fractional initiator probe (1190) and the second fractional initiator probe (1290) are both bound specifically to a target (1020).

While the term "adjacent" is used for binding of the first and second fractional initiator probes (and/or first and second target-binding sections), binding need not be immediately adjacent, as long as the first and second initiator probes are close enough to one another to form a full initiator capable of triggering HCR. In some embodiments, the first target-binding section binds at least within 10 amino acids of the second target binding section, for example within 5 amino acids or within 1 amino acid. In some embodiments, the first target-binding section binds at least within 10 nucleotides of the second target binding section, for example within 5 nucleotides or within 1 nucleotide. In some embodiments, the first and second fractional initiator probes (and/or first and second target-binding sections), are within 2, 5, 10, 50, 100, 1000, Angstroms of each other. In some embodiments, the first and second fractional initiator probes can include spacers-so as to allow more space between the two target sections. Such spacers could include additional nucleic acid sequence, to provide more flexibility for the location of the first and second (or additional) target sections. In some embodiments, each target section on the target is proximal enough to one another to allow the two or more fractional initiators to colocalize a full initiator.

In some embodiments, the first fractional initiator probe (1190) comprises more than one target-binding section, for example, two target binding sections, three target binding sections, four target binding sections, or five target binding sections. This can allow for assaying of multiple possible targets at a time. In some embodiments, the first fractional initiator probe (1190) comprises more than five target binding sections. In some embodiments, the second fractional initiator probe (1290) comprises more than one target-binding section, for example, two target binding sections, three target binding sections, four target binding sections, or five target binding sections. In some embodiments, the second fractional initiator probe (1290) comprises more than five target binding sections.

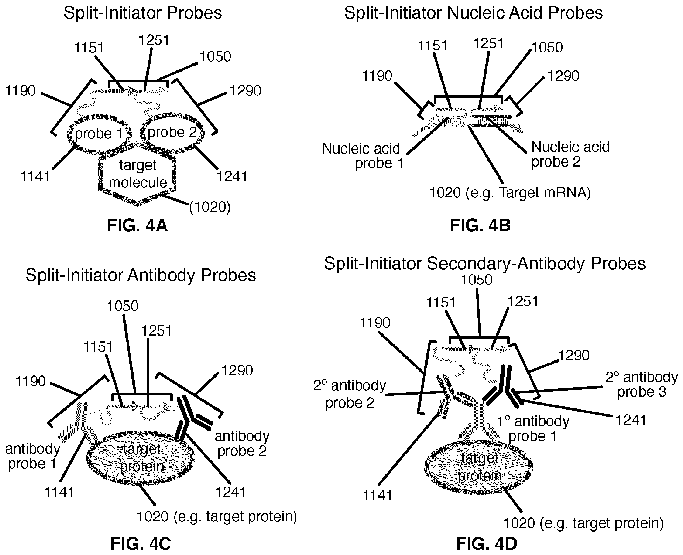

In some embodiments, the target binding section (e.g., region) within each of the fractional initiator (aka a split-initiator) probe pairs can be made of DNA, RNA, 2'OMe-RNA, PNA, amino acids, or any synthetic nucleic acid analog, or any synthetic amino acid analog. Figure 4 illustrates detection of a target molecule using fractional initiator (aka a split-initiator) probes (panel a), detection of a target mRNA using fractional initiator (aka a split-initiator) nucleic acid probes (panel b), detection of a target protein using fractional initiator (aka a split-initiator) antibody probes (panel c), and detection of a target protein using a primary-antibody probe and split-initiator secondary-antibody probes (panel d). In each case, selective binding of the probe pair to the cognate target molecule colocalizes the two halves of the full HCR initiator, triggering growth of a tethered HCR amplification polymer. The target molecule can be a protein or small molecule such that when the target binding sites within the fractional initiator (aka a split-initiator) probe pair bind specifically to their target sites on the protein or small molecule, the two halves of the HCR initiator are brought into proximity, such that the full initiator becomes capable of initiating HCR signal amplification.

In some embodiments, HCR hairpin monomers may be labeled with reporter molecules that are not fluorescent (e.g., isotopically pure rare earth elements, chromophores, etc.).

For Scheme E, each probe within a fractional initiator (aka a split-initiator) probe pair contains a target binding site and half of an HCR initiator such that when the fractional initiator (aka a split-initiator) probes base-pair specifically to their target sections (e.g., cognate proximal target sites), the two halves of the HCR initiator are co-localized; this functionality can be achieved by arranging the target binding sites and initiator fragments within the fractional initiator (aka a split-initiator) probe pair in a variety of configurations (Figure 3).

In some embodiments, the HCR initiator within a fractional initiator (aka a split-initiator) probe pair can be split between two probes (not necessarily half-and-half) in such a way that HCR amplification is only initiated if both probes are proximal.

In some embodiments, the HCR initiator can be split between two or more fractional initiator (aka a split-initiator) probes.

In some embodiments, the target mRNA can be detected using a fractional initiator probe set (or probe set) containing one or more fractional initiator (aka a split-initiator) probe pairs; each probe pair contains target-binding sites addressing different subsequences of the target mRNA. Within a fractional initiator probe set, each fractional initiator (aka a split-initiator) probe pair co-localizes to form the same HCR initiator sequence, thus enabling simultaneous growth of HCR amplification polymers off of multiple probe pairs bound to the same target mRNA.

In some embodiments, the target molecule could be an mRNA, a miRNA, a lncRNA, an rRNA, genomic DNA, or any nucleic acid molecule.

In some embodiments, the initiator that is split between the two fractional initiator (aka a split-initiator) probes within a pair could be made of DNA, RNA, 2'OMe-RNA, PNA, or any synthetic polymer capable of initiating HCR amplification.

In some embodiments, any of the methods described herein comprise adding more than a first fractional initiator (1151) and a second fractional initiator (1251), for example, adding 10, 20, 50, 100, 1000, 10,000 etc.

In some embodiments, any of the methods described herein can comprise a 2-stage approach with a wash after each stage. In some embodiments, the target is immobilized and/or the sample is fixed. In some embodiments, the first stage is a detection stage that comprises binding fractional initiator probe(s) to a target and washing away unbound fractional initiator probe(s) and the second stage is an amplification stage in which HCR amplification occurs. This can be followed by washing away unpolymerized HCR monomers (such as hairpin monomers). In some embodiments, there is more than one wash after each stage, for example, two washes, three washes, four washes, or five washes. In some embodiments, the HCR method comprises a single stage with no washes.

In some embodiments, the HCR hairpin monomers comprise a label-binding site, rather than directly incorporating a reporter molecule onto the hairpin monomer itself. In some embodiments, any of the methods described herein further comprises washing the sample to remove unpolymerized HCR hairpin monomers, adding a label probe that comprises a complement to the label binding site and a reporter molecule; washing away unbound label probe, and detecting a presence or absence of the reporter molecule. In some embodiments, the label probe is a hairpin molecule that further comprises a flourophore/quencher pair, such that the fluorophore is quenched when the hairpin monomer is closed, but when the label probe binds the label binding site on an HCR polymer, the fluorophore is unquenched. In some embodiments, the label probe is a duplex with one strand carrying a fluorophore and the other strand carrying a quencher such that when the label probe binds the label binding site on an HCR polymer, the quencher-labeled strand is displaced and the fluorophore-labeled strand is bound to the label binding site on the HCR polymer. In some embodiments, the hairpin monomers carry a reporter that is one part of a FRET pair, and the label probe carries the other part of a FRET pair, such that upon binding of the label probe to the label binding site on an HCR polymer, the two parts of the FRET pair are brought into proximity and can undergo FRET. In some embodiments removing unpolymerized HCR hairpin monomer through washing results in the removal of greater than 50% of the unpolymerized HCR hairpin monomers, for example, greater than 55%, greater than 60%, greater than 65%, greater than 70%, greater than 75%, greater than 80%, greater than 85%, greater than 90%, greater than 95%, greater than 99%, greater than 99.9%, greater than 99.99%, greater than 99.999%, or greater than 99.9999999%.

In some embodiments, any of the washes provided herein can result in the removal of greater than 50% of the fractional initiator (and/or fractional initiator probe) for example, greater than 55%, greater than 60%, greater than 65%, greater than 70%, greater than 75%, greater than 80%, greater than 85%, greater than 90%, greater than 95%, greater than 99%, greater than 99.9%, greater than 99.99%, greater than 99.999%, or greater than 99.9999999%.

In some embodiments, a wash results in removal of at least 50 to 99% of probes that are not bound specifically to the target. In some embodiments, the wash results in the removal of greater than 50% of the probes, for example, greater than 55%, greater than 60%, greater than 65%, greater than 70%, greater than 75%, greater than 80%, greater than 85%, greater than 90%, or greater than 95%, greater than 99%, greater than 99.9%, greater than 99.99%, greater than 99.999%, or greater than 99.9999999%..

Any of the methods described herein can further comprise a first wash. In some embodiments, the first wash removes unbound first fractional initiator probe (1190) and unbound second fractional initiator probe (1290) from a sample containing a first target molecule. In some embodiments, following the first wash colocalized first and second initiator probes on the target molecules are bound by the first hairpin monomer (1510), which is then bound by the second hairpin monomer (1610), resulting in HCR amplification. In some embodiments, following the first wash, unbound hairpin monomers are washed from the sample. In some embodiments, the method comprises additional washes, for example 2 washes, 3 washes, 4 washes, 5 washes, 6 washes, or 7 washes. In some embodiments, the method comprises more than 7 washes.

In some embodiments, a fractional initiator can be from 1-1000 nucleotides in length, e.g., 10-80, 10-60, 10-50, 20-50, 20-30 nucleotides in length. In some embodiments, it can be of any functional length.

In some embodiments, each target section can be from 1-1000 nucleotides in length, e.g., 10-80, 10-60, 10-50, 20-50, 20-30 nucleotides in length. In some embodiments, it can be of any functional length.

In some embodiments, each target binding section can be from 1-1000 nucleotides in length, e.g., 10-80, 10-60, 10-50, 20-50, 20-30 nucleotides in length. In some embodiments, it can be of any functional length. In some embodiments, the target binding section and/or the target section are not nucleic acids, and thus can be proteins etc. as described herein. In some embodiments, the target binding sections can each be from individual atoms up to hundreds of kDa (e.g., antibodies etc.) or larger.

In some embodiments, each full initiator can be as long as the sum of the fractional initiators that form the full initiator. In some embodiments, each fractional initiator can be about ½ the size of the full initiator. In some embodiments, the fractional size of the fractional initiator depends upon the number of fractional initiators to complete the full initiator. Thus, when 2, 3, 4, 5, etc. fractional initiators are employed in a single full initiator, then ½, 1/3, ¼, 1/5, etc. of the initiator will be present in each fractional initiator. In some embodiments, the size of the initiator need not be evenly divided for each fractional initiator. The size of the full initiator is adequate to allow for HCR to occur through the full initiator.

In some embodiments, any of the methods and/or compositions described herein comprise adding at least one additional fractional initiator probe, for example, adding three fractional initiators, adding four fractional initiators, adding five fractional initiators, adding six fractional initiators, adding seven fractional initiators, adding eight fractional initiators, a nine fractional initiators, adding 10 fractional initiators, adding 11 fractional initiators, adding 12 fractional initiators, or adding 14 fractional initiators, adding 15 fractional initiators. In some embodiments, more than 15 fractional initiators are added, for example, between 16 and 100 fractional initiators or more, e.g., 500, 1000, etc.

In some embodiments, the target molecule (1020) comprises a first target section (1100) and a second target section (1200). The first fractional initiator probe (1190) can comprise a first target-binding section (1141) and the second fractional initiator probe (1290) comprises a second target-binding section (1241). The first target section (1100) is configured to bind to (or be bound by) the first target-binding section (1141) and the second target section (1200) is configured to bind to (or be bound by) the second target-binding section (1241).

In some embodiments, any of the methods described herein further comprises: binding the first hairpin monomer (1510) to both the first fractional initiator (1151) and the second fractional initiator (1251), binding the second hairpin monomer (1610) to the first hairpin monomer (1510), and detecting a signal. The signal can be generated or amplified via the first and second hairpin monomers going through a hybridization chain reaction to thereby add and/or concentrate an amount of a reporter molecule that is associated with one or both of the first and/or second hairpin monomers.

Wash steps can be performed prior to the addition of the hairpin monomers and/or detection. In some embodiments, the method comprises a wash to remove unbound first and second fractional initiator probes from a sample that contains a target. In some embodiments, the method comprises more than one wash, for example, 2 washes, 3 washes, 4 washes, or 5 washes. In some embodiments, individual fractional initiator probes that remain within the sample and/or solution after the wash and are not specifically bound to the target do not colocalize a full initiator and hence do not trigger HCR.

In some embodiments, any of the methods described herein comprise binding an additional first hairpin monomer (1510) to the second hairpin monomer (1610) (which is already bound to a first hairpin monomer). Thus, extending the chain of monomers can be achieved via HCR polymerization. In some embodiments, there are at least 10 additional first hairpin monomers. In some embodiments there are at least 100 additional first hairpin monomers, e.g., 1000, 10000, etc. In some embodiments, the method comprises binding an additional second hairpin monomer to the additional first hairpin monomer, which is already bound to a second hairpin monomer). In some embodiments, there are at least 10 additional second hairpin monomers. In some embodiments, there are at least 100 additional second hairpin monomers, e.g., 1000, 10000, etc. In some embodiments, any of the methods described herein comprise an alternating cascade of polymerization events in which a full initiator binds a first hairpin monomer (1510) which in turn binds a second hairpin monomer (1610) which in turn binds another first hairpin monomer which in turn binds another second hairpin monomer, and so on, such that the polymer grows via alternating addition of first and second hairpin monomers. In some embodiments the alternating cascade comprises binding of more than two hairpin monomers, for example, three hairpin monomers, four hairpin monomers, five hairpin monomers, six hairpin monomers, seven hairpin monomers, eight hairpin monomers, nine hairpin monomers, 10 hairpin monomers, 100, 1000, 10000, 100000, 1000000, or more.

In some embodiments, in any of the binding reactions described herein, binding comprises hybridization. In some embodiments, binding comprises selective protein-protein interaction. In some embodiments, binding comprises selective nucleic acid-protein interaction. In some embodiments, binding comprises ionic binding. In some embodiments, binding comprises covalent binding.

In some embodiments, in any of the methods or compositions described herein, the reporter molecule is a fluorescent molecule. In some embodiments, the reporter molecule comprises a quenched or FRET arrangement, in which a fluorescent molecule on a hairpin monomer is dequenched when a polymer configuration is achieved by the first and second hairpin monomers. In some embodiments, the reporter molecule is a non-fluorescent molecule. In some embodiments, the reporter molecule is a rare earth element.

In some embodiments, the first and second hairpin monomers form a FRET pair when combined as a polymer. In some embodiments, the FRET pair is formed due to a change in quenching or FRET that occurs when the hairpin monomers open and polymerize. Thus, in some embodiments, the hairpin monomers are configured to allow for FRET (e.g., meeting proximity requirements and reporter molecule pairing requirements for changes in FRET to be monitored).

In some embodiments, the first fractional initiator probe (1190) can comprise an amino acid sequence and the second fractional initiator probe (1290) can comprises an amino acid sequence. In some embodiments, the probe will also include a nucleic acid sequence to hybridize to the target sequence(s). These can be the first target binding sections and the second target binding sections. In some embodiments, the target binding sections are complementary to the first and second target sections (1100 and 1200). In some embodiments, the first and second fractional initiator probes will also include first and second fractional initiators, which can comprise nucleotides, providing a nucleic acid sequence of adequate length, which when colocalized, forms the full initiator. In some embodiments, the first fractional initiator probe (1190) comprises a nucleic acid sequence and the second fractional initiator probe (1290) comprises a nucleic acid sequence. In some embodiments, the first and second fractional initiators (1151, 1251) can base-pair with the first hairpin monomer (1510). In some embodiments, the first fractional initiator probe comprises one or more of the following: DNA, RNA, 2'Ome-RNA, LNA, synthetic nucleic acid analog, amino acid, synthetic amino acid analog, and PNA and the second fractional initiator probe comprises one or more of the following: DNA, RNA, 2'Ome-RNA, LNA, synthetic nucleic acid analog, amino acid, synthetic amino acid analog, and PNA.

In some embodiments, any of the wash buffers described herein comprises formamide. In some embodiments, the percent concentration of formamide is between 0% and 80%, for example, between 10% and 70%, or between 30% and 60%. In some embodiments, the wash buffer comprises citric acid. In some embodiments, the molar concentration of citric acid is between 1nM and 30nM, for example, between 5nM and 15nM or between 8nM and 12nM. In some embodiments, the wash buffer comprises Tween. In some embodiments, the percent concentration of Tween is between 0% and 1.0%, for example, between 0.05% and 0.5%. In some embodiments, the wash buffer comprises heparin. In some embodiments, the concentration of heparin is between 20µg/mL and 80µgmL, for example, between 30µg/mL and 70 µg/mL, for example, between 40µg/mL and 60µg/mL.

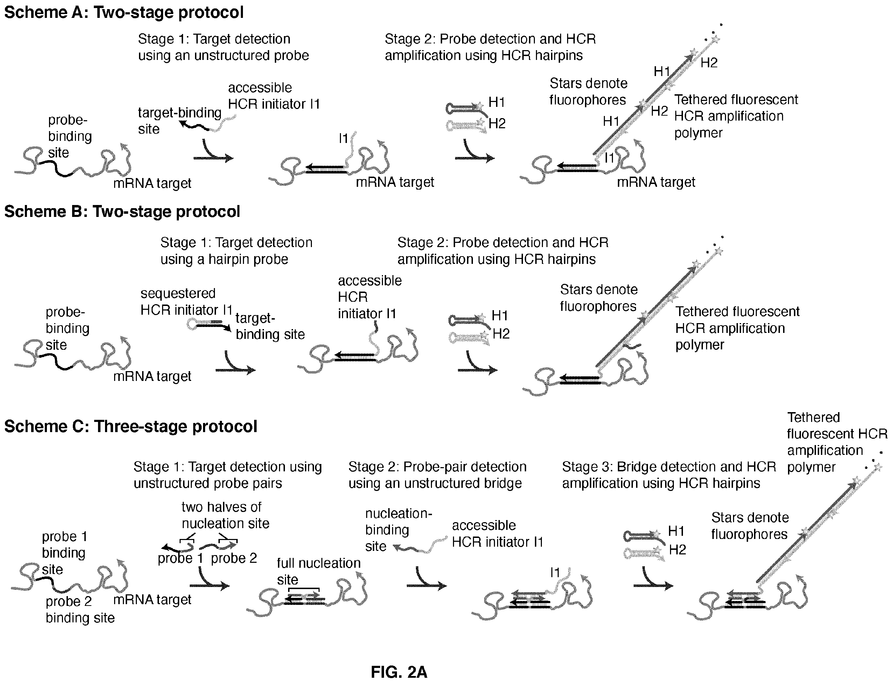

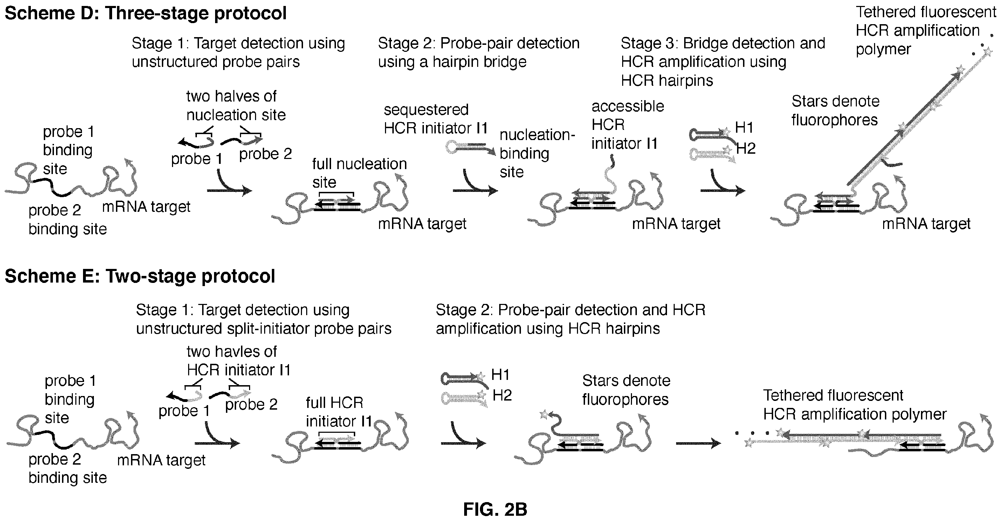

A fractional initiator is distinct from other conditional probes. For example, in some embodiments, a fractional initiator is not a conformation-changing probe (e.g., scheme B in FIG. 2). In some embodiments, the fractional initiator is one that involves two separate strands of nucleic acids. In some embodiments, the fractional initiator is dependent upon at least two separate binding events to at least two different target sections (although the two sections can be on a single molecule).

In some embodiments, the method provided herein can be used to improve the signal-to-background ratio for In Situ Signal Amplification via Hybridization Chain Reaction (HCR).

Figure 18C depicts further embodiments involving HCR, in particular, a three-stage in situ HCR protocol using fractional initiator probes with hairpin monomers that carry label binding sites. Detection stage: fractional initiator probe pairs are hybridized to the target mRNA and unused probes are washed from the sample. Each fractional initiator probe set contains one or more probe pairs that selectively bind to different subsequences along the target mRNA. Each probe within a pair carries a part of the HCR initiator I1 (e.g., ½). Selective hybridization of the two probes within a pair to their cognate target binding sites colocalizes the two halves of full HCR initiator I1. Amplification stage: full HCR initiator I1 triggers self-assembly of tethered amplification polymers and unused H1 and H2 hairpin monomers are washed from the sample. Each hairpin monomer carries a label binding site. The label binding sites decorate the resulting HCR amplification polymer. Labeling stage: label probes comprising a complement to the label binding site and additionally comprising a fluorophore reporter are hybridized to the amplification polymers and then unbound label probes are washed from the sample. Stars denote fluorophores. Each of the stages may be repeated, combined, or separated depending upon the desired results. In some embodiments, the stages occur in the listed order.

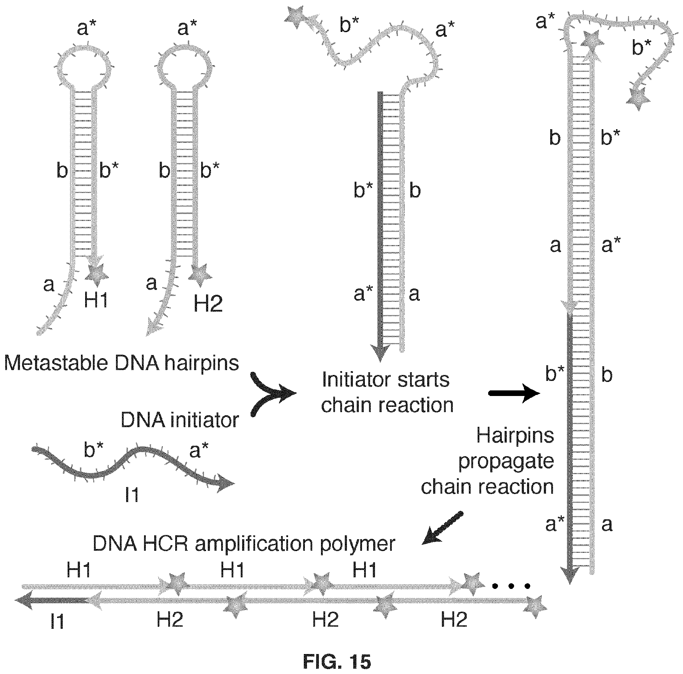

Figure 15 depicts an HCR mechanism using simplified HCR hairpins. Metastable fluorescent hairpins self-assemble into fluorescent amplification polymers upon detection of a cognate initiator. Initiator I1 nucleates with hairpin H1 via base-pairing to single-stranded toehold 'a', mediating a branch migration that opens the hairpin to form complex I1•H1 containing single-stranded segment 'a*-b*'. This complex nucleates with the second hairpin monomer (hairpin H2) by means of base-pairing to toehold 'a', mediating a branch migration that opens the hairpin to form complex I1•H1•H2 containing single-stranded segment 'b*-a*'. Thus, the initiator sequence is regenerated, providing the basis for a chain reaction of alternating H1 and H2 polymerization steps. Stars denote fluorophores. Arrowhead denotes 3' end of each strand. Note that the two hairpins have the same sequence domains but with opposite strand polarity (a-b-a*-b* for hairpin monomer H1 running 5' to 3') and (a-b-a*-b* for hairpin monomer H2 running 3' to 5').

In some embodiments, "domain a" is the same as domain "c" (e.g., as shown in FIG. 13). That is, both hairpins use the same loop and toehold sequences. This is a special case of the standard HCR case that uses less sequence space to achieve the same functionality.

### Fish

In some embodiments, the method is part of a biotechnology protocol. In some embodiments, the method can be part of Fluorescence In Situ Hybridization (FISH). Fluorescence in situ hybridization methods provide biologists with a crucial window into the spatial organization of endogenous biological circuitry by revealing the expression patterns of target mRNAs within cells, tissues, organs, organisms, and ecosystems (1-7). If autofluorescence within the sample is low, sufficient signal can be generated using a fractional initiator probe set containing one or more nucleic acid probes, each carrying one or more fluorescent reporter molecules, and each containing a target binding sequence complementary to a portion of the target mRNA (8-14); in many settings, including whole-mount vertebrate embryos and thick brain sections, this approach does not yield sufficient signal, so probes are instead used to mediate in situ signal amplification to increase the signal-to-background ratio (3, 6, 7, 11, 15-31).

In some embodiments, the method allows one to map target mRNAs within a sample with a high signal-to-background ratio. In some embodiments, a FISH method incorporates in situ signal amplification, in which case all fluorescence within the sample is either amplified signal or some form of background:
Amplified Signal. Amplified signal is generated when probes hybridized specifically to their cognate targets and then subsequently mediate generation of fluorescent amplification products at the site of the target molecule.
Background. All other fluorescence in the sample is some form of background:
Autofluorescence. Autofluorescence is background fluorescence inherent to the sample.
Amplified Background. Amplified background is generated when non-specific binding of a reagent in any stage of a protocol leads to generation of an amplification product in subsequent stages of the protocol.
Unamplified Background. Unamplified background is generated if a fluorescent reagent binds non-specifically in the sample, but does not lead to generation of amplification products.

Hence, the performance of the technique depends both on what goes right (generation of amplified signal) and on what goes wrong (generation of background from any of three sources: autofluorescence, unamplified background, amplified background). To achieve a high signal-to-background ratio in a voxel within an image, it is useful to generate amplified signal that is significantly higher than the total background within the voxel.

Programmable in situ amplification based on the mechanism of hybridization chain reaction (HCR) (32) allows straightforward multiplexing, deep sample penetration, high signal-to-background, and subcellular resolution in diverse organisms (33-35). An HCR amplifier includes two kinetically trapped nucleic acid hairpin molecules (H1 and H2) that co-exist metastably in the absence of a cognate initiator strand (I1; Figure 1a). Arrival of the initiator triggers a chain reaction in which H1 and H2 hairpins sequentially nucleate and open to assemble into a long nicked double-stranded amplification polymer (32). Using in situ HCR, DNA probes complementary to mRNA targets carry DNA HCR initiators that trigger chain reactions in which metastable fluorophore-labeled DNA hairpins self-assemble into tethered fluorescent amplification polymers (Figure 1b). The same two-stage in situ hybridization protocol is used independent of the number of target RNAs (Figure 1c): in the detection stage, all fractional initiator probe sets are hybridized in parallel; in the amplification stage, orthogonal HCR amplifiers operate in parallel.

HCR draws on principles from the disciplines of molecular programming and dynamic nucleic acid nanotechnology to provide isothermal enzyme-free signal amplification in diverse technological settings (36-39) and it is particularly well-suited to the demands of in situ amplification (33, 34). First, HCR is programmable, providing the basis for straightforward multiplexing using orthogonal amplifiers that operate independently and carry spectrally distinct fluorophores. Use of a two-stage protocol independent of the number of target mRNAs is convenient for any sample, but essential for delicate samples such as sea urchin embryos that are easily damaged during serial multiplexing protocols. Second, HCR hairpin monomers do not self-assemble until they encounter a probe carrying the cognate initiator, enabling deep sample penetration prior to growth of bright amplification polymers at the site of target molecules. The use of amplification reagents that are structured hairpins with a duplex stem reduces the potential for non-specific hybridization within the sample and also increases the ease of engineering multiple orthogonal amplifiers. The fact that amplification polymers can carry hundreds of fluorophores (34) makes it possible to achieve high signal-to-background even when autofluorescence is high (e.g., in whole-mount vertebrate embryos (34, 40, 41) or in bacteria contained within environmental samples or other organisms (42-44)). Third, HCR amplification polymers remain tethered to their initiating probes, preventing signal from diffusing away from targets, and leading to subcellular resolution. Fourth, because HCR amplifier sequences are independent of mRNA target sequences, previously validated amplifiers (34) can be used for new studies without modification. To map a new target mRNA, all that is needed is a new DNA fractional initiator probe set carrying DNA initiators for an existing DNA HCR amplifier. Taken together, the properties of in situ HCR lead to straightforward multiplexing, deep sample penetration, high signal-to-background, and subcellular resolution in diverse organisms, offering biologists a dramatically improved window into the spatial organization of biological circuitry. Below is described five Schemes (A, B, C, D, E) that adopt different approaches to optimize the signal-to-background ratio using HCR in situ signal amplification (Figure 2). A standard in situ HCR approach depicted in Figure 1b (34) corresponds to Scheme A.

Scheme A: Two-stage protocol: Target detection with an unstructured probe followed by probe detection and HCR amplification using HCR hairpin monomers. An unstructured probe contains a target-binding section (e.g., target binding sequence) and an unstructured HCR initiator sequence. The initiator is accessible to initiate HCR whether or not the probe is hybridized to the cognate target mRNA. HCR hairpin monomers predominantly do not interact except when they are initiated by a cognate HCR initiator; initiation triggers polymerization via sequential hairpin nucleation and opening, yielding an HCR amplification polymer base-paired to the initiator.

Stage 1: Target detection using an unstructured probe: Probes are hybridized within the fixed sample and unused probes are washed away. Probes predominantly bind to the cognate target mRNA but a non-negligible fraction of probes bind elsewhere in the sample. Probes that remain in the sample will trigger amplification during Stage 2, whether or not they are bound to the cognate target mRNA.

Stage 2: Probe detection and HCR amplification using HCR hairpin monomers: HCR hairpin monomers are hybridized within the fixed sample and unused hairpins are washed away. Probes within the sample initiate growth of tethered HCR amplification polymers, generating amplified signal at the site of probes bound to target molecules and amplified background at the site of probes bound elsewhere in the sample. HCR hairpin monomers that bind non-specifically in the sample do not trigger HCR polymerization and hence do not contribute to generation of amplified background, instead contributing a negligible amount of unamplified background.

Performance implications: Scheme A is vulnerable to non-specific probe binding during Stage 1, which leads to generation of amplified background during Stage 2. Because the probe is unstructured, Scheme A has the benefit that the target binding sequence and the HCR initiator sequence are independent; as a result, validated HCR initiators and HCR hairpin monomers can be used for diverse new probes and targets without changing the HCR initiator and HCR hairpin monomer sequences.

Scheme B: Two-stage protocol: Target detection using a hairpin probe followed by probe detection and HCR amplification using HCR hairpin monomers. A hairpin probe contains a target-binding section (e.g., target binding sequence) and an H CR initiator sequence; the HCR initiator is initially inaccessible due to base-pairing within the hairpin probe; if the hairpin probe base-pairs to its cognate target mRNA, the probe changes conformation and the HCR initiator becomes accessible and capable of initiating HCR amplification. HCR hairpin monomers predominantly do not interact except when they are initiated by a cognate HCR initiator; initiation triggers polymerization via sequential hairpin nucleation and opening, yielding an HCR amplification polymer base-paired to the initiator.

Stage 1: Target detection using a hairpin probe: Probes are hybridized within the fixed sample and unused probes are washed away. Probes predominantly bind to the cognate target mRNA but a non-negligible fraction of probes bind elsewhere in the sample. A probe hybridized to a cognate target mRNA changes conformation to expose an accessible HCR initiator, leading to generation of amplified signal in Stage 2. A probe bound elsewhere in the sample has an inaccessible HCR initiator and thus does not trigger HCR amplification, avoiding generation of amplified background in Stage 2.

Stage 2: Probe detection and HCR amplification using HCR hairpin monomers: HCR hairpin monomers are hybridized within the fixed sample and unused hairpins are washed away. A probe hybridized to its cognate target triggers growth of a tethered HCR amplification polymer, generating amplified signal at the site of the target molecule. A probe bound elsewhere in the sample does not trigger amplification, avoiding generation of amplified background. HCR hairpin monomers that bind non-specifically in the sample do not trigger HCR polymerization and hence do not contribute to generation of amplified background, instead contributing a negligible amount of unamplified background.

Performance Implications: Scheme B is not vulnerable to non-specific probe binding during Stage 1, as non-specifically-bound probes do not trigger HCR amplification during Stage 2. Hence, Scheme B has the property that even if reagents bind non-specifically at any stage of the protocol, amplified background will predominantly not be generated. The drawback of Scheme B is that using hairpin probes, there is some degree of sequence complementarity between the target -binding sequence and the HCR initiator sequence. As a result, changing the target-binding section (e.g., target binding sequence) necessitates changing the HCR initiator and HCR hairpin monomer sequences, which is a disadvantage compared to the simplicity of Scheme A.

Scheme C: Three-stage protocol: Target detection with unstructured probe pairs followed by probe-pair detection using an unstructured bridge followed by bridge detection and HCR amplification using HCR hairpin monomers. Unstructured probes come in pairs; each probe contains a target-binding section (e.g., target binding sequence) and half of an unstructured nucleation sequence; for the two probes within a probe pair, the two target-binding section (e.g., target binding sequence)s are complementary to target sections (e.g., proximal subsequences) of the target mRNA such that when the two probes bind specifically to their target sections (e.g. cognate target sites), the two halves of the nucleation sequence are brought into proximity. An unstructured bridge strand contains proximal binding sequences complementary to the two halves of the nucleation sequence; the unstructured bridge strand also contains an HCR initiator that is accessible to initiate HCR whether or not the bridge strand is specifically base-paired to its cognate nucleation site. HCR hairpin monomers predominantly do not interact except when they are initiated by a cognate HCR initiator; initiation triggers polymerization via sequential hairpin nucleation and opening, yielding an HCR amplification polymer base-paired to the initiator.

Stage 1: Target detection using unstructured probe pairs: Probes are hybridized within the fixed sample and unused probes are washed away. Probe pairs predominantly base-pair to their target sections (e.g., cognate target sites), co-localizing the two halves of the nucleation sequence; a non-negligible fraction of probes bind elsewhere in the sample, but probes that are bound non-specifically predominantly do not co-localize the two halves of the nucleation sequence.

Stage 2: Probe pair detection using an unstructured bridge: Unstructured bridge strands are hybridized within the fixed sample under experimental conditions such that a bridge strand predominantly base-pairs stably to the full nucleation sequence created by a cognate probe pair specifically base-paired to proximal target sections (e.g., cognate target sites); furthermore, the experimental conditions are such that the bridge strand predominantly does not base-pair stably to the half-nucleation site carried by an individual probe that is not proximal to its partner probe. A non-negligible fraction of bridges bind non-specifically in the sample; non-specifically bound bridges will trigger HCR amplification during Stage 3, generating amplified background.

Stage 3: Bridge detection and HCR amplification using HCR hairpin monomers: HCR hairpin monomers are hybridized within the fixed sample and unused hairpins are washed away. Bridge strands within the sample initiate growth of tethered HCR amplification polymers; for bridge strands base-paired to their cognate nucleation site formed by a cognate pair of probes base-paired to proximal target sections (e.g., cognate target sites), these polymers represent amplified signal. For bridge strands bound elsewhere in the sample, these polymers correspond to amplified background. HCR hairpin monomers that bind non-specifically in the sample do not trigger HCR polymerization and hence do not contribute to generation of amplified background, instead contributing a negligible amount of unamplified background.

Performance implications: Scheme C is not vulnerable to non-specific probe binding in Stage 1, as bridges predominantly do not base pair stably to isolated probes in Stage 2. However, Scheme C is vulnerable to non-specific binding of bridges in Stage 2, which leads to generation of amplified background in Stage 3. Note that the unstructured bridge in Stage 2 of Scheme C is subject to the same conceptual weakness as the unstructured probe in Stage 1 of Scheme A. The benefit of Scheme C relative to Scheme A is that using Scheme C a library of bridge sequences can be optimized for use in a given species and then those bridge sequences can be reused for diverse new probes and targets without changing the bridges sequences. By comparison, using Scheme A, each new probe sequence would need to be optimized for use in a given species. However, even using optimized bridge sequences with Scheme C, a non-negligible fraction of bridges will bind non-specifically in the sample, generating amplified background in the next stage in the protocol. Likewise, even using optimized probe sequences using Scheme A, a non-negligible fraction of probes will bind non-specifically in the sample, generating amplified background in the next stage in the protocol.

Scheme D: Three-stage protocol: Target detection with unstructured probe pairs followed by probe-pair detection using a hairpin bridge followed by bridge detection and HCR amplification using HCR hairpin monomers. Unstructured probes come in pairs; each probe contains a target-binding section (e.g., target binding sequence) and half of an unstructured nucleation sequence; for the two probes within a probe pair, the two target-binding section (e.g., target binding sequence)s are complementary to target sections (e.g., proximal subsequences) of the target mRNA such that when the two probes bind specifically to their target sections (e.g., cognate target sites), the two halves of the nucleation sequence are brought into proximity. A hairpin bridge contains proximal binding sequences complementary to the two halves of the nucleation sequence; the hairpin bridge also contains an HCR initiator that is initially inaccessible due to base-pairing within the hairpin bridge; if the hairpin bridge base-pairs to both halves of its cognate nucleation sequence, the bridge changes conformation and the HCR initiator becomes accessible and capable of initiating HCR amplification. HCR hairpin monomers predominantly do not interact except when they are initiated by a cognate HCR initiator; initiation triggers polymerization via sequential hairpin nucleation and opening, yielding an HCR amplification polymer base-paired to the initiator.

Stage 1: Target detection using unstructured probe pairs: Probes are hybridized within the fixed sample and unused probes are washed away. Probe pairs predominantly base-pair to their target sections (e.g., cognate target sites), co-localizing the two halves of the nucleation sequence; some probes bind elsewhere in the sample, but probes that are bound non-specifically predominantly do not co-localize the two halves of the nucleation sequence.

Stage 2: Probe -pair detection using a hairpin bridge: Hairpin bridge strands are hybridized within the fixed sample and unused bridges are washed away. Hairpin bridge strands predominantly base-pair stably to the full nucleation sequence created by a cognate probe pair specifically base-paired to proximal target sections (e.g., cognate target sites); a specifically bound hairpin bridge changes conformation to expose an accessible HCR initiator, leading to generation of amplified signal in Stage 3. A hairpin bridge bound elsewhere in the sample has an inaccessible HCR initiator and does not trigger HCR amplification, avoiding generation of amplified background in Stage 3.

Stage 3: Bridge detection and HCR amplification using HCR hairpin monomers: HCR hairpin monomers are hybridized within the fixed sample and unused hairpins are washed away. Specifically-bound hairpin bridges with exposed HCR initiators will trigger growth of tethered fluorescent amplification polymers, generating amplified signal at the site of target molecules. HCR hairpin monomers that bind non-specifically in the sample do not trigger HCR polymerization and hence do not contribute to generation of amplified background, instead contributing a negligible amount of unamplified background.

Performance implications: Scheme D is not vulnerable to non-specific probe binding in Stage 1, as hairpin bridges predominantly do not base pair stably to isolated probes in Stage 2. Furthermore, Scheme D is not vulnerable to non-specific hairpin bridge binding in Stage 2, as non-specifically-bound hairpin bridges do not trigger amplification during Stage 3. Hence, Scheme D shares the important property with Scheme B that even if reagents bind non-specifically at any stage in the protocol, amplified background will predominantly not be generated. The advantage of Scheme D relative to Scheme B is that the bridge nucleation sites, and hence the HCR initiator and HCR hairpin monomer sequences, are independent of the target binding sites in the probes; as a result, a validated HCR initiator and amplifier can be used for diverse new probes and targets without changing the HCR initiator and amplifier sequences.

A drawback of Scheme D relative to Scheme B is the increase in number of stages from two to three.

Scheme E: Two-stage protocol: Target detection with unstructured fractional initiator (aka a split-initiator) probe pairs followed by probe -pair detection and HCR amplification using HCR hairpin monomers. Unstructured fractional initiator (aka a split-initiator) (a.k.a., fractional initiator) probes come in pairs; each probe contains a target-binding section (e.g., target binding sequence) and half of an unstructured HCR initiator; for the two probes within a probe pair, the two target-binding section (e.g., target binding sequence)s are complementary to target sections (e.g., proximal subsequences) of the target mRNA such that when the two probes bind specifically to their target sections (e.g., cognate target sites), the two halves of the HCR initiator are brought into proximity. HCR hairpin monomers predominantly do not interact except when they are initiated by a cognate HCR initiator (full initiator); initiation triggers polymerization via sequential hairpin nucleation and opening, yielding an HCR amplification polymer base-paired to the initiator (full initiator).

Stage 1: Target detection using unstructured fractional initiator (aka a split-initiator) probe pairs: Probes are hybridized within the fixed sample and unused probes are washed away. Probe pairs predominantly base-pair to their target sections (e.g., cognate target sites), co-localizing the two halves of the HCR initiator; some probes bind elsewhere in the sample, but probes that are bound non-specifically predominantly do not co-localize the two halves of the HCR initiator.

Stage 2: Probe-pair detection and HCR amplification using HCR hairpin monomers: HCR hairpin monomers are hybridized within the fixed sample and unused hairpins are washed away. Probe pairs that are hybridized specifically to their target sections (e.g., cognate target sites) co-localize the two halves of an HCR initiator, cooperatively initiating growth of tethered fluorescent HCR amplification polymers, generating amplified signal at the site of target molecules. Probes bound non-specifically do not co-localize the two halves of an HCR initiator, do not trigger HCR amplification, and thus avoid generating amplified background. HCR hairpin monomers that bind non-specifically in the sample do not trigger HCR polymerization and hence do not contribute to generation of amplified background, instead contributing a negligible amount of unamplified background.

Performance Implications: Scheme E is not vulnerable to non-specific probe binding in Stage 1, as isolated fractional initiator (aka a split-initiator) probes do not initiate HCR amplification in Stage 2. Hence, Scheme E shares the important property with Schemes B and D that active background suppression is provided at every stage of the protocol: even if reagents bind non-specifically at any stage in the protocol, amplified background will predominantly not be generated.

Compared to Scheme D, Scheme E has, among other advantages, the advantage that an HCR hairpin monomer serves the role of the hairpin bridge, thus eliminating the need for a separate hairpin bridge and reducing the number of stages in the protocol from three to two. Compared to Scheme C, Schemes D and E have, among other advantages, the advantage that the bridge will not contribute to generation of amplified background if it binds non-specifically. Compared to Scheme B, Scheme E has, among other advantages, the advantage that the HCR initiator and HCR hairpin monomers sequences are independent of the target-binding section (e.g., target binding sequences) in the probes. As a result, validated HCR initiators and HCR hairpin monomers can be used for diverse new probes and targets without changing the HCR initiator and HCR hairpin monomer sequences. Overall, Scheme E has the all of the benefits and none of the drawbacks of Schemes A, B, C, and D: the simplicity of a two-stage protocol, the versatility to re-use validated HCR initiators and HCR hairpin monomers with new target sequences, and the robustness to avoid generating amplified background even if reagents bind non-specifically in the sample at any stage of the protocol.

Figure 1 describes in situ amplification via hybridization chain reaction (HCR). (a) HCR mechanism. Metastable fluorescent hairpins self-assemble into fluorescent amplification polymers upon detection of a cognate initiator. Initiator I1 nucleates with the first hairpin monomer (e.g., hairpin H1) via base- pairing to single-stranded toehold 'a', mediating a branch migration that opens the hairpin to form complex I1 H1 containing single-stranded segment 'c*-b*'. This complex nucleates with the second hairpin monomer (hairpin H2) by means of base-pairing to toehold 'c', mediating a branch migration that opens the hairpin to form complex I1 1 H2 containing single-stranded segment 'b*-a*'. Thus, the initiator sequence is regenerated, providing the basis for a chain reaction of alternating H1 and H2 polymerization steps. Stars denote fluorophores. Arrowhead denotes 3' end of each strand. (b) In situ hybridization protocol (using standard probes of Scheme A).

With regard to the detection stage: fractional initiator probe sets are hybridized to mRNA targets and unused probes are washed from the sample. With regard to the amplification stage: initiators trigger self-assembly of tethered fluorescent amplification polymers and unused hairpins are washed from the sample. (c) Experimental timeline. The same two-stage protocol is used independent of the number of target mRNAs. For multiplexed experiments (three-color example depicted), fractional initiator probe sets for different target mRNAs (five probes depicted per set) carry orthogonal initiators that trigger orthogonal HCR amplification cascades labeled by spectrally distinct fluorophores.

Figure 2 provides the above noted schematics for five schemes, in summary form. Scheme A: Two-stage protocol: Target detection with an unstructured probe followed by probe detection and HCR amplification using HCR hairpin monomers. Scheme B: Two-stage protocol: Target detection using a hairpin probe followed by probe detection and HCR amplification using HCR hairpin monomers. Scheme C: Three-stage protocol: Target detection with unstructured probe pairs followed by probe-pair detection using an unstructured bridge followed by bridge detection and HCR amplification using HCR hairpins monomer. Scheme D: Three-stage protocol: Target detection with unstructured probe pairs followed by probe-pair detection using a hairpin bridge followed by bridge detection and HCR amplification using HCR hairpin monomers. Scheme E: Two-stage protocol: Target detection with unstructured fractional initiator (aka a split-initiator) probe pairs followed by probe-pair detection and HCR amplification using HCR hairpin monomers. Arrowhead denotes 3' end of each strand.

Figure 3: Alternative arrangements for two target-binding sites and two fractional initiators (HCR initiator fragments) within a split initiator probe pair. For each Arrangement (1,2,3,4,5), each probe within a probe pair carries half of the full HCR initiator (aka half of the HCR initiator I1); specific binding of the two probes within a pair to the cognate target molecule leads to colocalization of the two halves of the full HCR initiator (aka half of the HCR initiator I1). Arrowhead denotes 3' end of each strand.

Figure 4 provides additional embodiments in which the split initiator probes are colocalized by a target molecule. (a) Fractional initiator (aka a split-initiator) probes. First fractional initiator probe (Probe 1) and second fractional initiator probe (probe 2) each carry half of HCR initiator I1; selective binding of first fractional initiator probe (Probe 1) and second fractional initiator probe (Probe 2) to the target molecule colocalizes the two halves of HCR initiator I1. (b) Fractional initiator (aka a split-initiator) nucleic acid probes. Nucleic acid probe 1 and nucleic acid probe 2 each carry half of HCR initiator I1; selective binding of nucleic acid probe 1 and nucleic acid probe 2 to the target mRNA colocalizes the two halves of HCR initiator I1. (c) Fractional initiator (aka a split-initiator) antibody probes. Antibody probe 1 and antibody probe 2 each carry half of HCR initiator I1; selective binding of antibody probe 1 and antibody probe 2 to the protein target colocalizes the two halves of HCR initiator I1. (d) Split-initiator secondary-antibody probes. Primary-antibody probe 1 binds selectively to the protein target; secondary-antibody probe 2 and secondary-antibody probe 3 each carry half of HCR initiator I1; selective binding of secondary- antibody probe 2 and secondary-antibody probe 3 to primary-antibody probe 1 colocalizes the two halves of HCR initiator I1.

Figure 5 provides additional embodiments regarding fractional initiator (aka a split-initiator) probes colocalized by a target complex. (a) Fractional initiator (aka a split-initiator) probes. Target complex comprises molecule 1 bound to molecule 2; first fractional initiator probe (probe 1) and second fractional initiator probe (probe 2) each carry half of HCR initiator I1; selective binding of probe 1 to molecule 1 within the target complex and selective binding of probe 2 to molecule 2 within the target complex colocalizes the two halves of HCR initiator I1. (b) Fractional initiator (aka a split-initiator) nucleic acid probes. Target complex comprises nucleic acid target 1 bound to nucleic acid target 2; nucleic acid probe 1 and nucleic acid probe 2 each carry half of HCR initiator I1; selective binding of nucleic acid probe 1 to nucleic acid target 1 within the target complex and selective binding of nucleic acid probe 2 to nucleic acid target 2 within the target complex colocalizes the two halves of HCR initiator I1. (c) Fractional initiator (aka a split-initiator) antibody probes.

Target complex comprises protein target 1 bound to protein target 2; antibody first fractional initiator probe (probe 1) and antibody second fractional initiator probe (probe 2) each carry half of HCR initiator I1; selective binding of antibody first fractional initiator probe (probe 1) to protein target 1 within the target complex and selective binding of antibody second fractional initiator probe(probe 2) to protein target 2 within the target complex colocalizes the two halves of HCR initiator I1. (d) Fractional initiator (aka a split-initiator) secondary-antibody probes. Target complex comprises protein target 1 bound to protein target 2; primary-antibody first fractional initiator probe (probe 1) binds selectively to protein target 1 within the target complex and primary-antibody second fractional initiator probe (probe 2) binds selectively to protein target 2 within the target complex; secondary-antibody probe 3 and secondary-antibody probe 4 each carry half of HCR initiator I1; selective binding of secondary-antibody probe 3 to primary-antibody first fractional initiator probe (probe 1) and selective binding of secondary-antibody probe 4 to primary-antibody second fractional initiator probe (probe 2) colocalizes the two halves of HCR initiator I1. (e) Fractional initiator (aka a split-initiator) antibody and nucleic acid probes. Target complex comprises protein target 1 bound to nucleic acid target 2; antibody probe 1 and nucleic acid probe 2 each carry half of HCR initiator I1; binding of antibody probe 1 to protein target 1 within the target complex and binding of nucleic acid probe 2 to nucleic acid target 2 within the target complex colocalizes the two halves of HCR initiator I1.

The schematic of Figure 8 summarizes in situ HCR for imaging nucleic acid target molecules using fractional initiator (aka a split-initiator) nucleic acid probes (Scheme E). In situ HCR using fractional initiator (aka a split-initiator) probes (Scheme E). (a) Two-stage in situ HCR protocol. Detection stage: fractional initiator (aka a split-initiator) probe pairs are hybridized to the target mRNA and unused probes are washed from the sample. Each fractional initiator probe set contains one or more probe pairs that selectively bind to different subsequences along the target mRNA. Each probe within a pair carries a fraction of HCR initiator I1. Selective hybridization of the two probes within a pair to their cognate target binding sites colocalizes the two fractions of HCR initiator I1. Amplification stage: full HCR initiator I1 triggers self-assembly of tethered fluorescent amplification polymers and unused H1 and H2 hairpins are washed from the sample. Stars denote fluorophores. (b) Experimental timeline for a multiplexed experiment. A two-stage protocol is used independent of the number of target mRNAs.

### Hairpin Monomers

Two or more distinct species of nucleic acid hairpin monomers are preferably utilized in an HCR reaction. Each hairpin monomer species typically comprises at least one region that is complementary to a portion of another hairpin monomer species. However, the monomers are designed such that they are kinetically trapped and the system is unable to equilibrate in the absence of an initiator molecule that can disrupt the secondary structure of one of the monomers. Thus, the monomers are unable to polymerize in the absence of the initiator. Introduction of a full initiator species triggers a chain reaction of alternating kinetic escapes by the two or more monomer species resulting in formation of a polymer. In some embodiments, two hairpin monomers polymerize in the presence of an initiator to form a nicked, double-stranded polymer.

In some embodiments, two or more hairpin monomers are employed that have a hairpin structure. The hairpin monomers can comprise loops protected by long stems. In some embodiments, monomers with a different secondary structure are provided. However, the secondary structure can be such that the monomers are metastable under the reaction conditions in the absence of an initiator nucleic acid. In the presence of a full initiator, the secondary structure of a first hairpin monomer changes such that it is able to hybridize to a sticky end of a second hairpin monomer species. This in turn leads to a change in the secondary structure of the second hairpin monomer, which is then able to hybridize to another first hairpin monomer and continue the process. In this way, once a single copy of the first hairpin monomer interacts with a single copy of the initiator, a chain reaction is produced such that the hairpin monomers are able to assemble into a polymer comprising alternating hairpin monomer species.

A number of criteria can be used to design the monomers to achieve the desired properties. These include, for example and without limitation, sequence symmetry minimization, the probability of adopting the target secondary structure at equilibrium, the ensemble defect corresponding to the average number of incorrectly paired nucleotides at equilibrium relative to the target structure, the test tube ensemble defect corresponding to the concentration of incorrectly paired nucleotides at equilibrium, and hybridization kinetics.

Monomers can be synthesized using standard methods, including commercially available nucleic acid synthesizers or obtained from commercial sources such as Integrated DNA Technologies (Coralville, IA).

In some embodiments, monomers are derivitized with a compound or molecule to increase the molecular weight of the polymer resulting from HCR. Preferably they are derivitized at a location that does not interfere with their ability to hybridize. In some embodiments, monomers are derivitized with label binding site. In other embodiments monomers comprise a fluorophore or colorimetric compound that allows the resulting polymers to be visualized.

### Initiator

The full initiator can be a nucleic acid molecule. The full initiator is complementary to a portion of a hairpin monomer, preferably a portion of the hairpin monomer that is available for hybridization with the full initiator while the hairpin monomer is in its kinetically trapped state. The full initiator also preferably comprises a sequence that is complementary to a portion of the hairpin monomer adjacent to the sticky end such that hybridization of the full initiator to the sticky end causes a conformational change in the hairpin monomer and begins the HCR chain reaction. For example, the full initiator can comprise a region that is complementary to the first complementary region of the hairpin monomer, as described above.

In some embodiments, the sequence of the full initiator is complementary to the sticky end (initiator complementary region) and first complementary region of a first hairpin monomer. As described herein, in some embodiments this will also influence the sequence of the second complementary region and the loop of the second hairpin monomer species.

In some embodiments, the full initiator is a nucleic acid that is to be detected in a sample or a portion of a nucleic acid that is to be detected. In this case, the sequence of the target nucleic acid is taken into consideration in designing the HCR hairpin monomers. For example, the initiator complement region, preferably a sticky end, of one hairpin monomer is designed to be complementary to a portion of the target nucleic acid sequence. Because the second hairpin monomer will hybridize to the first hairpin monomer, the sequence of the second hairpin monomer will also reflect at least a portion of the sequence of the target nucleic acid.

In some embodiments, amplification of diverse recognition events is achieved by coupling HCR to nucleic acid aptamer triggers. An aptamer is identified that is able to specifically bind an analyte of interest. The analyte is not limited to a nucleic acid but may be, for example, a polypeptide or small molecule. The aptamer is linked to a nucleic acid comprising an initiator region in such a way that the initiator is unavailable to stimulate HCR in the absence of analyte binding to the aptamer.

### Detecting HCR

The products of HCR are readily detectable by methods known to one of skill in the art for the detection of nucleic acids, including, for example, agarose gel electrophoresis, polyacrylamide gel electrophoresis, capillary electrophoresis, and gel-filled capillary electrophoresis. As the polymers comprise nucleic acids, they can be visualized by standard techniques, such as staining with ethidium bromide. Other methods also can be suitable including light scattering spectroscopy, such as dynamic light scattering (DLS), viscosity measurement, colorimetric systems and fluorescence spectroscopy. As discussed in more detail, in some methods for *in situ* imaging and detection, HCR products are fluorescently labeled.

In some embodiments HCR is monitored by fluorescence resonance energy transfer (FRET). Certain monomers are labeled with fluorescent dyes so that conformational changes resulting from HCR can be monitored by detecting changes in fluorescence. In one embodiment, one of a pair of hairpin molecules is labeled with a fluorophore at the junction of the region complementary to the initiator strand and the duplex region and labeled at the opposing side of the duplex region with a quencher molecule. Upon polymerization, the fluorophore and quencher are separated spatially in the aggregated nucleic acid structure, providing relief of the fluorescence quenching. In this case, the presence of a single initiator is amplified by the chain of fluorescent events caused by HCR. In the context of *in situ* imaging, the presence of a single target molecule can be amplified by the chain of fluorescence events. In addition, for *in situ* imaging the quenching of fluorescence in unreacted monomers reduces background noise. Thus, unreacted monomers do not need to be removed from the sample.

Relative target abundance can be quantified for different samples by immobilizing the targets (e.g., via fixation within an embryo, or via crosslinking to a blot, or via binding to a bead) and then detecting the targets using fractional initiator probes that are colocalized by the target to trigger HCR, generating a signal that scales linearly with target abundance. If one or more of the samples has known target abundance, absolute quantitation can be performed for the other samples.

### Application to in situ imaging

Some embodiments of HCR provide for an enzyme-free approach to in situ amplification that can be multiplexed in parallel. Furthermore, HCR amplification triggered by fractional initiator probes provides a means for reducing the background signal resulting from nonspecific probe binding. Fractional initiator probes colocalize a full initiator only if they bind specifically to the cognate target. Hence, they trigger HCR if and only the target is specifically detected, leading to self-assembly of a tethered (to the target) non-covalent 'polymer' built from HCR monomers, preferably hairpin monomers as described above. The HCR monomers can be fluorescently labeled so that the polymers can be detected and the presence and/or location of the target determined.

The HCR hairpin monomers are also referred to herein as "amplifiers" because the polymerization of the monomers upon triggering by a full HCR initiator produces a detectable signal, which is amplified compared to the signal that would be produced by the binding of a single probe to the target. The amplifiers can each be labeled with the same or different fluorophores. For example, the system can be designed to use more than two monomer species per target, with at least one species fluorescently labeled. An HCR amplifier comprising two types of hairpin monomers can have a different fluorophore type labeling each of the two hairpin monomer types. An HCR amplifier comprising four hairpin monomer types can have four different fluorophores, one for each type of hairpin monomer. In such cases, the HCR amplification polymers would be decorated with multiple types of fluorophores in a known ratio (e.g., 1:1 for a polymer made from two alternating types of hairpin monomer, or 1:1:1:1 for a polymer made from four alternating types of hairpin monomer). Fluorescent labels are well known to one of skill in the art and include those, for example, in the "The Handbook - A Guide to Fluorescent Probes and Labeling Technologies," 10th Edition.

In some embodiments, amplifiers within the system are labeled with both a fluorophore and a quencher to form a construct analogous to a "molecular beacon" (Tyagi et al. Nature Biotechnology 14:303-308, 1996). For example, a hairpin monomer can comprise both a fluorophore and a quencher, such that the quencher reduces fluorescence while the monomer is in the hairpin form but not when the monomer is incorporated into an HCR polymer. Thus, molecular beacon versions of HCR monomers can reduce background signal resulting from any unpolymerized monomers, such as those that bind non-specifically or that simply remain unreacted in the sample.

For imaging of biological samples, it can be advantageous to use amplifier components that are small in size to allow penetration into the sample. For example, in some embodiments HCR components less than about 20 nanometers are used, e.g., less than 15 nm, 10 nm, and between about 8 and 16 nm. In some embodiments the HCR hairpin monomers are less than about 8 nm. Standard procedures for *in situ* imaging can be used to cause the HCR products to enter the sample. The skilled artisan will be able to select the appropriate methods for causing the HCR components to enter the sample.

Advantages of HCR for *in situ* imaging include, without limitation, the ability to rapidly amplify a signal based on a small amount of analyte present and the ability to image a diversity of analytes in the same sample.

As described herein the use of HCR for *in situ* detection and imaging provides a number of advantages. Specificity can be achieved using fractional initiator probes that colocalize a full initiator only of two or more fractional initiator probes bind specifically to their target sections (e.g., cognate target sites). Specificity can be achieved by using triggered probes that protect the initiators until the probes bind specifically to targets. Self-quenching HCR monomers can be labeled with fluorophore/quencher pairs that become separated during self-assembly into tethered amplification polymers. This automatic background suppression is particularly useful for in vivo applications where unused amplification components cannot be washed away before imaging. Versatility can be achieved by selecting structure-switching aptamers (Ellinton et al. Nature 346:818-822, 1990 and Tuerk et al. Science 249:505-510, 1990) that generalize the triggered probe concept to the detection of proteins and small molecules. Small probe and amplification monomers, preferably with maximum dimensions of 8-16 nm facilitate sample penetration. Isothermal conditions are ideal for HCR amplification, avoiding damage to the morphology of fixed samples or their components and facilitating in vivo imaging. Multiplexing follows naturally from the use of independent HCR amplifiers that operate simultaneously, for example using spectrally distinct fluorophores to encode unique combinatorial signatures directly into the structure of each HCR product. Sensitive quantitative amplification can be achieved using nonlinear HCR mechanisms that offer exponential growth into tethered polymers of a prescribed finite size. Finally, biocompatibility for in vivo applications follows from the use of nucleic acid amplifier components.

### Imaging Multiple Analytes

HCR amplification allows one to amplify multiple targets simultaneously. HCR targeting a number of analytes (for example, gene transcripts or proteins) can be used simultaneously. In some embodiments, each HCR system is labeled with a spectrally distinguishable dye. Accordingly, the number of analytes is equal to the number of spectrally distinguishable dyes that are available. For many situations, this will be sufficient.

To study the expression of multiple mRNAs or proteins, it is desirable to perform multiplexed amplification of all recognition events simultaneously using orthogonal HCR amplifiers. To increase the number of distinct targets that can be imaged using a limited supply of spectrally distinct fluorophores, the unamplified combinatorial multiplexing approach of Levsky and co-workers (Levsky et al. Science 297:836-840, 2002) can be adapted for HCR amplification by labeling the monomers for each amplifier with different unique dye combinations. The use of barcodes with a minimum of two colors provides a basis for screening single-color signals resulting from probes that are not bound specifically.

Therefore, in some embodiments only a single probe pair is used for each target and combinatorial multiplexing is performed by labeling the monomers for each HCR amplifier with different unique dye combinations.

If the HI and H2 hairpins are end-labeled with different dyes, the HCR product will carry an equal number of each dye by construction. In general, N spectrally distinct fluorophores can be used to address T: N!/[(N-2)!2!] targets with dual-color amplifiers (e.g., 4 dyes for 6 targets, 5 dyes for 10 targets). However, since combinatorial barcodes are not employed as a background diagnostic using this approach, the number of targets can be increased to T= N![N-2]!2! + N by allowing single-color amplifiers (e.g., 4 dyes for 10 targets, 5 dyes for 15 targets). Furthermore, it is possible to label HCR monomers with more than one dye to increase the number of targets that can be addressed up to T = Σ_{(i=1,N)}N!/[(N-i)!i!] = 2^{N} - 1 (e.g., 4 dyes for 15 targets, 5 dyes for 31 targets). HCR systems also can be designed that used M hairpins per amplifier.

In some embodiments, any one or more of the optional steps of any one or more of the methods herein can be combined with any one or more of the other optional steps of any one or more of the methods herein.

In some embodiments, any one or more of the steps that are different for any one or more of the methods herein can be combined with any one or more of the other steps that are different for any one or more of the methods herein.

In some embodiments, any one or more of the optional steps that are different for any one or more of the methods herein can be combined with any one or more of the other optional steps that are different for any one or more of the methods herein.

### Repeated Application Embodiments-Method 1

In some embodiments, a method for repeated signal detection with a reporter-labeled hairpin is provided.

In some embodiments, the method comprises: a) providing a sample possibly containing up to N targets as well as possibly other molecules that are not targets; b) providing N probe sets (each targeting one of N target types) each comprising either: i) one or more HCR initiator-labeled probes, or ii) one or more probe units each comprising two or more HCR fractional initiator probes; c) optionally washing the sample; d) providing M HCR amplifiers (for M ≤ N; each labeled with a distinct reporter) corresponding to M of the N probe sets; e) optionally washing the sample; f) detecting M signals corresponding to the M reporters; g) removing the M signals from the sample; and h) optionally repeating one or more of steps b-g until signal detection has been performed for all N targets. In some embodiments, when multiple probe sets are used, some of option i) and some of option ii) are used (with respect to the probe sets).

In some embodiments of the method, the probe sets used for zero, one or more targets each comprise one or more initiator-labeled probes and the probe sets used for zero, one, or more other targets each comprise one or more probe units each comprising two or more fractional initiator probes. In some embodiments, each probe set comprises one or more initiator-labeled probes. In some embodiments, each probe set comprises one or more probe units each comprising two or more fractional initiator probes. In some embodiments, the type of probe set used for each target is selected independently for each target based on the details of that target. In some embodiments, the HCR amplifiers further mediate CARD or repeated CARD.

In some embodiments, the method comprises: a) providing a sample possibly containing up to N targets as well as possibly other molecules that are not targets; b) providing N probe sets (each targeting one of N target types) each comprising either: i) one or more HCR initiator-labeled probes, or ii) one or more probe units each comprising two or more HCR fractional initiator probes; c) washing the sample; d) providing M HCR amplifiers (for M ≤ N; each labeled with a distinct reporter) corresponding to M of the N probe sets; e) washing the sample; f) detecting M signals corresponding to the M reporters; g) removing the M signals from the sample; and h) repeating one or more of steps b-g until signal detection has been performed for all N targets. In some embodiments, when multiple probe sets are used, some of option i) and some of option ii) are used (with respect to the probe sets).

In some embodiments of the method, the probe sets used for zero, one or more targets each comprise one or more initiator-labeled probes and the probe sets used for zero, one, or more other targets each comprise one or more probe units each comprising two or more fractional initiator probes. In some embodiments, each probe set comprises one or more initiator-labeled probes. In some embodiments, each probe set comprises one or more probe units each comprising two or more fractional initiator probes. In some embodiments, the type of probe set used for each target is selected independently for each target based on the details of that target. In some embodiments, the HCR amplifiers further mediate CARD or repeated CARD.

In some embodiments, a probe set comprises one or more initiator-labeled probes. In some embodiments, the number of initiator-labeled probes in a probe set could be in the range 1 to 1000, or 10 to 100, or 20 to 50, or 30 to 40. Increasing the number of initiator-labeled probes in a probe set could increase the signal-to-background ratio by 2-fold, or 5-fold, or 10-fold, or 100-fold, or more. Increasing the number of initiator-labeled probes in a probe set could increase the signal-to-background ratio to be 2, or 5, or 10, or 50, or 100, or 200, or 500, or 1000 or more depending on the number of initiator-labeled probes in the probe set, the level of background inherent to the sample, and the abundance of the target within the sample.

In some embodiments, an initiator-labeled probe comprises one or more target-binding domains and one or more HCR initiators (for example, FIGs. 39A-39N and 42A-42F).

In some embodiments, a probe set comprises one or more probe units. In some embodiments, the number of probe units in a probe set could be in the range 1 to 1000, or 10 to 100, or 20 to 50, or 30 to 40. Increasing the number of probe units in a probe set could increase the signal-to-background ratio by 2-fold, or 5-fold, or 10-fold, or 100-fold, or more. Increasing the number of probe units in a probe set could increase the signal-to-background ratio to be 2, or 5, or 10, or 50, or 100, or 200, or 500, or 1000 or more depending on the number of probe units in the probe set, the level of background inherent to the sample, and the abundance of the target within the sample.

In some embodiments, when multiple probe sets are used, some of option i) (one or more HCR initiator-labeled probes) and some of option ii) (one or more probe units each comprising two or more HCR fractional initiator probes) are used together. In some embodiments, when N probe sets are used, all of them are option i) or all of them are option ii) or a mix of option i) and ii) are used, so as to result in a ratio of, for example, 1:1000, 1:100, 1:50, 1:25, 1:20, 1:10, 1:5, 1:4, 1:3, 1:2, 1:1, 2:1, 3:1, 4:1, 5:1, 10:1. 20:1, 25:1, 50:1, 100:1, 1000:1, or any range defined between any two of the preceding ratios. These mixes and ratios can be applied to any of the embodiments provided herein where more than one probe set is used.

In some embodiments, a probe unit comprises two or more HCR fractional initiator probes. In some embodiments, a probe unit comprises two fractional initiator probes that together generate a full HCR initiator (fraction f1 for probe P1 and fraction f2 for probe P2 such that f1 + f2 = 1).

In some embodiments, an HCR fractional initiator probe comprises a target-binding region and a fractional initiator. In some embodiments, binding of each probe within a probe unit to adjacent cognate binding sites on the target colocalizes the fractional initiators to form a full HCR initiator (for example, see the full HCR initiators of Figures 3A-5E, 8A-8B, 16A-16D, 21A-22, and 27) capable of hybridizing to an HCR hairpin to trigger HCR signal amplification. In some embodiments, the target-binding regions within a probe unit are configured to bind to overlapping or non-overlapping regions of the target (for example, see Figures 8A-8B, 21A-21B, 22, and 27). In some embodiments, the fractional initiators within a probe unit are designed to hybridize to overlapping or non-overlapping regions of an HCR hairpin (for example, see Figures 8A-8B, 21A-22, and 27). Individual probes that bind non-specifically in the sample do not colocalize a full HCR initiator, suppressing HCR signal amplification.

In some embodiments, an HCR amplifier comprises two or more HCR hairpins (for example, see the HCR amplifiers of Figures 8A-8B, 18A-18F, and 19A-19B).

In some embodiments, an HCR hairpin comprises an input domain comprising a single-stranded toehold and a stem section.

In some embodiments, an HCR hairpin further comprises an output domain comprising a single-stranded loop and a complement to the stem section.

In some embodiments, each HCR hairpin comprises an input domain with a single-stranded toehold and a stem section, and an output domain with a single-stranded loop and a complement to the stem section (for example, see the HCR hairpins of Figures 8A-8B, 13, 14, 18A-18F, and 19A-19B). In the absence of a full HCR initiator, HCR hairpins are kinetically trapped and do not polymerize, suppressing background. However, if the fractional initiator probes within a probe unit bind to their adjacent cognate binding sites on the target to colocalize a full HCR initiator, the full HCR initiator initiates a chain reaction of polymerization steps in which the full initiator hybridizes to the input domain of a first HCR hairpin, opening the first hairpin to expose its output domain, which in turn hybridizes to the input domain of a second HCR hairpin, opening the second hairpin to expose its output domain, and so on and so forth, leading to a chain reaction in which hairpins polymerize to yield an HCR amplification polymer tethered to the target (for example, see the amplification polymers of Figures 8A, 13, 14, 18C-18F, and 19A-19B). In some embodiments, an HCR hairpin further comprises one or more labels, each label comprising a reporter or comprising a substrate (or a fractional substrate) that recruits a label probe comprising one or more reporters (for example, see the reporter-labeled, substrate-labeled, and fractional-substrate-labeled HCR hairpins of Figure 18A-18F).

In some embodiments, a label probe comprises one or more reporters and further comprises a substrate-binding region complementary to a substrate on an HCR hairpin or complementary to a full substrate colocalized within an HCR amplification polymer (for example, see the label probes of Figure 20A-20F). In some embodiments, signal is generated by one or more reporters associated with an HCR amplification polymer tethered to the target within the sample. In some embodiments, signal is removed from the sample (for example, see FIGs. 23A-23O and 40A-40N). In some embodiments, HCR signal is generated, detected, and removed from the same sample one or more times (for example, see FIGs. 23A-23O, 26A-26T, and 40A-40N).

In some embodiments, an HCR hairpin further comprises a reporter. For example, a reporter could be a fluorophore, a chromophore, a luminophore, a phosphor, a FRET pair, a member of a FRET pair, a quencher, a fluorophore/quencher pair, a rare-earth element or compound, a radioactive molecule, a magnetic molecule, or any other molecule that facilitates measurement of a signal.

### Method 2

In some embodiments, a method for repeated signal detection with a reporter-labeled hairpin is provided.

In some embodiments, the method comprises: a) providing a sample possibly containing one or more targets as well as possibly other molecules that are not targets; b) providing one or more probe sets each comprising either: i) one or more HCR initiator-labeled probes, or ii) one or more probe units each comprising two or more HCR fractional initiator probes; c) optionally washing the sample; d) providing one or more HCR amplifiers (each labeled with one or more reporters); e) optionally washing the sample; f) detecting one or more signals from one or more reporters; g) optionally removing one or more probe sets from the sample; h) optionally removing one or more HCR amplifiers from the sample; i) optionally removing one or more reporters from the sample; and j) optionally removing one or more signals from the sample. In some embodiments, when multiple probe sets are used, some of option i) and some of option ii) are used (with respect to the probe sets).

In some embodiments of the method, the probe sets used for zero, one or more targets each comprise one or more initiator-labeled probes and the probe sets used for zero, one, or more other targets each comprise one or more probe units each comprising two or more fractional initiator probes. In some embodiments, each probe set comprises one or more initiator-labeled probes. In some embodiments, each probe set comprises one or more probe units each comprising two or more fractional initiator probes. In some embodiments, the type of probe set used for each target is selected independently for each target based on the details of that target. In some embodiments, the HCR amplifiers further mediate CARD or repeated CARD.

n some embodiments, the method comprises: a) providing a sample possibly containing one or more targets as well as possibly other molecules that are not targets; b) providing one or more probe sets each comprising either: i) one or more HCR initiator-labeled probes, or ii) one or more probe units each comprising two or more HCR fractional initiator probes; c) washing the sample; d) providing one or more HCR amplifiers (each labeled with one or more reporters); e) washing the sample; f) detecting one or more signals from one or more reporters; g) removing one or more probe sets from the sample; h) removing one or more HCR amplifiers from the sample; i) optionally removing one or more reporters from the sample; and j) removing one or more signals from the sample. In some embodiments, when multiple probe sets are used, some of option i) and some of option ii) are used (with respect to the probe sets).

In some embodiments of the method, the probe sets used for zero, one or more targets each comprise one or more initiator-labeled probes and the probe sets used for zero, one, or more other targets each comprise one or more probe units each comprising two or more fractional initiator probes. In some embodiments, each probe set comprises one or more initiator-labeled probes. In some embodiments, each probe set comprises one or more probe units each comprising two or more fractional initiator probes. In some embodiments, the type of probe set used for each target is selected independently for each target based on the details of that target. In some embodiments, the HCR amplifiers further mediate CARD or repeated CARD.

In some embodiments, a probe set comprises one or more initiator-labeled probes. In some embodiments, the number of initiator-labeled probes in a probe set could be in the range 1 to 1000, or 10 to 100, or 20 to 50, or 30 to 40. Increasing the number of initiator-labeled probes in a probe set could increase the signal-to-background ratio by 2-fold, or 5-fold, or 10-fold, or 100-fold, or more. Increasing the number of initiator-labeled probes in a probe set could increase the signal-to-background ratio to be 2, or 5, or 10, or 50, or 100, or 200, or 500, or 1000 or more depending on the number of initiator-labeled probes in the probe set, the level of background inherent to the sample, and the abundance of the target within the sample.

In some embodiments, an initiator-labeled probe comprises one or more target-binding domains and one or more HCR initiators (for example, FIGs. 39A-39N and 42A-42F).

In some embodiments, a probe set comprises one or more probe units. In some embodiments, the number of probe units in a probe set could be in the range 1 to 1000, or 10 to 100, or 20 to 50, or 30 to 40. Increasing the number of probe units in a probe set could increase the signal-to-background ratio by 2-fold, or 5-fold, or 10-fold, or 100-fold, or more. Increasing the number of probe units in a probe set could increase the signal-to-background ratio to be 2, or 5, or 10, or 50, or 100, or 200, or 500, or 1000 or more depending on the number of probe units in the probe set, the level of background inherent to the sample, and the abundance of the target within the sample.

In some embodiments, a probe unit comprises two or more HCR fractional initiator probes. In some embodiments, a probe unit comprises two fractional initiator probes that together generate a full HCR initiator (fraction f1 for probe P1 and fraction f2 for probe P2 such that f1 + f2 = 1).

In some embodiments, an HCR fractional initiator probe comprises a target-binding region and a fractional initiator. In some embodiments, binding of each probe within a probe unit to adjacent cognate binding sites on the target colocalizes the fractional initiators to form a full HCR initiator (for example, see the full HCR initiators of Figures 3A-5E, 8A-8B, 16A-16D, 21A-22, and 27) capable of hybridizing to an HCR hairpin to trigger HCR signal amplification. In some embodiments, the target-binding regions within a probe unit are configured to bind to overlapping or non-overlapping regions of the target (for example, see Figures 8A-8B, 21A-21B, 22, and 27). In some embodiments, the fractional initiators within a probe unit are designed to hybridize to overlapping or non-overlapping regions of an HCR hairpin (for example, see Figures 8A-8B, 21A-22, and 27). Individual probes that bind non-specifically in the sample do not colocalize a full HCR initiator, suppressing HCR signal amplification.

In some embodiments, an HCR amplifier comprises two or more HCR hairpins (for example, see the HCR amplifiers of Figures 8A-8B, 18A-18F, and 19A-19B).

In some embodiments, an HCR hairpin comprises an input domain comprising a single-stranded toehold and a stem section.

In some embodiments, an HCR hairpin further comprises an output domain comprising a single-stranded loop and a complement to the stem section.

In some embodiments, an HCR hairpin further comprises one or more reporters. In some embodiments, the one or more reporters is a fluorophore, a chromophore, a luminophore, a phosphor, a FRET pair, a member of a FRET pair, a quencher, a fluorophore/quencher pair, a rare-earth element or compound, a radioactive molecule, a magnetic molecule, or any other molecule that facilitates measurement of a signal.

### Method 3

In some embodiments, a method of repeated signal detection with substrate-labeled hairpins is provided.

In some embodiments, the method comprises: a) providing a sample possibly containing up to N targets as well as possibly other molecules that are not targets; b) providing N probe sets each comprising either: i) one or more HCR initiator-labeled probes, or ii) one or more probe units each comprising two or more HCR fractional initiator probes; c) optionally washing the sample; d) providing N HCR amplifiers (each labeled with a distinct substrate) corresponding to the N probe sets; e) optionally washing the sample; f) providing M label probes (for M ≤ N; each conjugated to a distinct reporter) corresponding to M of the N distinct substrates; g) optionally washing the sample; h) detecting M signals corresponding to the M distinct reporters; i) removing the M signals from the sample; and j) optionally repeating one or more of steps f-i until signal detection has been performed for all N targets. In some embodiments, when multiple probe sets are used, some of option i) and some of option ii) are used (with respect to the probe sets).

In some embodiments of the method, the probe sets used for zero, one or more targets each comprise one or more initiator-labeled probes and the probe sets used for zero, one, or more other targets each comprise one or more probe units each comprising two or more fractional initiator probes. In some embodiments, each probe set comprises one or more initiator-labeled probes. In some embodiments, each probe set comprises one or more probe units each comprising two or more fractional initiator probes. In some embodiments, the type of probe set used for each target is selected independently for each target based on the details of that target. In some embodiments, the HCR amplifiers further mediate CARD or repeated CARD.

In some embodiments, the method comprises: a) providing a sample possibly containing up to N targets as well as possibly other molecules that are not targets; b) providing N probe sets each comprising either: i) one or more HCR initiator-labeled probes, or ii) one or more probe units each comprising two or more HCR fractional initiator probes; c) washing the sample; d) providing N HCR amplifiers (each labeled with a distinct substrate) corresponding to the N probe sets; e) washing the sample; f) providing M label probes (for M ≤ N; each conjugated to a distinct reporter) corresponding to M of the N distinct substrates; g) washing the sample; h) detecting M signals corresponding to the M distinct reporters; i) removing the M signals from the sample; and j) repeating one or more of steps f-i until signal detection has been performed for all N targets. In some embodiments, when multiple probe sets are used, some of option i) and some of option ii) are used (with respect to the probe sets).

In some embodiments of the method, the probe sets used for zero, one or more targets each comprise one or more initiator-labeled probes and the probe sets used for zero, one, or more other targets each comprise one or more probe units each comprising two or more fractional initiator probes. In some embodiments, each probe set comprises one or more initiator-labeled probes. In some embodiments, each probe set comprises one or more probe units each comprising two or more fractional initiator probes. In some embodiments, the type of probe set used for each target is selected independently for each target based on the details of that target. In some embodiments, the HCR amplifiers further mediate CARD or repeated CARD.

In some embodiments, a probe set comprises one or more initiator-labeled probes. In some embodiments, the number of initiator-labeled probes in a probe set could be in the range 1 to 1000, or 10 to 100, or 20 to 50, or 30 to 40. Increasing the number of initiator-labeled probes in a probe set could increase the signal-to-background ratio by 2-fold, or 5-fold, or 10-fold, or 100-fold, or more. Increasing the number of initiator-labeled probes in a probe set could increase the signal-to-background ratio to be 2, or 5, or 10, or 50, or 100, or 200, or 500, or 1000 or more depending on the number of initiator-labeled probes in the probe set, the level of background inherent to the sample, and the abundance of the target within the sample.

In some embodiments, an initiator-labeled probe comprises one or more target-binding domains and one or more HCR initiators (for example, FIGs. 39A-39N and 42A-42F).

In some embodiments, a probe set comprises one or more probe units. In some embodiments, the number of probe units in a probe set could be in the range 1 to 1000, or 10 to 100, or 20 to 50, or 30 to 40. Increasing the number of probe units in a probe set could increase the signal-to-background ratio by 2-fold, or 5-fold, or 10-fold, or 100-fold, or more. Increasing the number of probe units in a probe set could increase the signal-to-background ratio to be 2, or 5, or 10, or 50, or 100, or 200, or 500, or 1000 or more depending on the number of probe units in the probe set, the level of background inherent to the sample, and the abundance of the target within the sample.

In some embodiments, a probe unit comprises two or more HCR fractional initiator probes. In some embodiments, a probe unit comprises two fractional initiator probes that together generate a full HCR initiator (fraction f1 for probe P1 and fraction f2 for probe P2 such that f1 + f2 = 1).

In some embodiments, an HCR fractional initiator probe comprises a target-binding region and a fractional initiator. In some embodiments, binding of each probe within a probe unit to adjacent cognate binding sites on the target colocalizes the fractional initiators to form a full HCR initiator (for example, see the full HCR initiators of Figures 3A-5E, 8A-8B, 16A-16D, 21A-22, and 27) capable of hybridizing to an HCR hairpin to trigger HCR signal amplification. In some embodiments, the target-binding regions within a probe unit are configured to bind to overlapping or non-overlapping regions of the target (for example, see Figures 8A-8B, 21A-21B, 22, and 27). In some embodiments, the fractional initiators within a probe unit are designed to hybridize to overlapping or non-overlapping regions of an HCR hairpin (for example, see Figures 8A-8B, 21A-22, and 27). Individual probes that bind non-specifically in the sample do not colocalize a full HCR initiator, suppressing HCR signal amplification.

In some embodiments, an HCR amplifier comprises two or more HCR hairpins (for example, see the HCR amplifiers of Figures 8A-8B, 18A-18F, and 19A-19B).

In some embodiments, an HCR hairpin comprises an input domain comprising a single-stranded toehold and a stem section.

In some embodiments, an HCR hairpin further comprises an output domain comprising a single-stranded loop and a complement to the stem section.

In some embodiments, an HCR hairpin further comprises a substrate. In some embodiments, the substrate serves to recruit a reporter entity that directly or indirectly mediates localization of reporters in the vicinity of the hairpin label.

In some embodiments, the hairpin label can comprise digoxigenin (DIG) that recruits anti-DIG antibody as the reporter entity, where the anti-DIG is directly labeled with one or more reporters, or with one or more substrates that serve to directly or indirectly mediate localization of reporters in the vicinity of the hairpin label.

In some embodiments, the hairpin label can comprise a nucleic acid domain that serves as a substrate with full or partial sequence complementarity to a domain within a label probe that further comprises one or more reporters,
In some embodiments, the hairpin label can comprise a nucleic acid domain that serves as a substrate with full or partial sequence complementarity to a domain within a label probe that carries one or more substrates that serve to mediate localization of reporters in the vicinity of the hairpin label.

In some embodiments, the hairpin label can comprise a nucleic acid domain that serves as a substrate for a reporter entity that directly or indirectly mediates localization of reporters in the vicinity of the hairpin label.

In some embodiments, the hairpin label can comprise a substrate that serves to recruit a reporter entity that indirectly mediates localization of reporters in the vicinity of the hairpin label.

In some embodiments, the hairpin label can comprise a substrate that serves to recruit a reporter entity that comprises an enzyme that catalyzes a CARD-substrate leading to deposition of reporter molecules in the vicinity of the hairpin (see for example, 33A-33E, 34A-34C, 36A-36B). For example:
1. the enzyme could be horseradish peroxidase (HRP) (or polymer HRP comprising multiple HRP enzymes) that acts on a CARD-substrate to catalyze deposition a chromogenic reporter such as AEC, DAB, TMB, or StayYellow, or that catalyzes a CARD-substrate to catalyze deposition of a fluorescent reporter such as fluophore-labeled tyramide, or that catalyzes deposition of a hapten-labeled CARD-substrate such as biotin-labeled tyramide, where the hapten serves to mediate localization of reporters in the vicinity of the hairpin label.
2. the enzyme could be alkaline phosphatase (AP) (or polymer AP comprising multiple AP enzymes) that acts on a CARD-substrate to catalyze deposition of reporters, for example a chromogenic reporter such as but not limited to BCIP/NBT, BCIP/TNBT, Napthol AS-MX phosphate + FastBlue BB, Napthol AS-MX phosphate + FastRed TR, StayGreen.
3. the enzyme could be glucose oxidase that acts on a CARD-substrate to catalyze deposition of reporters, for example NBT,
4. the enzyme could be any molecule or complex that directly or indirectly mediates localization of reporters in the vicinity of a hairpin label.

In some embodiments, a label probe comprises one or more reporters and further comprises a substrate-binding region complementary to a substrate on an HCR hairpin or complementary to a full substrate colocalized within an HCR amplification polymer (for example, see the label probes of Figure 20A-20F). In some embodiments, signal is generated by one or more reporters associated with an HCR amplification polymer tethered to the target within the sample. In some embodiments, signal is removed from the sample (for example, see FIGs. 23A-23O and 40A-40N). In some embodiments, HCR signal is generated, detected, and removed from the same sample one or more times (for example, see FIGs. 23A-23O, 26A-26T, and 40A-40N).

In some embodiments, an HCR hairpin further comprises a reporter. For example, a reporter could be a fluorophore, a chromophore, a luminophore, a phosphor, a FRET pair, a member of a FRET pair, a quencher, a fluorophore/quencher pair, a rare-earth element or compound, a radioactive molecule, a magnetic molecule, or any other molecule that facilitates measurement of a signal.

### Method 4

In some embodiments, a method of repeated signal detection with substrate-labeled hairpins is provided.

In some embodiments, the method comprises: a) providing a sample possibly containing one or more targets as well as possibly other molecules that are not targets; b) providing one or more probe sets each comprising either: i) one or more HCR initiator-labeled probes, or ii) one or more probe units each comprising two or more HCR fractional initiator probes; c) optionally washing the sample; d) providing one or more HCR amplifiers (each labeled with a substrate) corresponding to one or more probe sets; e) optionally washing the sample; f) providing one or more label probes (each conjugated to a reporter) corresponding to one or more substrates; g) optionally washing the sample; h) detecting one or more signals corresponding to one or more reporters; i) removing one or more signals from the sample; and j) optionally repeating any of steps b-i one or more times in any order. In some embodiments, when multiple probe sets are used, some of option i) and some of option ii) are used (with respect to the probe sets).

In some embodiments of the method, the probe sets used for zero, one or more targets each comprise one or more initiator-labeled probes and the probe sets used for zero, one, or more other targets each comprise one or more probe units each comprising two or more fractional initiator probes. In some embodiments, each probe set comprises one or more initiator-labeled probes. In some embodiments, each probe set comprises one or more probe units each comprising two or more fractional initiator probes. In some embodiments, the type of probe set used for each target is selected independently for each target based on the details of that target. In some embodiments, the HCR amplifiers further mediate CARD or repeated CARD.

In some embodiments, a method of repeated signal detection with substrate-labeled hairpins is provided. In some embodiments, the method comprises: a) providing a sample possibly containing one or more targets as well as possibly other molecules that are not targets; b) providing one or more probe sets each comprising either: i) one or more HCR initiator-labeled probes, or ii) one or more probe units each comprising two or more HCR fractional initiator probes; c) washing the sample; d) providing one or more HCR amplifiers (each labeled with a substrate) corresponding to one or more probe sets; e) washing the sample; f) providing one or more label probes (each conjugated to a reporter) corresponding to one or more substrates; g) washing the sample; h) detecting one or more signals corresponding to one or more reporters; i) removing one or more signals from the sample; and j) repeating any of steps b-i one or more times in any order. In some embodiments, when multiple probe sets are used, some of option i) and some of option ii) are used (with respect to the probe sets).

In some embodiments of the method, the probe sets used for zero, one or more targets each comprise one or more initiator-labeled probes and the probe sets used for zero, one, or more other targets each comprise one or more probe units each comprising two or more fractional initiator probes. In some embodiments, each probe set comprises one or more initiator-labeled probes. In some embodiments, each probe set comprises one or more probe units each comprising two or more fractional initiator probes. In some embodiments, the type of probe set used for each target is selected independently for each target based on the details of that target. In some embodiments, the HCR amplifiers further mediate CARD or repeated CARD.

In some embodiments, a probe set comprises one or more initiator-labeled probes. In some embodiments, the number of initiator-labeled probes in a probe set could be in the range 1 to 1000, or 10 to 100, or 20 to 50, or 30 to 40. Increasing the number of initiator-labeled probes in a probe set could increase the signal-to-background ratio by 2-fold, or 5-fold, or 10-fold, or 100-fold, or more. Increasing the number of initiator-labeled probes in a probe set could increase the signal-to-background ratio to be 2, or 5, or 10, or 50, or 100, or 200, or 500, or 1000 or more depending on the number of initiator-labeled probes in the probe set, the level of background inherent to the sample, and the abundance of the target within the sample.

In some embodiments, an initiator-labeled probe comprises one or more target-binding domains and one or more HCR initiators (for example, FIGs. 39A-39N and 42A-42F).

In some embodiments, a probe set comprises one or more probe units. In some embodiments, the number of probe units in a probe set could be in the range 1 to 1000, or 10 to 100, or 20 to 50, or 30 to 40. Increasing the number of probe units in a probe set could increase the signal-to-background ratio by 2-fold, or 5-fold, or 10-fold, or 100-fold, or more. Increasing the number of probe units in a probe set could increase the signal-to-background ratio to be 2, or 5, or 10, or 50, or 100, or 200, or 500, or 1000 or more depending on the number of probe units in the probe set, the level of background inherent to the sample, and the abundance of the target within the sample.

In some embodiments, a probe unit comprises two or more HCR fractional initiator probes. In some embodiments, a probe unit comprises two fractional initiator probes that together generate a full HCR initiator (fraction f1 for probe P1 and fraction f2 for probe P2 such that f1 + f2 = 1).

In some embodiments, an HCR fractional initiator probe comprises a target-binding region and a fractional initiator. In some embodiments, binding of each probe within a probe unit to adjacent cognate binding sites on the target colocalizes the fractional initiators to form a full HCR initiator (for example, see the full HCR initiators of Figures 3A-5E, 8A-8B, 16A-16D, 21A-22, and 27) capable of hybridizing to an HCR hairpin to trigger HCR signal amplification. In some embodiments, the target-binding regions within a probe unit are configured to bind to overlapping or non-overlapping regions of the target (for example, see Figures 8A-8B, 21A-21B, 22, and 27). In some embodiments, the fractional initiators within a probe unit are designed to hybridize to overlapping or non-overlapping regions of an HCR hairpin (for example, see Figures 8A-8B, 21A-22, and 27). Individual probes that bind non-specifically in the sample do not colocalize a full HCR initiator, suppressing HCR signal amplification.

In some embodiments, an HCR amplifier comprises two or more HCR hairpins (for example, see the HCR amplifiers of Figures 8A-8B, 18A-18F, and 19A-19B).

In some embodiments, an HCR hairpin comprises an input domain comprising a single-stranded toehold and a stem section.

In some embodiments, an HCR hairpin further comprises an output domain comprising a single-stranded loop and a complement to the stem section.

In some embodiments, an HCR hairpin further comprises a substrate. In some embodiments, the substrate that serves to recruit a reporter entity that directly or indirectly mediates localization of reporters in the vicinity of the hairpin label.

In some embodiments, a label probe comprises a substrate-binding region and a reporter (for example, see Figures 18C-E and 20). For example, a reporter could be a fluorophore, a chromophore, a luminophore, a phosphor, a FRET pair, a member of a FRET pair, a quencher, a fluorophore/quencher pair, a rare-earth element or compound, a radioactive molecule, a magnetic molecule, or any other molecule that facilitates measurement of a signal.

### Method 5

In some embodiments, method of repeated signal detection with reporter and/or substrate-labeled hairpins is provided.

In some embodiments, the method comprises: a) providing a sample possibly containing one or more targets as well as possibly other molecules that are not targets; b) providing one or more HCR probe sets each comprising either: i) one or more HCR initiator-labeled probes, or ii) one or more probe units each comprising two or more HCR fractional initiator probes; c) providing one or more HCR amplifiers (each labeled with one or more reporters and/or one or more substrates) corresponding to one or more probe sets; d) optionally providing one or more label probes (each conjugated to one or more reporters) corresponding to one or more substrates; e) detecting one or more signals; f) optionally washing the sample; g) optionally removing one or more signals from the sample; h) optionally removing one or more reporters from the sample; i) optionally removing one or more label probes from the sample; j) optionally removing one or more HCR amplifiers from the sample; k) optionally removing one or more probe sets from the sample; and l) optionally repeating any of the above steps in any order. In some embodiments, when multiple probe sets are used, some of option i) and some of option ii) are used (with respect to the probe sets).

In some embodiments of the method, the probe sets used for zero, one or more targets each comprise one or more initiator-labeled probes and the probe sets used for zero, one, or more other targets each comprise one or more probe units each comprising two or more fractional initiator probes. In some embodiments, each probe set comprises one or more initiator-labeled probes. In some embodiments, each probe set comprises one or more probe units each comprising two or more fractional initiator probes. In some embodiments, the type of probe set used for each target is selected independently for each target based on the details of that target. In some embodiments, the HCR amplifiers further mediate CARD or repeated CARD.

In some embodiments, a method of repeated signal detection with reporter and/or substrate-labeled hairpins is provided. In some embodiments, the method comprises: a) providing a sample possibly containing one or more targets as well as possibly other molecules that are not targets; b) providing one or more HCR probe sets each comprising either: i) one or more HCR initiator-labeled probes, or ii) one or more probe units each comprising two or more HCR fractional initiator probes; c) providing one or more HCR amplifiers (each labeled with one or more reporters and/or one or more substrates) corresponding to one or more probe sets; d) providing one or more label probes (each conjugated to one or more reporters) corresponding to one or more substrates; e) detecting one or more signals; f) washing the sample; g) removing one or more signals from the sample; h) optionally removing one or more reporters from the sample; i) removing one or more label probes from the sample; j) removing one or more HCR amplifiers from the sample; k) removing one or more probe sets from the sample; and l) repeating any of the above steps in any order. In some embodiments, when multiple probe sets are used, some of option i) and some of option ii) are used (with respect to the probe sets).

In some embodiments of the method, the probe sets used for zero, one or more targets each comprise one or more initiator-labeled probes and the probe sets used for zero, one, or more other targets each comprise one or more probe units each comprising two or more fractional initiator probes. In some embodiments, each probe set comprises one or more initiator-labeled probes. In some embodiments, each probe set comprises one or more probe units each comprising two or more fractional initiator probes. In some embodiments, the type of probe set used for each target is selected independently for each target based on the details of that target. In some embodiments, the HCR amplifiers further mediate CARD or repeated CARD.

In some embodiments, a probe set comprises one or more initiator-labeled probes. In some embodiments, the number of initiator-labeled probes in a probe set could be in the range 1 to 1000, or 10 to 100, or 20 to 50, or 30 to 40. Increasing the number of initiator-labeled probes in a probe set could increase the signal-to-background ratio by 2-fold, or 5-fold, or 10-fold, or 100-fold, or more. Increasing the number of initiator-labeled probes in a probe set could increase the signal-to-background ratio to be 2, or 5, or 10, or 50, or 100, or 200, or 500, or 1000 or more depending on the number of initiator-labeled probes in the probe set, the level of background inherent to the sample, and the abundance of the target within the sample.

In some embodiments, an initiator-labeled probe comprises one or more target-binding domains and one or more HCR initiators (for example, FIGs. 39A-39N and 42A-42F).

In some embodiments, a probe set comprises one or more probe units. In some embodiments, the number of probe units in a probe set could be in the range 1 to 1000, or 10 to 100, or 20 to 50, or 30 to 40. Increasing the number of probe units in a probe set could increase the signal-to-background ratio by 2-fold, or 5-fold, or 10-fold, or 100-fold, or more. Increasing the number of probe units in a probe set could increase the signal-to-background ratio to be 2, or 5, or 10, or 50, or 100, or 200, or 500, or 1000 or more depending on the number of probe units in the probe set, the level of background inherent to the sample, and the abundance of the target within the sample.

In some embodiments, a probe unit comprises two or more HCR fractional initiator probes. In some embodiments, a probe unit comprises two fractional initiator probes that together generate a full HCR initiator (fraction f1 for probe P1 and fraction f2 for probe P2 such that f1 + f2 = 1).

In some embodiments, an HCR fractional initiator probe comprises a target-binding region and a fractional initiator. In some embodiments, binding of each probe within a probe unit to adjacent cognate binding sites on the target colocalizes the fractional initiators to form a full HCR initiator (for example, see the full HCR initiators of Figures 3A-5E, 8A-8B, 16A-16D, 21A-22, and 27) capable of hybridizing to an HCR hairpin to trigger HCR signal amplification. In some embodiments, the target-binding regions within a probe unit are configured to bind to overlapping or non-overlapping regions of the target (for example, see Figures 8A-8B, 21A-21B, 22, and 27). In some embodiments, the fractional initiators within a probe unit are designed to hybridize to overlapping or non-overlapping regions of an HCR hairpin (for example, see Figures 8A-8B, 21A-22, and 27). Individual probes that bind non-specifically in the sample do not colocalize a full HCR initiator, suppressing HCR signal amplification.

In some embodiments, an HCR amplifier comprises two or more HCR hairpins (for example, see the HCR amplifiers of Figures 8A-8B, 18A-18F, and 19A-19B).

In some embodiments, an HCR hairpin comprises an input domain comprising a single-stranded toehold and a stem section.

In some embodiments, an HCR hairpin further comprises an output domain comprising a single-stranded loop and a complement to the stem section.

In some embodiments, an HCR hairpin further comprises one or more reporters and/or one or more substrates.

In some embodiments, a label probe comprises a substrate-binding region and one or more reporters.

### Method 6

In some embodiments, a method of repeated signal detection with reporter and/or substrate-labeled hairpins is provided.

In some embodiments, the method comprises: a) providing a sample possibly containing one or more targets as well as possibly other molecules that are not targets; b) performing any of steps c-g one or more times in any order; c) providing one or more HCR probe sets each comprising either: i) one or more HCR initiator-labeled probes, or ii) one or more probe units each comprising two or more HCR fractional initiator probes; d) providing one or more HCR amplifiers that directly or indirectly generate one or more signals; e) optionally washing the sample; f) detecting one or more signals; and g) optionally removing one or more signals. In some embodiments, when multiple probe sets are used, some of option i) and some of option ii) are used (with respect to the probe sets).

In some embodiments of the method, the probe sets used for zero, one or more targets each comprise one or more initiator-labeled probes and the probe sets used for zero, one, or more other targets each comprise one or more probe units each comprising two or more fractional initiator probes. In some embodiments, each probe set comprises one or more initiator-labeled probes. In some embodiments, each probe set comprises one or more probe units each comprising two or more fractional initiator probes. In some embodiments, the type of probe set used for each target is selected independently for each target based on the details of that target. In some embodiments, the HCR amplifiers further mediate CARD or repeated CARD.

In some embodiments, a method of repeated signal detection with reporter and/or substrate-labeled hairpins is provided. In some embodiments, the method comprises: a) providing a sample possibly containing one or more targets as well as possibly other molecules that are not targets; b) performing any of steps c-g one or more times in any order; c) providing one or more HCR probe sets each comprising either: i) one or more HCR initiator-labeled probes, or ii) one or more probe units each comprising two or more HCR fractional initiator probes; d) providing one or more HCR amplifiers that directly or indirectly generate one or more signals; e) washing the sample; f) detecting one or more signals; and g) removing one or more signals. In some embodiments, when multiple probe sets are used, some of option i) and some of option ii) are used (with respect to the probe sets).

In some embodiments of the method, the probe sets used for zero, one or more targets each comprise one or more initiator-labeled probes and the probe sets used for zero, one, or more other targets each comprise one or more probe units each comprising two or more fractional initiator probes. In some embodiments, each probe set comprises one or more initiator-labeled probes. In some embodiments, each probe set comprises one or more probe units each comprising two or more fractional initiator probes. In some embodiments, the type of probe set used for each target is selected independently for each target based on the details of that target. In some embodiments, the HCR amplifiers further mediate CARD or repeated CARD.

In some embodiments, a probe set comprises one or more initiator-labeled probes. In some embodiments, the number of initiator-labeled probes in a probe set could be in the range 1 to 1000, or 10 to 100, or 20 to 50, or 30 to 40. Increasing the number of initiator-labeled probes in a probe set could increase the signal-to-background ratio by 2-fold, or 5-fold, or 10-fold, or 100-fold, or more. Increasing the number of initiator-labeled probes in a probe set could increase the signal-to-background ratio to be 2, or 5, or 10, or 50, or 100, or 200, or 500, or 1000 or more depending on the number of initiator-labeled probes in the probe set, the level of background inherent to the sample, and the abundance of the target within the sample.

In some embodiments, an initiator-labeled probe comprises one or more target-binding domains and one or more HCR initiators (for example, FIGs. 39A-39N and 42A-42F).

In some embodiments, a probe set comprises one or more probe units. In some embodiments, the number of probe units in a probe set could be in the range 1 to 1000, or 10 to 100, or 20 to 50, or 30 to 40. Increasing the number of probe units in a probe set could increase the signal-to-background ratio by 2-fold, or 5-fold, or 10-fold, or 100-fold, or more. Increasing the number of probe units in a probe set could increase the signal-to-background ratio to be 2, or 5, or 10, or 50, or 100, or 200, or 500, or 1000 or more depending on the number of probe units in the probe set, the level of background inherent to the sample, and the abundance of the target within the sample.

In some embodiments, a probe unit comprises two or more HCR fractional initiator probes. In some embodiments, a probe unit comprises two fractional initiator probes that together generate a full HCR initiator (fraction f1 for probe P1 and fraction f2 for probe P2 such that f1 + f2 = 1).

In some embodiments, an HCR fractional initiator probe comprises a target-binding region and a fractional initiator. In some embodiments, binding of each probe within a probe unit to adjacent cognate binding sites on the target colocalizes the fractional initiators to form a full HCR initiator (for example, see the full HCR initiators of Figures 3A-5E, 8A-8B, 16A-16D, 21A-22, and 27) capable of hybridizing to an HCR hairpin to trigger HCR signal amplification. In some embodiments, the target-binding regions within a probe unit are configured to bind to overlapping or non-overlapping regions of the target (for example, see Figures 8A-8B, 21A-21B, 22, and 27). In some embodiments, the fractional initiators within a probe unit are designed to hybridize to overlapping or non-overlapping regions of an HCR hairpin (for example, see Figures 8A-8B, 21A-22, and 27). Individual probes that bind non-specifically in the sample do not colocalize a full HCR initiator, suppressing HCR signal amplification.

In some embodiments, an HCR amplifier comprises two or more HCR hairpins (for example, see the HCR amplifiers of Figures 8A-8B, 18A-18F, and 19A-19B).

In some embodiments, an HCR hairpin comprises an input domain comprising a single-stranded toehold and a stem section.

In some embodiments, an HCR hairpin further comprises an output domain comprising a single-stranded loop and a complement to the stem section.

In some embodiments, an HCR hairpin further comprises one or more reporters and/or one or more substrates.

### Method 7

In some embodiments, a method of HCR involving overlapping binding sites is provided.

In some embodiments, the method comprises: a) providing a sample possibly containing a target as well as possibly other molecules that are not targets; b) providing a probe set comprising one or more probe units each comprising two or more HCR fractional initiator probes, where the target-binding regions on the probes within each probe unit are configured to bind to overlapping binding sites on the target; c) optionally washing the sample; d) providing an HCR amplifier labeled with a reporter and/or a substrate; e) optionally washing the sample; f) optionally providing a label probe (conjugated to a reporter) corresponding to the substrate; g) optionally washing the sample; and h) detecting a signal from the reporter.

In some embodiments, the method comprises: a) providing a sample possibly containing a target as well as possibly other molecules that are not targets; b) providing a probe set comprising one or more probe units each comprising two or more HCR fractional initiator probes, where the target-binding regions on the probes within each probe unit are configured to bind to overlapping binding sites on the target; c) washing the sample; d) providing an HCR amplifier labeled with a reporter and/or a substrate; e) washing the sample; f) providing a label probe (conjugated to a reporter) corresponding to the substrate; g) washing the sample; and h) detecting a signal from the reporter. The overlap can be 1, 2, 3, 4 or 5 nucleotides, in some embodiments.

In some embodiments, a probe set comprises one or more probe units. In some embodiments, the number of probe units in a probe set could be in the range 1 to 1000, or 10 to 100, or 20 to 50, or 30 to 40. Increasing the number of probe units in a probe set could increase the signal-to-background ratio by 2-fold, or 5-fold, or 10-fold, or 100-fold, or more. Increasing the number of probe units in a probe set could increase the signal-to-background ratio to be 2, or 5, or 10, or 50, or 100, or 200, or 500, or 1000 or more depending on the number of probe units in the probe set, the level of background inherent to the sample, and the abundance of the target within the sample.

In some embodiments, a probe unit comprises two or more HCR fractional initiator probes. In some embodiments, a probe unit comprises two fractional initiator probes that together generate a full HCR initiator (fraction f1 for probe P1 and fraction f2 for probe P2 such that f1 + f2 = 1).

In some embodiments, an HCR fractional initiator probe comprises a target-binding region and a fractional initiator. In some embodiments, binding of each probe within a probe unit to adjacent cognate binding sites on the target colocalizes the fractional initiators to form a full HCR initiator (for example, see the full HCR initiators of Figures 3A-5E, 8A-8B, 16A-16D, 21A-22, and 27) capable of hybridizing to an HCR hairpin to trigger HCR signal amplification.

In some embodiments, the target-binding regions on the probes within each probe unit are configured to bind to overlapping or non-overlapping regions of the target (for example, see Figures 8A-8B, 21A-21B, 22, and 27). In some embodiments, the fractional initiators within a probe unit are designed to hybridize to overlapping or non-overlapping regions of an HCR hairpin (for example, see Figures 8A-8B, 21A-22, and 27). Individual probes that bind non-specifically in the sample do not colocalize a full HCR initiator, suppressing HCR signal amplification.

In some embodiments, an HCR amplifier comprises two or more HCR hairpins (for example, see the HCR amplifiers of Figures 8A-8B, 18A-18F, and 19A-19B).

In some embodiments, an HCR hairpin comprises an input domain comprising a single-stranded toehold and a stem section.

In some embodiments, an HCR hairpin further comprises an output domain comprising a single-stranded loop and a complement to the stem section.

In some embodiments, an HCR hairpin further comprises a reporter and/or a substrate.

In some embodiments, a label probe comprises a substrate-binding region and a reporter.

In some embodiments, the hairpin label can comprise a substrate that serves to recruit a reporter entity that comprises an enzyme that catalyzes a CARD-substrate leading to deposition of reporter molecules in the vicinity of the hairpin (see for example, 33A-33E, 34A-34C, 36A-36B). For example:
1. the enzyme could be horseradish peroxidase (HRP) (or polymer HRP comprising multiple HRP enzymes) that acts on a CARD-substrate to catalyze deposition a chromogenic reporter such as AEC, DAB, TMB, or StayYellow, or that catalyzes a CARD-substrate to catalyze deposition of a fluorescent reporter such as fluophore-labeled tyramide, or that catalyzes deposition of a hapten-labeled CARD-substrate such as biotin-labeled tyramide, where the hapten serves to mediate localization of reporters in the vicinity of the hairpin label.
2. the enzyme could be alkaline phosphatase (AP) (or polymer AP comprising multiple AP enzymes) that acts on a CARD-substrate to catalyze deposition of reporters, for example a chromogenic reporter such as but not limited to BCIP/NBT, BCIP/TNBT, Napthol AS-MX phosphate + FastBlue BB, Napthol AS-MX phosphate + FastRed TR, StayGreen.
3. the enzyme could be glucose oxidase that acts on a CARD-substrate to catalyze deposition of reporters, for example NBT,
4. the enzyme could be any molecule or complex that directly or indirectly mediates localization of reporters in the vicinity of a hairpin label.

### Method 8

In some embodiments, a method of HCR involving overlapping binding sites is provided.

In some embodiments, the method comprises: a) providing a sample possibly containing a target as well as possibly other molecules that are not targets; b) providing a probe set comprising one or more probe units each comprising two or more HCR fractional initiator probes, where the fractional initiators on the probes within each probe unit are configured to bind to overlapping binding sites on an HCR hairpin; c) optionally washing the sample; d) providing an HCR amplifier labeled with a reporter and/or a substrate; e) optionally washing the sample; f) optionally providing a label probe (conjugated to a reporter) corresponding to the substrate; g) optionally washing the sample; and h) detecting a signal from the reporter.

In some embodiments, the method comprises: a) providing a sample possibly containing a target as well as possibly other molecules that are not targets; b) providing a probe set comprising one or more probe units each comprising two or more HCR fractional initiator probes, where the fractional initiators on the probes within each probe unit are configured to bind to overlapping binding sites on an HCR hairpin; c) washing the sample; d) providing an HCR amplifier labeled with a reporter and/or a substrate; e) washing the sample; f) providing a label probe (conjugated to a reporter) corresponding to the substrate; g) washing the sample; and h) detecting a signal from the reporter.

In some embodiments, a probe set comprises one or more probe units. In some embodiments, the number of probe units in a probe set could be in the range 1 to 1000, or 10 to 100, or 20 to 50, or 30 to 40. Increasing the number of probe units in a probe set could increase the signal-to-background ratio by 2-fold, or 5-fold, or 10-fold, or 100-fold, or more. Increasing the number of probe units in a probe set could increase the signal-to-background ratio to be 2, or 5, or 10, or 50, or 100, or 200, or 500, or 1000 or more depending on the number of probe units in the probe set, the level of background inherent to the sample, and the abundance of the target within the sample.

In some embodiments, a probe unit comprises two or more HCR fractional initiator probes. In some embodiments, a probe unit comprises two fractional initiator probes that together generate a full HCR initiator (fraction f1 for probe P1 and fraction f2 for probe P2 such that f1 + f2 = 1).

In some embodiments, an HCR fractional initiator probe comprises a target-binding region and a fractional initiator. In some embodiments, binding of each probe within a probe unit to adjacent cognate binding sites on the target colocalizes the fractional initiators to form a full HCR initiator (for example, see the full HCR initiators of Figures 3A-5E, 8A-8B, 16A-16D, 21A-22, and 27) capable of hybridizing to an HCR hairpin to trigger HCR signal amplification. In some embodiments, the target-binding regions within a probe unit are configured to bind to overlapping or non-overlapping regions of the target (for example, see Figures 8A-8B, 2A-21B, 22, and 27). In some embodiments, the fractional initiators within a probe unit are designed to hybridize to overlapping or non-overlapping regions of an HCR hairpin (for example, see Figures 8A-8B, 21A-22, and 27). Individual probes that bind non-specifically in the sample do not colocalize a full HCR initiator, suppressing HCR signal amplification.

In some embodiments, an HCR amplifier comprises two or more HCR hairpins (for example, see the HCR amplifiers of Figures 8A-8B, 18A-18F, and 19A-19B).

In some embodiments, an HCR hairpin comprises an input domain comprising a single-stranded toehold and a stem section.

In some embodiments, an HCR hairpin further comprises an output domain comprising a single-stranded loop and a complement to the stem section.

In some embodiments, an HCR hairpin further comprises a reported and/or a substrate. In some embodiments, the reporter is a fluorophore, a chromophore, a luminophore, a phosphor, a FRET pair, a member of a FRET pair, a quencher, a fluorophore/quencher pair, a rare-earth element or compound, a radioactive molecule, a magnetic molecule, or any other molecule that facilitates measurement of a signal. In some embodiments, the substrate that serves to recruit a reporter entity that directly or indirectly mediates localization of reporters in the vicinity of the hairpin label.

In some embodiments, a label probe comprises a substrate-binding region and a reporter (for example, see Figures 18C-E and 20). For example, a reporter could be a fluorophore, a chromophore, a luminophore, a phosphor, a FRET pair, a member of a FRET pair, a quencher, a fluorophore/quencher pair, a rare-earth element or compound, a radioactive molecule, a magnetic molecule, or any other molecule that facilitates measurement of a signal.

In some embodiments, the hairpin label can comprise a substrate that serves to recruit a reporter entity that comprises an enzyme that catalyzes a CARD-substrate leading to deposition of reporter molecules in the vicinity of the hairpin (see for example, 33A-33E, 34A-34C, 36A-36B). For example:
1. the enzyme could be horseradish peroxidase (HRP) (or polymer HRP comprising multiple HRP enzymes) that acts on a CARD-substrate to catalyze deposition a chromogenic reporter such as AEC, DAB, TMB, or StayYellow, or that catalyzes a CARD-substrate to catalyze deposition of a fluorescent reporter such as fluophore-labeled tyramide, or that catalyzes deposition of a hapten-labeled CARD-substrate such as biotin-labeled tyramide, where the hapten serves to mediate localization of reporters in the vicinity of the hairpin label.
2. the enzyme could be alkaline phosphatase (AP) (or polymer AP comprising multiple AP enzymes) that acts on a CARD-substrate to catalyze deposition of reporters, for example a chromogenic reporter such as but not limited to BCIP/NBT, BCIP/TNBT, Napthol AS-MX phosphate + FastBlue BB, Napthol AS-MX phosphate + FastRed TR, StayGreen.
3. the enzyme could be glucose oxidase that acts on a CARD-substrate to catalyze deposition of reporters, for example NBT,
4. the enzyme could be any molecule or complex that directly or indirectly mediates localization of reporters in the vicinity of a hairpin label.

### Method 9

In some embodiments, a method of HCR involving overlapping binding sites with repeated signal detection is provided.

In some embodiments, the method comprises: a) providing a sample possibly containing one or more targets as well as possibly other molecules that are not targets b) providing one or more probe sets each comprising either: i) one or more HCR initiator-labeled probes, or ii) one or more probe units each comprising two or more HCR fractional initiator probes where target-binding regions on the probes within each probe unit are configured to bind to overlapping or non-overlapping binding sites on a target and where fractional initiators on the probes within each probe unit are configured to bind to overlapping or non-overlapping binding sites on an HCR hairpin; c) optionally washing the sample; d) providing one or more HCR amplifiers each labeled with one or more reporters and/or substrates; e) optionally washing the sample; f) optionally providing one or more label probes (each conjugated to one or more reporters) corresponding to one or more substrates; g) optionally washing the sample; h) detecting a signal from one or more reporters; i) optionally removing one or more signals from the sample; j) optionally removing one or more reporters from the sample; k) optionally removing one or more label probes from the sample; l) optionally removing one or more amplifiers from the sample; m) optionally removing one or more probe sets from the sample; and n) optionally repeating any of the above steps in any order. In some embodiments, when multiple probe sets are used, some of option i) and some of option ii) are used (with respect to the probe sets).

In some embodiments of the method, the probe sets used for zero, one or more targets each comprise one or more initiator-labeled probes and the probe sets used for zero, one, or more other targets each comprise one or more probe units each comprising two or more fractional initiator probes. In some embodiments, each probe set comprises one or more initiator-labeled probes. In some embodiments, each probe set comprises one or more probe units each comprising two or more fractional initiator probes. In some embodiments, the type of probe set used for each target is selected independently for each target based on the details of that target. In some embodiments, the HCR amplifiers further mediate CARD or repeated CARD.

In some embodiments, the method comprises: a) providing a sample possibly containing one or more targets as well as possibly other molecules that are not targets b) providing one or more probe sets each comprising either: i) one or more HCR initiator-labeled probes, or ii) one or more probe units each comprising two or more HCR fractional initiator probes, where target-binding regions on the probes within each probe unit are configured to bind to overlapping or non-overlapping binding sites on a target, and where fractional initiators on the probes within each probe unit are configured to bind to overlapping or non-overlapping binding sites on an HCR hairpin; c) washing the sample; d) providing one or more HCR amplifiers each labeled with one or more reporters and/or substrates; e) washing the sample; f) providing one or more label probes (each conjugated to one or more reporters) corresponding to one or more substrates; g) washing the sample; h) detecting a signal from one or more reporters; i) removing one or more signals from the sample; j) removing one or more reporters from the sample; k) removing one or more label probes from the sample; l) removing one or more amplifiers from the sample; m) optionally removing one or more probe sets from the sample; and n) repeating any of the above steps in any order. In some embodiments, when multiple probe sets are used, some of option i) and some of option ii) are used (with respect to the probe sets).

In some embodiments of the method, the probe sets used for zero, one or more targets each comprise one or more initiator-labeled probes and the probe sets used for zero, one, or more other targets each comprise one or more probe units each comprising two or more fractional initiator probes. In some embodiments, each probe set comprises one or more initiator-labeled probes. In some embodiments, each probe set comprises one or more probe units each comprising two or more fractional initiator probes. In some embodiments, the type of probe set used for each target is selected independently for each target based on the details of that target. In some embodiments, the HCR amplifiers further mediate CARD or repeated CARD.

In some embodiments, a probe set comprises one or more initiator-labeled probes. In some embodiments, the number of initiator-labeled probes in a probe set could be in the range 1 to 1000, or 10 to 100, or 20 to 50, or 30 to 40. Increasing the number of initiator-labeled probes in a probe set could increase the signal-to-background ratio by 2-fold, or 5-fold, or 10-fold, or 100-fold, or more. Increasing the number of initiator-labeled probes in a probe set could increase the signal-to-background ratio to be 2, or 5, or 10, or 50, or 100, or 200, or 500, or 1000 or more depending on the number of initiator-labeled probes in the probe set, the level of background inherent to the sample, and the abundance of the target within the sample.

In some embodiments, an initiator-labeled probe comprises one or more target-binding domains and one or more HCR initiators (for example, FIGs. 39A-39N and 42A-42F).

In some embodiments, a probe set comprises one or more probe units. In some embodiments, the number of probe units in a probe set could be in the range 1 to 1000, or 10 to 100, or 20 to 50, or 30 to 40. Increasing the number of probe units in a probe set could increase the signal-to-background ratio by 2-fold, or 5-fold, or 10-fold, or 100-fold, or more. Increasing the number of probe units in a probe set could increase the signal-to-background ratio to be 2, or 5, or 10, or 50, or 100, or 200, or 500, or 1000 or more depending on the number of probe units in the probe set, the level of background inherent to the sample, and the abundance of the target within the sample.

In some embodiments, a probe unit comprises two or more HCR fractional initiator probes. In some embodiments, a probe unit comprises two fractional initiator probes that together generate a full HCR initiator (fraction f1 for probe P1 and fraction f2 for probe P2 such that f1 + f2 = 1).

In some embodiments, an HCR fractional initiator probe comprises a target-binding region and a fractional initiator. In some embodiments, binding of each probe within a probe unit to adjacent cognate binding sites on the target colocalizes the fractional initiators to form a full HCR initiator (for example, see the full HCR initiators of Figures 3A-5E, 8A-8B, 16A-16D, 21A-22, and 27) capable of hybridizing to an HCR hairpin to trigger HCR signal amplification. In some embodiments, the target-binding regions within a probe unit are configured to bind to overlapping or non-overlapping regions of the target (for example, see Figures 8A-8B, 21A-21B, 22, and 27).

In some embodiments, the fractional initiators within a probe unit are designed to hybridize to overlapping or non-overlapping regions of an HCR hairpin (for example, see Figures 8A-8B, 21A-22, and 27). Individual probes that bind non-specifically in the sample do not colocalize a full HCR initiator, suppressing HCR signal amplification.

In some embodiments, an HCR amplifier comprises two or more HCR hairpins (for example, see the HCR amplifiers of Figures 8A-8B, 18A-18F, and 19A-19B).

In some embodiments, an HCR hairpin comprises an input domain comprising a single-stranded toehold and a stem section.

In some embodiments, an HCR hairpin further comprises an output domain comprising a single-stranded loop and a complement to the stem section.

In some embodiments, an HCR hairpin further comprises one or more reporters and/or one or more substrates.

In some embodiments, a label probe comprises a substrate-binding region and one or more reporters.

In some embodiments of the methods herein (e.g., Methods 1-9), the target is a nucleic acid sequence. Non-limiting examples are dsDNA, dsRNA, ssDNA, and ssRNA.

In some embodiments of any of the methods herein (e.g., Method 1 or Method 3), N is 1 to 1000. In some embodiments, N is 1 to 10, 1 to 50, 1 to 100, 1 to 250, 1 to 500, or 1 to 750.

In some embodiments of any of the methods herein (e.g., Method 1 or Method 3), M is 2-10,000. In some embodiments, M is 1 to 10, 1 to 50, 1 to 100, 1 to 250, 1 to 500, 1 to 750, 1 to 1000, 1 to 1250, 1 to 2500, 1 to 3750, 1 to 5000, or 1 to 7500.

In some embodiments of any of the methods herein, the substrate is selected from the group consisting of a hapten, digoxigenin (DIG), dinitrophenyl (DNP), a fluorophore, biotin, a nucleic acid domain comprising 4-50 nucleotides, a substrate that recruits an enzyme that catalyzes deposition of reporter molecules, a fractional substrate, a nucleic acid domain that directly or indirectly mediates localization of reporters.

In some embodiments of any of the methods herein, the reporter is a fluorophore, a chromophore, a luminophore, a phosphor, a FRET pair, a member of a FRET pair, a quencher, a fluorophore/quencher pair, a rare-earth element or compound, a radioactive molecule, a magnetic molecule.

In some embodiments of any of the methods herein, further comprise fixing the sample.

In some embodiments of any of the methods herein, further comprise permeabilizing the sample.

In some embodiments of any of the methods herein, further comprise removing one or more signals from the sample.

In some embodiments of any of the methods herein, further comprise removing one or more reporters from the sample.

In some embodiments of any of the methods herein, further comprise removing one or more label probes from the sample.

In some embodiments of any of the methods herein, further comprise removing one or more amplifiers from the sample.

In some embodiments of any of the methods herein, further comprise removing one or more probe sets from the sample.

In some embodiments of any of the methods herein, further comprise repeating any of the above steps in any order.

In some embodiments of any of the methods herein, the method is conducted in alphabetical order as lettered in the claim.

In some embodiments of any of the methods herein, the probe units each comprise two or more HCR fractional initiator probes, and wherein an HCR fractional initiator probe comprises a target-binding region and a fractional initiator.

In some embodiments of any of the methods herein, overlapping binding is involved and wherein the overlap is at least 1, 2, or 3 nucleotides.

In some embodiments of any of the methods herein, the hairpin label can comprise a substrate that serves to recruit a reporter entity that comprises an enzyme that catalyzes a CARD-substrate leading to deposition of reporter molecules in the vicinity of the hairpin (see for example, 33A-33E, 34A-34C, 36A-36B). For example:
1. the enzyme could be horseradish peroxidase (HRP) (or polymer HRP comprising multiple HRP enzymes) that acts on a CARD-substrate to catalyze deposition a chromogenic reporter such as AEC, DAB, TMB, or StayYellow, or that catalyzes a CARD-substrate to catalyze deposition of a fluorescent reporter such as fluophore-labeled tyramide, or that catalyzes deposition of a hapten-labeled CARD-substrate such as biotin-labeled tyramide, where the hapten serves to mediate localization of reporters in the vicinity of the hairpin label.
2. the enzyme could be alkaline phosphatase (AP) (or polymer AP comprising multiple AP enzymes) that acts on a CARD-substrate to catalyze deposition of reporters, for example a chromogenic reporter such as but not limited to BCIP/NBT, BCIP/TNBT, Napthol AS-MX phosphate + FastBlue BB, Napthol AS-MX phosphate + FastRed TR, StayGreen.
3. the enzyme could be glucose oxidase that acts on a CARD-substrate to catalyze deposition of reporters, for example NBT,
4. the enzyme could be any molecule or complex that directly or indirectly mediates localization of reporters in the vicinity of a hairpin label.

In some embodiments of any of the methods herein, the enzyme used to mediate CARD is inactivated after reporter deposition (for example, using chemical and/or heat denaturation).

In some embodiments of any of the methods herein, the hairpin label comprises one or more haptens that recruit one or more anti-hapten primary antibodies that further recruit one or more secondary antibodies labeled with reporter entities, wherein the reporter entity is an enzyme that mediates CARD.

In some embodiments of any of the methods herein, the hairpin label comprises one or more first haptens that recruit one or more anti-first-hapten primary antibodies that further recruit one or more first secondary antibodies labeled with one or more second haptens that further recruit one or more anti-second-hapten primary antibodies that further recruit one or more second secondary antibodies each comprising one or more reporter entities, where the reporter entity is an enzyme that mediates CARD.

In some embodiments of any of the methods herein, the hairpin label comprises one or more first haptens that recruit one or more anti-first-hapten primary antibodies that further recruit one or more first secondary antibodies labeled with one or more second haptens that further recruit one or more anti-second-hapten primary antibodies that further recruit one or more second secondary antibodies each comprising one or more reporter entities, where the reporter entity is an enzyme that mediates CARD. In some embodiments, the second hapten is also a reporter entity that is an enzyme that mediates CARD (for example, HRP or AP).

### Method 10

In some embodiments, a method comprises providing a first fractional initiator probe comprising a first fractional initiator, a second fractional initiator probe comprising a second fractional initiator, a first hairpin monomer, a second hairpin monomer, and a target molecule, and incubating the first fractional initiator probe and the second fractional initiator probe with the target. In some embodiments, the first hairpin monomer comprises a first input domain comprising a first toehold and a first stem section, a first output domain comprising a first hairpin loop and a complement to the first stem section, and a first hapten molecule. In some embodiments, the second hairpin monomer comprises a second input domain comprising a second toehold and a second stem section, a second output domain comprising a second hairpin loop and a complement to the second stem section, and a second hapten molecule (see for example, FIG. 34B).

In some embodiments of the method, the incubating binds the first fractional initiator probe to the target molecule and binds the second fractional initiator probe to the target molecule.

In some embodiments, the method further comprises binding the first hairpin monomer to both of the first fractional initiator and the second fractional initiator, binding the second hairpin monomer to the first hairpin monomer, providing an anti-hapten molecule labeled with one or more reporter entities. In some embodiments, the reporter entity is an enzyme that mediates CARD. In some embodiments, the method further comprises providing one or more CARD-substrates and measuring a signal from one or more deposited reporters generated from the CARD-substrate by the enzyme that mediates CARD (see for example, FIG. 34B).

In some embodiments of the method, the anti-hapten molecule is an anti-hapten antibody. In some embodiments of the method, the anti-hapten molecule is an anti-hapten nanobody. In some embodiments of the method, the anti-hapten molecule is a combination of anti-hapten antibody and anti-hapten nanobody.

In some embodiments of the method, the at least one target is a nucleic acid (*See,* for example, FIGs. 33B, 36A, and 36B).

In some embodiments of the method, the at least one target molecule is an RNA. In some embodiments, the RNA is single-stranded. In some embodiments, the RNA is double-stranded. In some embodiments, the RNA is both single-stranded and double-stranded.

In some embodiments of the method, the at least one target molecule is a DNA. In some embodiments, the DNA is single-stranded. In some embodiments, the DNA is double-stranded. In some embodiments, the DNA is both single-stranded and double-stranded.

In some embodiments of the method, the at least one target molecule is a complex of RNA molecules (for example a ribosomal RNA and an mRNA). In some embodiments, the RNA is single-stranded. In some embodiments, the RNA is double-stranded. In some embodiments, the RNA is both single-stranded and double-stranded.

### Method 11

In some embodiments, a method comprises providing at least one initiator-labeled probe comprising at least one initiator, a first hairpin monomer, a second hairpin monomer, and one or more target molecules, and incubating the at least one initiator-labeled probe comprising at least one initiator with the one or more target molecules. In some embodiments, the first hairpin monomer comprises a first input domain comprising a first toehold and a first stem section, a first output domain comprising a first hairpin loop and a complement to the first stem section, and a first hapten molecule. In some embodiments, a second hairpin monomer comprises a second input domain comprising a second toehold and a second stem section, a second output domain comprising a second hairpin loop and a complement to the second stem section, and a second hapten molecule (see for example, FIG. 34A). Optionally, each hairpin may have zero, one, two, or more haptens so long as at least one hairpin has at least one hapten.

In some embodiments of the method, the incubating binds the at least one initiator-labeled probe comprising at least one initiator to the one or more target molecules.

In some embodiments the method further comprises binding the first hairpin monomer to the at least one initiator, binding the second hairpin monomer to the first hairpin monomer, and providing an anti-hapten molecule labeled with one or more reporter entities. In some embodiments, the reporter entity is an enzyme that mediates CARD. In some embodiments, the method further comprises providing one or more CARD-substrates and measuring a signal from one or more deposited reporters generated from the CARD-substrate by the enzyme that mediates CARD (see for example, FIG. 34A).

In some embodiments of the method, the anti-hapten molecule is an anti-hapten antibody. In some embodiments of the method, the anti-hapten molecule is an anti-hapten nanobody. In some embodiments of the method, the anti-hapten molecule is an anti-hapten antibody fragment. In some embodiments of the method, the anti-hapten molecule is a combination of anti-hapten antibody and an anti-hapten nanobody.

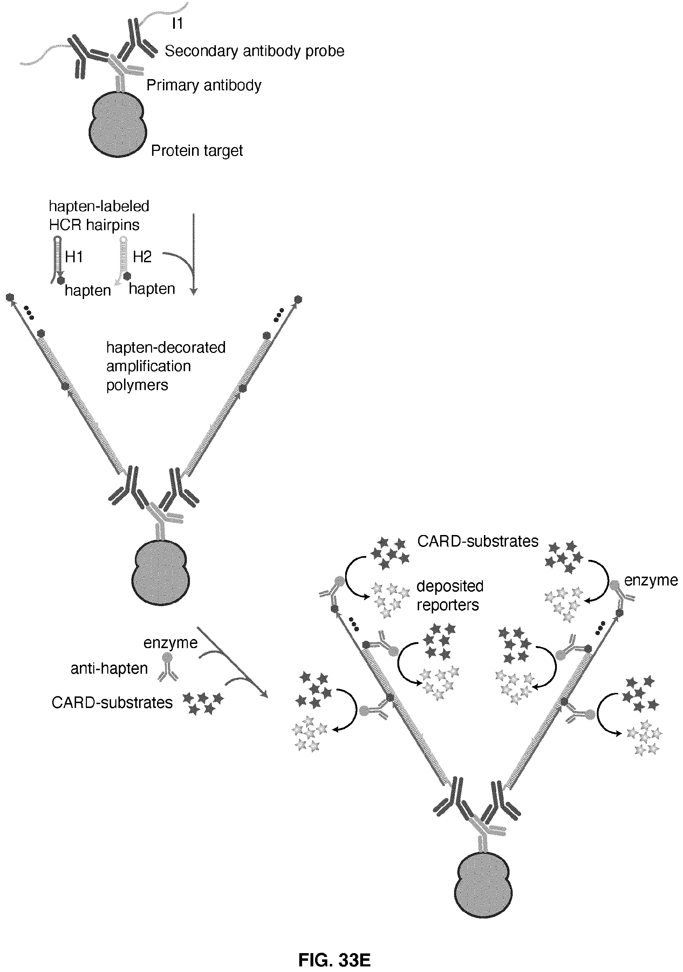

In some embodiments of the method, the one or more target molecules is a protein (see for example, FIGs. 33C and 33E). In some embodiments of the method, the one or more target molecules is a microRNA, a small RNA, or a non-coding RNA.

In some embodiments of the method, the anti-hapten molecule is an anti-hapten antibody. In some embodiments of the method, the anti-hapten molecule is an anti-hapten nanobody. In some embodiments of the method, the anti-hapten molecule is a combination of anti-hapten antibody and anti-hapten nanobody.

In some embodiments, the anti-hapten antibody is a primary antibody. In some embodiments, the method further includes or employs a secondary antibody. In some embodiments, the anti-hapten antibody comprises a primary antibody that binds the hapten and the method further comprises a secondary antibody (labeled with one or more reporter entities) that binds the primary antibody.

### Method 12

In some embodiments, a method comprises providing a first fractional initiator probe comprising a first fractional initiator, a second fractional initiator probe comprising a second fractional initiator, a first hairpin monomer, a second hairpin monomer, and a target molecule, and incubating the first fractional initiator probe and the second fractional initiator probe with the target molecule. In some embodiments, the first hairpin monomer comprises a first input domain comprising a first toehold and a first stem section, a first output domain comprising a first hairpin loop and a complement to the first stem section, and a substrate. In some embodiments, the second hairpin monomer comprises a second input domain, comprising a second toehold and a second stem section, a second output domain, comprising a second hairpin loop and a complement to the second stem section, and the substrate.

In some embodiments of the method, the incubating binds the first fractional initiator probe to the target molecule and binds the second fractional initiator probe to the target molecule.

In some embodiments, the method further comprises binding the first hairpin monomer to both of the first fractional initiator and the second fractional initiator, binding the second hairpin monomer to the first hairpin monomer, providing a substrate-binding region labeled with one or more reporter entities, wherein the substrate-binding region binds to the substrate, and wherein the reporter entity is an enzyme that mediates CARD, providing one or more CARD-substrates, measuring a signal from one or more deposited reporters generated from the CARD-substrate by the enzyme that mediates CARD.

In some embodiments of the method, the at least one target is a nucleic acid (see for example, FIGs. 33B, 36A, and 36B).

In some embodiments of the method, the at least one target molecule is an RNA. In some embodiment, the RNA is single-stranded. In some embodiments, the RNA is double-stranded. In some embodiments, the RNA is both single-stranded and double-stranded.

### Method 13

In some embodiments, a method comprises providing a first fractional initiator probe comprising a first fractional initiator, a second fractional initiator probe comprising a second fractional initiator, a first hairpin monomer, a second hairpin monomer, and a target molecule, and incubating the first fractional initiator probe and the second fractional initiator probe with the target. In some embodiments, the first hairpin monomer comprises a first input domain comprising a first toehold and a first stem section, a first output domain comprising a first hairpin loop and a complement to the first stem section, and a first fractional substrate. In some embodiments, a second hairpin monomer comprises a second input domain comprising a second toehold and a second stem section, a second output domain comprising a second hairpin loop and a complement to the second stem section, and a second fractional substrate.

In some embodiments of the method, the incubating binds the first fractional initiator probe to the target molecule and binds the second fractional initiator probe to the target molecule.

In some embodiments, the method further comprises binding the first hairpin monomer to both of the first fractional initiator and the second fractional initiator, binding the second hairpin monomer to the first hairpin monomer, obtaining a full substrate comprising the first and second fractional substrate, providing a substrate-binding region labeled with one or more reporter entities, wherein the substrate-binding region binds to the full substrate, and wherein the reporter entity is an enzyme that mediates CARD, providing one or more CARD-substrates, measuring a signal from one or more deposited reporters generated from the CARD-substrate by the enzyme that mediates CARD.

In some embodiments of the method, the at least one target is a nucleic acid (see for example, FIGs. 33B, 36A, and 36B).

In some embodiments of the method, the at least one target molecule is an RNA. In some embodiment, the RNA is single-stranded. In some embodiments, the RNA is double-stranded. In some embodiments, the RNA is both single-stranded and double-stranded.

### EXAMPLES

The following examples are non-limiting and other variants within the scope of the skill in the art are also contemplated.

### Example 1 - Imaging of two target mRNAs in whole-mount chicken embryos using unoptimized probe sets

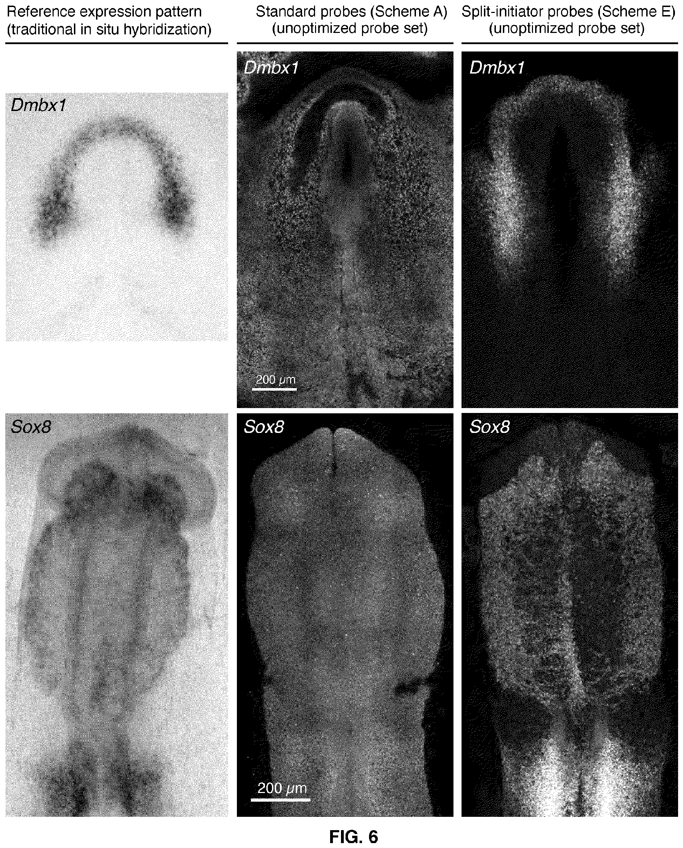

Figure 6 demonstrates imaging of two target mRNAs in whole-mount chicken embryos using unoptimized probe sets.

Using standard probes (aka initiator-labeled probes) (Scheme A), non-specific probe binding leads to amplified background, resulting in low signal-to-background. Using fractional initiator (aka a split-initiator) probes (Scheme E), automatic background suppression during both stages of the protocol ensures that non-specific probe binding does not lead to amplified background, resulting in high signal-to-background even using an unoptimized fractional initiator probe set. The automatic background suppression property of fractional initiator (aka a split-initiator) probes (Scheme E) is extremely beneficial when mapping the expression pattern of a new target mRNA, allowing rapid generation of high-quality results without requiring tedious fractional initiator probe set optimization (i.e., removal of bad probes observed to bind non-specifically in the sample).

The benefits of active background suppression are illustrated in Figure 6, depicting imaging of target mRNAs in whole-mount chicken embryos using unoptimized probe sets with either standard probes (aka initiator-labeled probes) (Scheme A) or fractional initiator (aka a split-initiator) probes (Scheme E). Target mRNA Dmbx1: Stage HH 7 embryo; target mRNA Sox8: Stage HH10 embryo. With standard probes (aka initiator-labeled probes) (Scheme A), low signal-to-background was observed using an unoptimized fractional initiator probe set due to non-specific probe binding, leading to generation of amplified background. With fractional initiator (aka a split-initiator) probes (Scheme E), high signal-to-background is achieved even using an unoptimized fractional initiator probe set; probes that bind non-specifically in the sample do not co-localize the two halves of the HCR initiator, and thus do not trigger HCR signal amplification, avoiding generation of amplified background. Scheme A uses a probe set containing 12 unstructured probes, each containing a target binding site for a different subsequence of the target mRNA. Scheme E uses a fractional initiator probe set containing 12 probe pairs; these 12 pairs address the same target subsequences as the 12 probes for Scheme A. Reference images from GEISHA (Darnell, D.K., Kaur, S., Stanislaw, S., Davey, S., Konieczka, J.H., Yatskievych, T.A., and Antin, P.B. (2007). GEISHA: An In situ hybridization gene expression resource for the chicken embryo. Cytogenet. Genome Res. 117:30-35).

### Example 2 - Cooperative initiation of HCR using fractional initiator (aka a split-initiator) probes

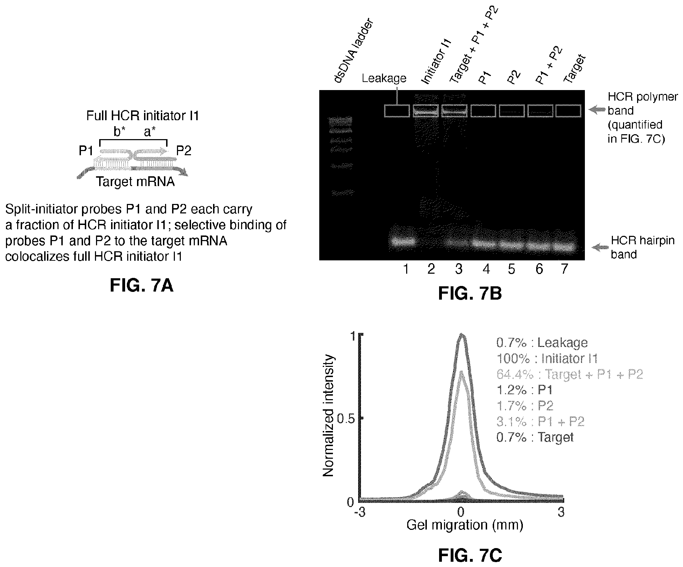

Cooperative initiation of HCR using fractional initiator (aka a split-initiator) probes (Scheme E) (FIG. 7). (a) Target-mediated colocalization of fractional initiator (aka a split-initiator) probes (Scheme E). Fractional initiator (aka a split-initiator) probes P1 and P2 each carry half of HCR initiator I1. Selective binding of P1 and P2 to the target mRNA colocalizes the two halves of HCR initiator I1, allowing cooperative initiation of the HCR amplification cascade.

(b) In vitro validation of cooperative initiation of HCR using split-initiator probes P1 and P2 (Scheme E). Reaction conditions: hairpins H1 and H2 at 0.5 µM each (Lanes 1-7); oligos I1, P1, P2, and/ or Target at 5 nM each (Lanes as noted on the gel); 5 SSCT buffer; overnight reaction at room temperature. Hairpins H1 and H2 labeled with Alexa 647 fluorophore. dsDNA 1 kb ladder pre-stained with SYBR Gold. Lane 1: Metastable hairpins H1 and H2 exhibit minimal leakage out of their kinetically trapped states in the absence of HCR initiator I1. Lane 2: Full conversion of HCR hairpin monomers H1 and H2 into amplification polymer in the presence of HCR initiator I1 (I1 as an oligo). Lane 3: Strong conversion of hairpins H1 and H2 to polymer in the presence of Target and both fractional initiator (aka a split-initiator) probes P1 and P2, demonstrating cooperative initiation of HCR. Lanes 4-6: Minimal conversion of HCR hairpin monomers H1 and H2 into polymer in the presence of probe P1, probe P2, or both probes P1 and P2, demonstrating active background suppression. Lane 7: Minimal conversion of HCR hairpin monomers H1 and H2 into polymer in the presence of Target alone.

(c) Quantification of the polymer bands in panel c. Multi Gauge software (Fuji Photo Film) was used to calculate the Alexa 647 intensity profile surrounding the polymer band for Lanes 1-7. Each intensity profile is displayed for ± 3 mm of gel migration distance with the peak value centered at 0. The quantification percentages were calculated using Multi Gauge with auto-detection of signal and background; the calculated values were normalized to the measured value for Lane 2.

The gel study of Figure 7b demonstrated cooperative initiation of HCR using fractional initiator (aka a split-initiator) probes (Scheme E). Lane 1 shows that there is minimal leakage of hairpins H1 and H2 out of their kinetically trapped states in the absence of HCR initiator I1. As a positive control, Lane 2 demonstrates conversion of metastable HCR hairpin monomers H1 and H2 into polymer in the presence of full HCR initiator I1 (where I1 is a single oligo).

Using fractional initiator (aka a split-initiator) probes, strong conversion of metastable HCR hairpin monomers H1 and H2 into polymer were observed if the target was present together with fractional initiator (aka a split-initiator) probes P1 and P2. However, minimal conversion to polymer was observed if P1 or P2 is present alone (Lanes 4 and 5), or if P1 and P2 were present together but in the absence of the Target (Lane 6).

These results demonstrated the active background suppression properties of fractional initiator (aka a split-initiator) probes (Scheme E): probes that are not colocalized via selective hybridization to the target predominantly do not trigger HCR amplification. The fact that in the absence of the target, P1 and P2 do not trigger HCR even when they are both present in solution (Lane 6), indicates that fractional initiator probes provide automatic background suppression even in the absence of washes.

### Example 3 - Comparison of performance of standard probes and fractional initiator (aka split-initiator) probes

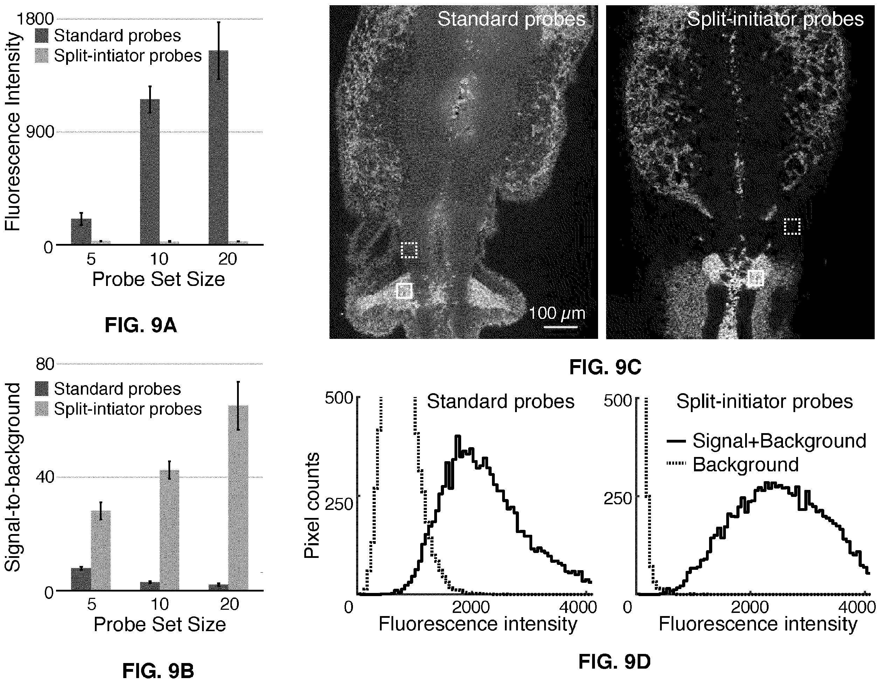

Figure 9 compares the performance of standard probes (aka initiator-labeled probes) (Scheme A) and fractional initiator (aka a split-initiator) probes (Scheme E) as the size of the probe set is increased for imaging of a mRNA target in whole-mount chicken embryos. For tests with standard probes, an optimized probe set of 5 probes was used, and then these probes were augmented with additional unoptimized probes to form probe sets of 10 probes and 20 probes. For tests with fractional initiator (aka a split-initiator) probes, probe sets were used with 5, 10, and 20 probe pairs, where each probe pair targets approximately the same binding site as the corresponding standard probe. Using standard probes, increasing the probe set size resulted in a substantial increase in background (panel a) and a corresponding decrease in signal-to-background as a result of some subset of the additional probes binding non-specifically within the sample.

These data illustrate the importance of probe set optimization using standard probes (aka initiator-labeled probes) (Scheme A) that do not provide active background suppression: if there are any bad probes in the probe set, they will undermine performance by generating amplified background. In contrast, using fractional initiator (aka a split-initiator) probes (Scheme E), as the number of probe pairs increases from 5 to 10 to 20, the background remains approximately constant (panel a) and the signal-to-background ratio increases monotonically (panel b).

These data illustrate the significant benefit of automatic background suppression using fractional initiator (aka a split-initiator) probes: even if there are bad probes in the fractional initiator probe set, they do not generate amplified background, making it straightforward to increase signal-to-background by increasing the number of probes without performing probe set optimization. Representative images using the probe sets with 20 probes (standard probes) or 20 probe pairs (fractional initiatory (split-initiator) probes) are shown in panel c. Representative pixel intensity distributions for these images are shown in panel d. With fractional initiator (aka a split-initiator) probes, the pixel intensity distributions for background and signal + background are predominantly non-overlapping.

The following is provided for slightly more detail for FIG. 9, which presents background and signal-to-background using standard probes (aka initiator-labeled probes) (Scheme A) and fractional initiator (aka a split-initiator) probes (Scheme E). (a) Fluorescent background using probe sets with 5, 10, or 20 probes (standard probes) vs 5, 10, or 20 probe pairs (fractional initiator (aka a split-initiator) probes). Unoptimized standard probes resulted in non-specific probe binding, leading to generation of amplified background. Unoptimized fractional initiator (aka a split-initiator) probes that bind non-specifically in the sample did not co-localize the two halves of the HCR initiator, and thus did not trigger HCR signal amplification, avoiding generation of amplified background. (b) Signal-to-background ratio for the probe sets of panel a. Fractional initiator (aka a split-initiator) probes with active background suppression outperformed unoptimized standard probes in signal-to-background measurements. (c) Confocal micrographs in the neural crest of fixed whole-mount chicken embryos. Probe sets: 20 probes for standard probes of Scheme A, 20 probe pairs for fractional initiator (aka a split-initiator) probes of Scheme E. (d) Pixel intensity histograms for Sign al + Background (pixels with in solid boundary in panel c) and Background (pixels within dashed boundary in panel c). For each image, the total number of pixels within solid and dashed boundaries is the same. Embryos fixed: stage HH10. Target: Sox10.

### Example 4 - Imaging four target mRNAs in whole-mount chicken embryos with high signal-to-background using unoptimized fractional initiator probes

Figure 10 demonstrates imaging for four target mRNAs in whole-mount chicken embryos with high signal-to-background using unoptimized fractional initiator (aka a split-initiator) probes (Scheme E).

Due to the automatic background suppression property of fractional initiator (aka a split-initiator) probes, the signal-to-background ranges from approximately 25 to 60 for the four target mRNAs without performing any probe set optimization.

Figure 10. Multiplexed imaging of mRNA expression with high signal-to-background in a fixed whole-mount chicken embryo using fractional initiator (aka a split-initiator) probes without probe set optimization (Scheme E). (a) Expression schematics for four target mRNAs: FoxD3, EphA4, Sox10, Dmbx1. (b) Four-channel confocal micrographs in the head and neural crest. (c) Zoom of depicted region of panel b. (b) Four individual channels from panel c with signal-to-background measurements. Probe sets: 12-20 pairs of unoptimized fractional initiator (aka a split-initiator) probes per target. Amplifiers: four orthogonal HCR amplifiers carrying spectrally distinct fluorophores (one HCR amplifier per target). Embryo fixed: stage HH10.

### Example 5 - In situ HCR using fractional initiator (aka a split-initiator) probes for quantitative analysis of mRNA expression with subcellular resolution

Figure 11 demonstrates that in situ HCR using fractional initiator (aka a split-initiator) probes (Scheme E) allows for quantitative analysis of mRNA expression with subcellular resolution within whole-mount chicken embryos.

Each target mRNA was redundantly detected using two fractional initiator probe sets that each triggered a different spectrally-distinct HCR amplifier. Plotting a 2-channel scatter plot of normalized voxel intensities resulted in a tight linear relationship with approximately zero intercept, indicating that HCR signal scales linearly with the number of target mRNAs per imaging voxel. Accuracy improves as the distribution becomes linear and the intercept vanishes; precision improves as the scatter becomes tighter. The 2×2 µm voxels provided subcellular resolution.

These results demonstrate that in situ HCR with fractional initiator (aka a split-initiator) probes allows accurate and precise relative quantitation of mRNA expression with subcellular resolution in an anatomical context without the need for probe set optimization.

Figure 11. Quantitative imaging of mRNA expression with subcellular resolution in fixed whole-mount chicken embryos. (a) Two-channel redundant detection of target mRNAs. Targets: Dmbx1 and EphA4. Confocal microscopy: 0.2×0.2 µm pixels. Probe sets: 20 pairs of fractional initiator (aka a split-initiator) probes per channel for each target. Amplifiers: two orthogonal HCR amplifiers carrying spectrally distinct fluorophores for each target. Embryos fixed: stage HH10. (b) Highly correlated normalized signal (Pearson correlation coefficient, r) for 2×2 µm voxels in the selected regions of panel b.

### Example 6 - Comparison of performance of fractional initiator probes that are complementary to overlapping regions of HCR hairpins to fractional initiator probes that are complementary to non-overlapping regions of HCR hairpins

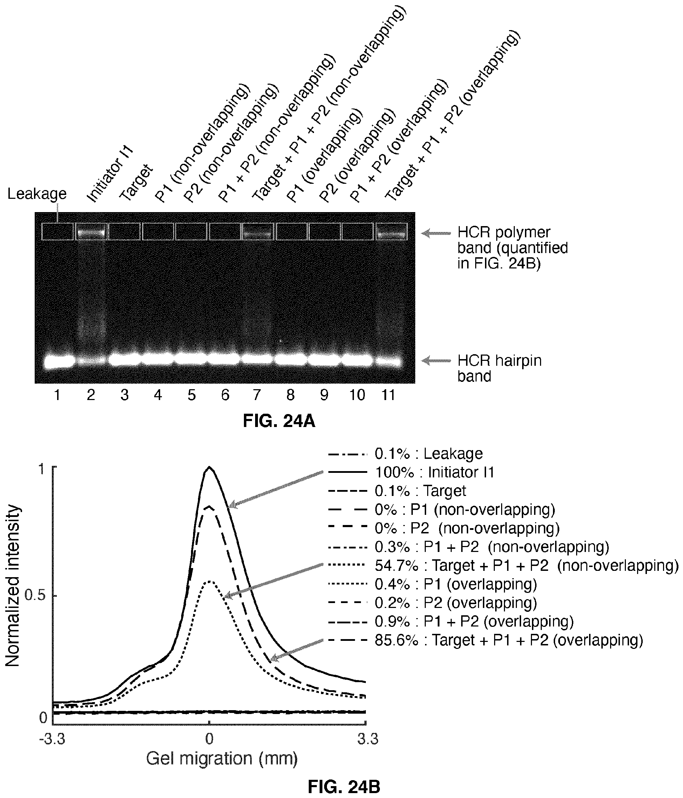

The gel studies of FIG. 24 compare the performance of fractional initiator probes that are complementary to overlapping regions of HCR hairpins H1 (for example, Figure 21) to fractional initiator probes that are complementary to non-overlapping regions of HCR hairpins H1 (for example, Figure 7A).

Reaction conditions: hairpins H1 and H2 at 0.6 µM each (Lanes 1-11); oligos I1, P1, P2, and/or Target at 6 nM each (Lanes as noted on the gel); 5 SSCT buffer; overnight reaction at room temperature. Hairpins H1 and H2 labeled with Alexa 647 fluorophore. Lane 1: Metastable hairpins H1 and H2 exhibit minimal leakage out of their kinetically trapped states in the absence of HCR initiator I1. Lane 2: Conversion of HCR hairpin monomers H1 and H2 into amplification polymer in the presence of HCR initiator I1. Lane 3: Minimal conversion of HCR hairpin monomers H1 and H2 into polymer in the presence of Target alone. Lanes 4-6, 8-10: Minimal conversion of HCR hairpin monomers H1 and H2 into polymer in the presence of probe P1, probe P2, or both probes P1 and P2, demonstrating active background suppression using the fractional initiator probes that are complementary to non-overlapping (Lanes 4-6) or overlapping (Lanes 8-10) regions of HCR hairpin H1. Lanes 7 and 11: Conversion of hairpins H1 and H2 to polymer in the presence of Target and both probes P1 and P2 that are complementary to non-overlapping (Lane 7) or overlapping (Lane 11) regions of HCR hairpin H1.

Quantification of the polymer bands. MATLAB was used to calculate the Alexa 647 intensity profile surrounding the polymer band for Lanes 1-11. Each intensity profile is displayed for ± 3.3 mm of gel migration distance with the peak value centered at 0. The quantification percentages were calculated using MATLAB with auto-detection of signal and background; the calculated values were normalized to the measured value for Lane 2.

These results demonstrated conversion of hairpins H1 and H2 to polymer in the presence of Target and probes P1 and P2 that are complementary to overlapping regions of HCR hairpins H1 (Lane 11; 85.6% conversion to polymer) is stronger than that of probes P1 and P2 that are complementary to non-overlapping regions of HCR hairpins H1 (Lane 7; 54.7% conversion to polymer). The active background suppression properties of fractional initiator probes remain comparable for both probe types.

### Example 7 - Dehybridization of HCR hairpins from HCR amplification polymers using an auxiliary strand

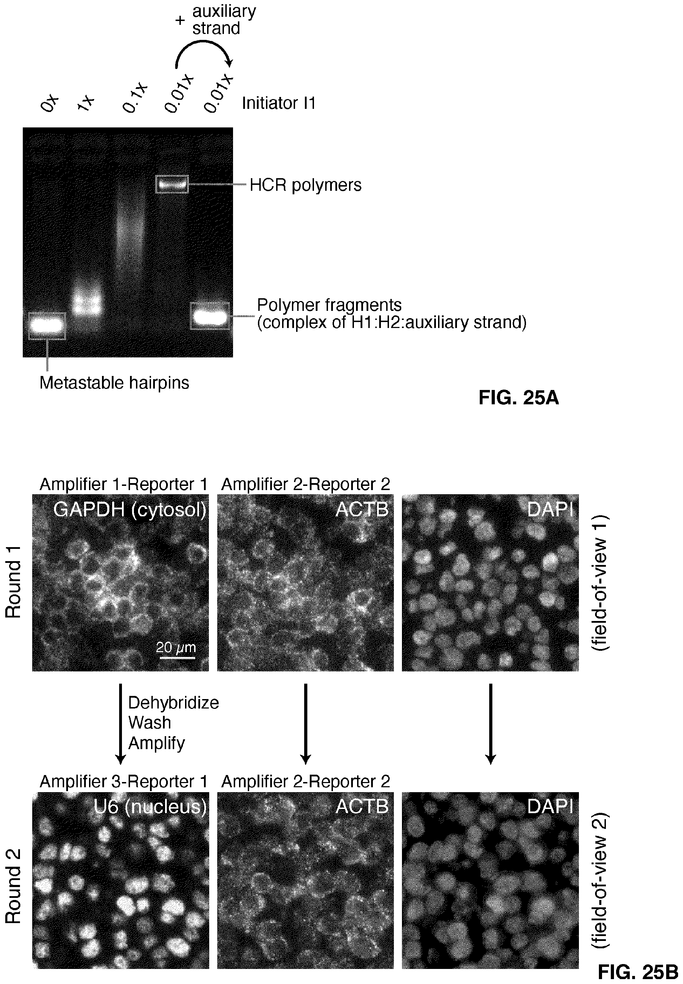

FIG. 25A depicts the in vitro validation of dehybridization of HCR hairpins from HCR amplification polymers using an auxiliary strand (for example, see Figure 23F). Reaction conditions: hairpins H1 and H2 at 0.6 µM each (Lanes 1-5); HCR initiator I1 at 0.6 µM (Lane 2), 60 nM (Lane 3), and 6 nM (Lanes 4 and 5); auxiliary strand at 6 µM (Lane 5); 5 SSCT buffer; overnight HCR amplification at room temperature; 1 hour auxiliary strand incubation at room temperature. Hairpins H1 and H2 labeled with Alexa 647 fluorophore. Lane 1: Metastable hairpins H1 and H2 exhibit minimal leakage in the absence of HCR initiator I1. Lane 2: Formation of short HCR amplification polymers at equimolar HCR initiator I1 concentration. Lanes 3 and 4: Formation of longer HCR amplification polymers at lower HCR initiator concentrations. Lane 5: Dehybridization of hairpins from HCR amplification polymers using an auxiliary strand to yield polymer fragments consisting of H1, H2, and the auxiliary strand.

Some embodiments of sequences used for dehybridization of HCR hairpins from HCR amplification polymers (for example, FIG. 25A) are shown below in TABLE 2. The sequences can be used for any of the embodiments of the methods herein.

**TABLE 2**

| | |
|---|---|
| **Hairpin H1 including toehold for auxiliary strand nucleation** | 5'- CGGGTTAAAGTTGAGTGGAGATATAGAGGCAGGGACAAAGTCTAATC CGTCCCTGCCTCTATATCTCCACTCTATCAT-3' (SEQ ID NO: 1) |
| **Hairpin H2** | 5'- GTCCCTGCCTCTATATCTCCACTCAACTTTAACCCGGAGTGGAGATAT AGAGGCAGGGACGGATTAGACTTT-3' (SEQ ID NO: 2) |
| **Initiator I1** | 5'-GTCCCTGCCTCTATATCTCCACTCAACTTTAACCCG-3' (SEQ ID NO: 3) |
| **Auxiliary strand** | 5'-ATGATAGAGTGGAGATATAGAGGCAGGGACGGATTAGACTTT-3' (SEQ ID NO: 4) |

FIG. 25B demonstrated imaging of 3 target mRNAs in FFPE cultured human cell (HEK293) pellet on a slide using repeated reporter detection (see Figures 23F and 26G). Target mRNAs: GAPDH, ACTB, U6. Probe sets for all three targets were introduced simultaneously in the detection stage. In the first round of amplification, only GAPDH and ACTB were detected using amplifiers labeled with spectrally distinct fluorophores (Amplifier 1 labeled with Reporter 1 (Alexa647) for GAPDH, Amplifier 2 labeled with Reporter 2 (Alexa546) for ACTB). Only Amplifier 1 used for GAPDH contained the toehold for auxiliary strand nucleation. After image acquisition, the sample was incubated with Amplifier 1 auxiliary strands for 1 hour at room temperature to dehybridize Alexa647-labeled hairpins from amplification polymers tethered to GAPDH mRNAs; resulting polymer fragments were washed from the sample. In the second round of amplification, a different HCR amplifier labeled with the same reporter as GAPDH (Amplifier 3 labeled with Reporter 1 (Alexa647)) was used for U6 signal amplification.

The images demonstrated the cytoplasmic Reporter 1 (Alexa 647) signal for GAPDH was completely removed by the addition of the Amplifier 1 auxiliary strand followed by washes and was replaced by the nuclear Reporter 1 (Alexa 647) signal of U6 in the second round of imaging. ACTB signal strength (Amplifier 2 labeled with Reporter 2 (Alexa 546)) remains comparable between imaging rounds.

### Example 8 - Optimization of cooperative probe junctions to enhance fractional initiator HCR suppression and conversion

FIG. 28 demonstrates the cooperative probe junctions to enhance fractional initiator HCR suppression (OFF state) and conversion (ON state). In FIG. 28A, fractional initiator probes p1 and p2 are complementary to overlapping regions of HCR hairpin h1 (each contains domains u* and v* complementary to domains u and v in h1) to enable relaxation of the junction into an energetically favorable conformation. In FIG. 28B, leakage (OFF state) is assayed by agarose gel using probes with varying degrees of overlap (u* = 0, 1, or 2 nucleotides, v* = 0, 1, or 2 nucleotides). The OFF state is characterized by testing HCR initiation by individual probes p1 or p2 (FIG. 28B left), and by quantifying the resulting polymerization bands in the gel (FIG. 28B right). In FIG. 28C, conversion (ON state) is assayed by agarose gel using probes with varying degrees of overlap (u* = 0, 1, or 2 nucleotides, v* = 0, 1 or 2 nucleotides). The ON state is characterized by testing HCR initiation by probes p1 and p2 plus target (FIG. 28C left) and by quantifying the resulting polymerization bands in the gel (FIG. 28C right). The reaction conditions for FIG. 28 were hairpins h1 and h2 at 500 nM each for OFF state and 60 nM each for ON state (all lanes); initiator i1, probes p1 and p2, and/or DNA target at 0.01× hairpin concentration.

Testing different junction designs, yields probes p1 and p2 with a clean OFF state (fractional initiator HCR suppression ≈0.5% of standard-initiator HCR suppression; boxed FIG. 28B) and a strong ON state (fractional initiator HCR conversion ≈99% of standard-initiator HCR conversion; boxed in FIG. 28C). These results indicate that replacement of a standard probe (v2.0) with a pair of fractional initiator probes (v3.0) can be engineered to dramatically decrease amplified background (lane 2 vs lanes 4 and 9 in FIG. 28B) without significantly decreasing amplified signal (lane 2 vs lane 6 in FIG. 28C). For this probe junction, the domain dimensions are u* = 0 nucleotides and v* = 2 nucleotides, corresponding to fractional initiators within a probe unit that hybridize to binding sites in HCR hairpin h1 that overlap by 2 nucleotides.

### Example 9 - Multiplexed immunohistochemistry (IHC) HCR imaging of protein targets

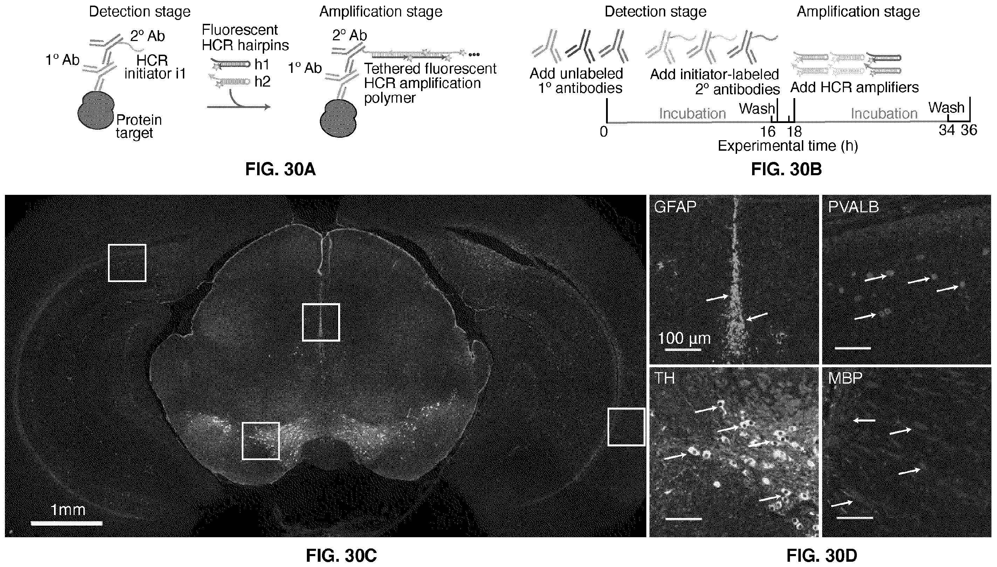

FIG. 29 and FIG. 30 demonstrate multiplexed immunohistochemistry (IHC) HCR imaging of protein targets in formalin-fixed paraffin-embedded (FFPE) mouse brain sections. FIG.

FIG. 29 employs initiator-labeled primary antibody probes. FIG. 29A displays the 2-stage IHC protocol consisting of a protein detection stage (initiator-labeled primary antibody probes bind to protein targets; wash) and an amplification stage (initiators trigger self-assembly of fluorophore-labeled HCR hairpins into tethered fluorescent amplification polymers; wash). FIG. 29B displays the multiplexing timeline in which the same 2-stage protocol is used independent of the number of target proteins. FIG. 29C displays a four-channel epifluorescence micrograph of an FFPE mouse brain section (four target proteins: TH, GFAP, MBP, MAP2) and FIG. 29D displays zooms for the depicted regions of FIG. 29D. The approach of FIG. 29 has the advantage that high levels of multiplexing can be achieved using initiator-labeled primary antibodies where the antibody probes for different protein targets carry orthogonal HCR initiators that operate independently in the sample at the same time. There is no requirement for the primary antibodies to be raised in different organisms, providing flexibility in establishing a large library of primary antibodies for different targets.

FIG. 30 employs unlabeled primary antibody probes and initiator-labeled secondary antibody probes. FIG. 30A displays the 2-stage IHC protocol consisting of a protein detection stage (unlabeled primary antibody probes bind to protein targets; wash; initiator-labeled secondary antibody probes bind to primary antibody probes; wash) and an amplification stage (initiators trigger self-assembly of fluorophore-labeled HCR hairpins into tethered fluorescent amplification polymers; wash). FIG. 30B displays the multiplexing timeline in which the same 2-stage protocol is used independent of the number of target proteins. 4-plex IHC in FFPE mouse brain sections. FIG. 30C displays a four-channel epifluorescence micrograph of an FFPE mouse brain section (four target proteins: TH, GFAP, PVALB, MBP) and FIG. 30D displays zooms of the depicted regions of FIG. 30C.

The approach of FIG. 30 has the advantage that unlabeled primary antibodies can be used without modification, enabling use of a validated library of initiator-labeled secondary antibodies, where different secondary antibodies carry orthogonal HCR initiators that operate independently in the sample at the same time. With this approach, the primary antibodies must be raised in different organisms so that only one type of initiator-labeled secondary antibody detects each primary antibody.

### Example 10 - Quantitative analysis of protein expression with subcellular resolution by HCR IHC

FIG. 41 demonstrates that HCR IHC allows for quantitative analysis of protein expression with subcellular resolution in an anatomical context. Each target protein was redundantly detected using two initiator-labeled primary antibody probes that each triggered a different spectrally-distinct HCR amplifier. Plotting a 2-channel scatter plot of normalized voxel intensities resulted in a tight linear relationship with approximately zero intercept, indicating that HCR signal scales linearly with the number of target proteins per imaging voxel.

FIG. 41A illustrates 2-channel redundant detection of a target protein. The target protein is detected using two initiator-labeled primary antibody probes that bind different epitopes, each initiating an orthogonal spectrally distinct HCR amplifier (Ch1: Alexa 546, Ch2: Alexa 647). FIG. 41B displays epifluorescence micrographs of an FFPE mouse brain section. FIG. 41C displays highly correlated normalized signal (Pearson correlation coefficient, r) for subcellular 2 ×2 µm voxels in the boxed region of FIG. 41B. In this scatter plot, accuracy corresponds to linearity with zero intercept and precision corresponds to scatter around the line. The 2×2 µm voxels provided subcellular resolution.

These results demonstrate that HCR IHC allows accurate and precise relative quantitation of protein expression with subcellular resolution in an anatomical context.

### Example 11 - Multiplexed HCR RNA-ISH and HCR IHC with HCR signal amplification for all mRNA and protein targets simultaneously

FIG. 31 and FIG. 32 demonstrate multiplexed HCR RNA-ISH and HCR IHC with HCR signal amplification performed for all mRNA and protein targets simultaneously.

FIG. 31 demonstrates multiplexed HCR IHC + RNA-ISH using initiator-labeled primary antibody probes and fractional initiator DNA probes. FIG. 31A displays a 3-stage IHC + ISH protocol consisting of a protein detection stage (initiator-labeled primary antibody probes bind to protein targets; wash), an RNA detection stage (fractional initiator DNA probes bind to RNA targets; wash), and an amplification stage (initiators trigger self-assembly of fluorophore-labeled HCR hairpins into tethered fluorescent amplification polymers; wash). The same 3-stage protocol is used independent of the number of target proteins and target RNAs. FIG. 31B displays a four-channel epifluorescence micrograph of an FFPE mouse brain section (two target proteins (TH, MBP) and two target mRNAs (Prkca, Slc17a7) and FIG. 31C displays zooms of the depicted regions of FIG. 31B.

FIG. 32 demonstrates multiplexed HCR IHC + RNA-ISH using unlabeled primary antibody probes, initiator-labeled secondary antibody probes, and fractional initiator DNA probes. FIG. 32A displays a 3-stage IHC + ISH protocol consisting of a protein detection stage (unlabeled primary antibody probes bind to protein targets; wash; initiator-labeled secondary-antibody probes bind to primary-antibody probes; wash), an RNA detection stage (fractional initiator DNA probes bind to RNA targets; wash), and an amplification stage (initiators trigger self-assembly of fluorophore-labeled HCR hairpins into tethered fluorescent amplification polymers; wash). The same 3-stage protocol is used independent of the number of target proteins and target RNAs. FIG. 32B displays a four-channel epifluorescence micrograph of an FFPE mouse brain section (two target proteins (TH, MBP) and two target mRNAs (Prkca, Slc17a7)) and FIG. 32C displays a zoom of depicted region of FIG. 32B.

### Example 12 - HCR-mediated CARD RNA-ISH

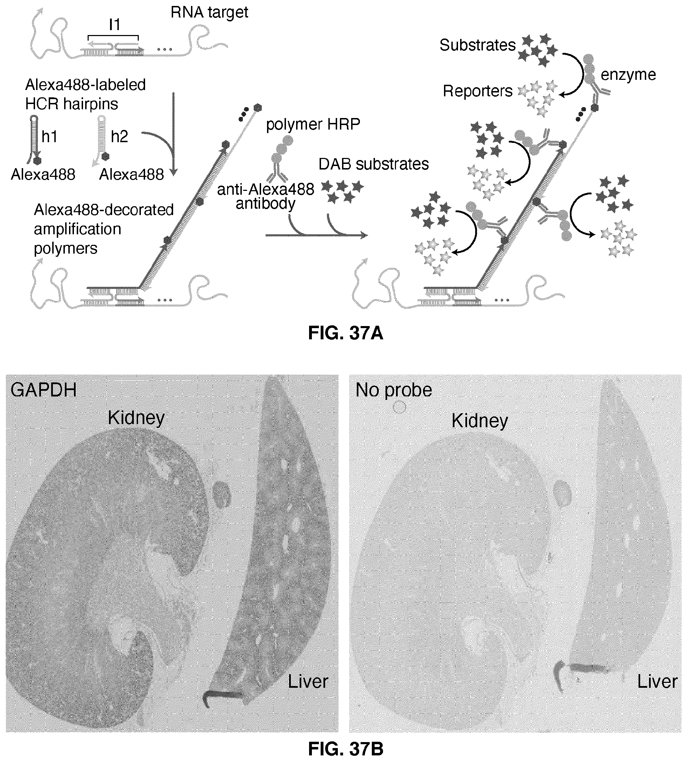

FIG. 37 demonstrates HCR-mediated CARD RNA-ISH in FFPE mouse liver and kidney sections. FIG. 37A displays a schematic summarizing the HCR-mediated CARD RNA-ISH protocol, including: detection of the RNA target using a probe set comprising multiple probe units each comprising two HCR fractional initiator probes, HCR signal amplification using hairpins h1 and h2 labeled with fluorophore Alexa488 as a hapten, binding to the hapten using an anti-Alexa488 antibody labeled with polymer HRP enzyme, providing CARD-substrate DAB which was catalytically deposited as a reporter in the vicinity of the HCR amplification polymer by the HRP. FIG. 37B (left panel) depicts brightfield microscopy of GAPDH mRNA expression in FFPE mouse kidney and liver sections; in the absence of probes, the staining is absent (right panel).

### Example 13 - Multiplexed microRNA and mRNA RNA-ISH using initiator-labeled probes

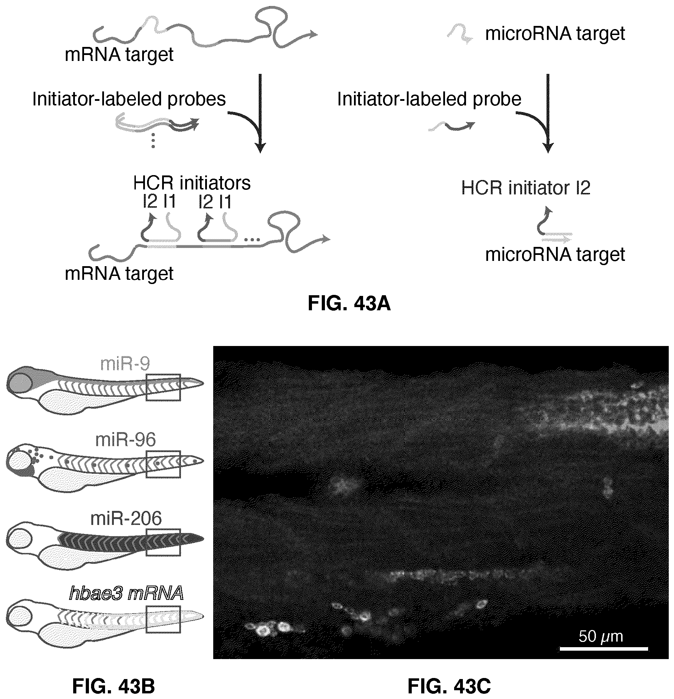

FIG. 43 demonstrates multiplexed microRNA and mRNA RNA-ISH using initiator-labeled probes. microRNA targets were detected using initiator-labeled probes comprising a 2'OMe-RNA target-binding domain and a DNA initiator (FIG. 43A). mRNA targets were detected using initiator-labeled probes comprising a DNA target-binding domain and two DNA initiators (one at each end of the target-binding domain; FIG 43A). HCR signal amplification was performed for both classes of target RNAs (microRNAs and mRNAs) simultaneously, achieving high signal-to-background in whole-mount zebrafish embryos. FIG. 43B depicts expression atlases for three target microRNAs (miR-9, miR-96, miR-206) and one target mRNA (*hbae3).* The embryo was fixed 72 hours post-fertilization and imaged by confocal microscopy (FIG. 43C).

### Example 14 - Reduction of background using shielded initiator-labeled antibody probes

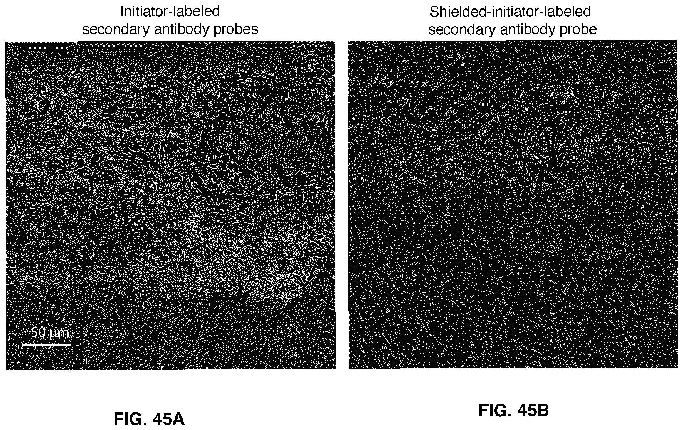

FIG. 45 demonstrates reduction of background using shielded initiator-labeled antibody probes compared to initiator-labeled antibody probes without shielding for HCR IHC in whole-mount zebrafish embryos. In either case, the target protein Desmin as detected with an unlabeled primary antibody, which in turn is bound with either an initiator-labeled secondary antibody (FIG. 45A) or a shielded initiator-labeled secondary antibody (FIG. 45B) in which the initiator is partially shielded by base-pairing to the initiator. Reduced background is observed using shielded initiator-labeled probes compared to initiator-labeled probes, illustrating that shielding the initiators on a probe can be used to reduce non-specific binding. Embryos fixed 27 hours post-fertilization and imaged via confocal microscopy.

### Example 15 - Multiplexed analysis using repeated reporter detection (Method A) (which can optionally be modified further by CARD, enzyme deactivation, and/or repeated CARD)

The present example outlines the process in Figure 26A. Step A1: one provides a sample possibly containing a target as well as possibly other molecules that are not targets. Step A2: One fixes the sample; Step A3: one optionally permeabilizes the sample. Step A4: provide a probe set comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17). Step A5: washing the sample. Step A6: providing an HCR amplifier labeled with a reporter (for example, see the reporter-labeled amplifiers of Figures 8 and 18). Step A7: washing the sample; Step A8: Detecting a signal from the reporter.

### Example 16 - Multiplexed analysis using repeated reporter detection (Method B) (which can optionally be modified further by CARD, enzyme deactivation, and/or repeated CARD)

The present example outlines the process in Figure 26B. Step B1: Provide a sample possibly containing up to N targets as well as possibly other molecules that are not targets. Step B2: optionally fixing the sample. Step B3: optionally permeabilizing the sample. Step B4: providing N probe sets each comprising either: a) one or more HCR initiator-labeled probes (for example, see the probes of Figures 39A-39N, 41A, 42A-42F, and 43A), or b) one or more probe units (for example see the probe sets of Figures 8 and 16) each comprising two or more HCR fractional initiator probes (for example, see the probe units of Figures 3, 4, 5, and 17). Step B5: optionally washing the sample. Step B6: providing N HCR amplifiers (each labeled with a distinct reporter) corresponding to the N probe sets (for example, see the reporter-labeled HCR amplifiers of Figures 8 and 18). Step B7: optionally washing the sample. Step B8: detecting N signals from the N distinct reporters.

### Example 17 - Multiplexed analysis using repeated reporter detection (Method C) (which can optionally be modified further by CARD, enzyme deactivation, and/or repeated CARD)

The present example outlines the process in Figure 26C. Step C1: providing a sample possibly containing up to N targets as well as possibly other molecules that are not targets. Step C2: optionally fixing the sample. Step C3: optionally permeabilizing the sample. Step C4: providing a probe set (targeting one of the N target types) comprising either: a) one or more HCR initiator-labeled probes (for example, see the probes of Figures 39A-39N, 41A, 42A-42F, and 43A), or b) one or more probe units (for example see the probe sets of Figures 8 and 16) each comprising two or more HCR fractional initiator probes (for example, see the probe units of Figures 3, 4, 5, and 17). Step C5: optionally washing the sample. Step C6: providing an HCR amplifier (labeled with a reporter) corresponding to the provided probe (for example, see the reporter-labeled HCR amplifiers of Figures 8 and 18). Step C7: optionally washing the sample. Step C8: detecting a signal from the reporter. Step C9: removing the signal from the sample (for example, see Figure 23). Step C10: optionally repeating one or more of Steps C4-C9 until signal detection has been performed for all N targets.

### Example 18 - Multiplexed analysis using repeated reporter detection (Method D) (which can optionally be modified further by CARD, enzyme deactivation, and/or repeated CARD)

The present example outlines the process in Figure 26D. Step D1: providing a sample possibly containing up to N targets as well as possibly other molecules that are not targets. Step D2: optionally fixing the sample. Step D3: optionally permeabilizing the sample. Step D4: providing M probe sets (for M ≤ N; each targeting one of M target types) each comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17). Step D5: optionally washing the sample. Step D6: providing M HCR amplifiers (each labeled with a distinct reporter) corresponding to the M probe sets (for example, see the reporter-labeled HCR amplifiers of Figures 8 and 18); Step D7: Optionally washing the sample. Step D8: detecting M signals corresponding to the M distinct reporters. Step D10: removing the M signals from the sample (for example, see Figure 23). Step D11: optionally repeating one or more of steps 4-9 until signal detection has been performed for all N targets.

### Example 19 - Multiplexed analysis using repeated reporter detection (Method E) (which can optionally be modified further by CARD, enzyme deactivation, and/or repeated CARD)

The present example outlines the process in Figure 26E. Step E1: providing a sample possibly containing up to N targets as well as possibly other molecules that are not targets. Step E2: optionally fixing the sample Step E3: optionally permeabilizing the sample. Step E4: providing N probe sets (each targeting one of N target types) each comprising either: a) one or more HCR initiator-labeled probes (for example, see the probes of Figures 39A-39N, 41A, 42A-42F, and 43A), or b) one or more probe units (for example, see the probe sets of Figure 8 and 16) each comprising two or more HCR fractional initiator probes (for example, see the probe units of Figures 3, 4, 5, and 17). Step E5: optionally washing the sample. Step E6: providing an HCR amplifier (labeled with a reporter) corresponding to one of the probe sets (for example, see the reporter-labeled amplifiers of Figure 8 and 18). Step E7: optionally washing the sample. Step E8: detecting a signal from the reporter. Step E9: removing the signal from the sample (for example, see Figure 23). Step E10: optionally repeating one or more of steps 6-9 until signal detection has been performed for all N targets.

### Example 20 - Multiplexed analysis using repeated reporter detection (Method F) (which can optionally be modified further by CARD, enzyme deactivation, and/or repeated CARD)

The present example outlines the process in Figure 26F. Step F1: providing a sample possibly containing up to N targets as well as possibly other molecules that are not targets. Step F2: optionally fixing the sample. Step F3: optionally permeabilizing the sample. Step F4: providing N probe sets (each targeting one of N target types) each comprising either: a) one or more HCR initiator-labeled probes (for example, see the probes of Figures 39A-39N, 41A, 42A-42F, and 43A), or b) one or more probe units (for example, see the probe sets of Figure 8 and 16) each comprising two or more HCR fractional initiator probes (for example, see the probe units of Figures 3, 4, 5, and 17). Step F5: optionally washing the sample. Step F6: providing M HCR amplifiers (for M ≤ N; each labeled with a distinct reporter) corresponding to M of the N probe sets (for example, see the reporter-labeled amplifiers of Figure 8 and 18). Step F7: optionally washing the sample. Step F8: detecting M signals corresponding to the M reporters. Step F9: removing the M signals from the sample (for example, see Figure 23). Step F10: optionally repeating one or more of steps 6-9 until signal detection has been performed for all N targets.

### Example 21 - Multiplexed analysis using repeated reporter detection (Method G) (which can optionally be modified further by CARD, enzyme deactivation, and/or repeated CARD)

The present example outlines the process in Figure 26G. Step G1: providing a sample possibly containing one or more targets as well as possibly other molecules that are not targets. Step G2: optionally fixing the sample. Step G3: optionally permeabilizing the sample. Step G4: providing one or more probe sets each comprising either: a) one or more HCR initiator-labeled probes (for example, see the probes of Figures 39A-39N, 4A, 42A-42F, and 43A), or b) one or more probe units (for example, see the probe sets of Figures 8 and 16) each comprising two or more HCR fractional initiator probes (for example, see the probe units of Figures 3, 4, 5, and 17). Step G5: optionally washing the sample. Step G6: providing one or more HCR amplifiers (each labeled with one or more reporters) (for example, see the reporter-labeled HCR amplifiers of Figure 8 and 18). Step G7: optionally washing the sample. Step G8: detecting one or more signals from one or more reporters. Step G9: optionally removing one or more probe sets from the sample. Step G10; optionally removing one or more HCR amplifiers from the sample. Step G11: optionally removing one or more reporters from the sample. Step G12: optionally removing one or more signals from the sample (for example, see Figure 23). Step G13: optionally repeating any of steps 2-12 one or more times in any order.

### Example 22 - Multiplexed analysis using repeated reporter detection (Method H) (which can optionally be modified further by CARD, enzyme deactivation, and/or repeated CARD)

he present example outlines the process in Figure 26H. Step H1: providing a sample possibly containing a target as well as possibly other molecules that are not targets. Step H2: optionally fixing the sample. Step H3: optionally permeabilizing the sample. Step H4: providing a probe set comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17). Step H5: optionally washing the sample. Step H6: providing an HCR amplifier labeled with a substrate (for example, see the substrate-labeled HCR amplifiers of Figure 18). Step H7: optionally washing the sample. Step H8: providing a label probe (conjugated to a reporter) corresponding to the substrate (for example, see the label probes of Figure 20). Step H9: optionally washing the sample. Step H10: detecting a signal from the reporter.

### Example 23 - Multiplexed analysis using repeated reporter detection (Method I) (which can optionally be modified further by CARD, enzyme deactivation, and/or repeated CARD)

The present example outlines the process in Figure 26I. Step I1: providing a sample possibly containing up to N targets as well as possibly other molecules that are not targets. Step I2: optionally fixing the sample. Step I3: optionally permeabilizing the sample. Step I4: providing N probe sets each comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17). Step I5: optionally washing the sample. Step I6: providing N HCR amplifiers (each labeled with a distinct substrate) corresponding to the N probe sets (for example, see the substrate-labeled HCR amplifiers of Figure 18). Step I7: optionally washing the sample. Step I8: providing N label probes (each conjugated to a distinct reporter) corresponding to the N distinct substrates (for example, see the label probes of Figure 20). Step I9: detecting the N signals from the N distinct reporters.

### Example 24 - Multiplexed analysis using repeated reporter detection (Method J) (which can optionally be modified further by CARD, enzyme deactivation, and/or repeated CARD)

The present example outlines the process in Figure 26J. Step J1: providing a sample possibly containing up to N targets as well as possibly other molecules that are not targets. Step J2: optionally fixing the sample. Step J3: optionally permeabilizing the sample. Step J4: providing N probe sets each comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17). Step J5: optionally washing the sample. Step J6: providing N HCR amplifiers (each labeled with a distinct substrate) corresponding to the N probe sets (for example, see the substrate-labeled HCR amplifiers of Figure 18). Step J7: optionally washing the sample. Step J8: providing M label probes (for M ≤ N; each conjugated to a distinct reporter) corresponding to M of the N distinct substrates (for example, see the label probes of Figure 20). Step J9: optionally washing the sample. Step J10: detecting M signals corresponding to the M distinct reporters. Step J11: removing the M signals from the sample (for example, see Figure 23). Step J12: optionally repeating one or more of steps J8-J11 until signal detection has been performed for all N targets.

### Example 25 - Multiplexed analysis using repeated reporter detection (Method K) (which can optionally be modified further by CARD, enzyme deactivation, and/or repeated CARD)

The present example outlines the process in Figure 26K. Step K1: providing a sample possibly containing one or more targets as well as possibly other molecules that are not targets. Step K2: optionally fixing the sample. Step K3: optionally permeabilizing the sample. Step K4: providing one or more probe sets each comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17). Step K5: optionally washing the sample. Step K6: providing one or more HCR amplifiers (each labeled with a substrate) corresponding to one or more probe sets (for example, see the substrate-labeled HCR amplifiers of Figure 18). Step K7: optionally washing the sample. Step K8: providing one or more label probes (each conjugated to a reporter) corresponding to one or more substrates (for example, see the label probes of Figure 20). Step K9: optionally washing the sample. Step K10: detecting one or more signals corresponding to one or more reporters. Step K11: removing one or more signals from the sample (for example, see Figure 23). Step K12: optionally repeating any of steps K4-K11 one or more times in any order.

### Example 26 - Multiplexed analysis using repeated reporter detection (Method L) (which can optionally be modified further by CARD, enzyme deactivation, and/or repeated CARD)

The present example outlines the process in Figure 26L. Step L1: providing a sample possibly containing one or more targets as well as possibly other molecules that are not targets. Step L2: optionally fixing the sample. Step L3: optionally permeabilizing the sample. Step L4: providing one or more HCR probe sets each comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17). Step L5: providing one or more HCR amplifiers (each labeled with one or more reporters and/or one or more substrates) corresponding to one or more probe sets (for example, see the HCR amplifiers of Figures 8 and 18). Step L6: optionally providing one or more label probes (each conjugated to one or more reporters) corresponding to one or more substrates (for example, see the label probes of Figure 20). Step L7: detecting one or more signals. Step L8: optionally washing the sample. Step L9: optionally removing one or more signals from the sample (for example, see Figure 23). Step L10: optionally removing one or more reporters from the sample. Step L11: optionally removing one or more label probes from the sample. Step L12: optionally removing one or more HCR amplifiers from the sample. Step L13: optionally removing one or more probe sets from the sample. Step L14: optionally repeating any of the above steps in any order.

### Example 27 - Multiplexed analysis using repeated reporter detection (Method M) (which can optionally be modified further by CARD, enzyme deactivation, and/or repeated CARD)

The present example outlines the process in Figure 26M. Step M1: providing a sample possibly containing one or more targets as well as possibly other molecules that are not targets. Step M2: optionally fixing the sample. Step M3: optionally permeabilizing the sample. Step M4: performing any of Steps M5-M9 one or more times in any order. Step M5: providing one or more HCR probe sets each comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes (for example, see the probe units of Figures 3, 4, 5, and 17). Step M6: providing one or more HCR amplifiers that directly or indirectly generate one or more signals (for example, see Figures 8, 18, and 20). Step M7: optionally washing the sample. Step M8: detecting one or more signals. Step M9: optionally removing one or more signals (for example, see Figure 23).

### Example 28 - Target analysis (Method N) (which can optionally be modified further by multiplexing, CARD, enzyme deactivation, repeated CARD, repeated reporter detection, and/or repeated signal removal)

The present example outlines the process in Figure 26N. Step N1: providing a sample possibly containing a target as well as possibly other molecules that are not targets. Step N2: optionally fixing the sample. Step N3: optionally permeabilizing the sample. Step N4: providing a probe set comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes where the target-binding regions on the probes within each probe unit are configured to bind to overlapping binding sites on the target (for example, see the probe units of Figure 22). Step N5: optionally washing the sample. Step N6: providing an HCR amplifier labeled with a reporter and/or a substrate (for example, see the HCR amplifiers of Figures 8 and 18). Step N7: optionally washing the sample. Step N8: optionally providing a label probe (conjugated to a reporter) corresponding to the substrate (for example, see the label probes of Figure 20). Step N9: optionally washing the sample. Step N10: detecting a signal from the reporter.

### Example 29 - Target analysis (Method O) (which can optionally be modified further by multiplexing, CARD, enzyme deactivation, repeated CARD, repeated reporter detection, and/or repeated signal removal)

The present example outlines the process in Figure 26O. Step O1: providing a sample possibly containing a target as well as possibly other molecules that are not targets. Step O2: optionally fixing the sample. Step O3: optionally permeabilizing the sample. Step O4: providing a probe set comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes where the fractional initiators on the probes within each probe unit are configured to bind to overlapping binding sites on an HCR hairpin (for example, see the probe units of Figure 21). Step O5: optionally washing the sample. Step O6: providing an HCR amplifier labeled with a reporter and/or a substrate (for example, see the HCR amplifiers of Figures 8 and 18). Step O7: optionally washing the sample. Step O8: optionally providing a label probes (conjugated to a reporter) corresponding to the substrate (for example, see the label probes of Figure 20). Step O9: optionally washing the sample. Step O10: detecting a signal from the reporter.

### Example 30 - Target analysis (Method P) (which can optionally be modified further by multiplexing, CARD, enzyme deactivation, repeated CARD, repeated reporter detection, and/or repeated signal removal)

The present example outlines the process in Figure 26P.: Step P1: providing a sample possibly containing a target as well as possibly other molecules that are not targets. Step P2: optionally fixing the sample. Step P3: optionally permeabilizing the sample. Step P4: providing a probe set comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes where the target binding regions on the probes within each probe unit are configured to bind to overlapping binding sites on the target and where the fractional initiators on the probes within each probe unit are configured to bind to overlapping binding sites on an HCR hairpin (for example, see the probe units of Figure 27). Step P5: optionally washing the sample. Step P6: providing an HCR amplifier labeled with a reporter and/or a substrate (for example, see the HCR amplifiers of Figures 8 and 18). Step P7: optionally washing the sample. Step P8: optionally providing a label probe (conjugated to a reporter) corresponding to the substrate (for example, see the label probes of Figure 20). Step P9: optionally washing the sample. Step P10: detecting a signal from the reporter.

### Example 31 - Target analysis (Method Q) (which can optionally be modified further by multiplexing, CARD, enzyme deactivation, repeated CARD, repeated reporter detection, and/or repeated signal removal)

The present example outlines the process in Figure 26Q Step Q1: providing a sample possibly containing a target as well as possibly other molecules that are not targets. Step Q2: optionally fixing the sample. Step Q3: optionally permeabilizing the sample. Step Q4: providing a probe set comprising either: a) one or more HCR initiator-labeled probes (for example, see the probes of Figures 39A-39N, 4A, 42A-42F, and 43A), or b) one or more probe units each comprising two or more HCR fractional initiator probes where the target binding regions on the probes within each probe unit are configured to bind to non-overlapping binding sites on the target and where the fractional initiators on the probes within each probe unit are configured to bind to overlapping binding sites on an HCR hairpin (for example, see the probe units of Figure 21). Step Q5: optionally washing the sample. Step Q6: providing an HCR amplifier labeled with a reporter and/or a substrate (for example, see the HCR amplifiers of Figures 8 and 18). Step Q7: optionally washing the sample. Step Q8: optionally providing a label probe (conjugated to a reporter) corresponding to the substrate (for example, see the label probes of Figure 20). Step Q9: optionally washing the sample. Step Q10: detecting a signal from the reporter.

### Example 32 - Target analysis (Method R) (which can optionally be modified further by multiplexing, CARD, enzyme deactivation, repeated CARD, repeated reporter detection, and/or repeated signal removal)

The present example outlines the process in Figure 26R. Step R1: providing a sample possibly containing a target as well as possibly other molecules that are not targets. Step R2: optionally fixing the sample. Step R3: optionally permeabilizing the sample. Step R4: providing a probe set comprising either: a) one or more HCR initiator-labeled probes (for example, see the probes of Figures 39A-39N, 41A, 42A-42F, and 43A), or b) one or more probe units each comprising two or more HCR fractional initiator probes where the target binding regions on the probes within each probe unit are configured to bind to overlapping binding sites on the target and where the fractional initiators on the probes within each probe unit are configured to bind to non-overlapping binding sites on an HCR hairpin (for example, see the probe units of Figure 22). Step R5: optionally washing the sample. Step R6: providing an HCR amplifier labeled with a reporter and/or a substrate (for example, see the HCR amplifiers of Figures 8 and 18). Step R6: optionally washing the sample. Step R8: optionally providing a label probe (conjugated to a reporter) corresponding to the substrate (for example, see the label probes of Figure 20). Step R9: optionally washing the sample. Step R10: detecting a signal from the reporter.

### Example 33 - Target analysis (Method S) (which can optionally be modified further by multiplexing, CARD, enzyme deactivation, repeated CARD, repeated reporter detection, and/or repeated signal removal)

The present example outlines the process in Figure 26S. Step S1: providing a sample possibly containing a target as well as possibly other molecules that are not targets. Step2: optionally fixing the sample. Step S3: optionally permeabilizing the sample. Step S4: providing a probe set comprising either: a) one or more HCR initiator-labeled probes (for example, see the probes of Figures 39A-39N, 41A, 42A-42F, and 43A), or b) one or more probe units (for example, see the probe sets of Figures 8 and 16) each comprising two or more HCR fractional initiator probes where the target binding regions on the probes within each probe unit are configured to bind to non-overlapping binding sites on the target and where the fractional initiators on the probes within each probe unit are configured to bind to non-overlapping binding sites on an HCR hairpin (for example, see the probe units of Figures 3, 4, 5, 17). Step S5: optionally washing the sample. Step S6: providing an HCR amplifier labeled with a reporter and/or a substrate (for example, see the HCR amplifiers of Figures 8 and 18). Step S7: optionally washing the sample. Step S8: optionally providing a label probe (conjugated to a reporter) corresponding to the substrate (for example, see the label probes of Figure 20). Step S9: optionally washing the sample. Step S10: detecting a signal from the reporter.

### Example 34 - Multiplexed analysis using repeated reporter detection (Method T) (which can optionally be modified further by CARD, enzyme deactivation, and/or repeated CARD)

The present example outlines the process in Figure 26T). Step T1: providing a sample possibly containing one or more targets as well as possibly other molecules that are not targets. Step T2: optionally fixing the sample. Step T3: optionally permeabilizing the sample. Step T4: providing one or more probe sets each comprising either: a) one or more HCR initiator-labeled probes (for example, see the probes of Figures 39A-39N, 41A, 42A-42F, and 43A), or b) one or more probe units each comprising two or more HCR fractional initiator probes where the target binding regions on the probes within each probe unit are configured to bind to overlapping or non-overlapping binding sites on a target and where the fractional initiators on the probes within each probe unit are configured to bind to overlapping or non-overlapping binding sites on an HCR hairpin (for example, see the probe units of Figure 3, 4, 5, 17, 21, 22, 27). Step T5: optionally washing the sample. Step T6: providing one or more HCR amplifiers each labeled with one or more reporters and/or substrates (for example, see the HCR amplifiers of Figures 8 and 18). Step T7: optionally washing the sample. Step T8: optionally providing one or more label probes (each conjugated to one or more reporters) corresponding to one or more substrates (for example, see the label probes of Figure 20). Step T9: optionally washing the sample. Step T10: detecting a signal from one or more reporters. Step T11: optionally removing one or more signals from the sample (for example, see Figure 23). Step T12: optionally removing one or more reporters from the sample. Step T13: optionally removing one or more label probes from the sample. Step T14: optionally removing one or more amplifiers from the sample. Step T15: optionally removing one or more probe sets from the sample. Step T16: optionally repeating any of the above steps in any order.

Although the foregoing invention has been described in terms of certain preferred embodiments, other embodiments will be apparent to those of ordinary skill in the art. Additionally, other combinations, omissions, substitutions and modification will be apparent to the skilled artisan, in view of the disclosure herein. Accordingly, the present invention is not intended to be limited by the recitation of the preferred embodiments, but is instead to be defined by reference to the appended claims. All references cited herein are incorporated by reference in their entirety.

The terminology used in the description presented herein is not intended to be interpreted in any limited or restrictive manner and unless otherwise indicated refers to the ordinary meaning as would be understood by one of ordinary skill in the art in view of the specification. Furthermore, embodiments may comprise, consist of, consist essentially of, several novel features, no single one of which is solely responsible for its desirable attributes or is believed to be essential to practicing the embodiments herein described. As used herein, the section headings are for organizational purposes only and are not to be construed as limiting the described subject matter in any way. All literature and similar materials cited in this application, including but not limited to, patents, patent applications, articles, books, treatises, and internet web pages are expressly incorporated by reference in their entirety for any purpose. When definitions of terms in incorporated references appear to differ from the definitions provided in the present teachings, the definition provided in the present teachings shall control. It will be appreciated that there is an implied "about" prior to the temperatures, concentrations, times, etc. discussed in the present teachings, such that slight and insubstantial deviations are within the scope of the present teachings herein.

Although this disclosure is in the context of certain embodiments and examples, those of ordinary skill in the art will understand that the present disclosure extends beyond the specifically disclosed embodiments to other alternative embodiments and/or uses of the embodiments and obvious modifications and equivalents thereof. In addition, while several variations of the embodiments have been shown and described in detail, other modifications, which are within the scope of this disclosure, will be readily apparent to those of ordinary skill in the art based upon this disclosure. It is also contemplated that various combinations or sub-combinations of the specific features and aspects of the embodiments may be made and still fall within the scope of the disclosure. It should be understood that various features and aspects of the disclosed embodiments can be combined with, or substituted for, one another in order to form varying modes or embodiments of the disclosure. Thus, it is intended that the scope of the present disclosure herein disclosed should not be limited by the particular disclosed embodiments described above.

### REFERENCES

1. Gall JG & Pardue ML (1969) Formation and Detection of RNA-DNA Hybrid Molecules in Cytological Preparations. Proc Natl Acad Sci USA 63:378-383.
2. Cox KH, Deleon DV, Angerer LM, & Angerer RC (1984) Detection of mRNAs in Sea Urchin Embryos by Insitu Hybridization Using Asymmetric RNA Probes. Dev Biol 101(2):485-502.
3. Tautz D & Pfeifle C (1989) A non-radioactive in situ hybridization method for the localization of specific RNAs in Drosophila embryos reveals translational control of the segmentation gene hunchback. Chromosoma 98:81-85.
4. Rosen B & Beddington RSP (1993) Whole-Mount Insitu Hybridization in the Mouse Embryo: Gene-Expression in three Dimensions. Trends Genet 9(5):162-166.
5. Wallner G, Amann R, & Beisker W (1993) Optimizing Fluorescent In situ Hybridization with rRNA-Targeted Oligonucleotide Probes for Flow Cytometric Identification of Microorganisms. Cytometry 14(2):136-143.
6. Nieto MA, Patel K, & Wilkinson DG (1996) In situ hybridization analysis of chick embryos in whole mount and tis sue sections. Methods Cell Biol, (Academic Press), Vol 51, pp 219-235.
7. Thisse C & Thisse B (2008) High-resolution in situ hybridization to whole-mount zebrafish embryos. Nat Protoc 3(1):59-69.
8. Kislauskis EH, Li Z, Singer RH, & Taneja KL (1993) Isoform-Specific 3'-Untranslated Sequences Sort α-Cardiac and β-Cytoplasmic Actin Messenger RNAs to Different Cytoplasmic Compartments. J Cell Biol 123(1):165-172.
9. Femino A, Fay FS, Fogarty K, & Singer RH (1998) Visualization of Single RNA Transcripts In Situ. Science 280(5363):585-590.
10. Levsky JM, Shenoy SM, Pezo RC, & Singer RH (2002) Single-Cell Gene Expression Profiling. Science 297:836-840.
11. Kos man D, et a l. (2004) Multiplex Detection of RNA Expression in Drosophila Embryos. Science 305:846.
12. Capodieci P, et a l. (2005) Gene Expression Profiling in Single Cells within Tissue. Nat Methods 2(9):663-665.
13. Chan PM, Yuen T, Ruf F, Gonzalez-Maeso J, & Sealfon SC (2005) Method for Multiplex Cellular Detection of mRNAs Using Quantum Dot Fluorescent In Situ Hybridization. Nucleic Acids Res 33(18):e 161.
14. Raj A, van den Bogaard P, Rifkin SA, van Oudenaarden A, & Tyagi S (2008) Imaging Individual mRNA Molecules Using Multiple Singly Labeled Probes. Nat Methods 5(10):877-879.
15. Harland RM (1991) In Situ Hybridization: An Improved Whole-Mount Method for Xenopus Embryos. Methods Cell Biol 36:685-695.
16. Lehmann R & Tautz D (1994) In Situ Hybridization to RNA. Methods Cell Biol, (Academic Press), Vol 44, pp 575-598.
17. Kerstens HMJ, Poddighe PJ, & Hanselaar AGJM (1995) A Novel in-Situ Hybridization Signal Amplification Method Based on the Deposition of Biotinylated Tyramine. J Histochem Cytochem 43(4):347-352.
18. Wiedorn KH, Kuhl H, Galle J, Caselitz J, & Vollmer E (1999) Comparison of In-Situ Hybridization, Direct and Indirect In-Situ PCR as well as Tyramide Signal Amplification for the Detection of HPV. Histochem Cell Biol 111:89-95.
19. Player AN, Shen LP, Kenny D, Antao VP, & Kolberg JA (2001) Single-Copy Gene Detection Using Branched DNA (bDNA) In Situ Hybridization. J Histochem Cytochem 49(5):603-611.
20. Pernthaler A, Pernthaler J, & Amann R (2002) Fluorescence in situ hybridization and catalyzed reporter deposition for the identification of marine bacteria. Appl Environ Microbiol 68(6):3094-3101.
21. Thisse B, et a l. (2004) Spatial and Temporal Expression of the Zebrafish Genome by Large-Scale In Situ Hybridization Screening. The Zebrafish: 2nd Edition Genetics Genomics and Informatics, Methods in Cell Biology, eds Detrich III HWD, Zon LI, & Westerfield M (Elsevier Academic Press, San Diego, CA), Vol 77, pp 505-519.
22. Denkers N, Garcia-Villalba P, Rodesch CK, Nielson KR, & Mauch TJ (2004) FISHing for Chick Genes: Triple-Label Whole-Mount Fluorescence In Situ Hybridization Detects Simultaneous and Overlapping Gene Expression in Avian Embryos. Dev Dyn 229(3):651-657.
23. Zhou H, et a l. (2004) Two-Color, Rolling-Circle Amplification on Antibody Microarrays for Sensitive, Multiplexed Serum-Protein Measurements. Genome Biol 5(4):R28.
24. Larsson C, e t a l. (2004) In Situ Genotyping Individual DNA Molecules by Target-Primed Rolling-Circle Amplification of Padlock Probes. Nat Methods 1(3):227-232.
25. Clay H & Ramakrishnan L (2005) Multiplex Fluorescent In Situ Hybridization in Zebrafish Embryos Using Tyramide Signal Amplification. ZebrafisH2(2):105-111.
26. Barroso-Chinea P, et al. (2007) Detection of Two Different mRNAs in a Single Section by Dual In Situ Hybridization: A Comparison Between Colorimetric and Fluorescent Detection. J Neurosci Methods 162(1-2):119-128.
27. Acloque H, Wilkinson DG, & Nieto MA (2008) In Situ Hybridization Analysis of Chick Embryos in Whole-Mount and Tissue Sections. Avian Embryology, 2nd Edition, Methods in Cell Biology, ed Bronner-Fraser M (Elsevier Academic Press, San Diego, CA), Vol 87, pp 169-185.
28. Piette D, Hendrickx M, Willems E, Kemp CR, & Leyns L (2008) An optimized procedure for whole-mount in situ hybridization on mouse embryos and embryoid bodies. Nat Protoc 3(7):1194-1201.
29. Weiszmann R, Hammonds AS, & Celniker SE (2009) Determination of gene expression patterns using high-throughput RNA in situ hybridization to whole-mount Drosophila embryos. Nat Protoc 4(5):605-618.
30. Larsson C, Grundberg I, Soderberg O, & Nilsson M (2010) In Situ Detection and Genotyping of Individual mRNA Molecule s. Nat Methods 7(5):395-397.
31. Wang F, et a l. (2012) RNAscope: A Novel in Situ RNA Analysis Platform for Formalin-Fixe d, Paraffin-Embedded Tissues. J Mol Diagnostics 14(1):22-29.
32. Dirks RM & Pierce NA (2004) Triggered Amplification by Hybridization Chain Reaction. Proc Natl Acad Sci USA 101(43):15275-15278.
33. Choi HMT, et a l. (2010) Programmable in situ amplification for multiplexed imaging of mRNA expression. Nat Biotechnol 28(11):1208-1212.
34. Choi HMT, Beck VA, & Pierce NA (2014) Next-generation in situ hybridization chain reaction: higher gain, lower cost, greater durability. ACS Nano 8(5):4284-4294.
35. Shah S, et a l. (2016) Single-molecule RNA detection a t depth via hybridization chain reaction and tis sue hydrogel embedding and clearing. Development.
36. Zhang HQ, Li F, Dever B, Li XF, & Le XC (2013) DNA-Media ted Homogeneous Binding Assays for Nucleic Acids and Proteins. Chem Rev 113(4):2812-2841.
37. Wang F, Lu CH, & Willner I (2014) From Cascaded Catalytic Nucleic Acids to Enzyme-DNA Nanostructures: Controlling Reactivity, Sensing, Logic Operations, and Assembly of Complex Structures. Chem Rev 114(5):2881-2941.
38. Jung C & Ellington AD (2014) Diagnostic applications of nucleic acid circuits. Acc Chem Res 47(6):1825-1835.
39. Ikbal J, Lim GS, & Gao ZQ (2015) The hybridization chain reaction in the development of ultrasensitive nucleic acid assays. Trac-Trends in Analytical Chemistry 64:86-99.
40. McLennan R, et a l. (2015) Neural crest migration is driven by a few trailblazer cells with a unique molecular signature narrowly confined to the invasive front. Development 142(11):2014-2025.
41. Huss D, et a l. (2015) Combinatorial analysis of mRNA expression patterns in mouse embryos using hybridization chain reaction. Cold Spring Harbor Protocols.
42. Rosenthal AZ, et al. (2013) Localizing transcripts to single cells suggests an important role of uncultured deltaproteobacteria in the termite gut hydrogen economy. Proc Natl Acad Sci USA 110(40):16163-16168.
43. Yamaguchi T, et al. (2015) In situ DNA-hybridization chain reaction (HCR): a facilitated in situ HCR system for the detection of environmental microorganisms. Environmental Microbiology 17(7):2532-2541.
44. Nikolakakis K, Lehnert E, McFall-Ngai MJ, & Ruby EG (2015) Use of Hybridization Chain Reaction-Fluorescent In Situ Hybridization To Track Gene Expression by Both Partners during Initiation of Symbiosis. Appl Environ Microbiol 81(14):4728-4735.
45. Lan HY, Mu W, NG YY, Nikolic-Paterson DH, & Atkins RC (1996) A simple, reliable, and sensitive method for nonradioactive in situ hybridization: use of microwave heating to improve hybridization efficiency and preserve tissue morphology. J Histochem Cytochem 44(3):281-287.
46. Smith JJ, Gunasekera TS, Barardi CR, Veal D, Vesey G (2004) Determination of Cryptosporidium parvum oocyst viability by fluorescence in situ hybridization using a ribosomal RNA-directed probe. J Appl Microbiol 96(2):409-417.
47. Hybridization chain reaction methods for in situ molecular detection, Evan R. DAUGHARTHY, George M. Church, https://patents.google "dot" com/patent/WO2017189525A1/en
48. Nanoscale imaging of proteins and nucleic acids via expansion microscopy, Asmamaw Wassie, Fei Chen, Edward Stuart Boyden, Shahar Alon, George Church, Evan Daugharthy, https://patents.google.com/patent/CA2994958A1/en
49. Chen, F., A. T. Wassie, A. J. Cote, A. Sinha, S. Alon, S. Asano, E. R. Daugharthy, et al. 2016. "Nanoscale Imaging of RNA with Expansion Microscopy." Nature Methods 13 (8): 679-84.
50. Husain, N. 2016. "Mapping MRNA and Protein Expression with High Signal-to-Background in Diverse Organisms." PhD Thesis, California Institute of Technology.
51. Koos, B., G. Cane, K. Grannas, L. Löf, L. Arngården, J. Heldin, C. M. Clausson, et al. 2015. "Proximity-Dependent Initiation of Hybridization Chain Reaction." Nature Communications 6: 7294. https://doi.org/ARTN 7294 10.1038/ncomms8294.
52. Leino, Mattias, Johan Heldin, Marie Rubin Sander, Despoina Kermpatsou, Doroteya Raykova, Björn Koos, and Ola Söderberg. 2019. "Optimization of Proximity-Dependent Initiation of Hybridization Chain Reaction for Improved Performance." Molecular Systems Design & Engineering 4 (5): 1058-65. https://doi.org/10.1039/C9ME00079H.
53. Lin, R., Q. Feng, P. Li, P. Zhou, R. Wang, Z. Liu, Z. Wang, et al. 2018. "A Hybridization-Chain-Reaction-Based Method for Amplifying Immunosignals." Nature Methods 15 (4): 275-78. https://doi.org/10.1038/nmeth.4611.
54. Wang, Y., W. Xie, R. E. Kohman, and G. M. Church. 2018. "Multiplexed Imaging Using Same Species Primary Antibodies with Signal Amplification." BioRxiv, January, 274456. https://doi.org/10.1101/274456.
55. Wang, Y., Y. Zeng, S. K. Saka, W. Xie, I. Goldaracena, R. E. Kohman, P. Yin, and G. M. Church. 2020. "Multiplexed in Situ Protein Imaging Using DNA-Barcoded Antibodies with Extended Hybridization Chain Reactions." BioRxiv, January, 274456. https://doi.org/10.1101/274456.
56. Wolfe, B. R., N. J. Porubsky, J. N. Zadeh, R. M. Dirks, and N. A. Pierce. 2017. "Constrained Multistate Sequence Design for Nucleic Acid Reaction Pathway Engineering." Journal of the American Chemical Society 139 (8): 3134-44.
57. Zadeh, J. N., C. D. Steenberg, J. S. Bois, B. R. Wolfe, M. B. Pierce, A. R. Khan, R. M. Dirks, and N. A. Pierce. 2011. "NUPACK: Analysis and Design of Nucleic Acid Systems." Journal of Computational Chemistry 32 (1): 170-73. https://doi.org/10.1002/jcc.21596.
58. Acharya, A. 2016. "Multiplexed Analysis of Diverse RNA Classes via Hybridization Chain Reaction." PhD Thesis, California Institute of Technology.
In the following, particular items of the present disclosure are summarized:
1. A method for repeated signal detection with a reporter-labeled hairpin, the method comprising:
   a) providing a sample possibly containing up to N targets as well as possibly other molecules that are not targets;
   b) providing N probe sets (each targeting one of N target types) each comprising either: i) one or more HCR initiator-labeled probes, or ii) one or more probe units each comprising two or more HCR fractional initiator probes;
   c) optionally washing the sample;
   d) providing M HCR amplifiers (for M ≤ N; each labeled with a distinct reporter) corresponding to M of the N probe sets;
   e) optionally washing the sample;
   f) detecting M signals corresponding to the M reporters;
   g) removing the M signals from the sample; and
   h) optionally repeating one or more of steps b-g until signal detection has been performed for all N targets;

   wherein a probe set comprises either:
      a) one or more HCR initiator-labeled probes, or
      b) one or more probe units,
   wherein an HCR initiator-labeled probe comprises:
      one or more target-binding regions and
      one or more initiators,
   wherein a probe unit comprises: two or more HCR fractional initiator probes,
   wherein an HCR fractional initiator probe comprises:
      a target-binding region and
      a fractional initiator, and
   wherein an HCR amplifier comprises two or more HCR hairpins,
   wherein an HCR hairpin comprises an input domain comprising:
      a single-stranded toehold; and
      a stem section,
   wherein an HCR hairpin further comprises an output domain comprising:
      a single-stranded loop; and
      a complement to the stem section, and
   wherein an HCR hairpin further comprises a reporter.
2. A method for repeated signal detection with a reporter-labeled hairpin, the method comprising:
   a) providing a sample possibly containing one or more targets as well as possibly other molecules that are not targets;
   b) providing one or more probe sets each comprising either: i) one or more HCR initiator-labeled probes, or ii) one or more probe units each comprising two or more HCR fractional initiator probes;
   c) optionally washing the sample;
   d) providing one or more HCR amplifiers (each labeled with one or more reporters);
   e) optionally washing the sample;
   f) detecting one or more signals from one or more reporters;
   g) optionally removing one or more probe sets from the sample;
   h) optionally removing one or more HCR amplifiers from the sample;
   i) optionally removing one or more reporters from the sample; and
   j) optionally removing one or more signals from the sample;

   wherein a probe set comprises either:
      a) one or more HCR initiator-labeled probes, or
      b) one or more probe units,
   wherein an HCR initiator-labeled probe comprises:
      one or more target-binding regions and
      one or more initiators,
   wherein a probe unit comprises two or more HCR fractional initiator probes,
   wherein an HCR fractional initiator probe comprises:
      a target-binding region and
      a fractional initiator;
   wherein an HCR amplifier comprises two or more HCR hairpins;
   wherein an HCR hairpin comprises: an input domain comprising:
      a single-stranded toehold and
      a stem section,
   wherein an HCR hairpin further comprises an output domain comprising:
      a single-stranded loop and
      a complement to the stem section, and
   wherein an HCR hairpin further comprises one or more reporters.
3. A method of repeated signal detection with substrate-labeled hairpins, the method comprising:
   a) providing a sample possibly containing up to N targets as well as possibly other molecules that are not targets;
   b) providing N probe sets each comprising either: i) one or more HCR initiator-labeled probes, or ii) one or more probe units each comprising two or more HCR fractional initiator probes;
   c) optionally washing the sample;
   d) providing N HCR amplifiers (each labeled with a distinct substrate) corresponding to the N probe sets;
   e) optionally washing the sample;
   f) providing M label probes (for M ≤ N; each conjugated to a distinct reporter) corresponding to M of the N distinct substrates;
   g) optionally washing the sample;
   h) detecting M signals corresponding to the M distinct reporters;
   i) removing the M signals from the sample; and
   j) optionally repeating one or more of steps f-i until signal detection has been performed for all N targets;

   wherein a probe set comprises either:
      a) one or more HCR initiator-labeled probes, or
      b) one or more probe units;
   wherein an HCR initiator-labeled probe comprises:
      one or more target-binding regions and
      one or more initiators,
   wherein a probe unit comprises: two or more HCR fractional initiator probes;
   wherein an HCR fractional initiator probe comprises:
      a target-binding region and
      a fractional initiator;
   wherein an HCR amplifier comprises two or more HCR hairpins;
   wherein an HCR hairpin comprises an input domain comprising:
      a single-stranded toehold and
      a stem section;
   wherein an HCR hairpin further comprises an output domain comprising:
      a single-stranded loop and
      a complement to the stem section;
   wherein an HCR hairpin further comprises a substrate, and
   wherein a label probe comprises:
      a substrate-binding region and
      a reporter.
4. A method of repeated signal detection with substrate-labeled hairpins, the method comprising:
   a) providing a sample possibly containing one or more targets as well as possibly other molecules that are not targets;
   b) providing one or more probe sets each comprising either: i) one or more HCR initiator-labeled probes, or ii) one or more probe units each comprising two or more HCR fractional initiator probes;
   c) optionally washing the sample;
   d) providing one or more HCR amplifiers (each labeled with a substrate) corresponding to one or more probe sets;
   e) optionally washing the sample;
   f) providing one or more label probes (each conjugated to a reporter) corresponding to one or more substrates;
   g) optionally washing the sample;
   h) detecting one or more signals corresponding to one or more reporters;
   i) removing one or more signals from the sample; and
   j) optionally repeating any of steps b-i one or more times in any order;

   wherein a probe set comprises either:
      a) one or more HCR initiator-labeled probes, or
      b) one or more probe units,
   wherein an HCR initiator-labeled probe comprises:
      one or more target-binding regions and
      one or more initiators,
   wherein a probe unit comprises two or more HCR fractional initiator probes,
   wherein an HCR fractional initiator probe comprises:
      a target-binding region and
      a fractional initiator,
   wherein an HCR amplifier comprises two or more HCR hairpins,
   wherein an HCR hairpin comprises an input domain comprising:
      a single-stranded toehold and
      a stem section,
   wherein an HCR hairpin further comprises an output domain comprising:
      a single-stranded loop and
      a complement to the stem section,
   wherein an HCR hairpin further comprises a substrate, and
   wherein a label probe comprises:
      a substrate-binding region and
      a reporter.
5. A method of repeated signal detection with reporter and/or substrate-labeled hairpins, the method comprising:
   a) providing a sample possibly containing one or more targets as well as possibly other molecules that are not targets;
   b) providing one or more HCR probe sets each comprising either: i) one or more HCR initiator-labeled probes, or ii) one or more probe units each comprising two or more HCR fractional initiator probes;
   c) providing one or more HCR amplifiers (each labeled with one or more reporters and/or one or more substrates) corresponding to one or more probe sets;
   d) optionally providing one or more label probes (each conjugated to one or more reporters) corresponding to one or more substrates;
   e) detecting one or more signals;
   f) optionally washing the sample;
   g) optionally removing one or more signals from the sample;
   h) optionally removing one or more reporters from the sample;
   i) optionally removing one or more label probes from the sample;
   j) optionally removing one or more HCR amplifiers from the sample;
   k) optionally removing one or more probe sets from the sample; and
   l) optionally repeating any of the above steps in any order;

   wherein a probe set comprises either:
      a) one or more HCR initiator-labeled probes, or
      b) one or more probe units,
   wherein an HCR initiator-labeled probe comprises:
      one or more target-binding regions and
      one or more initiators,
   wherein a probe unit comprises two or more HCR fractional initiator probes,
   wherein an HCR fractional initiator probe comprises:
      a target-binding region and
      a fractional initiator,
   wherein an HCR amplifier comprises two or more HCR hairpins,
   wherein the HCR hairpin comprises an input domain comprising:
      a single-stranded toehold and
      a stem section,
   wherein an HCR hairpin further comprises an output domain comprising:
      a single-stranded loop and
      a complement to the stem section,
   wherein an HCR hairpin further comprises:
      one or more reporters and/or
      one or more substrates, and
   wherein a label probe comprises:
      a substrate-binding region and
      one or more reporters.
6. A method of repeated signal detection with reporter and/or substrate-labeled hairpins, the method comprising:
   a) providing a sample possibly containing one or more targets as well as possibly other molecules that are not targets;
   b) performing any of steps c-g one or more times in any order;
   c) providing one or more HCR probe sets each comprising either: i) one or more HCR initiator-labeled probes, or ii) one or more probe units each comprising two or more HCR fractional initiator probes;
   d) providing one or more HCR amplifiers that directly or indirectly generate one or more signals;
   e) optionally washing the sample;
   f) detecting one or more signals; and
   g) optionally removing one or more signals;

   wherein a probe set comprises either:
      a) one or more HCR initiator-labeled probes, or
      b) one or more probe units;
   wherein an HCR initiator-labeled probe comprises:
      one or more target-binding regions and
      one or more initiators,
   wherein a probe unit comprises two or more HCR fractional initiator probes,
   wherein an HCR fractional initiator probe comprises:
      a target-binding region and
      a fractional initiator,
   wherein an HCR amplifier comprises two or more HCR hairpins,
   wherein an HCR hairpin comprises an input domain comprising:
      a single-stranded toehold and
      a stem section,
   wherein an HCR hairpin further comprises an output domain comprising:
      a single-stranded loop and
      a complement to the stem section, and
   wherein an HCR hairpin further comprises:
      one or more reporters and/or
      one or more substrates.
7. A method of HCR involving overlapping binding sites, the method comprising:
   a) providing a sample possibly containing a target as well as possibly other molecules that are not targets;
   b) providing a probe set comprising one or more probe units each comprising two or more HCR fractional initiator probes where the target-binding regions on the probes within each probe unit are configured to bind to overlapping binding sites on the target;
   c) optionally washing the sample;
   d) providing an HCR amplifier labeled with a reporter and/or a substrate;
   e) optionally washing the sample;
   f) optionally providing a label probe (conjugated to a reporter) corresponding to the substrate;
   g) optionally washing the sample; and
   h) detecting a signal from the reporter.

   wherein a probe set comprises one or more probe units,
   wherein a probe unit comprises two or more HCR fractional initiator probes,
   wherein an HCR fractional initiator probe comprises:
      a target-binding region and
      a fractional initiator,
   wherein the target binding regions on the probes within each probe unit are configured to bind to overlapping binding sites on the target,
   wherein an HCR amplifier comprises two or more HCR hairpins,
   wherein an HCR hairpin comprises an input domain comprising:
      a single-stranded toehold and
      a stem section,
   wherein an HCR hairpin further comprises an output domain comprising:
      a single-stranded loop and
      a complement to the stem section,
   wherein an HCR hairpin further comprises:
      a reporter and/or
      a substrate;
   wherein a label probe comprises:
      a substrate-binding region and
      a reporter.
8. A method of HCR involving overlapping binding sites, the method comprising:
   a) providing a sample possibly containing a target as well as possibly other molecules that are not targets
   b) providing a probe set comprising one or more probe units each comprising two or more HCR fractional initiator probes where the fractional initiators on the probes within each probe unit are configured to bind to overlapping binding sites on an HCR hairpin;
   c) optionally washing the sample;
   d) providing an HCR amplifier labeled with a reporter and/or a substrate;
   e) optionally washing the sample;
   f) optionally providing a label probe (conjugated to a reporter) corresponding to the substrate;
   g) optionally washing the sample; and
   h) detecting a signal from the reporter.

   wherein a probe set comprises one or more probe units;
   wherein a probe unit comprises two or more HCR fractional initiator probes,
   wherein an HCR fractional initiator probe comprises:
      a target-binding region and
      a fractional initiator,
   wherein the fractional initiators on the probes within each probe unit are configured to bind to overlapping binding sites on an HCR hairpin,
   wherein an HCR amplifier comprises two or more HCR hairpins,
   wherein an HCR hairpin comprises an input domain comprising:
      a single-stranded toehold and
      a stem section,
   wherein an HCR hairpin further comprises an output domain comprising:
      a single-stranded loop and
      a complement to the stem section,
   wherein an HCR hairpin further comprises:
      a reporter and/or
      a substrate, and
   wherein a label probe comprises:
      a substrate-binding region and
      a reporter.
9. A method of HCR involving overlapping binding sites with repeated signal detection, the method comprising:
   a) providing a sample possibly containing one or more targets as well as possibly other molecules that are not targets
   b) providing one or more probe sets each comprising either: i) one or more HCR initiator-labeled probes, or ii) one or more probe units each comprising two or more HCR fractional initiator probes where target-binding regions on the probes within each probe unit are configured to bind to overlapping or non-overlapping binding sites on a target and where fractional initiators on the probes within each probe unit are configured to bind to overlapping or non-overlapping binding sites on an HCR hairpin;
   c) optionally washing the sample;
   d) providing one or more HCR amplifiers each labeled with one or more reporters and/or substrates;
   e) optionally washing the sample;
   f) optionally providing one or more label probes (each conjugated to one or more reporters) corresponding to one or more substrates;
   g) optionally washing the sample;
   h) detecting a signal from one or more reporters;
   i) optionally removing one or more signals from the sample;
   j) optionally removing one or more reporters from the sample;
   k) optionally removing one or more label probes from the sample;
   l) optionally removing one or more amplifiers from the sample;
   m) optionally removing one or more probe sets from the sample; and
   n) optionally repeating any of the above steps in any order;

   wherein a probe set comprises either:
      a) one or more HCR initiator-labeled probes, or
      b) one or more probe units,
   wherein an HCR initiator-labeled probe comprises:
      one or more target-binding regions and
      one or more initiators,
   wherein a probe unit comprises two or more HCR fractional initiator probes,
   wherein an HCR fractional initiator probe comprises:
      a target-binding region and
      a fractional initiator,
   wherein the target-binding regions within a probe unit are configured to bind to overlapping or non-overlapping binding sites on the target,
   wherein the fractional initiators on the probes within each probe unit are configured to bind to overlapping or non-overlapping binding sites on an HCR hairpin,
   wherein an HCR amplifier comprises two or more HCR hairpins,
   wherein an HCR hairpin comprises an input domain comprising:
      a single-stranded toehold and
      a stem section,
   wherein an HCR hairpin further comprises an output domain comprising:
      a single-stranded loop and
      a complement to the stem section,
   wherein an HCR hairpin further comprises:
      one or more reporters and/or
      one or more substrates,
   wherein a label probe comprises:
      a substrate-binding region and
      one or more reporters.
10. The method of any one of items 1-9, wherein the target is a nucleic acid sequence.
11. The method of any one of items 1 or 3, wherein N is 1 to 1000.
12. The method of items 1 or 3, wherein M is 2-10,000
13. The method of any one of items 1-12, wherein the substrate is selected from the group consisting of a hapten, digoxigenin (DIG), dinitrophenyl (DNP), biotin, a fluorophore, a nucleic acid domain comprising 4-50 nucleotides, a substrate that recruits an enzyme that catalyzes deposition of reporter molecules, a fractional substrate, a nucleic acid domain that directly or indirectly mediates localization of reporters.
14. The method of any one of items 1-13, wherein the reporter is a fluorophore, a chromophore, a luminophore, a phosphor, a FRET pair, a member of a FRET pair, a quencher, a fluorophore/quencher pair, a rare-earth element or compound, a radioactive molecule, a magnetic molecule.
15. The method of any one of items 1-14, further comprising fixing the sample.
16. The method of any one of items 1-15, further comprising permeabilizing the sample.
17. The method of any one of items 1-16 further comprising removing one or more signals from the sample.
18. The method of any one of items 1-17, further comprising removing one or more reporters from the sample.
19. The method of any one of items 1-18 further comprising removing one or more label probes from the sample.
20. The method of any one of items 1-19 further comprising removing one or more amplifiers from the sample.
21. The method of any one of items 1-20 further comprising removing one or more probe sets from the sample.
22. The method of any one of items 1-21 further comprising repeating any of the above steps in any order.
23. The method of any one of items 1-21, wherein the method is conducted in alphabetical order as lettered in the item.
24. The method of any one of items 1-23, wherein the probe units each comprise two or more HCR fractional initiator probes, and wherein an HCR fractional initiator probe comprises a target-binding region and a fractional initiator.
25. The method of any one of items 1-24, wherein overlapping binding is involved and wherein the overlap is at least 1, 2, or 3 nucleotides.
26. A method, comprising:
   providing:
      a first fractional initiator probe comprising a first fractional initiator;
      a second fractional initiator probe comprising a second fractional initiator;
      a first hairpin monomer, comprising:
         a first input domain, comprising a first toehold and a first stem section,
         a first output domain, comprising a first hairpin loop and a complement to the first stem section, and
         a first hapten molecule;
      a second hairpin monomer, comprising:
         a second input domain, comprising a second toehold and a second stem section,
         a second output domain, comprising a second hairpin loop and a complement to the second stem section, and
         a second hapten molecule;
      a target molecule; and
   incubating the first fractional initiator probe and the second fractional initiator probe with the target.
27. The method of item 26, wherein the incubating binds the first fractional initiator probe to the target molecule and binds the second fractional initiator probe to the target molecule.
28. The method of any one of items 26-27, further comprising:
   binding the first hairpin monomer to both of the first fractional initiator and the second fractional initiator;
   binding the second hairpin monomer to the first hairpin monomer;
   providing an anti-hapten molecule labeled with one or more reporter entities, wherein the reporter entity is an enzyme that mediates CARD;
   providing one or more CARD-substrates;
   measuring a signal from one or more deposited reporters generated from the CARD-substrate by the enzyme that mediates CARD.
29. The method of any one of items 26-28, wherein the anti-hapten molecule is an anti-hapten antibody or an anti-hapten nanobody.
30. The method of any one of items 26-29, wherein the at least one target is a nucleic acid.
31. The method of any one of items 26-30, wherein the at least one target molecule is an RNA.
32. A method, comprising:
   providing:
      at least one initiator-labeled probe comprising at least one initiator;
      a first hairpin monomer, comprising:
         a first input domain, comprising a first toehold and a first stem section,
         a first output domain, comprising a first hairpin loop and a complement to the first stem section, and
         a first hapten molecule;
      a second hairpin monomer, comprising:
         a second input domain, comprising a second toehold and a second stem section,
         a second output domain, comprising a second hairpin loop and a complement to the second stem section, and
         a second hapten molecule,
      a target molecule; and
   incubating the at least one initiator-labeled probe comprising at least one initiator with the target.
33. The method of item 32, wherein the incubating binds the at least one initiator-labeled probe comprising at least one initiator to the target molecule.
34. The method of any one of items 32-33, further comprising:
   binding the first hairpin monomer to the at least one initiator;
   binding the second hairpin monomer to the first hairpin monomer;
   providing an anti-hapten molecule labeled with one or more reporter entities, wherein the reporter entity is an enzyme that mediates CARD;
   providing one or more CARD-substrates;
   measuring a signal from one or more deposited reporters generated from the CARD-substrate by the enzyme that mediates CARD.
35. The method of any one of items 32-34, wherein the target molecule is a protein.
36. The method of any one of items 32-35, wherein the anti-hapten molecule is an anti-hapten antibody or an anti-hapten nanobody.
37. The method of item 36, wherein the anti-hapten antibody is a primary antibody.
38. The method of item 36, wherein the anti-hapten antibody comprises a primary antibody that binds the hapten and the method further comprises a secondary antibody (labeled with one or more reporter entities) that binds the primary antibody.
39. A method, comprising:
   providing:
      a first fractional initiator probe comprising a first fractional initiator;
      a second fractional initiator probe comprising a second fractional initiator;
      a first hairpin monomer, comprising:
         a first input domain, comprising a first toehold and a first stem section,
         a first output domain, comprising a first hairpin loop and a complement to the first stem section, and
         a substrate;
      a second hairpin monomer, comprising:
         a second input domain, comprising a second toehold and a second stem section,
         a second output domain, comprising a second hairpin loop and a complement to the second stem section, and
         the substrate,
      a target molecule; and
   incubating the first fractional initiator probe and the second fractional initiator probe with the target.
40. The method of item 39, wherein the incubating binds the first fractional initiator probe to the target molecule and binds the second fractional initiator probe to the target molecule.
41. The method of any one of items 39-40, further comprising:
   binding the first hairpin monomer to both of the first fractional initiator and the second fractional initiator;
   binding the second hairpin monomer to the first hairpin monomer;
   providing a substrate-binding region labeled with one or more reporter entities, wherein the substrate-binding region binds to the substrate, and wherein the reporter entity is an enzyme that mediates CARD;
   providing one or more CARD-substrates;
   measuring a signal from one or more deposited reporters generated from the CARD-substrate by the enzyme that mediates CARD.
42. The method of any one of items 39-41, wherein the at least one target is a nucleic acid.
43. The method of any one of items 39-42, wherein the at least one target molecule is an RNA.
44. A method, comprising:
   providing:
      a first fractional initiator probe comprising a first fractional initiator;
      a second fractional initiator probe comprising a second fractional initiator;
      a first hairpin monomer, comprising:
         a first input domain, comprising a first toehold and a first stem section,
         a first output domain, comprising a first hairpin loop and a complement to the first stem section, and
         a first fractional substrate;
      a second hairpin monomer, comprising:
         a second input domain, comprising a second toehold and a second stem section,
         a second output domain, comprising a second hairpin loop and a complement to the second stem section, and
         a second fractional substrate,
      a target molecule; and
   incubating the first fractional initiator probe and the second fractional initiator probe with the target molecule.
45. The method of item 44, wherein the incubating binds the first fractional initiator probe to the target molecule and binds the second fractional initiator probe to the target molecule.
46. The method of any one of items 44-45, further comprising:
   binding the first hairpin monomer to both of the first fractional initiator and the second fractional initiator;
   binding the second hairpin monomer to the first hairpin monomer;
   obtaining a full substrate comprising the first and second fractional substrate;
   providing a substrate-binding region labeled with one or more reporter entities, wherein the substrate-binding region binds to the full substrate, and wherein the reporter entity is an enzyme that mediates CARD;
   providing one or more CARD-substrates;
   measuring a signal from one or more deposited reporters generated from the CARD-substrate by the enzyme that mediates CARD.
47. The method of any one of items 44-46, wherein the at least one target is a nucleic acid.
48. The method of any one of items 44-47, wherein the at least one target molecule is an RNA.

## Claims

1. A method, comprising:
providing:
a probe set comprising either:
a) a first fractional initiator probe comprising a first fractional initiator, and
a second fractional initiator probe comprising a second fractional initiator; or
b) an initiator-labeled probe comprising an initiator;
a first hairpin monomer, comprising:
a first input domain, comprising a first toehold and a first stem section,
a first output domain, comprising a first hairpin loop and a complement to the first stem section;
a second hairpin monomer, comprising:
a second input domain, comprising a second toehold and a second stem section,
a second output domain, comprising a second hairpin loop and a complement to the second stem section;
wherein at least one hairpin monomer further comprises a substrate;
a target; and
incubating the probe set with the target.

2. The method of claim 1, wherein the incubating binds the first fractional initiator probe of a) to the target and binds the second fractional initiator probe of a) to the target; or binds the initiator-labeled probe of b) to the target.

3. The method of either of claims 1 or 2, further comprising:
binding the first hairpin monomer to both of the first fractional initiator of a) and the second fractional initiator of a); or binding the first hairpin monomer to the initiator of b);
binding the second hairpin monomer to the first hairpin monomer.

4. The method of any one of claims 1-3, wherein the substrate directly or indirectly mediates localization of one or more reporters in the vicinity of the substrate.

5. The method of any one of claims 1-4, wherein the substrate directly or indirectly mediates localization of one or more reporters in the vicinity of the substrate via enzyme-mediated CARD.

6. The method of any one of claims 1-5, further comprising:
providing a substrate-binding region labeled with one or more reporter entities, wherein the substrate-binding region binds to the substrate, and wherein the reporter entity is an enzyme that mediates CARD;
providing one or more CARD-substrates;
measuring a signal directly or indirectly from one or more deposited reporters generated from the CARD-substrate by the enzyme that mediates CARD.

7. The method of either of claims 5 or 6 wherein the one or more reporters comprise a fluorophore-labeled tyramide.

8. The method of either of claims 5 or 6 wherein the one or more reporters comprise an auxiliary CARD-substrate that mediates localization of auxiliary reporters in the vicinity of the target, and wherein optionally the auxiliary CARD-substrate comprises a tyramide.

9. The method of either of claims 5 or 6 wherein the enzyme that mediates CARD is deactivated after reporter deposition.

10. The method of either of claims 5 or 6 wherein the enzyme that mediates CARD comprises horse radish peroxidase, alkaline phosphatase, or glucose oxidase.

11. The method of any one of claims 1-10, wherein the target comprises a nucleic acid, a protein, a small molecule, or a complex of molecules.

12. The method of any one of claims 1-11, wherein the target comprises an RNA.

13. The method of any one of claims 1-12 wherein the probe set is a secondary probe set and wherein the target is a secondary target, and wherein the secondary target comprises a primary probe that binds the primary target, optionally wherein the primary probe comprises an antibody, a nanobody, an amino acid, or a nucleotide.

14. The method of any one of claims 1-13 wherein the probe set further comprises a third fractional initiator probe comprising a third fractional initiator.

15. The method of any one of claims 5-14 wherein the method further comprises deactivating the enzyme that mediates CARD and repeating CARD using: a) the same enzyme in combination with different CARD substrates for different targets to enable multiplexed target analysis, or b) different enzymes in combination with different CARD substrates for different targets to enable multiplexed target analysis.
